(19) Europäisches Patentamt
European Patent Office
Office européen des brevets

(11) Numéro de publication: **0 472 449 B1**

(12) # FASCICULE DE BREVET EUROPEEN

(45) Date de publication de fascicule du brevet: **14.09.94**

(51) Int. Cl.$^5$: **C07C 311/19**, C07C 311/20, C07C 311/29, A61K 31/18, C07D 203/26, C07C 255/45, C07C 211/35, C07C 211/17

(21) Numéro de dépôt: **91402122.5**

(22) Date de dépôt: **30.07.91**

(54) **Nouveaux sulfonamides substitués procédés de préparation et médicaments les contenant.**

(30) Priorité: **31.07.90 FR 9009737**

(43) Date de publication de la demande:
**26.02.92 Bulletin 92/09**

(45) Mention de la délivrance du brevet:
**14.09.94 Bulletin 94/37**

(84) Etats contractants désignés:
**AT BE CH DE DK ES FR GB GR IT LI LU NL SE**

(56) Documents cités:
**EP-A- 0 312 906**
**EP-A- 0 358 398**
**EP-A- 0 361 113**
**US-A- 4 350 685**

**CHEMISCHE BERICHTE, vol. 121, 1988, pages 757-780, VCH Verlagsgesellschaft mbH, Weinheim, DE; B. ZIPPERER et al.: "cis-/trans-(1.1.1)-O,N,C-Tris-sigma-homobenzole - synthesen, (sigma2+sigma2+sigma2)-cycloreversionen, O,N-heteronine"**

(73) Titulaire: **LIPHA, LYONNAISE INDUSTRIELLE PHARMACEUTIOUE**
**34, rue Saint Romain**
**F-69379 Lyon Cedex 08 (FR)**

(72) Inventeur: **Lardy, Claude**
**84, bd Ambroise Paré**
**F-69008 Lyon (FR)**
Inventeur: **Guerrier, Daniel**
**35, route de Charly**
**F-69230 Saint Genis Laval (FR)**
Inventeur: **Chavernac, Gilles**
**4A, Chemin de la Bastero,**
**Résidence le Vert Galant**
**F-69350 La Mulatière (FR)**
Inventeur: **Collonges, François**
**Le Grand Champ,**
**Chemin de Halage**
**F-01700 Beynost Miribel (FR)**

(74) Mandataire: **Moncheny, Michel et al**
**c/o Cabinet Lavoix**
**2 Place d'Estienne d'Orves**
**F-75441 Paris Cedex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

JOURNAL OF THE AMERICAN CHEMICAL SOCIETY, vol. 104, 1982, pages 2444-2451, American Chemical Society, Washington, D.C., US; L. S. HEGEDUS et al.: "Palladium-catalyzed cyclization of omega-olefinic tosamides. Synthesis of nonaromatic nitrogen heterocycles"

JOURNAL OF ORGANIC CHEMISTRY, vol. 43, no. 12, 1978, pages 2544-2548, American Chemical Society, Washington, D.C., US; E. HERRANZ et al.: "Improvements in the osmium-catalyzed oxyamination of olefins by chloramine-T"

CHEMICAL AND PHARMACEUTICAL BULLETIN, vol. 24, no. 11, 1976, pages 2841-2849, Tokyo, JP; A. YAMASAKI et al.: "Reaction of N-haloamide. XXVII. Reaction of N,N-dihaloamides with dienes"

CHEMICAL AND PHARMACEUTICAL BULLETIN, vol. 24, no. 5, 1976, pages 1083-1089, Tokyo, JP; K. IMAI et al.: "Grignard reaction and products. V. Reduction of ketoximes to aziridines with Grignard reagents"

CHEMICAL AND PHARMACEUTICAL BULLETIN, vol. 23, no. 12, 1975, pages 3162-3169, Tokyo, JP; H. TERAUCHI et al.: "Reaction of N-haloamide. XXV. Addition reaction of N,N-dibromobenzenesulfonamide with asymmetric olefins and alpha,beta-unsaturated carboxylic acid esters"

CHEMICAL AND PHARMACEUTICAL BULLETIN, vol. 23, no. 10, 1975, pages 2410-2414, Tokyo, JP; H. TERAUCHI et al.: "Reaction of N-haloamide. XXIV. N,N-dibromo-o-carbomethoxybenzenesulfonamide as a reagent for the preparation of beta-substituted amines from olefins"

JOURNAL OF ORGANIC CHEMISTRY, vol. 35, no. 12, 1970, pages 4084-4087, Washington, D.C., US; I. J. BURNSTEIN et al.: "Aziridines. XVIII. The pyrolytic and iodide ion catalyzed rearrangements of the cis- and trans-13-p-nitrobenzoyl-13-azabicyclo(10.1.0) tridecanes"

JOURNAL OF HETEROCYCLIC CHEMISTRY, vol. 7, no. 4, août 1970, pages 755-759, Provo, US; H. ZACHARIS et al.: "Crystal and molecular structure of cis 11-(p-iodobenzenesulfonyl)-11-azabicyclo(8.1.0) undecane"

JOURNAL OF ORGANIC CHEMISTRY, vol. 34, no. 10, octobre 1969, pages 2866-2878, Washington, D.C., US; L. A. PAOUETTE et al.: "Unsaturated heterocyclic systems. LII. A general synthetic entry to derivative of 1H-azepine"

JOURNAL OF HETEROCYCLIC CHEMISTRY, vol. 5, juin 1968, pages 419-421, Provo, US; P. E. FANTA et al.: "Aziridines. XVII. Reactions of 6-azabicyclo(3.1.0) hexane (1)"

JOURNAL OF HETEROCYCLIC CHEMISTRY, vol. 3, décembre 1966, pages 404-408, Provo, US; L. M. TREFONAS et al.: "Crystal and molecular structure of 6-(p-iodobenzenesulfonyl)-3-oxa-6-azabicyclo(3.1.0) hexane"

CHEMICAL ABSTRACTS, vol. 68, no. 3, 15 janvier 1968, page 1193, abstract no. 12617y, Columbus, Ohio, US; S. TAKEMURA et al.: "Reaction of DL-trans- and DL-cis-2-halo-N-(phenylsulfonylamino)cyclohexanes. Formation and the reaction of N-(phenylsulfonyl)cyclohexenimine" & CHEM. PHARM. BULL. (TOKYO) 15(9), 1322-7(1967)

JOURNAL OF ORGANIC CHEMISTRY, vol. 31, janvier 1966, pages 59-62, Washington, D.C., US; P. E. FANTA et al.: "Aziridines. XIV. 3-oxa-6-azabicyclo(3.1.0)hexane"

JOURNAL OF ORGANIC CHEMISTRY, vol. 30, octobre 1965, pages 3574-3575, Washington, D.C. US; P. E. FANTA et al.: "Aziridines. XIII. Reactions of cyclohexenimine derivatives"

JOURNAL OF HETEROCYCLIC CHEMISTRY, vol. 2, mars 1965, pages 80-86, Provo, US; L. M. TREFONAS et al.: "Crystal and molecular structure of 7-(p-iodobenzenesulfonyl)-7-azabicyclo 4.1.0 heptane"

## Description

La présente invention se rapporte à de nouveaux sulfonamides substitués, à leur préparation et à leur utilisation comme médicaments et notamment dans le traitement de maladies où la participation du thromboxane $A_2$ est reconnue.

L'acide arachidonique exogène ou libéré des phospholipides par action de la phospholipase $A_2$ est converti en endoperoxydes (prostaglandine $G_2$ ($PGG_2$) puis en prostaglandine $H_2$ ($PGH_2$)) par l'intermédiaire de la cyclooxygénase. La thromboxane synthétase catalyse ensuite la conversion de $PGH_2$ en thromboxane $A_2$ ($TXA_2$). Ce dernier est capable, à des concentrations très faibles, d'induire de sérieux désordres biologiques, tels que l'agrégation plaquettaire, la vasoconstriction, la bronchoconstriction, de participer à la perte de l'intégrité de la paroi vasculaire, lui conférant ainsi des implications dans certaines maladies de l'appareil circulatoire et de l'appareil respiratoire. Ces actions pathophysiologiques passent par l'intermédiaire de récepteurs pouvant accueillir le thromboxane $A_2$ ainsi que les endopéroxydes $PGG_2/PGH_2$.

Une méthode d'inhibition des effets du thromboxane $A_2$ est l'utilisation d'antagonistes sélectifs des récepteurs $TXA_2/PGH_2$ et plusieurs produits agissant selon ce mécanisme sont décrits dans la littérature : voir par exemple les brevets U.S. 4,443,477 et U.S. 4,861,913.

Le brevet U.S. 4,443,477 décrit des acides sulfonamidobenzènecarboxyliques qui inhibent l'agrégation plaquettaire, abaissent les lipides sériques et qui possèdent une structure générale :

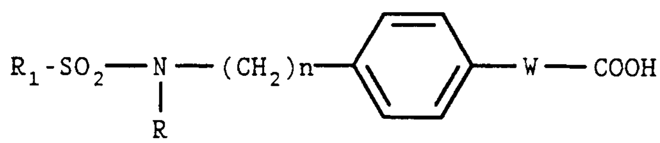

où R est un atome d'hydrogène ou un radical alkyle inférieur, $R_1$ est un radical alkyle ou aryle ou aralkyle ou aralkenyle, le groupement aryle peut être éventuellement dans chaque cas substitué par un ou plusieurs des groupes suivants : hydroxyle, halogène, trifluorométhyle, alkyle inférieur ou alkoxy ou acyle, carboxy ou alkoxycarbonyle; n est 1,2 ou 3 et W est une liaison ou une chaîne hydrocarbonée aliphatique linéaire ou non, contenant une double liaison ou saturée, de même que les sels, esters et amides physiologiquement acceptables.

Le brevet U.S. 4,861,913 décrit des dérivés bicycliques sulfonamides inhibiteurs de l'agrégation plaquettaire induite par le thromboxane $A_2$, inhibiteurs de la vasoconstriction et de la bronchoconstriction et qui possèdent la structure générale suivante :

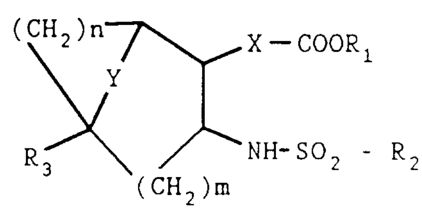

où $R_1$ est un atome d'hydrogène ou un groupe alkyle inférieur; $R_2$ est un radical alkyle ou un résidu aryle éventuellement substitué ou un reste aralkyle ou un hétérocycle; $R_3$ représente un atome d'hydrogène ou un groupe méthyle; X est un enchaînement alkylène ou alkenylène éventuellement substitué par un atome de fluor et pouvant contenir de l'oxygène ou du soufre et/ou un groupement phénylène dans la chaîne; Y représente un enchaînement alkylène ou alkenylène ou un atome d'oxygène ou de soufre; m est 0 ou 1; n est 0,1 ou 2.

Les produits de la présente invention, leurs sels, complexes, esters et amides physiologiquement acceptables répondent à la formule générale (I):

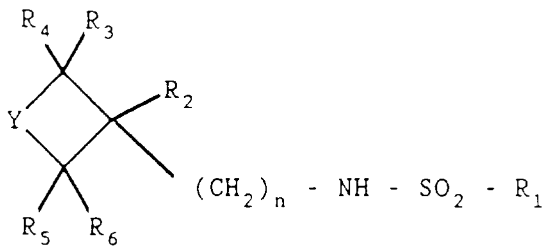

(I)

dans laquelle

$R_1$ est un radical aryle ou un hétérocycle, éventuellement substitué par un ou plusieurs des groupes suivants: halogène, trifluorométhyle, trifluorométhoxy, alkyle inférieur éventuellement substitué par un cycloalkyle, alkoxy inférieur, alkoxycarbonyle, carboxy, alkylthio inférieur, alkylsulfinyle inférieur, alkylsulfonyle inférieur, acyle inférieur, thioacyle inférieur, hydroxyle, amine éventuellement (poly)substituée par un radical alkyle inférieur, acétamide, nitro, nitrile, azide, aryle;

$R_2$ et $R_3$ sont différents; l'un des deux représente W ;

lorsque $R_2$ est W, $R_3$ représente l'hydrogène ou un alkyle inférieur et lorsque $R_3$ est W, $R_2$ représente l'hydrogène ; W représente un enchaînement $-Z-Ar-(CH_2)_q-A$, dans lequel A est $CO_2H$ ou un groupement hydrolysable en $CO_2H$, $SO_3H$, $PO_3H_2$, un hétérocycle, un radical acyle inférieur, un radical oxoalkylcarboxylique ou le groupe hydroxyéthyle ; q est égal à 0, 1, 2, 3 ou 4 ; Ar est un radical aryle. L'enchaînement $-(CH_2)_q-A$ se positionne en ortho, méta ou para lorsque Ar représente un radical phényle. Z est un atome d'oxygène, un groupement méthylène ou une liaison ;

lorsque $R_3$ est W, $R_2$ est toujours différent de W et les produits formés pourront avoir la conformation *cis* ou *trans* ou être sous forme racémique ;

$R_4$, $R_5$ et $R_6$ peuvent être, simultanément ou indépendamment les uns des autres, hydrogène, alkyle inférieur ;

Y est un groupe $-(CH_2)_s-B-(CH_2)_t$, dans lequel s vaut 0, 1 ou 2, t vaut 0, 1 ou 2 ; B est soit une liaison, un atome d'oxygène, de soufre oxydé ou non, ou d'azote éventuellement substitué par un radical alkyle inférieur, un radical acyle inférieur ou un radical aryle, soit une fonction carbonyle ou thiocarbonyle. Lorsque s et t sont égaux à 0 et B est une liaison, le groupement $-(CR_3R_4)-$est directement lié à $-(CR_5R_6)$ ;

n est 0 ou 1.

Les sels physiologiquement acceptables des composés de la formule (I) comprennent les sels formés avec des métaux (tels que le sodium, le potassium, le calcium, le magnésium), ou avec des bases comme l'ammoniac ou des amines substituées (telles que la diéthylamine, la triéthylamine, la pipéridine, la pipérazine, la morpholine), ou avec des acides aminés basiques (tels que la lysine, l'arginine) ou avec des osamines (telles que la méglumine).

Les complexes physiologiquement acceptables des composés de la formule (I) sont réalisés à partir des composés de la formule (I) ou de leurs sels et d'une $\alpha$-, $\beta$- ou $\gamma$-cyclodextrine non substituée ou substituée, hydratée ou non hydratée, et plus particulièrement la $\beta$-cyclodextrine.

Le terme "aryle" représente un groupement mono ou bicyclique aromatique comprenant 6 à 10 atomes de carbone, tel que phényle ou naphtyle.

Le terme "hétérocycle" désigne un cycle à caractère aromatique ou non, comprenant 3 à 10 atomes dont 1 à 4 hétéroatomes de même nature ou différents parmi oxygène, soufre et azote, comme par exemple les radicaux aziridinyle, oxiranyle, oxazolyle, furyle, tétrahydrofuranyle, thiényle, imidazolyle, pyridyle, pyrazinyle, benzopyranyle, benzofuranyle, pipéronyle, pyrimidinyle, pyridazinyle, pipéridinyle, quinolyle, tétrahydroquinolyle, tétrazolyle, 4,5-dihydro-3(2H)-pyridazinonyle, phtalazinyle, purinyle, indolyle, chroményle, chromanyle, isochromanyle, pyrrolyle.

Le terme "cycloalkyle" désigne des groupements hydrocarbonés saturés contenant 3 à 12 carbones, de préférence 3 à 8, parmi cyclopropyle, cyclobutyle, cyclopentyle, cyclohexyle, cycloheptyle, cyclooctyle, cyclononyle, cyclodecyle, cycloundécyle et cyclododécyle.

Le terme "halogène" représente un atome de fluor, de chlore, de brome ou d'iode.

Le terme "inférieur" appliqué à un radical alkyle, signifie que le radical peut être linéaire ou ramifié et qu'il peut comprendre de 1 à 7 atomes de carbone, tel que méthyle, éthyle, propyle, isopropyle, butyle, tert-butyle, isobutyle, pentyle, hexyle, heptyle.

Le terme "alkoxy inférieur" désigne un groupe alkyle inférieur lié à un atome d'oxygène, tel que les radicaux méthoxy, éthoxy, butoxy, hexyloxy.

Les termes "alkylthio inférieur", "alkylsulfinyle inférieur" et "alkylsulfonyle inférieur" représentent un groupe alkyle inférieur lié à un atome de soufre respectivement non oxydé, mono-oxydé ou di-oxydé, tel que méthylthio, méthylsulfinyle ou méthylsulfonyle.

Les termes "acyle inférieur" et "thioacyle inférieur" représentent un groupe alkyle inférieur lié respectivement à une fonction carbonyle ou thiocarbonyle, tel que les radicaux acétyle, propionyle, thioacétyle.

Le terme "oxoalkylcarboxylique" représente un groupe acyle inférieur porteur d'une fonction acide carboxylique, tel que le radical 4-(4-oxobutanoïque).

Le terme "groupement hydrolysable" employé dans la définition de A signifie que l'acide organique peut se trouver sous forme de sel avec un métal (tel que Na, K, Li, Ca ou Mg) ou sous forme d'ester avec un radical alkoxy inférieur, avec des radicaux alkoxy inférieurs aminés tels que N,N-diéthylaminoéthoxy, N,N-diméthylaminoéthoxy, ou sous forme d'amide avec des amines telles que l'ammoniac, la morpholine, la pipéridine, la pipérazine ou avec des acides aminés tels que la glycine, la $\beta$-alanine.

Le terme "*trans*" utilisé pour les composés porteurs de carbones asymétriques dans le groupement cycloalkyle est appliqué lorsque les 2 substituants se trouvent de part et d'autre du plan formé par le cycle.

Le terme "*cis*" pour les composés porteurs de carbones asymétriques dans le groupement cycloalkyle est appliqué lorsque les 2 substituants se trouvent du même côté du plan formé par le cycle.

Une famille préférée, de composés de l'invention, dans laquelle $R_3$ est le groupement W et $R_2$ est différent de W, est représentée par la formule générale (Ia).

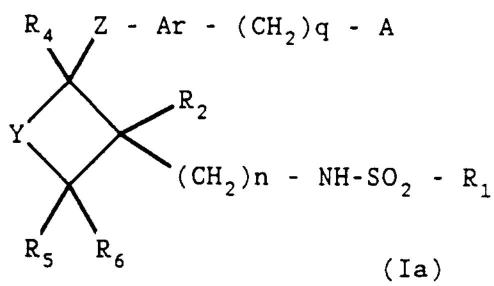

(Ia)

Une autre famille préférée, de composés de l'invention, dans laquelle $R_2$ est le groupement W et $R_3$ est différent de W, est représentée par la formule générale (Ib).

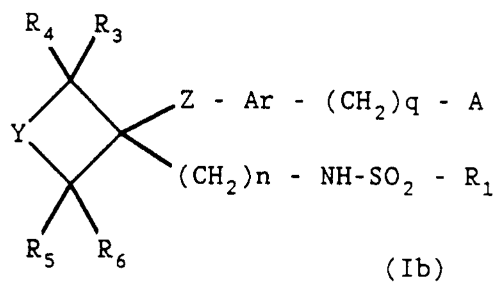

(Ib)

Les substituants $R_{1-6}$, les paramètres A, Y, Z, Ar, n et q possèdent les valeurs énoncées dans la formule (I).

Dans ces deux formules, (Ia) et (Ib), les produits préférés sont ceux pour lesquels Ar est un radical phényle, et A est une fonction acide carboxylique ou un radical tétrazolyle.

Dans les structures du type (Ia), il existe plusieurs familles préférées, en particulier lorsque n est égal à 0 et Z est $CH_2$, lorsque n est 0 et Z est une liaison et lorsque n est 0 et Z est un atome d'oxygène.

Dans les structures du type (Ib), il existe plusieurs familles préférées, en particulier lorsque n est égal à 0 et Z est une liaison, lorsque n est 0 et Z est $CH_2$, lorsque n est égal à 1 et Z est une liaison, lorsque n est 1 et Z est $CH_2$ et lorsque n est 1 et Z est un atome d'oxygène.

Parmi les composés préférés de l'invention on citera :
acide *trans*-4-[[2-[[(4-chlorophényl)sulfonyl]amino]cyclopentyl]méthyl]benzèneacétique,
*trans*-4-[[2-[[(4-chlorophényl)sulfonyl]amino]cyclopentyl]méthyl]benzèneacétate de sodium,
acide *cis*-4-[[2-[[(4-chlorophényl)sulfonyl]amino]cyclopentyl]méthyl]benzèneacétique,

acide *trans*-4-[[2-[[(4-fluorophényl)sulfonyl]amino]cyclopentyl]méthyl]benzèneacétique,
acide *trans*-4-[[2-[[(phényl)sulfonyl]amino]cyclopentyl]méthyl]benzèneacétique,
acide *trans*-4-[[2-[[(4-méthylphényl)sulfonyl]amino]cyclopentyl]méthyl]benzèneacétique,
*trans*-4-[[2-[[(4-chlorophényl)sulfonyl]amino]cyclopentyl]méthyl]benzèneacétate d'éthyle,
acide 4-[2-[[(4-chlorophényl)sulfonyl]amino]cyclopentyloxy]benzèneacétique,
acide *trans*-4-[2-[[(4-chlorophényl)sulfonyl]amino]cyclohexyl]benzèneacétique,
acide *trans*-4-[[2-[[(4-chlorophényl)sulfonyl]amino]cyclohexyl]méthyl]benzèneacétique,
acide 4-[1-[[[(4-chlorophényl)sulfonyl]amino]méthyl]cyclopropyl]benzèneacétique,
acide 4-[1-[[[(4-chlorophényl)sulfonyl]amino]méthyl]cyclobutyl]benzèneacétique,
acide 4-[1-[[[(4-chlorophényl)sulfonyl]amino]méthyl]cyclopentyl]benzèneacétique,
acide 4-[[1-[[[(phényl)sulfonyl]amino]méthyl]cyclopentyl]méthyl]benzèneacétique,
acide 4-[[1-[[[(4-fluorophényl)sulfonyl]amino]méthyl]cyclopentyl]méthyl]benzèneacétique,
N-[[1-[(4-acétylphényl)méthyl]cyclopentyl]méthyl]-4-chlorobenzènesulfonamide,
4-[[1-[[[(4-chlorophényl)sulfonyl]amino]méthyl]cyclopentyl]méthyl]benzèneacétate de méthyle,
acide 4-[[1-[[[(4-chlorophényl)sulfonyl]amino]méthyl]cyclopentyl]méthyl]benzèneacétique,
4-[[1-[[[(4-chlorophényl)sulfonyl]amino]méthyl]cyclopentyl]méthyl]benzèneacétate de sodium,
complexe *trans*-4-[[2-[[(4-chlorophényl)sulfonyl]amino]cyclopentyl]méthyl]benzèneacétate de sodium et $\beta$-cyclodextrine (1:1),
complexe 4-[[1-[[[(4-chlorophényl)sulfonyl]amino]méthyl]cyclopentyl]méthyl]benzèneacétate de sodium et $\beta$-cyclodextrine (1:1),
acide 4-[[1-[[[(4-méthylphényl)sulfonyl]amino]méthyl]cyclopentyl]méthyl]benzèneacétique,
acide 4-[[1-[[[(4-chlorophényl)sulfonyl]amino]méthyl]cyclopentyl]méthyl]benzoïque,
acide 4-[[1-[[[(3,4-dichlorophényl)sulfonyl]amino]méthyl]cyclopentyl]méthyl]benzèneacétique,
acide 4-[[1-[[[(4-chlorophényl)sulfonyl]amino]méthyl]cyclobutyl]méthyl]benzèneacétique,
4-chloro-N-[[1-[[4-(2-hydroxyéthyl)phényl]méthyl]cyclopentyl]méthyl]benzènesulfonamide,
acide 4-[4-[[1-[[[(4-chlorophényl)sulfonyl]amino]méthyl]cyclopentyl]méthyl]phényl]-4-oxobutanoique,
acide 4-[[1-[[[(2-chlorophényl)sulfonyl]amino]méthyl]cyclopentyl]méthyl]benzèneacétique,
3-chloro-N-[[1-[[4-(2-hydroxyéthyl)phényl]méthyl]cyclopentyl]méthyl]benzènesulfonamide,
4-acétyl-N-[[1-[[4-(2-hydroxyéthyl)phényl]méthyl]cyclopentyl]méthyl]benzènesulfonamide,
N-[[1-[[4-(2-hydroxyéthyl)phényl)méthyl]cyclopentyl]méthyl]-4-(méthylsulfonyl)benzènesulfonamide,
N-[[1-[[4-(2-hydroxyéthyl)phényl)méthyl]cyclopentyl]méthyl]-4-(trifluorométhoxy)benzènesulfonamide,
acide 4-[[1-[[[(4-bromophényl)sulfonyl]amino]méthyl]cyclopentyl]méthyl]benzèneacétique,
acide 4-[[1-[[[(4-iodophényl)sulfonyl]amino]méthyl]cyclopentyl]méthyl]benzèneacétique,
acide 4-[[1-[[[(4-trifuorométhylphényl)sulfonyl]amino]méthyl]cyclopentyl]méthyl]benzèneacétique,
acide 4-[[1-[[[(4-cyanophény)sulfonyl)amino]méthyl]cyclopentyl]méthyl]benzèneacétique,
acide 4-[[1-[[[(2,4-dichlorophényl)sulfonyl]amino]méthyl]cyclopentyl]méthyl]benzèneacétique,
acide 4-[[1-[[[(4-trifluorométhoxyphényl)sulfonyl]amino]méthyl]cyclopentyl]méthyl]benzèneacétique,
acide 4-[[1-[[[(4-méthoxyphényl)sulfonyl]amino]méthyl]cyclopentyl]méthyl]benzèneacétique,
acide 4-[[1-[[[(thien-2-yl)sulfonyl]amino]méthyl]cyclopentyl]méthyl]benzèneacétique,
acide 4-[[1-[[[(5-chlorothien-2-yl)sulfonyl]amino]méthyl]cyclopentyl]méthyl]benzéneacétique,
acide 4-[[1-[[[(4-hydroxyphényl)sulfonyl]amino]méthyl]cyclopentyl]méthyl]benzèneacétique,
acide 4-[[1-[[[(4-chlorophényl)sulfonyl]amino]méthyl]cyclopropyl]méthyl]benzèneacétique,
acide 4-[[1-[[[(4-chlorophényl)sulfony]amino]méthyl]cycloheptyl]méthyl]benzèneacétique,
acide 3-[[2-[4-[[1-[[[4-chlorophényl)sulfonyl]amino]méthyl]cyclopentyl]méthyl]phényl]-1-oxoéthyl]amino]-propionique,
4-[[1-[[[(4-chlorophényl)sulfonyl]amino]méthyl]cyclopentyl]méthyl]benzèneacétate d'éthyle,
4-[[1-[[[(4-chlorophényl)sulfonyl]amino]méthyl]cyclopentyl]méthyl]benzèneacétate de 2-(diéthylamino)éthyle,
acide 4-[[1-[[[4-chlorophényl)sulfonyl]amino]méthyl]cyclopentyl]méthyl]benzèneméthanesulfonique,
4-Chloro-N-[1-[[[4-[(1H-tétrazol-5-yl)méthyl]phényl)méthyl]cyclopentyl]méthyl]-benzènesulfonamide,
acide 4-[[1-[[[(4-chlorophényl)sulfonyl]amino]méthyl]cyclopentyl]méthyl]phénylméthanephosphonique,
acide 4-[[1-[[[(4-acétamidophényl)sulfonyl]amino]méthyl]cyclopentyl]méthyl]benzèneacétique,
acide 4-[[1-[[[(4-aminophényl)sulfonyl]amino]méthyl]cyclopentyl]méthyl]benzèneacétique,
acide 4-[[1-[[[(4-chlorophényl)sulfonyl]amino]méthyl]cyclohexyl]méthyl]benzèneacétique,
acide 4-[[4-[[[(4-chlorophényl)sulfonyl]amino]méthyl]-2,3,5,6-tétrahydropyran-4-yl]méthyl]benzéneacétique,
acide 4-[[1-[[[(4-chlorophényl)sulfonyl]amino]méthyl]-3,3-diméthylcyclobutyl]méthyl]benzèneacétique,
acide 2-[[1-[[[(4-chlorophényl)sulfonyl]amino]méthyl]cyclopentyl]méthyl]benzèneacétique,
acide 3-[[1-[[[(4-chlorophényl)sulfonyl]amino]méthyl]cyclopentyl]méthyl]benzèneacétique,
acide 4-[[1-[[[(4-nitrophényl)sulfonyl]amino]méthylcyclopentyl]méthyl]benzèneacétique,

acide 4-[[1-[[[(4-bromophényl)sulfonyl]amino]méthyl]cyclobutyl]méthyl]benzèneacétique,
acide 4-[[1-[[[(2-fluorophényl)sulfonyl]amino]méthyl]cyclopentyl]méthyl]benzèneacétique,
acide 4-[[1-[[[(naphtalen-2-yl)sulfonyl]amino]méthyl]cyclopentyl]méthyl]benzèneacétique,
acide 4-[[1-[[[(4-chloro-2-fluorophényl)sulfonyl]amino]méthyl]cyclopentyl]méthyl)benzèneacétique,
acide 4-[[1-[[[(quinonyl-8-yl)sulfonyl]amino]méthyl]cyclopentyl]méthyl]benzèneacétique.

L'invention vise également un procédé de préparation des composés de formule (I). Il s'agit d'un procédé de sulfonation décrit ci-dessous:

les produits de formule (I) sont préparés à partir de l'amine (II) que l'on fait réagir avec un chlorure d'arylsulfonyle ou d'hétéroarylsulfonyle dans les proportions molaires (II)/$R_1$-$SO_2Cl$ de 1:0,5 à 1:5, de préférence 1:0,95 à 1:1,05 pour donner (I).

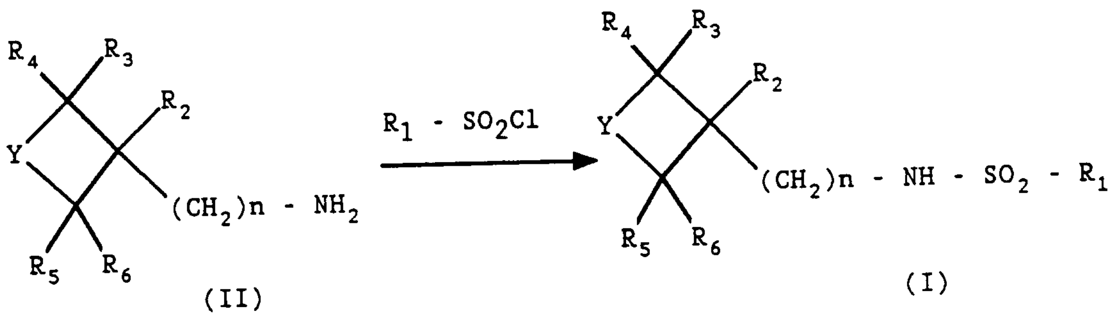


Dans les structures (II) et (I), W représenté par $R_2$ ou $R_3$ possède les valeurs définies précédement et peut prendre aussi la valeur -Z-Ar, soit plus précisément Ar, -$CH_2$-Ar ou -O-Ar. Les substituants $R_{1-6}$ et les paramètres Y, Z, Ar et n possèdent les valeurs définies précédement. Lorsque W contient A valant $CO_2H$, la fonction carboxylique est éventuellement protégée sous forme d'ester avec un radical alkoxy inférieur. Lorsque W contient A représentant un radical acyle inférieur, q étant égal à 0, la fonction carbonyle est bloquée par un groupement protecteur classique, tel que diméthylacétal, 1,3-dioxane, 5,5-diméthyl-1,3-dioxane.

La réaction de sulfonation est réalisée en présence d'une base telle que la triéthylamine, la pyridine, le carbonate de potassium, la soude, la potasse ou le butyllithium, en proportions molaires (II)/base 1:1 à 1:5, plus particulièrement 1:1,2 à 1:2,2 dans un solvant inerte vis à vis de la réaction, comme l'eau, le benzène, le tétrahydrofurane, l'éther éthylique ou le dichlorométhane ou un mélange éther/dichlorométhane dans des proportions v/v 1:1 à 1:30 et plus particulièrement de 1:2 à 1:15. La température de la réaction est comprise entre -78 °C et la température du reflux du solvant ou du mélange de solvants et de préférence entre -20 °C et la température du reflux du solvant ou du mélange de solvants, pendant une durée comprise entre 2 et 72 heures, de préférence entre 2 et 16 heures.

Les amines utilisées lors du procédé général de synthèse des composés (I) peuvent être formées selon plusieurs processus en fonction des substituants déjà présents dans la molécule:

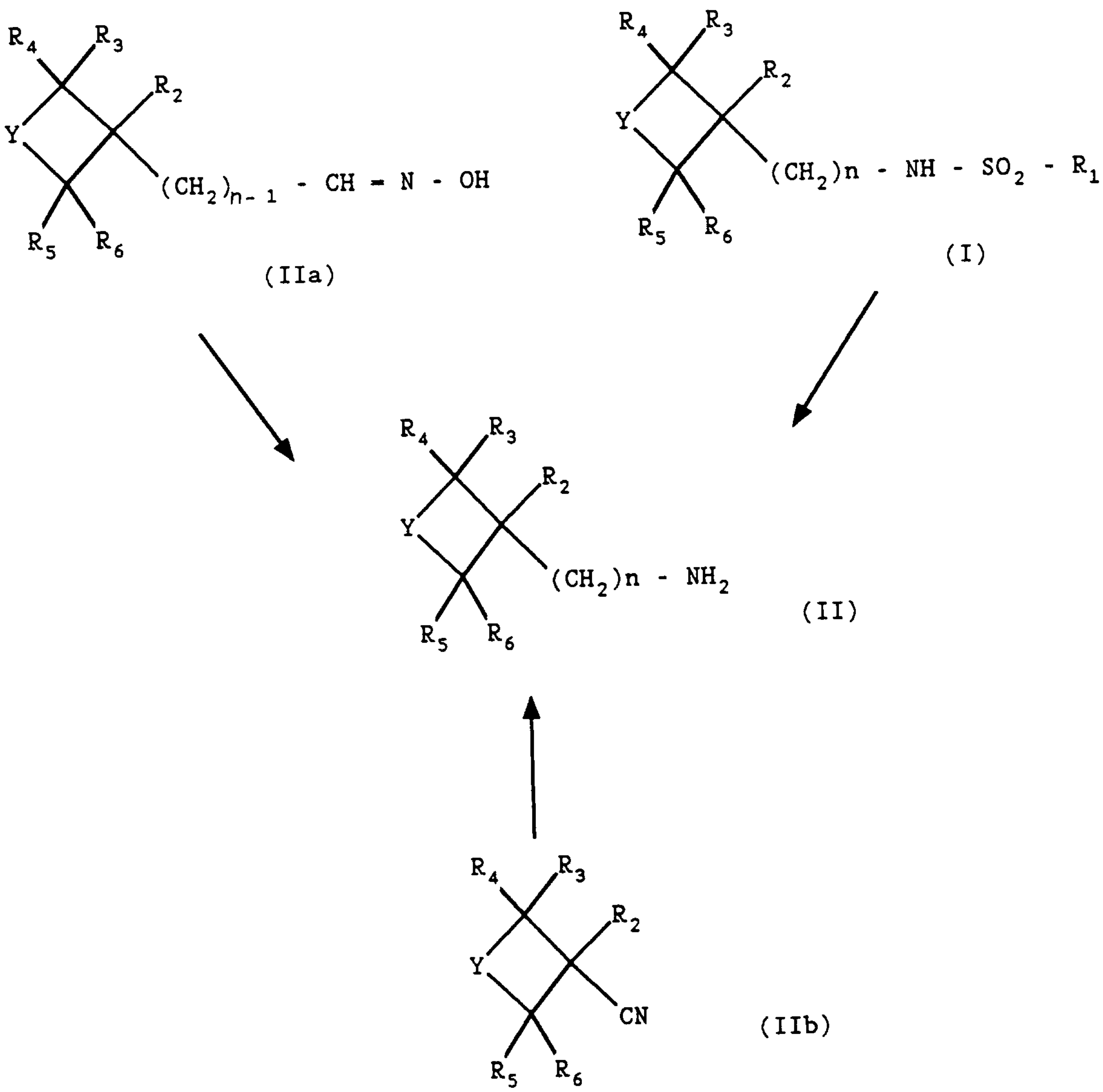

(IIa)

(I)

(II)

(IIb)

soit par réduction de l'oxime (IIa), par exemple lors d'une hydrogénation catalytique sous pression dans un mélange éthanol-ammoniaque; soit par coupure de la liaison sulfonamide de composés de formule (I), par exemple à l'aide d'une base telle que le naphtalène sodé dans un solvant de type 1,2-diméthoxyéthane; soit par réduction du nitrile (IIb), par exemple par hydrogénation catalytique sous pression dans un mélange méthanol-ammoniaque ou par réduction par $LiAlH_4$ dans l'éther, le tétrahydrofurane ou le toluène à une température comprise entre -30 ° C et le reflux du solvant et plus particulièrement entre 0 ° C et le reflux du solvant. Dans les formules (I), (II), (IIa) et (IIb), W représenté par $R_2$ ou $R_3$ possèdent les significations énoncées précédemment et peut prendre en plus la valeur -Z-Ar. Les substituants $R_{1-6}$ et les paramètres Y, Z, Ar et n possèdent les significations énoncées précédemment.

Les nitriles de la formule (IIb) sont obtenus après alkylation d'un nitrile (IIc) par un composé halogéné $R_2$-X.

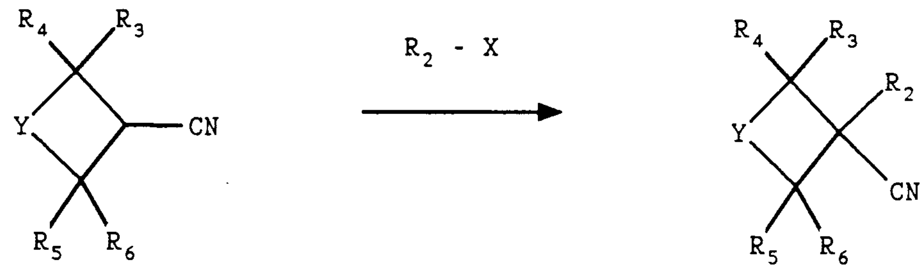


(IIc) (IIb)

X est un atome d'halogène, $R_2$-X étant plus précisement X-$CH_2$-Ar ou X-$CH_2$-Ar-$(CH_2)_q$-A; les substituants $R_{2-6}$ et les paramètres Y, Ar, A et q possèdent les significations énoncées précédemment. La réaction est réalisée en présence d'une base, telle que le butyllithium, le lithium diisopropylamide ou l'amidure de sodium dans un solvant comme l'éther diéthylique ou le tétrahydrofurane en présence d'hexaméthylphosphoramide ou de N,N-diméthylimidazolidinone, à une température comprise entre -78 °C et le reflux du solvant ou du mélange de solvants, pendant une durée comprise entre 4 heures et 24 heures.

Pour les composés de formule (IIb) où W, représenté par $R_2$, contient un alcool primaire, celui-ci est protégé par un groupement permettant d'effectuer la réaction d'alkylation, comme par exemple un groupement protecteur labile tel que les radicaux silyles, en particulier -$SiMe_3$. Le groupement protecteur est éliminé ensuite par traitement acide en milieu aqueux ou par l'utilisation de fluorure (par exemple le fluorure de tétrabutylammonium) dans le tétrahydrofurane, à une température comprise entre -20 °C et 150 °C, de préférence à 25 °C. Le nitrile-alcool (IIb) obtenu est alors réduit en composé (II) amino-alcool utilisable dans le procédé général de sulfonation. Après sulfonation, on aboutit alors au composé (I) porteur d'une fonction alcool primaire.

Tous les composés de formule (I), (Ia) et (Ib), porteurs d'une fonction alcool primaire peuvent être transformés en composés (I) porteurs d'une fonction acide carboxylique,

$$- Z - Ar - (CH_2)_{q+1} - OH \rightarrow - Z - Ar - (CH_2)_q - COOH$$

par oxydation de la fonction alcool, par exemple par la méthode de Jones ($CrO_3/H_2SO_4$) dans un solvant comme l'acétone, à des températures comprises entre -78 °C et 30 °C, et plus particulièrement entre -20 °C et 20 °C, durant 1 à 96 heures, de préférence entre 2 et 16 heures.

Dans les produits de la formule (I), et plus précisement dans ceux correspondant à la formule (Ia), pour lesquels $R_3$ représente W, les isomères stéréochimiquement purs, ***cis*** ou ***trans***, sont obtenus soit par réaction sur une amine stéréochimiquement pure, ***cis*** ou ***trans***, soit par réaction sur une amine racémique, mélange d'isomères ***cis*** et ***trans***, suivie d'une séparation des isomères par recristallisations sélectives ou par méthode chromatographique (comme par exemple la chromatographie liquide à haute performance préparative, la chromatographie liquide, la flash chromatographie, la chromatographie sur couche mince préparative). Les mélanges d'isomères et les produits purs sont dosés par chromatographie liquide à haute performance (C.L.H.P.), par détection ultra-violette.

Une autre possibilité pour accéder directement et uniquement aux isomères ***trans***, purs, est la synthèse stéréosélective, réalisable pour les produits de la formule (I), et plus précisement ceux de la formule (Ia), dans lesquels $R_3$ est W et $n=0$. A partir de l'aziridine de formule (III) par réaction avec un dérivé organométallique d'un halogénure W-Cl, tel que le chlorure de benzyle, on obtient un produit d'isomérie ***trans***, de structure (IV) dans laquelle W est Ar ou -$CH_2$-Ar; $R_{1-2}$, $R_{4-6}$, Ar, Y possèdent les significations énoncées précédemment.

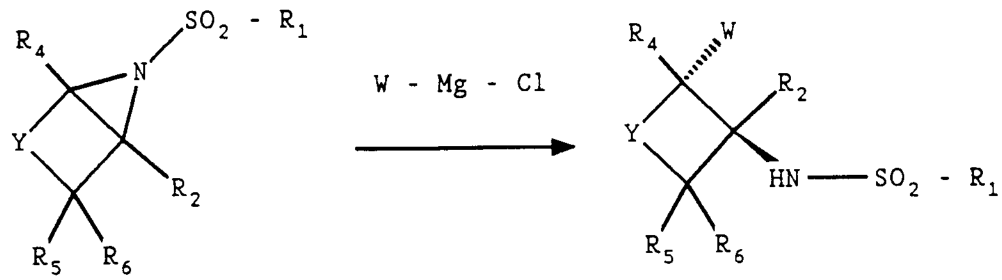


(III) (IV)

Cette réaction est réalisée dans un solvant tel que l'éther diéthylique, l'éther dibutylique, le tétrahydrofurane ou le dioxane, à une température comprise entre -78 ° C et la température de reflux du solvant, et plus particulièrement entre 0 ° C et la température de reflux du solvant, en utilisant un rapport molaire (III)-/halogénure de 1:0,25 à 1:5, de préférence 1:1 à 1:4. Le métal nécessaire à la formation de l'organométallique est de préférence le magnésium. Le temps de réaction est généralement compris entre 2 et 96 heures et plus particulièrement entre 16 et 48 heures.

Les aziridines de formule (III) utilisées dans la synthèse stéréospécifique des isomères *trans* sont préparées à partir d'arylsulfonamides ou d'hétérocycles sulfonamides (IIIa) selon le principe décrit par Ueno Y. et coll., Chem. Pharm. Bull. (1967) 15, 1193-7. Les dérivés sulfonamides (IIIa) transformés en dérivés N,N-dibromosulfonamides (IIIb) par action du brome en présence d'une base, telle que la soude aqueuse, réagissent spontanément sur des composés insaturés, comme les alcènes de formule (IIIc) en excès molaire, dans un solvant, en général le chloroforme, à une température comprise entre -30 ° C et le reflux du solvant, et plus particulièrement entre 0 ° C et 50 ° C, pendant une durée comprise entre 5 et 72 heures, et plus précisement entre 16 à 24 heures. Les intermédiaires bromés (V) sont ensuite cyclisés par action d'une base en excès, telle que la soude ou la potasse, dans des solvants tels que méthanol, éthanol, acétone, purs ou en mélange avec de l'eau, à une température comprise entre -20 ° C et le reflux du solvant, et de préférence à 20 ° C pendant une durée comprise entre 5 et 48 heures, de préférence entre 14 à 16 heures, pour donner l'aziridine (III). Les substituants $R_1$, $R_2$, $R_{4-6}$ et le paramètre Y, cités dans les formules (IIIa), (IIIb), (IIIc), (III) et (V) possèdent les valeurs définies précédemment.

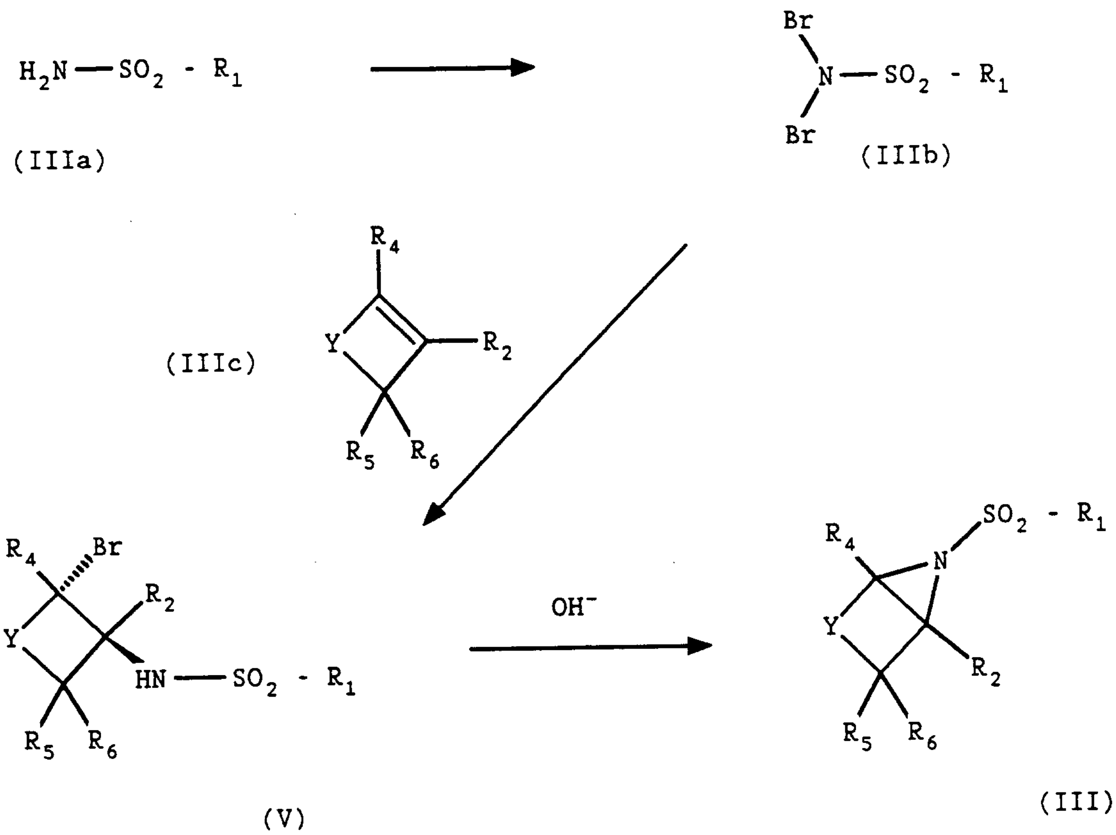


(IIIa) (IIIb) (IIIc) (V) (III)

Les intermédiaires bromés de formule (V) peuvent servir aussi de réactifs de départ pour la synthèse des composés de formule (I) et plus particulièrement de formule (Ia) pour lesquels $R_3$ représente W et Z compris dans W est un atome d'oxygène. Les composés bromés de formule

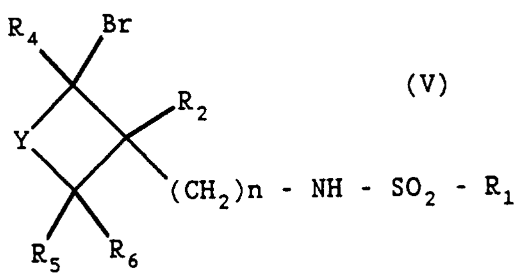

(V)

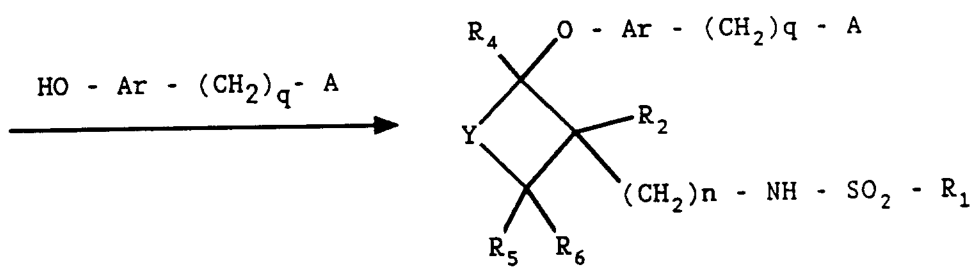


(VI)

(V), par O-alkylation d'un phénol fournissent directement des composés de formule (VI). Dans les structures (V) et (VI), les substituants $R_1$, $R_2$, $R_{4-6}$ et les paramètres Y, Ar, A, n et q possèdent les valeurs définies précédemment. Dans la formule (VI), lorsque A est $CO_2H$, la fonction carboxylique est protégée sous forme d'ester avec un radical alkoxy inférieur. La réaction se déroule avec un excès molaire de dérivé phénolique, dans un solvant classique des réactions d'alkylation comme le N,N-diméthylformamide, en présence d'une

base telle que le carbonate de potassium, la soude, la potasse, en proportions molaires phénol/base 1:0,5 à 1:10, de préférence 1:2. La réaction est chauffée à une température comprise entre 40 et 200 °C, de préférence à 80 °C pendant une durée comprise entre 1/2 heure et 16 heures, de préférence de 3 à 6 heures.

Les composés intermédiaires dans la préparation des composés de formule (I), (Ia) et (Ib), en particulier ceux de formule (I), (IIb) ou (IV), dans lesquels W représenté par $R_2$ ou $R_3$ est un radical Ar ou $-CH_2$-Ar, et plus particulièrement lorsque Ar représente le radical phényle, peuvent être convertis en composés de type (I) où W contiendra une fonction acide carboxylique. Par acylation de Friedel et Crafts, on obtient des dérivés para-acylés qui seront oxydés en esters (cas où A est un groupement hydrolysable en $CO_2H$) puis saponifiés pour donner des acides carboxyliques (I). La réaction de Friedel et Crafts est pratiquée en faisant réagir des halogénures d'acyle, tels que le chlorure d'acétyle, le 3-chloroformylpropionate d'éthyle, ou des anhydrides tels que l'anhydride succinique, en présence d'un acide de Lewis tel que $SnCl_4$, $TiCl_4$ ou $AlCl_3$, dans un solvant comme le

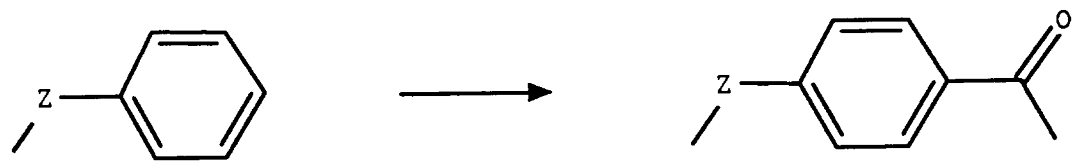

dichlorométhane ou le 1,2-dichloroéthane, à une température comprise entre -78 °C et le reflux du solvant et de préférence entre -30 °C et le reflux du solvant, durant 4 à 30 heures. On utilise un excès molaire d'acide de Lewis de 1,1 à 11 équivalents et plus particulièrement de 3,3 à 7 équivalents ainsi qu'un excès molaire d'halogénure d'acyle ou d'anhydride, de 1,1 à 4 équivalents, et de préférence 1,2 à 2,2 équivalents.

Tous les composés acylés peuvent se transformer en esters par oxydation avec le nitrate de thallium ($Tl(NO_3)_3$) ou le tétraacétate de plomb en présence de méthanol et d'un acide de Lewis comme l'acide perchlorique ou l'éthérate de trifluorure de bore,

$$- Z - Ar - CO - CH_3 \rightarrow - Z - Ar - CH_2 - CO - O - CH_3$$

dans un solvant tel que le dichlorométhane, le benzène , le toluène ou directement dans le méthanol, entre -50 °C et le reflux du solvant et plus particulièrement entre 20 °C et le reflux du solvant, durant 5 à 40 heures. On utilise un excès molaire d'acide de Lewis, de 1,1 à 11 équivalents. Le complexe nitrate de thallium-montmorillonite K-10 a aussi été utilisé en remplacement de $Tl(NO_3)_3$ dans les mêmes conditions et proportions, mais en l'absence d'acide perchlorique.

Tous les esters méthyliques sont saponifiés dans des conditions classiques, par un équivalent ou un excès molaire de base (soude, potasse, hydroxyde de lithium ou bicarbonate de sodium)

$$- Z - Ar - CH_2 - CO - O - CH_3 \rightarrow - Z - Ar - CH_2 - COOH$$

dans l'eau ou dans un mélange hydroalcoolique (méthanol ou éthanol), en présence ou en absence de tétrahydrofurane, à une température comprise entre 0 °C et le reflux du solvant ou du mélange de solvants, et de préférence entre 30 °C et le reflux du solvant ou du mélange de solvants.

Les composés (I) porteurs d'une fonction acide carboxylique peuvent aussi être formés à partir des dérivés acylés par transformation en dérivés thiomorpholides (par reflux dans la morpholine en présence d'un excès de soufre selon la méthode dite de Willgerodt), suivie d'une hydrolyse basique classique.

Les composés de l'invention possèdent notamment d'excellentes propriétés antithrombotiques et antiasthmatiques chez les mammifères, en particulier l'homme, le chat, le chien, dues à leur effet sur les récepteurs $TXA_2$.

L'activité antagoniste $TXA_2$ a été démontrée par l'effet antiagrégant plaquettaire et par l'effet spasmolytique vasculaire.

(A) Inhibition de l'agrégation plaquettaire induite par U46619, un analogue stable de $TXA_2$ (9,11-didéoxy-11$\alpha$,9$\alpha$-époxyméthanoprostaglandine $F_{2\alpha}$):

l'activité antagoniste du $TXA_2$ est démontrée dans le test d'agrégation plaquettaire décrit par BORN G.V.R. (Nature (1962) 194, 927-9), sur plaquettes de cobaye. Les tests déterminent une concentration de composé qui inhibe à 50% ($CI_{50}$) une agrégation liminaire induite par le U46619. Les activités des composés de la présente invention sont données dans l'exemple 108.

(B) Inhibition de la vasoconstriction de l'aorte de rat induite par U46619 :

des rats mâles d'environ 300 g sont euthanasiés; on prélève rapidement l'aorte thoracique. Un fragment d'environ 1 cm est coupé en spirale, puis placé dans une cuve renfermant 20 ml de milieu nutritif (Krebs-Henseleit) maintenu à 37 °C et oxygéné ($O_2$ 95%, $CO_2$ 5%). Les contractions sont enregistrées par une jauge de contrainte isotonique. Après un repos de 45 mn, des contractions supramaximales sont déclenchées par addition d'un analogue du $TXA_2$ (U46619 : 142 nM) au milieu de survie. Un plateau est en général atteint après 10 mn de contact, ce dernier ne dépassant pas 25 mn. Un repos de 30 mn et de nombreux rinçages séparent chaque contraction. Les produits sont ajoutés 10 mn après le U46619 et laissés 15 mn, délai au bout duquel l'inhibition est mesurée. On détermine pour chaque composé étudié et sur un minimum de 3 aortes, la concentration inhibitrice 50 ($CI_{50}$). Les activités des composés de la présente invention sont décrites dans l'exemple 108.

Les produits de cette invention possèdent aussi une activité hypocholestérolémiante, par action sur la biosynthèse du cholestérol, en inhibant par exemple l'HMG CoA réductase.

Les produits de cette invention possèdent aussi une activité contre les complications liées à la pathologie diabétique, comme les neuropathies, les néphropathies, les rétinopathies, les cataractes dues à l'accumulation de sorbitol. Les produits de l'invention empêche la synthèse du sorbitol, par inhibition de l'aldose réductase.

Les composés de cette invention sont caractérisés par leur absence de toxicité. A titre d'illustration, pour le composé décrit dans l'exemple 20, la dose létale 50 déterminée sur le rat, par voie intraveineuse est supérieure à 150 mg/kg.

Les produits de l'invention sont caractérisés, en ce qu'ils entrent dans des compositions renfermant une quantité efficace d'au moins un composé de formule (I), en association avec un véhicule pharmacologiquement acceptable. Ces compositions pharmaceutiques administrables chez les mammifères, sont utilisées en administration orale, intraveineuse, intraartérielle, cutanée, intestinale ou en aérosol. De plus ces nouveaux produits possèdent une longue durée d'action, quelque soit le mode d'administration.

Les produits de la formule générale (I) seront associés dans la forme pharmaceutique à des excipients, des arômes et des colorants adéquats pour former par exemple des comprimés, en outre sous forme liposomale, microcapsules ou nanocapsules, des comprimés pelliculés, des gélules, des solutions, des solutés injectables, des suppositoires, des aérosols ou des crèmes. Les excipients utilisés peuvent être par exemple la cellulose microcristalline, le lactose, la polyvidone, le glycolate sodique d'amidon, le talc, le stéarate de magnésium. Les excipients pour les formes liposomales, microcapsules et nanocapsules pourront être des polyalkylcyanoacrylates, des phospholipides. L'enrobage des comprimés peut être réalisé avec des additifs tels que l'hydroxypropylméthylcellulose, différents polymères acryliques, le propylèneglycol et le dioxyde de titane. Les préparations pour administration orale pourront contenir des arômes artificiciels et des édulcorants tels que le sucre, l'aspartam. Les préparations pour soluté injectable seront réalisées avec de l'eau qui contiendra des agents de stabilisation, de solubilisation, comme le chlorure de sodium, le mannitol et le sorbitol et/ou des tampons nécessaires aux solutions injectables. Les préparations pour suppositoires pourront utiliser des excipients tels que les glycérides semi-synthétiques. Les préparations pour crèmes seront réalisées entre autres par l'addition d'agents tensio-actifs non ioniques. Les préparations pour administration par aérosol pourront être réalisées à partir du principe actif micronisé, associé à un agent tensio-actif comme le trioléate de sorbitan, dans un gaz vecteur comme les CFC 11 et 12.

Les produits de cette invention inhibent fortement les activités biologiques du $TXA_2$, par effet antagoniste sur les récepteurs $TXA_2$. Ils peuvent être utilisés pour le traitement ou la prophylaxie des maladies ischémiques (telles que l'infarctus du myocarde, l'angine de poitrine, la thrombose), des maladies cérébrovasculaires (telles que l'attaque ischémique transitoire, la migraine, l'hémorragie cérébrale, l'infarctus cérébral), des maladies vasculaires périphériques et des maladies provoquées par des déséquilibres lipidiques (telles que l'athérosclérose, la convulsion capillaire, les désordres de la circulation périphérique, l'hypertension, l'avortement, le retard de croissance intra-utérin, la néphropathie diabétique, la rétinopathie, les troubles de la menstruation, l'embolisme pulmonaire), des maladies allergiques et inflammatoires (telles que l'asthme bronchique, la bronchite, la pneumonie, le syndrome de détresse respiratoire, le choc allergique, l'ulcère gastrique, la néphrite glomérulaire, le lupus érythémateux, l'hydronéphrite). Ils sont utilisés contre la formation de thrombus par exemple dans la circulation sanguine extracorporelle, en prophylaxie ou en traitement des complications thrombotiques péri et post-opératoires suites aux transplantations d'organes et à la néphrotoxicité de la cyclosporine, aux pontages aorto-coronariens, à l'angioplastie, à l'endartérectomie, à la thrombolyse et aux effets secondaires de la neutralisation de l'héparine par la protamine.

Les produits de cette invention peuvent aussi être utilisés en association avec tout autre substance d'utilisation thérapeutique, par exemple les thrombolytiques (streptokinase, urokinase, activateurs du plasminogène...), les inhibiteurs de phosphodiestérases, les analogues stables de la prostacycline, les inhibiteurs de la cyclooxygénase, les inhibiteurs de la thromboxane synthétase, les anticoagulants (antivitaminiques K, héparines, héparines de bas poids moléculaire), les vasodilatateurs périphériques et centraux, les antagonistes des récepteurs $S_2$ de la sérotonine, les antihistaminiques, les antagonistes du PAF-acéther, les activateurs des canaux potassiques.

Les doses journalières de principe actif, administrées en une ou plusieurs fois, seront comprises entre 0,01 et 100 mg/kg de poids corporel, et de préférence entre 0,1 et 50 mg/kg.

Les exemples suivants illustrent l'invention à titre non limitatif. Dans les données de résonance magnétique nucléaire (R.M.N.), les abréviations suivantes ont été utilisées : s pour singulet, d pour doublet, t pour triplet, q pour quadruplet et m pour massif complexe ; les déplacements chimiques $\delta$ sont exprimés en ppm. Les analyses par chromatographie liquide à haute performance (C.L.H.P.), ont été réalisées sur colonne Sphérisorb 5 $\mu$m, longueur = 15 cm, diamètre = 0,46 cm, dont la nature est précisée par les abréviations suivantes : Si pour silice et ODS-2 pour phase inverse C-18 "end-capped". La phase mobile utilisée sur la colonne de silice est un mélange hexane-acétate d'éthyle, dans les proportions précisées à chaque fois ; sur colonne ODS-2, on utilise un mélange méthanol-eau-acide acétique [57 : 43 : 0,01]. Le débit utilisé est de 1 ml/mn et la détection est réalisée par spectrophotométrie à 254 nm. Les temps de rétention, $t_R$, sont exprimés en minutes.

## Exemple 1

### Acide *trans*-4-[[2-[[(4-chlorophényl)sulfonyl]amino]cyclopentyl]méthyl]benzèneacétique

a) 4-[(2-Hydroxyiminocyclopentyl)méthyl]benzèneacétate d'éthyle

Une suspension de 10 g (0,04 mole) d'acide 4-[(2-hydroxyiminocyclopentyl)méthyl]benzèneacétique (préparé selon Terada A. et coll., J. Med. Chem. (1984) 27, 212-6) dans 70 ml d'éthanol absolu est saturée par HCl gaz, en maintenant la température du milieu réactionnel à environ 10 °C par un bain de glace + sel. Après saturation, la solution obtenue est agitée à température ambiante 1 heure, avant d'être concentrée à sec sous vide. Le résidu repris dans l'acétate d'éthyle est lavé successivement par une solution saturée de $NaHCO_3$ et par $H_2O$. La phase organique séchée sur $Na_2SO_4$ et évaporée, fournit 11,05 g (Rdt = 99,5 %) d'une huile épaisse brune partiellement cristallisée que l'on utilise sans autre purification.
I.R. (film) : $\nu$ (N-OH) = 3250 $cm^{-1}$ ; (C=O) = 1725 $cm^{-1}$.
R.M.N. ($CDCl_3$) : $\delta$ = 1,2 (3H,t, J = 6,75 Hz) ; 1,6-3,2 (9H, m) ; 3,5 (2H,s) ; 4,1 (2H,q, J = 6,75 Hz) ; 7,1 (4H,s) ; 9,0 (1H,s large, échangeable avec $CF_3COOD$).
Une autre méthode est également possible : on porte à reflux durant 6 heures, dans un réacteur surmonté d'un séparateur dit de Dean-Stark, un mélange de 5,8 g d'acide 4-[(2-hydroxyiminocyclopentyl)méthyl]-benzèneacétique, de 4,1 ml d'éthanol 98° et de 2 gouttes d'$H_2SO_4$ concentré dans 20 ml de toluène. Après refroidissement le toluène est lavé par une solution de $NaHCO_3$ puis par $H_2O$. La phase organique est séchée sur $Na_2SO_4$ et concentrée. Le produit obtenu se présente sous forme d'une huile jaune.

b) 4-[(2-Aminocyclopentyl)méthyl]benzèneacétate d'éthyle (*cis + trans*)

Dans un autoclave, une solution de 75,1 g (0,27 mole) de 4-[(2-hydroxyiminocyclopentyl)méthyl]-benzèneacétate d'éthyle dans 800 ml d'éthanol absolu saturé par $NH_3$ est additionnée d'environ 37,5 g de Nickel de Raney dans l'eau. Le mélange est agité sous 80 atmosphères d'hydrogène, à 80 °C pendant 4 heures. Après refroidissement, le catalyseur est filtré et rincé à l'éthanol. Le filtrat concentré sous pression réduite, fournit 69,3 g (Rdt = 98,3 %) d'une huile orangée qui cristallise mais que l'on utilise sans autre purification.
I.R. (film) : $\nu$ ($NH_2$) = 3300 $cm^{-1}$ ; (C=O) = 1715 $cm^{-1}$.
R.M.N. ($CDCl_3$) : $\delta$ = 1,2 (3H,t, J = 6,75 Hz) ; 1,5 (2H, m) ; 1,6 - 3,25 (10H,m,dont 2H échangeables par $CF_3CO_2D$) ; 3,55 (2H,s) ; 4,1 (2H,q, J = 6,75 Hz) ; 7,1 (4H,s).
Ce composé peut aussi être obtenu par hydrogénation sous faible pression d'hydrogène (3 atmosphères) à 25 °C.

c) 4-[[2-[[(4-Chlorophényl)sulfonyl]amino]cyclopentyl]méthyl]benzèneacétate d'éthyle (*cis* + *trans*)

Un mélange de 2,25 g (8,6 mmoles) de l'amine préparée dans l'exemple 1b, de 1,45 ml (10,3 mmoles) de triéthylamine et de 70 ml de $CH_2Cl_2$, est amené à 10 °C par un bain de glace-eau. Une solution de 1,9 g (9 mmoles) de chlorure de 4-chlorobenzènesulfonyle dans 5 ml d'éther est ajoutée goutte à goutte en 3 mn. On laisse revenir à température ambiante et on agite 2 heures. On verse alors sur 150 g de glace-eau et on acidifie à pH 1 sous agitation par HCl 6N. On décante $CH_2Cl_2$ et on extrait à nouveau par 100 ml de $CH_2Cl_2$. Les phases organiques réunies sont lavées par $H_2O$, puis séchées sur $Na_2SO_4$ avant d'être concentrées. L'huile jaune obtenue est purifiée par flash chromatographie sur colonne de silice avec un mélange hexane-acétate d'éthyle 5/1, pour donner 3,35 g (Rdt = 89,3 %) d'une huile jaune très pâle, qui cristallise lentement.
I.R. (film) : $\nu$ (NH) = 3250 $cm^{-1}$ ; (C=O) = 1725 $cm^{-1}$; ($SO_2$) = 1320 $cm^{-1}$ ; ($SO_2$) = 1150 $cm^{-1}$.
R.M.N. ($CDCl_3$) : $\delta$ = 1,2 (3H,t, J = 6,75 Hz) ; 1,15-1,9 (6H,m) ; 1,9-3,9 (4H,m) ; 3,6 (2H,s) ; 4,15 (2H,q, J = 6,75 Hz) ; 5,4 (1H, dd, J = 8,25 Hz,échangeable dans $D_2O$) ; 7,0 (4H,m) ; 7,4 (2H,m) ; 7,8 (2H,m).
C.L.H.P. (Si ; hexane-acétate d'éthyle 8:1) : $t_R$ = 14,1 (42 %) ; 16,0 (58 %).

d) Acide *trans*-4-[[2-[[(4-chlorophényl)sulfonyl]amino]cyclopentyl]méthyl]benzèneacétique

A une solution de 95,25 g (0,218 mole) d'ester éthylique préparé dans l'exemple 1c, et de 1200 ml d'éthanol 98°, sont ajoutés 24,4 g (0,435 mole) de KOH dissous dans 360 ml d'eau. Le mélange est agité 3 heures à 40 °C. L'éthanol est évaporé sous pression réduite. Le résidu repris par $H_2O$ est lavé par l'acétate d'éthyle. La phase aqueuse est acidifiée à froid, à pH 1, par HCl 6N. Le précipité formé est repris par l'acétate d'éthyle. Cette phase organique lavée par $H_2O$ jusqu'à neutralité, est séchée sur $Na_2SO_4$, puis concentrée sous vide. On obtient 75,9 g d'un solide crème qui est recristallisé quatre fois dans l'acétate d'éthyle pour donner 11 g de paillettes blanches - isomère *trans* - (Rdt = 12,4 %). F = 164 - 5 °C. Le traitement des eaux-mères permet de récupérer 1,2 g de produit de même pureté (Rdt global = 13,9 %).

| Analyse centésimale : $C_{20}H_{22}ClNO_4S$ (M = 407,912) | | | | | |
|---|---|---|---|---|---|
| | C % | H % | Cl % | N % | S % |
| Calculé | 58,89 | 5,44 | 8,69 | 3,43 | 7,86 |
| Trouvé | 58,75 | 5,30 | 8,74 | 3,47 | 7,73 |

I.R. (KBr) : $\nu$ (NH) = 3250 $cm^{-1}$ ; (C=O) = 1690 $cm^{-1}$; ($SO_2$) = 1320 $cm^{-1}$; ($SO_2$) = 1150 $cm^{-1}$.
R.M.N. (acétone-$d_6$) : $\delta$ = 1,15-1,9 (6H,m) ; 1,9-2,05 (1H,m) ; 2,3 (1H,m, $J_{H-H}$gem = 13 Hz) ; 2,8 (1H,m, $J_{H-H}$gem = 13 Hz) ; 3,3 (1H,m, $J_{CH-NH}$ = 7,8 Hz) ; 3,6 (2H,s) ; 6,7 (1H,d, $J_{NH-CH}$ = 7,8 Hz,échangeable avec $CF_3COOD$) ; 7,0-7,1 (2H,m) ; 7,15-7,25 (2H,m) ; 7,55-7,65 (2H,m) ; 7,8-7,9 (2H,m) ; 10,55 (1H,s,échangeable avec $CF_3COOD$).
C.L.H.P. (ODS-2) : $t_R$ = 30,15 (100 %). Avant recristallisation $t_R$ = 27,0 ; 30,0 ; ces deux pics d'égale intensité correspondent aux isomères *cis* et *trans*.

**Exemple 2**

***Trans*-4-[[2-[[(4-chlorophényl)sulfonyl]amino]cyclopentyl]méthyl]benzène acétate de sodium**

A une suspension de 3,9 g (9,6 mmoles) d'acide *trans*-4-[[2-[[(4-chlorophényl)sulfonyl]amino]-cyclopentyl]méthyl]benzèneacétique dans 50 ml d'eau distillée sont ajoutés 0,38 g (9,6 mmoles) de NaOH dissous dans 30 ml d'eau distillée. Le mélange est chauffé au bain-marie à 50 °C jusqu'à solubilisation complète, avant d'être filtré, et abandonné quelques heures au réfrigérateur. Le précipité blanc obtenu est filtré, rincé à l'eau distillée. L'eau résiduelle est éliminée par distillation azéotropique avec du toluène. Le produit obtenu est recristallisé dans l'eau distillée. Solide blanc. (Rdt = 57,0 %). F = 212 - 4 °C.

| Analyse centésimale : $C_{20}H_{21}ClNNaO_4S$ (M = 429,894) | | | | | | |
|---|---|---|---|---|---|---|
| | C % | H % | Cl % | N % | Na % | S % |
| Calculé | 55,88 | 4,92 | 8,25 | 3,26 | 5,35 | 7,46 |
| Trouvé | 55,67 | 5,02 | 8,31 | 3,16 | 5,50 | 6,99 |

I.R. (KBr) : $\nu$ (NH) = 3260 $cm^{-1}$ ; ($SO_2$) = 1315 $cm^{-1}$ ; ($SO_2$) = 1140 $cm^{-1}$.
R.M.N. (DMSO $d_6$) : $\delta$ = 0,7-1,6 (6H,m) ; 1,6-2,4 (3H,m) ; 2,6-3,7 (2H,m,dont 1H échangeable avec $CF_3COOD$) ; 3,2 (2H,s) ; 6,7-7,35 (4H,m) ; 7,6-8,1(4H,m).
C.L.H.P. (ODS-2) : 1 seul pic possèdant un $t_R$ identique au produit de l'exemple 1d.

**Exemple 3**

**Acide *cis*-4-[[2-[[(4-chlorophényl)sulfonyl]amino]cyclopentyl]méthyl]benzèneacétique**

A partir des eaux-mères de la première recristallisation du produit de l'exemple 1d et par relargage de l'isomère *trans* dans un mélange acétate d'éthyle-hexane, on isole 1,1 g d'isomère *cis* pur. Solide blanc. F = 142-5 °C.

| Analyse centésimale : $C_{20}H_{22}ClNO_4S$ (M = 407,912) | | | | | |
|---|---|---|---|---|---|
| | C % | H % | Cl % | N % | S % |
| Calculé | 58,89 | 5,44 | 8,69 | 3,43 | 7,86 |
| Trouvé | 58,80 | 5,56 | 8,90 | 3,41 | 7,70 |

I.R. (KBr) : $\nu$ (NH) = 3230 $cm^{-1}$ ; (C=O) = 1690 $cm^{-1}$ ; ($SO_2$) = 1315 $cm^{-1}$ ; ($SO_2$) = 1150 $cm^{-1}$.
R.M.N. (acétone $d_6$) : $\delta$ = 1,15-1,85 (6H,m) ; 2,05-2,25 (1H,m) ; 2,35(1H,m) ; 2,8 (1H,m) ; 3,6 (2H,s) ; 3,7 (1H,m,$J_{CH-NH}$ = 8,7 Hz) ; 6,6 (1H,d, $J_{NH-CH}$ = 8,7 Hz, echangeable avec $CF_3COOD$) ; 7,0-7,1 (2H,m) ; 7,15-7,25 (2H,m) ; 7,6-7,7 (2H,m) ; 7,85-7,95 (2H,m) ; 9,5-10,4 (1H,s large, échangeable dans $CF_3COOD$).
C.L.H.P. (ODS-2 : $t_R$ = 25,2 (99,4 % - isomère *cis*) ; 28,2 (0,6 % - isomére *trans*).

**Exemple 4**

**Acide *trans*-4-[[2-[[(4-fluorophényl)sulfonyl]amino]cyclopentyl]méthyl]benzèneacétique**

a) 4-[[2-[[(4-Fluorophényl)sulfonyl]amino]cyclopentyl]méthyl]benzèneacétate d'éthyle (*cis* + *trans*)

Obtenu en opérant comme dans l'exemple 1c à partir de 5,9 g (22,6 mmoles) de 4-[(2-aminocyclopentyl)méthyl]benzèneacétate d'éthyle préparé dans l'exemple 1b, et de 4,6 g (23,6 mmoles) de chlorure de 4-fluorobenzènesulfonyle en présence de 3,7 ml (26,7 mmoles) de triéthylamine dans 190 ml d'un mélange dichlorométhane-éther (8,5:1); on agite 16 heures à température ambiante. Purification par chromatographie sur colonne de silice avec un mélange hexane-acétate d'éthyle (4 : 1) pour obtenir 5,1 g (Rdt = 53,7 %) d'une huile jaune.
I.R. (film) : $\nu$ (NH) = 3250 $cm^{-1}$ ; (C=O) = 1715 $cm^{-1}$ ; ($SO_2$) = 1325 $cm^{-1}$ ; ($SO_2$) = 1150 $cm^{-1}$.
R.M.N. ($CDCl_3$) : $\delta$ = 1,3 (3H,t, J = 6,75 Hz) ; 1,0-1,9 (6H,m) ; 1,9-3,5 (4H,m) ; 3,6 (2H,d) ; 4,15 (2H,q, J = 6,75 Hz) ; 5,2 (1H,dd, J = 8,25 Hz, échangeable avec $CF_3COOD$) ; 6,8-7,4 (4H,m) ; 7,5-8,0 (4H,m).
C.L.H.P. (Si, hexane-acétate d'éthyle 4:1) : $t_R$ = 6,05 (41,8 %) ; 6,6 (58,2 %)

b) Acide *trans*-4-[[2-[[(4-fluorophényl)sulfonyl]amino]cyclopentyl]méthyl]benzèneacétique

Obtenu en opérant comme dans l'exemple 1d à partir de 4,5 g (10,7 mmoles) d'ester éthylique obtenu dans l'exemple 4a et de 1,2 g (21,4 mmoles) de KOH dans un mélange éthanol-eau. Après plusieurs recristallisations dans hexane-acétate d'éthyle, on obtient 0,3 g (Rdt = 7,1 %) d'un solide blanc. F = 145-8 °C.

| Analyse centésimale : $C_{20}H_{22}FNO_4S$ (M = 391,457) | | | | | |
|---|---|---|---|---|---|
| | C % | H % | F % | N % | S % |
| Calculé | 61,37 | 5,66 | 4,85 | 3,58 | 8,19 |
| Trouvé | 61,37 | 5,67 | 4,93 | 3,57 | 8,24 |

I.R. (KBr) : ν (NH) = 3250 $cm^{-1}$ ; (C=O) = 1685 $cm^{-1}$ ; ($SO_2$) = 1320 $cm^{-1}$ ; ($SO_2$) = 1150 $cm^{-1}$.
R.M.N. (DMSO $d_6$) : δ = 0,8-1,6 (6H,m) ; 1,6-2,4 (3H,m) ; 2,4-3,7 (2H,m, dont 1H échangeable par $CF_3CO_2D$ ; 3,5 (2H,s) ; 6,8-7,3 (4H,m) ; 7,4-8,1 (5H,m,dont 1H échangeable par $CF_3CO_2D$).
C.L.H.P. (ODS-2 : $t_R$ = 17,1 (0,9 % - isomère *cis*) ; 18,8 (99,1 % - isomère *trans*).

**Exemple 5**

**Acide *trans*-4-[[2-[[(4-chlorophényl)sulfonyl]amino]cyclopentyl]méthyl]benzèneacétique**

a) N-(2-Bromocyclopentyl)-4-chlorobenzènesulfonamide

Une solution de soude 20 % (270 ml, 1,80 mole) est coulée goutte à goutte en 30 minutes sur un mélange de 128 g (0,67 mole) de 4-chlorobenzènesulfonamide et de 400 g (2,5 moles) de brome sous forte agitation. Durant l'addition la température est maintenue à 5°C par un bain glace-eau. Le milieu réactionnel est ensuite agité 30 minutes à température ambiante, avant de filtrer le précipité jaune orange formé. Après lavage à l'eau, on obtient un solide jaune, fondant à 104-8°C (F = 102°C selon Baxter R.R. et Chattaway F.D., J. Chem. Soc. (1915) 1814-23) que l'on reprend par 600 ml de chloroforme tiédi. L'eau résiduelle est décantée. Cette phase chloroformique est ajoutée goutte à goutte en 45 minutes à 235 ml (2,67 moles) de cyclopentène maintenu à une température de 5°C. La solution obtenue est agitée ensuite 22 heures à température ambiante, avant d'être lavée par $H_2O$, séchée sur $Na_2SO_4$ et concentrée sous pression réduite. Le résidu est purifié par recristallisation dans un mélange d'acétate d'éthyle et d'hexane. On obtient 140,2 g d'un solide blanc. (Rdt = 62 %). F = 113-8°C.
I.R. (KBr) : ν (NH) = 3240 $cm^{-1}$.
R.M.N. ($CDCl_3$) : δ = 1,25-2,5 (6H,m) ; 3,7 (1H,m) ; 4,1 (1H,m) ; 5,3 (1H,d, J = 6,75 Hz, échangeable dans $CF_3COOD$) ; 7,35-7,7 (2H,m) ; 7,7 - 8,05 (2H,m).

b) 6-[(4-Chlorophényl)sulfonyl-6-azabicyclo[3.1.0]hexane

Une suspension de 140 g (0,413 mole) du produit préparé dans l'exemple 5a, dans 670 ml d'éthanol est chauffée jusqu'à solubilisation. Une solution de soude 20 % (80,5 ml, 0,537 mole) est alors ajoutée rapidement. On laisse revenir le milieu réactionnel à température ambiante, sous agitation, avant de le concentrer à sec sous pression réduite. Le résidu repris par l'acétate d'éthyle est lavé par l'eau, séché sur sulfate de sodium et concentré sous vide. Le résidu est purifié par chromatographie sur colonne de silice (éluant : hexane-acétate d'éthyle 9/1) pour donner 54 g d'un solide blanc (Rdt = 50,8 %). F = 75-6°C.
I.R. (KBr) : ν ($SO_2$) = 1310 $cm^{-1}$ ; ($SO_2$) = 1150 $cm^{-1}$.
R.M.N. ($CDCl_3$) : δ = 1,3-2,3 (6H,m) ; 3,4 (2H,s) ; 7,3-7,6 (2H,m) ; 7,7-8,0 (2H,m).

c) *Trans*-4-chloro-N-[2-[(phényl)méthyl]cyclopentyl]-benzènesulfonamide

A une solution de 0,27 mole de chlorure de phénylméthylmagnésium (préparé à partir de 6,7 g de magnésium en tournures et de 31,5 ml de chlorométhylbenzène dans 105 ml d'éther anhydre) sous courant d'azote, maintenue à 0°C, on ajoute goutte à goutte 35,3 g (0,137 mole) du composé préparé dans l'exemple 5b dissous dans 300 ml d'éther anhydre. Le milieu réactionnel est agité 20 heures à 20°C. Après refroidissement à 0°C, le magnésien en excès est détruit par l'addition de 210 ml d'une solution saturée de chlorure d'ammonium. Après dissolution du précipité blanc inorganique formé par de l'eau saturée en NaCl, on extrait 2 fois par l'acétate d'éthyle. La phase organique lavée par $H_2O$, séchée sur $Na_2SO_4$ et concentrée fournit une huile qui, après trituration dans l'hexane, donne 30,5 g (Rdt = 63,7 %) d'un solide blanc utilisé sans autre purification. F = 66-8°C.
I.R. (KBr) : ν (NH) = 3260 $cm^{-1}$ ; ($SO_2$) = 1320 $cm^{-1}$ ; ($SO_2$) = 1150 $cm^{-1}$.
R.M.N. ($CDCl_3$) : δ = 0,9-2,3 (7H,m) ; 2,35-2,95 (2H,m) ; 3,2 (1H,m) ; 4,9 (1H,d, J = 6,75 Hz,échangeable dans $CF_3COOD$) ; 6,8-7,5 (7H,m) ; 7,6-7,9 (2H,m).

d) *Trans*-N-[2-[(4-acétylphényl)méthyl]cyclopentyl]4-chlorobenzènesulfonamide

A un mélange de 30,5 g (87 mmoles) du composé préparé dans l'exemple 5c et de 690 ml de dichlorométhane maintenu entre -30 et -20 °C, on ajoute 15 g (191 mmoles) de chlorure d'acétyle, puis par portions en 1 heure, 46,4 g (348 mmoles) de chlorure d'aluminium anhydre. Le milieu réactionnel orangé est maintenu à -30 °C pendant 3,5 heures avant d'être versé sur un mélange glace + acide chlorhydrique concentré, et extrait au dichlorométhane. La phase organique est lavés à l'eau, séchée sur $Na_2SO_4$ et concentrée. Le résidu obtenu est purifié par chromatographie sur colonne de silice (éluant : hexane-acétate d'éthyle 10/1 puis 2/1) pour donner 14,8 g (Rdt = 43,5 %) d'une huile brune orangée.
I.R. (film) : $\nu$ (NH) = 3250 $cm^{-1}$ ; (C=O) = 1660 $cm^{-1}$ ; ($SO_2$) = 1320 $cm^{-1}$ ; ($SO_2$) = 1150 $cm^{-1}$.
R.M.N. ($CDCl_3$) : $\delta$ = 0,8-2,1 (7H,m) ; 2,1-2,95 (2H,m) ; 2,55 (3H,s) ; 3,2 (1H,m) ; 5,2 (1H,d, J = 8,25 Hz,échangeable avec $CF_3COOD$) ; 6,8-8,0 (8H,m).

e) *Trans*-4-[[2-[[(4-chlorophényl)sulfonyl]amino]cyclopentyl]méthyl]benzèneacétatede méthyle

A un mélange de 14,8 g (38 mmoles) du composé préparé dans l'exemple 5d, de 18 ml de méthanol et de 90 ml de dichlorométhane, sous courant d'azote, et maintenu à 0 °C on ajoute goutte à goutte 18,7 ml (152 mmoles) d'éthérate de trifluorure de bore. Ce mélange est agité 15 minutes à température ambiante avant d'être coulé, en une seule fois sur une suspension de 17,5 g (39,5 mmoles) de tétraacétate de plomb dans 105 ml de benzène, sous azote, maintenue à 10 °C. Le milieu réactionnel orangé est agité 22 heures à température ambiante avant d'être jeté sur de la glace, et extrait au dichlorométhane. La phase organique lavée par $H_2O$, séchée sur $Na_2SO_4$ et concentrée fournit une huile qui après trituration dans l'hexane donne 13,2 g (Rdt = 82,5 %) d'un solide beige utilisé sans autre purification. F = 80 °C.
I.R. (KBr) : $\nu$ (NH) = 3200 $cm^{-1}$ ; (C=O) = 1690 $cm^{-1}$ ; ($SO_2$) = 1320 $cm^{-1}$ ; ($SO_2$) = 1150 $cm^{-1}$.
R.M.N. ($CDCl_3$) : $\delta$ = 0,9-2,2 (7H,m) ; 2,3-2,9 (2H,m) ; 3,0-3,4 (1H,m) ; 3,6 (2H,s) ; 3,65 (3H,s) ; 5,0 (1H,d, J = 8,25 Hz,échangeable dans $CF_3COOD$) ; 6,8-7,25 (4H,m) ; 7,3-7,5 (2H,m) ; 7,6-7,9 (2H,m).
C.L.H.P. (Si, hexane-acétate d'éthyle 8:1) : $t_R$ = 19,0 (1 seul pic).

f) Acide *trans*-4-[[2-[[(4-chlorophényl)sulfonyl]amino]cyclopentyl]méthyl]benzèneacétique

Un mélange de 3,5 g (62,5 mmoles) de KOH en pastilles dans 120 ml d'eau et de 13,2 g (31 mmoles) du composé préparé dans l'exemple 5e dissous dans 300 ml d'éthanol est agité à 40 °C pendant 2 heures. L'éthanol est ensuite concentré sous pression réduite et la phase aqueuse résiduelle lavée à l'éther. L'acidification de la phase aqueuse permet d'obtenir un précipité beige, qui après décoloration au noir végétal et recristallisation dans le toluène, puis dans l'acétate d'éthyle fournit 4,6 g (Rdt = 36,5 %) de paillettes blanches possédant toutes les caractéristiques physiques, spectrales et chromatographiques du produit décrit dans l'exemple 1d.

**Exemple 6**

**Acide *trans*-4-[[2-[[(phényl)sulfonyl]amino]cyclopentyl]méthyl]benzèneacétique**

a) 6-(Phénylsulfonyl)-6-azabicyclo[3.1.0]hexane

Une solution de 93 g (0,306 mole) de N-(DL-*trans*-2-bromocyclopentyl)benzènesulfonamide (préparé selon Ueno Y. et coll., Chem. Pharm. Bull. (1967) 15, 1328-30), de 610 ml d'acétone et de 61 ml de NaOH 30 % est porté à reflux 2 mn, puis agitée 16 heures à température ambiante. Après concentration sous pression réduite, le résidu est repris par l'eau et extrait à l'éther. La phase organique lavée à l'eau est séchée sur $Na_2SO_4$ et concentrée sous vide pour donner 67,5 g (Rdt = 99,0 %) d'une huile jaune clair, utilisée sans autre purification. Une fraction de ce produit est purifié par chromatographie sur silice (mélange hexane-acétate d'éthyle 2/1) pour fournir une huile incolore. $Eb_{0,5}$ = 137-9 °C.

| Analyse centésimale : $C_{11}H_{13}NO_2S$ (M = 223,29) | | | | |
|---|---|---|---|---|
| | C % | H % | N % | S % |
| Calculé | 59,17 | 5,87 | 6,27 | 14,36 |
| Trouvé | 59,47 | 5,88 | 6,16 | 14,37 |

I.R. (film) : ν ($SO_2$) = 1310 $cm^{-1}$ ; ($SO_2$) = 1150 $cm^{-1}$.
R.M.N. ($CDCl_3$) : δ = 1,0-2,35 (6H,m) ; 3,4 (2H,s) : 7,25-7,75 (3H,m) ; 7,75-8,2 (2H,m).

b) *Trans*-N-[2-[(phényl)méthyl]cyclopentyl]-benzènesulfonamide

Obtenu en opérant comme dans l'exemple 5c à partir de 1,65 mode de chlorure de phénylméthylmagnésium et de 184,3 g (0,825 mole) du composé préparé dans l'exemple 6a, dans 2250 ml d'éther anhydre. On obtient 249,8 g (Rdt = 96,0 %) d'un solide blanc utilisé sans autre purification. F = 89-91 ° C. Une portion de ce produit a été recristallisée deux fois dans un mélange hexane-acétate d'éthyle pour donner un solide blanc. F = 96-98 ° C.

| Analyse centésimale : $C_{18}H_{21}NO_2S$ (M = 315,43) | | | | |
|---|---|---|---|---|
| | C % | H % | N % | S % |
| Calculé | 68,54 | 6,71 | 4,44 | 10,16 |
| Trouvé | 68,69 | 6,74 | 4,33 | 10,13 |

I.R. (KBr) : ν (NH) = 3210 $cm^{-1}$ ; ($SO_2$) = 1310 $cm^{-1}$ ; ($SO_2$) = 1140 $cm^{-1}$.
R.M.N. ($CDCl_3$) : δ = 1,0-2,3 (7H,m) ; 2,3-2,8 (2H,m) ; 3,1 - 3,5 (1H,m) ; 4,9 (1H,d, J = 7,5 Hz,echangeable dans $CF_3COOD$) ; 6,85-7,35 (5H,m) ; 7,4-7,65 (3H,m) ; 7,7-8,0 (2H,m).

c) *Trans*-N-[2-[(4-acétylphényl)méthyl]cyclopentyl]-benzènesulfonamide

Obtenu en opérant comme dans l'exemple 5d à partir de 13,2 g (41,8 mmoles) du composé préparé dans l'exemple 6b, de 6,5 ml (91,9 mmoles) de chlorure d'acétyle, de 22,3 g (167 mmoles) de chlorure d'aluminium anhydre dans 330 ml de 1,2-dichloroéthane. Agitation 4,5 heures à -20 ° C. La purification par chromatographie sur colonne de silice (éluant = hexane-acétate d'éthyle 2/1) fournit 13,2 g (Rdt = 88,6 %) d'un solide jaune pâle, F = 78 ° C, utilisé sans autre purification. Un échantillon recristallisé dans un mélange hexane-acétate d'éthyle fournit un solide blanc à reflets jaunes fondant à 90-2 ° C.

| Analyse centésimale : $C_{20}H_{23}NO_3S$ (M = 357,468) | | | | |
|---|---|---|---|---|
| | C % | H % | N % | S % |
| Calculé | 67,20 | 6,49 | 3,92 | 8,97 |
| Trouvé | 67,40 | 6,34 | 3,97 | 9,29 |

I.R. (film) : ν (NH) = 3250 $cm^{-1}$ ; (C=O) = 1660 $cm^{-1}$ ; ($SO_2$) = 1315 $cm^{-1}$ ; ($SO_2$) = 1150 $cm^{-1}$.
R.M.N. ($CDCl_3$) : δ = 0,75-1,9 (7H,m) ; 1,9-2,9 (2H,m) ; 2,5 (3H,s) ; 2,95-3,4 (1H,m) ; 5,35 (1H,d, J = 8,25 Hz,échangeable dans $CF_3COOD$) ; 6,7-7,25 (2H,m) ; 7,35-8,1 (7H,m).

d) *Trans*-4-[[2-[[(phényl)sulfonyl]amino]cyclopentyl]méthyl]benzèneacétate de méthyle

A un mélange de 12,9 g (36 mmoles) du composé préparé dans l'exemple 6c, de 110 ml de méthanol et de 18 ml d'acide perchlorique à 70 % est ajouté par portions 19,2 g (43,2 mmoles) de nitrate de thallium trihydrate. Après agitation 25 heures à température ambiante, le précipité formé est filtré et rincé au méthanol. Le filtrat dilué à l'eau est extrait au dichlorométhane. La phase organique rincée à l'eau, séchée sur $Na_2SO_4$ est concentrée sous pression réduite. Le résidu purifié par chromatographie sur colonne de silice (éluant : hexane-acétate d'éthyle 2/1) donne 7,4 g (Rdt = 53,2 %) d'un solide beige. F = 88-90 ° C.
I.R. (KBr : ν (NH) = 3205 $cm^{-1}$ ; (C=O) = 1695 $cm^{-1}$ ; ($SO_2$) = 1320 $cm^{-1}$ ; ($SO_2$) = 1150 $cm^{-1}$.

R.M.N. ($CDCl_3$) : δ = 1,0-2,2 (7H,m) ; 2,2-2,9 (2H,m) ; 3,0-3,4 (1H,m) ; 3,5 (2H,s) ; 3,6 (3H,s) ; 5,05 (1H,d, J = 7,5 Hz, échangeable avec $CF_3COOD$) ; 6,75-7,25 (4H,m) ; 7,3-7,6 (3H,m) ; 7,65-7,9 (2H,m).

On obtient un produit identique par oxydation du composé de l'exemple 6c avec le tétraacétate de plomb dans les conditions décrites dans l'exemple 5e, suivie d'une purification par chromatographie sur colonne de silice. Une recristallisation supplémentaire dans un mélange hexane-acétate d'éthyle donne un solide blanc. F = 91-3 °C.

| Analyse centésimale : $C_{21}H_{25}NO_4S$ (M = 387,494) | | | | |
|---|---|---|---|---|
| | C % | H % | N % | S % |
| Calculé | 65,09 | 6,50 | 3,61 | 8,27 |
| Trouvé | 65,18 | 6,53 | 3,58 | 8,20 |

e) Acide *trans*-4-[[2-[[(phényl)sulfonyl]amino]cyclopentyl]méthyl]benzèneacétique

Obtenu en opérant comme dans l'exemple 5f à partir de 4,6 g (11,9 mmoles) du composé préparé dans l'exemple 6d et de 1,3 g (23,2 mmoles) de KOH dans un mélange éthanol-eau. Après recristallisation dans le toluène, on obtient 3,4 g (Rdt = 77,3 %) d'un solide blanc cassé. F = 118-20 °C.

| Analyse centésimale : $C_{20}H_{23}NO_4S$ (M = 373,467) | | | | |
|---|---|---|---|---|
| | C % | H % | N % | S % |
| Calculé | 64,32 | 6,21 | 3,75 | 8,58 |
| Trouvé | 64,67 | 6,18 | 3,74 | 8,69 |

I.R. (KBr) : ν (NH) = 3250 $cm^{-1}$ ; (C=O) = 1680 $cm^{-1}$ ; ($SO_2$) = 1315 $cm^{-1}$ ; ($SO_2$) = 1145 $cm^{-1}$.
R.M.N. (DMSO $d_6$) : δ = 0,75-1,7 (7H,m) ; 1,7-2,3 (2H,m) ; 3,1-3,35 (1H,m) ; 3,5 (2H,s) ; 6,65-7,4 (4H,m) ; 7,4-8,1 (6H,m, dont 1H échangeable par $CF_3COOD$), 12,2 (1H,s, échangeable par $CF_3COOD$).
C.L.H.P. (ODS-2 : $t_R$ = 17,65 (1 seul pic).

**Exemple 7**

**Acide*trans*-4-[[2-[[(4-méthylphényl)sulfonyl]amino]cyclopentyl]méthyl]benzèneacétique**

a) *Trans*-4-méthyl-N-[2-[(phényl)méthyl]cyclopentyl]-benzènesulfonamide

Obtenu en opérant comme dans l'exemple 5c à partir de 0,3 mole de chlorure de phénylméthylmagnésium et de 35 g (0,147 mole) de 6-[(4-méthylphényl)sulfonyl]-6-azabicyclo[3.1.0]hexane (préparé selon Hegedus L.S. et Mc Kearin J.M., J. Am. Chem. Soc. (1982) 104, 2444-51, mais obtenu sous forme d'un solide. Rdt = 70,2 % - F = 70 °C) dans 500 ml d'éther anhydre. On obtient 40,1 g (Rdt = 83,0 %) d'un solide blanc utilisé sans autre purification. F = 73-8 °C.
I.R. (KBr) : ν (NH = 3240 $cm^{-1}$ : ($SO_2$) = 1320 $cm^{-1}$ ; ($SO_2$) = 1140 $cm^{-1}$.
R.M.N. ($CDCl_3$) : δ = 0,75-2,85 (9H,m) ; 2,4 (3H,s) ; 3,0 -3,45 (1H,m) ; 5,1 (1H,d, J = 7,5 Hz, échangeable dans $CF_3COOD$) ; 6,75-7,4 (7H,m) ; 7,55-7,9 (2H,m).

b) *Trans*-N-[2-[(4-acétylphényl)méthyl]cyclopentyl]-4-méthylbenzènesulfonamide

Obtenu en opérant comme dans l'exemple 5d à partir de 10 g (30,3 mmoles) du composé préparé dans l'exemple 7a, de 4,7 ml (66 mmoles) de chlorure d'acétyle, de 16,2 g (122 mmoles) de chlorure d'aluminium anhydre, dans 240 ml de dichlorométhane. Agitation 5 h à -30/-20 °C. La purification par chromatographie sur colonne de silice (éluant = hexane-acétate d'éthyle 4/1) fournit 4,7 g (Rdt = 42,0 %) d'une huile qui cristallise partiellement.
I.R. (film) : ν (NH) = 3250 $cm^{-1}$ ; (C=O) = 1660 $cm^{-1}$ ; ($SO_2$) = 1320 $cm^{-1}$ ; ($SO_2$) = 1140 $cm^{-1}$.
R.M.N. ($CDCl_3$) : δ = 0,65-3,6 (10H, m) ; 2,4 (3H,s) ; 2,55 (3H,s) ; 5,3 (1H,d, J = 7,5 Hz, échangeable dans $CF_3COOD$) ; 6,9-7,4 (4H,m) ; 7,55-8,05 (4H,m).

c) *Trans*-4-[[2-[[(4-méthylphényl)sulfonyl]amino]cyclopentyl]méthyl]benzèneacétate de méthyle

Obtenu en opérant comme dans l'exemple 5e à partir de 4,7 g (12,6 mmoles) du composé préparé dans l'exemple 7b, de 6 ml de méthanol, de 6,2 ml (50,4 mmoles) d'éthérate de trifluorure de bore dans 30 ml de dichlorométhane, puis de 5,8 g (13,1 mmoles) de tétraacétate de plomb dans 35 ml de benzène. La purification par chromatographie sur colonne de silice (éluant = hexane-acétate d'éthyle 4/1) fournit 2,7 g (Rdt = 54,0 %) d'une huile jaune clair.
I.R. (film) : $\nu$ (NH = 3240 $cm^{-1}$ ; (C=O) = 1710 $cm^{-1}$ ; ($SO_2$) = 1310 $cm^{-1}$ ; ($SO_2$) = 1140 $cm^{-1}$.
R.M.N. ($CDCl_3$) : $\delta$ = 0,9-3,4 (10H,m) ; 2,4 (3H,s) ; 3,6 (2H,s) ; 3,7 (3H,s) ; 4,8 (1H,d, J = 7 Hz, échangeable dans $CF_3COOD$) ; 6,8-7,4 (6H,m) ; 7,6-7,85 (2H,m).

d) Acide *trans*-4-[[2-[[(4-méthylphényl)sulfonyl]amino]cyclopentyl]méthyl]benzèneacétique

Obtenu en opérant comme dans l'exemple 5f à partir de 2,7 g (6,7 mmoles) du composé préparé dans l'exemple 7c et de 0,75 g (13,4 mmoles) de KOH en pastilles dans un mélange éthanol-eau. Par recristallisation dans le toluène on obtient 0,6 g (Rdt = 23,1 %) d'un solide blanc. F = 134-5 ° C.

| Analyse centésimale : $C_{21}H_{25}NO_4S$ (M = 387,494) | | | | |
|---|---|---|---|---|
| | C % | H % | H % | S % |
| Calculé<br>Trouvé | 65,09<br>65,53 | 6,50<br>6,55 | 3,61<br>3,61 | 8,27<br>8,17 |

I.R. (KBr) : $\nu$ (NH) = 3250 $cm^{-1}$ ; (C=O) = 1690 $cm^{-1}$ ; ($SO_2$) = 1320 $cm^{-1}$ ; ($SO_2$) = 1145 $cm^{-1}$.
R.M.N. (DMSO $d_6$) : $\delta$ = 1,0-2,25 (8H,m) ; 2,4 (3H,s) ; 2,75-3,4 (2H,m) ; 3,5 (2H,s) ; 6,8-8,0 (6H,m) ; 8,0-8,5 (3H,m, dont 1H échangeable dans $CF_3COOD$) ; 12,3 (1H,s large, échangeable dans $CF_3COOD$).
C.L.H.P. (ODS-2) : $t_R$ = 33,5 (1 seul pic).

## Exemple 8

### *Trans*-4-[[2-[[(4-chlorophényl)sulfonyl]amino]cyclopentyl]méthyl]benzèneacétate d'éthyle

a) *Trans*-4-[(2-aminocyclopentyl)méthyl]benzèneacétate d'éthyle

On ajoute 0,5 g (21 mmoles) de sodium à un mélange de 2,6 g (21 mmoles) de naphtalène et de 20 ml de 1,2-diméthoxyéthane, sous courant d'azote. Après agitation 1 heure à 20 ° C, une solution de 2 g (5,2 mmoles) de *trans*-4-[[2-[[(phényl)sulfonyl]amino]cyclopentyl]méthyl]benzèneacétate de méthyle dans 20 ml de 1,2-diméthoxyéthane est ajoutée goutte à goutte en 1 heure, à température ambiante. Après agitation 1 heure à 20 ° C, on ajoute goutte à goutte 40 ml d'eau, avant de laver par l'acétate d'éthyle. La phase aqueuse acidifiée par HCl dilué est lavée par l'acétate d'éthyle, avant d'être concentrée à sec sous pression réduite. Le résidu est repris par 50 ml d'éthanol absolu et la suspension blanche obtenue est saturée à 0 ° C par HCl gaz. Après agitation 16 h à 20 °, le milieu réactionnel est concentré sous pression réduite, repris par $H_2O$, basifié par $NH_4OH$ et extrait par l'acétate d'éthyle. Cette phase organique après lavage par $H_2O$ saturée par NaCl est séchée sur $Na_2SO_4$, concentrée, pour donner 0,5 g (Rdt = 39,3 %) d'une huile brune partiellement cristallisée, utilisée sans autre purification.
I.R. (film) : $\nu$ ($NH_2$) = 3330 $cm^{-1}$ ; (C=O) = 1710 $cm^{-1}$.
R.M.N. ($CDCl_3$) : $\delta$ = 1,2 (3H,t, J = 6,75 Hz) ; 1,4-3,2 (12H,m, 2H échangeables avec $D_2O$) ; 3,55 (2H,s) ; 4,1 (2H,q, J = 6,75 Hz) ; 7,1 (4H,s).

b) *Trans*-4-[[2-[[(4-chlorophényl)sulfonyl]amino]cyclopentyl]méthyl]benzèneacétated'éthyle

Obtenu en opérant comme dans l'exemple 1c à partir de 0,5 g (1,9 mmole) du composé préparé dans l'exemple 8a, de 0,25 g (2,5 mmoles) de triéthylamine dans 20 ml de dichlorométhane et de 0,4 g (1,9 mmole) de chlorure de 4-chlorobenzènesulfonyle dans 15 ml d'éther. Agitation 20 heures à 20 ° C. On obtient 0,6 g (Rdt = 72,3 %) d'une huile épaisse.
I.R. (film) : $\nu$ (NH) = 3260 $cm^{-1}$ ; (C=O) = 1720 $cm^{-1}$ ; ($SO_2$) = 1325 $cm^{-1}$ ; ($SO_2$) = 1150 $cm^{-1}$.
R.M.N. ($CDCl_3$) : $\delta$ = 1,2 (3H,t, J = 6,75 Hz) ; 1,2-3,4 (10H,m) ; 3,55 (2H,s) ; 4,1 (2H,q, J = 6,75 Hz) ; 4,9

(1H,d, J = 7,5 Hz, échangeable dans $D_2O$) ; 6,8-7,25 (4H,m) ; 7,3-7,5 (2H,m) ; 7,6-7,85 (2H,m).

**Exemple 9**

**Acide 4-[2-[[(4-chlorophényl)sulfonyl]amino]cyclopentyloxy]benzèneacétique**

a) 4-[2-[[(4-Chlorophényl)sulfonyl]amino]cyclopentyloxy]benzèneacétate d'éthyle

Un mélange de 2,5 g (15 mmoles) de 4-hydroxybenzèneacétate de méthyle, de 4 g (30 mmoles) de $K_2CO_3$ et de 30 ml de DMF est porté à 80°C. On y ajoute goutte à goutte une solution de 5 g (14,8 mmoles) de N-(2-bromocyclopentyl)-4-chlorobenzènesulfonamide préparé dans l'exemple 5a, dans 50 ml de DMF. On agite 5 h à 80°C. Après refroidissement, le solide présent est filtré et rincé par l'acétate d'éthyle. Le filtrat dilué à l'eau est extrait par l'acétate d'éthyle. La phase organique est lavée à l'eau, séchée sur $Na_2SO_4$ et concentrée sous pression réduite. Le résidu purifié par chromatographie sur colonne de silice (éluant : hexane-acétate d'éthyle 1/1) donne 6,2 g (Rdt = 99,5 %) d'un liquide jaune.
I.R. (film) : $\nu$ (NH) = 3250 $cm^{-1}$ ; (C=O) = 1710 $cm^{-1}$ ; ($SO_2$) = 1320 $cm^{-1}$ ; ($SO_2$) = 1150 $cm^{-1}$.
R.M.N. ($CDCl_3$) : $\delta$ = 1,0-2,35 (6H,m) ; 2,75-3,4 (1H,m) ; 3,55 (2H,s) ; 3,7 (3H,s) ; 4,5-4,9 (1H,m) ; 5,2-5,7 (1H,m, échangeable par $CF_3COOD$) ; 6,5 - 7,6 (6H,m) ; 7,6-7,95 (2H,m).

b) Acide 4-[2-[[(4-chlorophényl)sulfonyl]amino]cyclopentyloxy]benzèneacétique

Obtenu en opérant comme dans l'exemple 1d, à partir de 6,2 g (14,6 mmoles) du composé préparé dans l'exemple 9a dans 60 ml d'éthanol et de 1,6 g (28,6 mmoles) de KOH dans 15 ml d'eau. Après recristallisation dans le toluène on obtient 1,6 g (Rdt = 27,1 %) d'un solide blanc cassé. F = 136-9°C.

| Analyse centésimale : $C_{19}H_{20}ClNO_5S$ (M = 409,884) | | | | | |
|---|---|---|---|---|---|
| | C % | H % | Cl % | N % | S % |
| Calculé<br>Trouvé | 55,68<br>55,80 | 4,92<br>5,01 | 8,65<br>8,95 | 3,42<br>3,37 | 7,82<br>7,94 |

I.R. (KBr) : $\nu$ (NH) = 3280 $cm^{-1}$ ; (C=O) = 1690 $cm^{-1}$ ; ($SO_2$) = 1315 $cm^{-1}$ ; ($SO_2$) = 1140 $cm^{-1}$.
R.M.N. (acétone $d_6$) : $\delta$ = 1,1-2,35 (6H,m) ; 3,2-3,8 (1H,m) ; 3,5 (2H,s) ; 4,3-4,65 (1H,m) ; 6,0-6,9 (3H,m, dont 1H échangeable dans $CF_3COOD$) ; 7,0-7,25 (2H,m) ; 7,3-7,6 (2H,m) ; 7,7-7,9 (2H,m) ; 10,0-10,9 (1H,s large, échangeable dans $CF_3COOD$).
C.L.H.P. (ODS-2 : $t_R$ = 24,8 (1 seul pic).

**Exemple 10**

**Acide*trans*-4-[2-[[(4-chlorophényl)sulfonyl]amino]cyclohexyl]benzèneacétique**

a) *Trans*-N-[2-(4-acétylphényl)cyclohexyl]-4-chlorobenzènesulfonamide

A un mélange maintenu à 0°C de 4,0 g (12 mmoles) de trans-4-chloro-N-(2-phénylcyclohexyl)-benzènesulfonamide (préparé selon Das P.C. et coll., Indian J. Chem. (1974), 12, 1139-40), de 80 ml de dichlorométhane anhydre et de 2,4 g (31 mmoles) de chlorure d'acétyle, on ajoute par fractions 5,3 g (40 mmoles) de chlorure d'aluminium. Après 4 heures à 0°C, le milieu réactionnel est jeté sur un mélange glace-HCl avant d'être extrait par le dichlorométhane. La phase organique est lavée par l'eau jusqu'à neutralité, séchée sur $Na_2SO_4$ et concentrée. Le résidu est purifié par chromatographie sur colonne de silice (éluant : hexane-acétate d'éthyle 4/1) pour donner 2 g d'huile incolore (Rdt : 42,5 %).
I.R. (film) : $\nu$ (NH) = 3260 $cm^{-1}$ ; (C=O) = 1665 $cm^{-1}$ ; ($SO_2$) = 1315 $cm^{-1}$ ; ($SO_2$) = 1150 $cm^{-1}$.
R.M.N. ($CDCl_3$) : $\delta$ = 0,7-2,45 (8H,m) ; 2,6 (3H,s) ; 2,75-3,75 (2H,m) ; 4,8 (1H,d, J = 7,5 Hz, échangeable dans $CF_3COOD$) ; 6,8-7,1 (2H,m) ; 7,1-7,4 (4H,m) ; 7,5-7,8 (2H,m).

b) *Trans*-4-[2-[[(4-chlorophényl)sulfonyl]amino]cyclohexyl]benzèneacétate de méthyle

A un mélange de 4,7 g (12 mmoles) de *trans*-N-[2-(4-acétylphényl)cyclohexyl]-4-chlorobenzènesulfonamide et de 40 ml de méthanol, on ajoute goutte à goutte à température ambiante 6,1 ml (100 mmoles) d'acide perchlorique à 70 % puis 6,4 g (14,4 mmoles) de nitrate de thallium (III) par portions. Le milieu réactionnel obtenu est agité 48 heures à température ambiante. Après filtration et lavage au méthanol du précipité blanc formé, le filtrat est jeté dans l'eau et extrait par le dichlorométhane. La phase organique est rincée à l'eau, séchée sur $Na_2SO_4$ puis concentrée pour donner 4 g d'huile incolore (Rdt = 79,0 %). Cette huile critallise dans l'éther après trituration, pour donner des cristaux blancs. F = 107-110 °C.
I.R. (KBr) : $\nu$ (NH) = 3260 $cm^{-1}$ ; (C=O) = 1705 $cm^{-1}$ ; ($SO_2$) = 1315 $cm^{-1}$ ; ($SO_2$) = 1150 $cm^{-1}$.
R.M.N. ($CDCl_3$) : $\delta$ = 1,0-3,4 (10H,m) ; 3,55 (2H,s) ; 3,7 (3H,s) ; 4,15-4,5 (1H,m, échangeable avec $CF_3COOD$ ; 6,6-7,15 (4H,m) ; 7,2 (4H,s).

c) Acide *trans*-4-[2-[[(4-chlorophényl)sulfonyl]amino]cyclohexyl]benzèneacétique

Un mélange de 1,8 g (4,3 mmoles) de *trans*-4-[2-[[(4-chlorophényl)sulfonyl]amino]cyclohexyl]-benzèneacétate de méthyle, de 18 ml d'éthanol, de 18 ml d'eau et de 0,5 g (8,5 mmoles) de KOH en pastilles est chauffé 4 heures à 40 °C sous agitation. L'éthanol est ensuite éliminé sous pression réduite. Le résidu dilué par $H_2O$ est lavé à l'éther et acidifié à froid par 5 ml d'acide chlorhydrique concentré. Le précipité formé est essoré, rincé par $H_2O$ et séché à l'étuve une nuit à 50 °C. Le produit est purifié par 2 recristallisations dans un mélange hexane-acétate d'éthyle pour donner 0,8 g (Rdt = 47,0 %) d'un solide blanc. F = 147-9 °C.

| Analyse centésimale : $C_{20}H_{22}ClNO_4S$ (M = 407,91) | | | | | |
|---|---|---|---|---|---|
| | C % | H % | Cl % | N % | S % |
| Calculé | 58,89 | 5,44 | 8,69 | 3,43 | 7,86 |
| Trouvé | 59,12 | 5,35 | 8,86 | 3,36 | 8,20 |

I.R. (KBr) : $\nu$ (NH) = 3170 $cm^{-1}$ ; (C=O) = 1700 $cm^{-1}$ ; ($SO_2$) = 1300 $cm^{-1}$ ; ($SO_2$) = 1140 $cm^{-1}$.
R.M.N. (DMSO $d_6$) : $\delta$ = 0,8-2,3 (9H,m) ; 2,75-3,4 (1H,m) ; 3,45 (2H,s) ; 6,9 (4H,s) ; 7,3 (4H,s) ; 7,6 (1H,d, J = 8,25 Hz, échangeable avec $CF_3COOD$) ; 12,15 (1H,s, échangeable avec $CF_3COOD$).

## Exemple 11

### Acide *trans*-4-[[2-[[(4-chlorophényl)sulfonyl]amino]cyclohexyl]méthyl]benzèneacétique

a) 4-[(2-Hydroxyiminocyclohexyl)méthyl]benzèneacétate d'éthyle

Obtenu en opérant comme dans l'exemple la à partir de 16,4 g (62,7 mmoles) d'acide 4-[(2-hydroxyiminocyclohexyl)méthyl]benzèneacétate (préparé selon Terada A. et coll., J. Med. Chem. (1984) 27, 212-6) dans 320 ml d'éthanol (Rdt = 90,6 %).
I.R. (film) : $\nu$ (N-OH) = 3220 $cm^{-1}$ ; (C=O) = 1715 $cm^{-1}$.
R.M.N. ($CDCl_3$) : $\delta$ = 1,25 (3H,t, J = 7,1 Hz) ; 1,1-3,4 (12H,m, dont 1H échangeable avec $CF_3COOD$) ; 3,6 (2H,s) ; 4,15 (2H,q, J = 7,1 Hz) ; 7,1 (4H,m).

b) 4-[(2-Aminocyclohexyl)méthyl]benzèneacétate d'éthyle (*cis* + *trans*)

Obtenu en opérant comme dans l'exemple 1b à partir de 15,5 g (56,7 mmoles) de 4-[(2-hydroxyiminocyclohexyl)méthyl]benzèneacétate d'éthyle dans l'éthanol saturé par $NH_3$. Après traitement on isole 6,1 g (Rdt = 39,1 %) d'une huile jaune-vert que l'on utilise sans autre purification.
I.R. (film) : $\nu$ ($NH_2$) = 3360 $cm^{-1}$ ; (C=O) = 1720 $cm^{-1}$.
R.M.N. ($CDCl_3$) : $\delta$ = 1,25 (3H,t, J = 7,1 Hz) ; 0,75-2,1 (11H,m, dont 2H échangeables par $D_2O$) ; 2,1-2,75 (2H,m) ; 2,8-3,4 (1H,m) ; 3,6 (2H,s) ; 4,15 (2H,q, J = 7,1 Hz) ; 7,1 (4H,s).

c) 4-[[2-(4-Chlorophényl)sulfonyl]amino]cyclohexyl]méthyl]benzèneacétate d'éthyle (*cis + trans*)

Obtenu en opérant comme dans l'exemple 1c à partir de 6,1 g (22 mmoles) de 4-[(2-aminocyclohexyl)-méthyl]benzèneacétate d'éthyle, de 2,7 g (26,5 mmoles) de triéthylamine dans 150 ml de dichlorométhane et de 4,6 g (22 mmoles) de chlorure de 4-chlorobenzènesulfonyle dans 50 ml d'éther. L'huile incolore obtenue (Rdt = quantitatif) est utilisée sans autre purification.
I.R. (film) : $\nu$ (NH) = 3280 $cm^{-1}$ ; (C=O) = 1710 $cm^{-1}$ ; ($SO_2$) = 1320 $cm^{-1}$ ; ($SO_2$) = 1150 $cm^{-1}$.
R.M.N. ($CDCl_3$) : $\delta$ = 1,25 (3H,t, J = 6,75 Hz) ; 0,6-2,75 (11H,m) ; 2,75-3,3 (1H,m) ; 3,55 (2H,s) ; 4,1 (2H,q, J = 6,75 Hz) ; 4,5-5,3 (1H,m, échangeable par $CF_3COOD$) ; 6,8-7,25 (4H,m) ; 7,4 (2H,m) ; 7,8 (2H,m).

d) Acide *trans*-4-[[2-[[(4-chlorophényl)sulfonyl]amino]cyclohexyl]méthyl]benzèneacétique

Obtenu en opérant comme dans l'exemple 1d à partir de 9,9 g (22 mmoles) de 4-[[2-[[(4-chlorophényl)-sulfonyl]amino]cyclohexyl]méthyl]benzèneacétate d'éthyle, de 2,5 g (44 mmoles) de KOH en pastilles dans 212 ml d'un mélange éthanol-eau (1:1). Après 5 recristallisations dans l'isopropanol, on isole 0,9 g (Rdt = 9,7 %) d'un solide blanc. F = 166-8 °C.

| Analyse centésimale : $C_{21}H_{24}ClNO_4S$ (M = 421,94) | | | | | |
|---|---|---|---|---|---|
| | C % | H % | Cl % | N % | S % |
| Calculé | 59,78 | 5,73 | 8,40 | 3,32 | 7,60 |
| Trouvé | 59,71 | 5,56 | 8,07 | 3,31 | 7,31 |

I.R. (KBr) : $\nu$ (NH) = 3270 $cm^{-1}$ ; (C=O) = 1690 $cm^{-1}$ ; ($SO_2$) = 1310 $cm^{-1}$ ; ($SO_2$) = 1140 $cm^{-1}$.
R.M.N. (DMSO $d_6$) : $\delta$ = 0,65-2,2 (10H,m) ; 2,6-3,4 (3H,m, dont 1H échangeable par $CF_3COOD$) ; 3,5 (2H,s) ; 6,8-7,3 (4H,m) ; 7,5-8,0 (4H,m), 12,0 (1H, s large, échangeable avec $CF_3COOD$).
C.L.H.P. (ODS-2) : $t_R$ = 15,6.

Avant recristallisation $t_R$ = 12,7 ; 15,6 ; ces deux pics d'égale intensité correspondent aux isomères *cis* et *trans*.

**Exemple 12**

**Acide 4-[1-[[[(4-chlorophényl)sulfonyl]amino]méthyl]cyclopropyl]benzèneacétique**

a) 1-(4-Acétylphényl)cyclopropanecarbonitrile

Une solution de 5 g (31,4 mmoles) de 4-acétylbenzèneacétonitrile (préparé selon Rorig K., J. Am. Chem. Soc. (1953) 75, 5381-3) dans 10 ml de DMSO, est coulée goutte à goutte à température ambiante dans une suspension de 3,4 g (environ 78,5 mmoles) d'hydrure de sodium (à 55-60 % dans l'huile minérale) dans 60 ml de DMSO sous courant d'azote. Après agitation 45 minutes à température ambiante, on ajoute goutte à goutte 8,85 g (47,1 mmoles) de 1,2-dibromoéthane dissous dans 10 ml de DMSO, en maintenant la température inférieure à 50 °C à l'aide d'un bain d'eau glacée. Après agitation 16 heures à température ambiante le milieu réactionnel est versé sur 300 ml de glace-eau. Le précipité formé est essoré, lavé par l'eau et séché sous vide, avant d'être purifié par recristallisation dans un mélange d'acétate d'éthyle et d'hexane pour donner 3,7 g (Rdt = 64,0 %) d'un solide violet gris. F = 74 °C.
I.R. (KBr) : $\nu$ (C≡N) = 2210 $cm^{-1}$ ; (C=O) = 1660 $cm^{-1}$.
R.M.N. ($CDCl_3$) : $\delta$ = 1,3-1,65 (2H,m) ; 1,75-2,0 (2H,m) ; 2,6 (3H,s) ; 7,15-7,5 (2H,m) ; 7,75-8,1 (2H,m).

b) 4-(1-Cyanocyclopropyl)benzèneacétate de méthyle

A un mélange de 3,7 g (20 mmoles) du produit préparé dans l'exemple 12 a, de 6,5 ml (160 mmoles) de méthanol et de 35 ml de dichlorométhane, sous courant d'azote, on ajoute goutte à goutte à température ambiante 9,8 ml (80 mmoles) d'éthérate de trifluorure de bore, puis 9,75 g (22 mmoles) de tétraacétate de plomb en suspension dans 55 ml de benzène. Après avoir agité le milieu réactionnel 17 heures à température ambiante, on le verse sur 150 ml de glace-eau. L'extraction est réalisée par le dichlorométhane. La phase organique est ensuite lavée par une solution saturée de $NaHCO_3$, puis par l'eau jusqu'à neutralité, avant d'être séchée sur $Na_2SO_4$. Après concentration sous vide, on obtient 3,85 g (Rdt =

89,5 %) d'une huile fluide orange utilisée sans autre purification.
I.R. (film) : ν (C≡N) = 2010 $cm^{-1}$ ; (C=O) = 1725 $cm^{-1}$.
R.M.N. ($CDCl_3$) : δ = 1,2-1,55 (2H,m) ; 1,55-1,9 (2H,m) ; 3,6 (2H,s) ; 3,65 (3H,s) ; 7,2 (4H,s).

c) 4-[1-(Aminométhyl)cyclopropyl]benzèneacétate de méthyle

Un mélange de 2,75 g (12,8 mmoles) du composé préparé dans l'exemple 12b, de 8,4 ml d'ammoniac liquide, de 80 ml de méthanol et de 1 g de nickel de Raney lavé par le méthanol est soumis à une pression de 90 atmosphères d'hydrogène, sous agitation et à température ambiante. Après 1,5 heure, l'hydrogénation s'arrête. Après filtration et rinçage du catalyseur, on concentre le filtrat sous pression réduite pour obtenir 2,8 g (Rdt = quantitatif) d'une huile jaune-vert utilisée sans autre purification.
I.R. (film) : ν ($NH_2$) = 3300 $cm^{-1}$ ; (C=O) = 1740 $cm^{-1}$.
R.M.N. ($CDCl_3$) : δ = 0,5-1,4 (4H,m) ; 1,5 (2H,s, échangeables dans $CF_3COOD$) ; 1,9 (2H,s) ; 3,5 (2H,s) ; 3,6 (3H,s) ; 6,9-7,55 (4H,m).

d) 4-[1-[[[(4-Chlorophényl)sulfonyl]amino]méthyl]cyclopropyl]benzèneacétate de méthyle

Obtenu en opérant comme dans l'exemple 1c à partir de 2,9 g (13,2 mmoles) du composé préparé dans l'exemple 12c, de 2,2 ml (15,8 mmoles) de triéthylamine dans 70 ml de dichlorométhane et de 2,8 g (13,2 mmoles) de chlorure de 4-chlorobenzènesulfonyle dans 7,5 ml d'éther. Le milieu réactionnel est agité 2,5 jours à température ambiante. Le produit est purifié par flash chromatographie sur colonne de silice avec un mélange hexane-acétate d'éthyle 4/1 puis 3/1, puis par recristallisation dans un mélange hexane-acétate d'éthyle, pour donner 1,6 g (Rdt = 30,8 %) d'un solide beige. F = 88-89,5 °C.
I.R. (KBr) : ν (NH) = 3280 $cm^{-1}$ ; (C=O) = 1720 $cm^{-1}$ ; ($SO_2$) = 1330 $cm^{-1}$ ; ($SO_2$) = 1150 $cm^{-1}$.
R.M.N. ($CDCl_3$) : δ = 0,8 (4H,s) ; 3,1 (2H,d, J = 6 Hz, se transforme en singulet avec $CF_3COOD$) ; 3,55 (2H,s) ; 3,7 (3H,s) ; 4,7 (1H,t, J = 6 Hz, échangeable avec $CF_3COOD$) ; 7,05 (4H,s) ; 7,2-7,75 (4H,m).

e) Acide 4-[1-[[[(4-chlorophényl)sulfonyl]amino]méthyl]cyclopropyl]benzèneacétique

Un mélange de 1,6 g (4 mmoles) du composé préparé dans l'exemple 12d, de 20 ml de méthanol, de 0,5 g (8,9 moles) de KOH en pastilles et de 6,7 ml d'eau est agité pendant 2 heures à 40 °C. Après concentration à sec sous pression réduite, le résidu est repris par 40 ml d'eau, lavé par l'acétate d'éthyle et acidifié par HCl 5N pour donner un précipité beige qui est repris à l'acétate d'éthyle. Cette phase organique est lavée par $H_2O$ puis extraite par une solution saturée de $NaHCO_3$. La phase aqueuse obtenue est acidifiée par HCl 5N pour donner un précipité blanc cassé qui est filtré, lavé par l'eau et séché une nuit à 50 °C. Après deux recristallisations dans un mélange hexane-acétate d'éthyle on recueille 0,55 g (Rdt = 36,7 %) d'un solide blanc. F = 132-3 °C.

| Analyse centésimale : $C_{18}H_{18}ClNO_4S$ (M = 379,858) | | | | | |
|---|---|---|---|---|---|
| | C % | H % | Cl % | N % | S % |
| Calculé | 56,92 | 4,78 | 9,33 | 3,69 | 8,44 |
| Trouvé | 56,64 | 4,73 | 9,45 | 3,55 | 8,37 |

I.R. (KBr) : ν (NH) = 3250 $cm^{-1}$ ; (C=O) = 1680 $cm^{-1}$ ; ($SO_2$) = 1305 $cm^{-1}$ ; ($SO_2$) = 1150 $cm^{-1}$.
R.M.N. (acétone $d_6$) : δ = 0,6-1,0 (4H,m) ; 3,15 (2H,d, J = 6 Hz, se transforme en singulet avec $CF_3COOD$) ; 3,6 (2H,s) ; 6,6 (1H,t, J = 6 Hz, échangeable avec $CF_3COOD$) ; 7,2 (4H,s) ; 7,4-7,6 (2H,m) ; 7,6-7,9 (2H,m) ; 10,2-10,9 (1H,s large, échangeable avec $CF_3COOD$).

**Exemple 13**

**Acide 4-[1-[[[(4-chlorophényl)sulfonyl]amino]méthy]cyclobutyl]benzèneacétique**

a) 1-Phénylcyclobutaneméthanamine

Une suspension commerciale de $LiAlH_4$ à 13 % dans un mélange toluène-THF, sous azote (0,595 mole ; 175 ml), diluée par 270 ml de THF est maintenue à 0 °C. Un mélange de 90 g (0,572 mole) de 1-

phénylcyclobutanecarbonitrile et de 800 ml de THF est ajouté goutte à goutte en 20 minutes. Après addition on laisse remonter la température lentement avant de porter à reflux durant 1 heure. Après refroidissement vers 5 °C, on coule goutte à goutte avec précaution 150 ml d'eau, 150 ml de NaOH 10 % puis encore 560 ml d'eau. On extrait par l'acétate d'éthyle après avoir saturé la phase aqueuse par NaCl. La phase organique est extraite par une solution HCl N. Cette phase aqueuse est basifiée par NaOH 30 % et extraite par le dichlorométhane, qui après lavage à l'eau, séchage sur $Na_2SO_4$ et concentration fournit un liquide rougeâtre purifié par distillation. On obtient 68,65 g (Rdt = 81,3 %) d'un liquide incolore. $Eb_6$ = 84-92 °C.

I.R. (film) : $\nu$ ($NH_2$) = 3350 $cm^{-1}$.
R.M.N. ($CDCl_3$) : $\delta$ = 0,9 (2H,s, échangeables avec $CF_3COOD$) ; 1,65-2,5 (6H,m) ; 2,9 (2H,s) ; 6,8-7,7 (5H,m).

b) 4-Chloro-N-[[1-(phényl)cyclobutyl]méthyl]-benzènesulfonamide

Obtenu en procédant comme dans l'exemple 1c à partir de 75 g (465 mmoles) de 1-phénylcyclobutaneméthanamine préparée dans l'exemple 13a, de 77,8 ml (558 mmoles) de triéthylamine dans 2600 ml de dichlorométhane et de 98,2 g (465 mmoles) de chlorure de 4-chlorobenzènesulfonyle dans 260 ml d'éther. Après agitation durant 62 heures et traitement usuel on obtient 154,4 g (Rdt = 98,8 %) d'un solide crème utilisé sans autre purification. F = 107-8 °C.

I.R. (KBr) : $\nu$ (NH) = 3260 $cm^{-1}$ ; ($SO_2$) = 1325 $cm^{-1}$ ; ($SO_2$) = 1165 $cm^{-1}$.
R.M.N. ($CDCl_3$) : $\delta$ = 1,75-2,5 (6H,m) ; 3,2 (2H,d, J = 6,75 Hz, se transforme en singulet avec $CF_3COOD$) ; 4,15 (1H,t, J = 6,75 Hz, échangeable avec $CF_3COOD$) ; 6,65-7,7 (9H,m).

c) N-[[1-(4-Acetylphényl)cyclobutyl]méthyl]-4-chlorobenzènesulfonamide

A un mélange sous azote, de 90 g (268 mmoles) du composé préparé dans l'exemple 13b, de 52,6 ml (697 mmoles) de chlorure d'acétyle dans 1300 ml de dichlorométhane, on ajoute par portions en 5 minutes, 188 g (1407 mmoles) de chlorure d'aluminium en maintenant la température à -5 °C. Ensuite on agite durant 4 heures à 5 °C puis 2 heures à 20 °C. Le milieu réactionnel est alors jeté sur 1,5 kg de glace contenant 250 ml d'acide chlorhydrique concentré. On extrait par le dichlorométhane (2 x 500 ml) que l'on lave ensuite jusqu'à neutralité par $H_2O$ et que l'on sèche sur $Na_2SO_4$. Après concentration sous pression réduite, le résidu solide obtenu est recristallisé deux fois dans l'acétate d'éthyle pour donner 32,3 g (Rdt = 31,9 %) d'aiguilles beige. F = 142-3 °C.

I.R. (KBr) : $\nu$ (NH) = 3140 $cm^{-1}$ ; (C=O) = 1655 $cm^{-1}$ ; ($SO_2$) = 1330 $cm^{-1}$ ; ($SO_2$) = 1155 $cm^{-1}$.
R.M.N. ($CDCl_3$) : $\delta$ - 1,5-2,65 (6H,m) ; 2,5 (3H,s) ; 3,2 (2H,d, J = 6,75 Hz, se transforme en singulet avec $CF_3COOD$) ; 4,55 (1H,t, J = 6,75 Hz, échangeable avec $CF_3COOD$) ; 6,8-7,85 (8H,m).

d) 4-[1-[[[(4-Chlorophényl)sulfonyl]amino]méthyl]cyclobutyl]benzèneacétate de méthyle

Obtenu en opérant comme dans l'exemple 5e à partir de 5 g (13,2 mmoles) du composé préparé dans l'exemple 13c, de 5 ml de méthanol dans 25 ml de dichlorométhane, de 6,5 ml d'éthérate de trifluorure de bore et de 6,2 g de tétraacétate de plomb dans 35 ml de benzène. Le produit obtenu, 4,05 g (Rdt = 75,0 %), après recristallisation dans un mélange hexane-acétate d'éthyle est un solide blanc cassé. F = 88-90 °C.

I.R. (KBr) : $\nu$ (NH) = 3250 $cm^{-1}$ ; (C=O) = 1720 $cm^{-1}$ ; ($SO_2$) = 1325 $cm^{-1}$ ; ($SO_2$) = 1160 $cm^{-1}$.
R.M.N. ($CDCl_3$) : $\delta$ = 1,75-2,6 (6H,m) ; 3,2 (2H,d, J = 6,75 Hz, se transforme en singulet avec $CF_3COOD$) ; 3,6 (2H,s) ; 3,7 (3H,s) ; 4,2 (1H,t, J = 6,75 Hz, échangeable avec $CF_3COOD$) ; 6,8-7,05 (2H,m) ; 7,05-7,25 (2H,m) ; 7,25-7,5 (2H,m) ; 7,5-7,8 (2H,m).

e) Acide 4-[1-[[[(4-Chlorophényl)sulfonyl]amino]méthyl]cyclobutyl]benzèneacétique

Obtenu en opérant comme dans l'exemple 12e à partir de 4 g (9,8 mmoles) de l'ester préparé dans l'exemple 13d, de 50 ml d'éthanol, de 1,1 g (19,6 mmoles) de KOH et de 16,5 ml d'eau. Après extraction, le produit est purifié par flash chromatographie sur colonne de silice (éluant $CH_2Cl_2$-méthanol 95/5), puis par recristallisation dans un mélange acétate d'éthyle-cyclohexane. On obtient 1,2 g (Rdt = 31,1 %) d'un solide blanc. F = 138-9 °C.

| Analyse centésimale : $C_{19}H_{20}ClNO_4S$ (M = 393,88) | | | | | |
|---|---|---|---|---|---|
| | C % | H % | Cl % | N % | S % |
| Calculé | 57,94 | 5,12 | 9,00 | 3,56 | 8,14 |
| Trouvé | 57,94 | 5,31 | 9,05 | 3,62 | 8,17 |

I.R. (KBr) : ν (NH) = 3250 $cm^{-1}$ ; (C=O) = 1680 $cm^{-1}$ ; ($SO_2$) = 1330 $cm^{-1}$ ; ($SO_2$) = 1160 $cm^{-1}$.
R.M.N. ($CDCl_3$) : δ = 1,6-2,7 (6H,m) ; 3,2 (2H,d, J = 6,75 Hz, se transforme en singulet avec $CF_3COOD$ ; 3,6 (2H,s) ; 4,3 (1H,t, J = 6,75 Hz, échangeable avec $CF_3COOD$ ; 6,8-7,1 (2H,m) ; 7,1-7,3 (2H,m) ; 7,3-7,5 (2H,m) ; 7,5-7,75 (2H,m) ; 9,3-10,15 (1H,s large, échangeable avec $CF_3COOD$).

**Exemple 14**

**Acide 4-[1-[[[(4-chlorophényl)sulfonyl]amino]méthyl]cyclopentyl]benzèneacétique**

a) 4-Chloro-N-[[1-(phényl)cyclopentyl]méthyl]-benzènesulfonamide

Obtenu en procédant comme dans l'exemple 1c à partir de 40,2 g (229 mmoles) de 1-phénylcyclopentaneméthanamine, de 38,3 ml (275 mmoles) de triéthylamine dans 1300 ml de dichlorométhane et de 49,3 g (233 mmoles) de chlorure de 4-chlorobenzènesulfonyle dans 130 ml d'éther, sous atmosphère inerte. Après purification par chromatographie sur colonne de silice avec un mélange hexane-acétate d'éthyle 9/1 on obtient 74 g (Rdt = 92,3 %) d'un solide crème, sous forme d'aiguilles. F = 88-91 °C.
I.R. (KBr) : ν (NH) = 3250 $cm^{-1}$ ; ($SO_2$) = 1340 $cm^{-1}$ ; ($SO_2$) = 1170 $cm^{-1}$.
R.M.N. ($CDCl_3$) : δ = 1,5-2,4 (8H,m) ; 3,0 (2H,d, J = 6,4 Hz, se transforme en singulet avec $CF_3COOD$) ; 4,2 (1H,t, J = 6,4 Hz, échangeable avec $CF_3COOD$ ; 6,7-8,0 (9H,m).

b) N-[[1-(4-Acétylphényl)cyclopentyl]méthyl]-4-chlorobenzènesulfonamide

Obtenu en opérant comme dans l'exemple 13 c à partir de 30 g (85,7 mmoles) du composé obtenu dans l'exemple 14a, de 16,8 ml (222,8 mmoles) de chlorure d'acétyle dans 500 ml de dichlorométhane, et de 60 g (450 mmoles) de chlorure d'aluminium, sous atmosphère inerte. Après purification par flash chromatographie sur colonne de silice par des mélanges hexane-acétate d'éthyle 3/1 à 1/4, on obtient 12,9 g (Rdt = 38,4 %) d'un solide blanc. F = 135-6,5 °C.
I.R. (KBr) : ν (NH) = 3190 $cm^{-1}$ (C=O) = 1655 $cm^{-1}$ ; ($SO_2$) = 1330 $cm^{-1}$ ; ($SO_2$) = 1150 $cm^{-1}$.
R.M.N. ($CDCl_3$) : δ = 1,5-2,25 (8H,m) ; 2,55 (3H,s) ; 3,0 (2H,d, J = 6,75 Hz, se transforme en singulet avec $CF_3COOD$) ; 4,4 (1H,t, J = 6,75 Hz, échangeable avec $CF_3COOD$ ; 6,9-8,35 (8H,m).

c) 4-Chloro-N-[[1-[4-[2-(morpholin-4-yl)-2-thioxoéthyl]phényl]cyclopentyl]méthyl]-benzènesulfonamide

Un mélange de 7 g (17,9 mmoles) du composé préparé dans l'exemple 14b, de 1 g (31,2 mmoles) de soufre et de 15 ml de morpholine est porté à reflux 16 heures. Le milieu réactionnel est alors versé sur 100 g de glace. On extrait par l'acétate d'éthyle (3 x 50 ml) après saturation de la phase aqueuse par NaCl. La phase organise est lavée par une solution HCl N (2 x 50 ml) puis à l'eau saturée par NaCl jusqu'à neutralité avant d'être séchée sur $Na_2SO_4$ et concentrée sous pression réduite. Après purification du résidu obtenu par flash chromatographie sur colonne de silice dans un mélange hexane-acétate d'éthyle 2/1, puis recristallisation dans un mélange hexane-acétate d'éthyle, on isole 2,95 g (Rdt = 33,5 %) d'un solide beige. F = 124-5 °C.
I.R. (KBr) : ν (NH) = 3260 $cm^{-1}$ ; ($SO_2$) = 1335 $cm^{-1}$ ; ($SO_2$)) = 1160 $cm^{-1}$.
R.M.N. ($CDCl_3$) : δ = 1,3-2,1 (8H,m) ; 2,95 (2H,d, J = 6,75 Hz, se transforme en singulet avec $CF_3COOD$) ; 3,3-4,5 (9H,m, dont 1H échangeable avec $CF_3COOD$) ; 4,3 (2H,s) ; 6,85-7,85 (8H,m).

d) Acide 4-[1-[[[(4-chlorophényl)sulfonyl]amino]méthyl]cyclopentyl]benzèneacétique

Un mélange de 2,9 g (5,9 mmoles) du composé préparé dans l'exemple 14c, de 1,5 g (37,5 mmoles) de soude en pastilles et de 37,5 ml d'eau est porté à reflux durant 15h. Après refroidissement le milieu réactionnel dilué par environ 65 ml de glace-eau, acidifié par HCl 5N à pH 1 et saturé par NaCl, est extrait par l'acétate d'éthyle (3 x 100 ml). La phase organique lavée par l'eau saturée par NaCl jusqu'à neutralité

est séchée sur $Na_2SO_4$ et concentrée sous pression réduite. Le résidu obtenu est purifié par flash chromatographie sur colonne de silice par un mélange de dichlorométhaneméthanol 97/3, puis par recristallisation dans le cyclohexane en présence de charbon végétal, pour donner 0,5 g (Rdt = 20,8 %) d'un solide blanc. F = 145-6,5 °C.

| Analyse centésimale : $C_{20}H_{22}ClNO_4S$ (M = 407,912) | | | | | |
|---|---|---|---|---|---|
| | C % | H % | Cl % | N % | S % |
| Calculé | 58,89 | 5,44 | 8,69 | 3,43 | 7,86 |
| Trouvé | 58,45 | 5,58 | 8,84 | 3,41 | 8,07 |

I.R. (KBr : ν (NH) = 3280 $cm^{-1}$ ; (C=O) = 1695 $cm^{-1}$ ; ($SO_2$) = 1320 $cm^{-1}$ ; ($SO_2$) = 1150 $cm^{-1}$.
R.M.N. (acétone $d_6$) δ = 1,3-2,25 (8H,m) ; 3,05 (2H,d, J = 6,75 Hz, se transforme en singulet avec $CF_3COOD$) ; 3,5 (2H,s) ; 6,1 (1H,t, J = 6,75 Hz, échangeable avec $CF_3COOD$) ; 7,15 (4H,s) ; 7,3-7,9 (4H,m).

**Exemple 15**

**Acide 4-[[1-[[[(phényl)sulfonyl]amino]méthyl]cyclopentyl]méthyl]benzèneacétique**

a) 1-[(Phényl)méthyl]cyclopentaneméthanamine

A une suspension de 68,8 g (1,81 mole) de $LiAlH_4$ dans 1350 ml d'éther anhydre, sous atmosphère d'azote, on coule goutte à goutte en 1,5 heure, en maintenant la température du milieu réactionnel entre 10 et 20 °C, 279,9 g (1,51 mole) de 1-[(phényl)méthyl]cyclopentanecarbonitrile (préparé selon Campaigne E. et Forsh R.A., J. Org. Chem. (1978) 43, 1044-50). Après agitation à température ambiante pendant 18 heures, on détruit l'excès d'hydrure par addition de 344 ml d'eau. La phase organique est séchée sur $Na_2SO_4$ et filtrée. Le filtrat concentré sous pression réduite est distillé pour donner 273,9 g (Rdt = 95,8 %) d'un liquide incolore. $Eb_2$ = 122 °C.

| Analyse centésimale : $C_{13}H_{19}N$ (M = 189,3) | | | |
|---|---|---|---|
| | C % | H % | N % |
| Calculé | 82,48 | 10,12 | 7,40 |
| Trouvé | 82,23 | 9,98 | 7,39 |

I.R. (film) : ν ($NH_2$) = 3370 $cm^{-1}$.
R.M.N. ($CCl_4$) : δ = 0,75 (2H,s large, échangeables dans $D_2O$) ; 1,0-2,0 (8H,m) ; 2,4 (2H,s) ; 2,6 (2H,s) ; 7,05 (5H,s).

b) N-[[1-[(Phényl)méthyl]cyclopentyl]méthyl]-benzènesulfonamide

Obtenu en opérant comme dans l'exemple 1c, à partir de 17,9 g (94,5 mmoles) de l'amine préparée dans l'exemple 15a, de 11,5 g (113 mmoles) de triéthylamine dans 360 ml de dichlorométhane et de 16,7 g (94,5 mmoles) de chlorure de benzènesulfonyle dans 50 ml d'éther. Après agitation 16 heures à température ambiante et extraction, on obtient un résidu que l'on triture dans l'hexane pour donner 28,7 g (Rdt = 92,2 %) d'un solide blanc utilisé sans autre purification. F = 105-6 °C.
I.R. (KBr) : ν (NH) = 3260 $cm^{-1}$ ; ($SO_2$) = 1315 $cm^{-1}$ ; ($SO_2$) = 1150 $cm^{-1}$.
R.M.N. ($CDCl_3$) : δ = 1,0-2,0 (8H,m) ; 2,6 (2H,s) ; 2,7 (2H,d, J = 6,75 Hz, se transforme en singulet avec $CF_3COOD$) ; 5,1 (1H,t, J = 6,75 Hz, échangeable avec $CF_3COOD$) ; 6,7-7,3 (5H,m) ; 7,3-7,7 (3H,m) ; 7,7-8,0 (2H,m).

c) N-[[1-[(4-Acétylphényl)méthyl]cyclopentyl]méthyl]-benzènesulfonamide

Une solution de 28,7 g (87,1 mmoles) du composé préparé dans l'exemple 15b et de 500 ml de dichlorométhane est maintenue entre -20 °C et -10 °C. On y ajoute 15 g (191 mmoles) de chlorure

d'acétyle, puis par portions 46,4 g (348 mmoles) de chlorure d'aluminium. Après agitation 4 heures à la même température on jette sur environ 1,5 l de glace-eau-HCl. Après extraction par le dichlorométhane, lavage à l'eau jusqu'à neutralité, séchage sur $Na_2SO_4$, concentration sous pression réduite et trituration du résidu obtenu, dans l'hexane, on isole 28,6 g (Rdt = 88,3 %) d'un solide blanc utilisé sans autre purification. F = 88-9 °C.
I.R. (KBr) : $\nu$ (NH) = 3250 $cm^{-1}$ ; (C=O) = 1660 $cm^{-1}$ ; ($SO_2$) = 1310 $cm^{-1}$ ; ($SO_2$) = 1145 $cm^{-1}$.
R.M.N. ($CDCl_3$) ; $\delta$ = 1,0-2,0 (8H,m) ; 2,45-2,9 (7H,m) ; 4,55-5,2 (1H,m, échangeable avec $CF_3COOD$) ; 6,75-8,0 (9H,m).

d) 4-[[1-[[[(Phényl)sulfonyl]amino]méthyl]cyclopentyl]méthyl]benzèneacétate de méthyle

Obtenu en opérant comme dans l'exemple 5e à partir de 28,6 g (76,9 mmoles) du composé préparé dans l'exemple 15c, de 35 ml de méthanol, de 43,6 g (308 mmoles) d'éthérate de trifluorure de bore dans 175 ml de dichlorométhane et de 35,8 g (80,7 mmoles) de tétraacétate de plomb dans 205 ml de benzène. Après trois recristallisations dans un mélange hexane-acétate d'éthyle, on obtient 6,3 g (Rdt = 20,4 %) d'un solide blanc. F = 129-30 °C.
I.R. (KBr) : $\nu$ (NH) = 3240 $cm^{-1}$ ; (C=O) = 1700 $cm^{-1}$ ; ($SO_2$) = 1340 $cm^{-1}$ ; ($SO_2$) = 1150 $cm^{-1}$.
R.M.N. ($CDCl_3$) : $\delta$ = 0,8-2,1 (8H,m) ; 2,6 (2H,s) ; 2,7 (2H,d, J = 6,75 Hz, se transforme en singulet avec $CF_3COOD$) ; 3,55 (2H,s) ; 3,65 (3H,s) ; 4,9 (1H,t, J = 6,75 Hz, échangeable avec $CF_3COOD$) ; 6,7-8,0 (9H,m).

e) Acide 4-[[1-[[[(phényl)sulfonyl]amino]méthyl]cyclopentyl]méthyl]benzèneacétique

Obtenu en opérant comme dans l'exemple 5f à partir de 6,3 g (15,7 mmoles) de l'ester préparé dans l'exemple 15d, de 1,8 g (31,4 mmoles) de KOH en pastilles, de 63 ml d'éthanol et 63 ml d'eau. Après deux recristallisations dans un mélange hexane-acétate d'éthyle, on obtient 4,2 g (Rdt = 65,7 %) d'un solide blanc. F = 154-6 °C.

| Analyse centésimale : $C_{21}H_{25}NO_4S$ (M = 387,494) | | | | |
|---|---|---|---|---|
| | C % | H % | N % | S % |
| Calculé | 65,09 | 6,50 | 3,61 | 8,27 |
| Trouvé | 64,92 | 6,57 | 3,62 | 8,05 |

I.R. (film) : $\nu$ (NH) = 3260 $cm^{-1}$ ; (C=O) = 1690 $cm^{-1}$ ; ($SO_2$) = 1315 $cm^{-1}$ ; ($SO_2$) = 1155 $cm^{-1}$.
R.M.N. (DMSO $d_6$) : $\delta$ = 0,8-1,75 (8H,m) ; 2,4-2,85 (5H,m, dont 1H échangeable avec $CF_3COOD$) ; 3,45 (2H,s) ; 7,0 (4H,s) ; 7,4-8,0 (5H,m) ; 12,1 (1H,s, échangeable avec $CF_3COOD$).

**Example 16**

**Acide 4-[[1-[[[(4-fluorophényl)sulfonyl]amino]méthyl]cyclopentyl]méthyl]benzèneacétique**

a) 4-Fluoro-N-[[1-[(phényl)méthyl]cyclopentyl]méthyl]-benzènesulfonamide

Obtenu en opérant comme dans l'exemple 1c, à partir de 9,4 g (48,3 mmoles) de 1-[(phényl)méthyl]-cyclopentaneméthanamine préparée dans l'exemple 15a, de 8,1 ml (57,9 mmoles) de triéthylamine dans 200 ml de dichlorométhane et de 9,4 g (48,3 mmoles) de chlorure de 4-fluorobenzènesulfonyledans 60 ml de dichlorométhane. Après agitation 3 jours à température ambiante et extraction, on obtient un résidu que l'on triture dans l'hexane pour donner 15,2 g (Rdt = 90,5 %) d'un solide blanc utilisé sans autre purification. F = 115-7 °C.
I.R. (KBr) : $\nu$ (NH) = 3260 $cm^{-1}$ ; ($SO_2$) = 1320 $cm^{-1}$ ; ($SO_2$) = 1150 $cm^{-1}$.
R.M.N. ($CDCl_3$) : $\delta$ = 1,0-1,85 (8H,m) ; 2,6 (2H,s) ; 2,7 (2H,d, se transforme en singulet avec $CF_3COOD$) ; 5,6 (1H,t, échangeable avec $CF_3COOD$) ; 6,75-8,0 (9H,m).

b) N-[[1-[(4-Acétylphényl)méthyl]cyclopentyl]méthyl]-4-fluorobenzènesulfonamide

A un mélange de 15,2 g (43,7 mmoles) du composé préparé dans l'exemple 16a, de 4,4 g (56,8 mmoles) de chlorure d'acétyle et de 290 ml de dichlorométhane, maintenu à 0 °C on ajoute par portions 19,2 g (144 mmoles) de chlorure d'aluminium. Après avoir agité 3 heures à 0 °C, on jette sur un mélange glace-HCl concentré. On extrait par le dichlorométhane, qui est ensuite lavé par l'eau jusqu'à neutralité, séché sur $Na_2SO_4$, puis concentré sous pression réduite pour donner une huile qui, après trituration dans l'hexane, fournit 9,7 g (Rdt = 57,0 %) d'un solide blanc cassé utilisé sans autre purification. F = 79-82 °C.
I.R. (KBr) : $\nu$ (NH) = 3260 $cm^{-1}$ ; (C=O) : 1670 $cm^{-1}$ ; ($SO_2$) = 1315 $cm^{-1}$ ; ($SO_2$) = 1160 $cm^{-1}$.
R.M.N. ($CDCl_3$) : $\delta$ = 1,0-2,0 (8H,m) ; 2,55 (3H,s) ; 2,7 (2H,d, J = 6,75 Hz, se transforme en singulet avec $CF_3COOD$) ; 2,7 (2H,s) ; 5,1 (1H,t, J = 6,75 Hz, échangeable avec $CF_3COOD$) ; 6,85-7,4 (4H,m) ; 7,6-8,0 (4H,m).

c) 4-[[1-[[[(4-Fluorophényl)sulfonyl]amino]méthyl]cyclopentyl]méthyl]benzèneacétate de méthyle

Obtenu en opérant comme dans l'exemple 5e à partir de 9,7 g (24,9 mmoles) du composé préparé dans l'exemple 16b, de 11,3 ml de méthanol dans 56 ml de dichlorométhane, de 14,1 g (99,6 mmoles) d'éthérate de trifluorure de bore et de 13,2 g (29,8 mmoles) de tétraacétate de plomb dans 60 ml de benzène. Après recristallisation dans l'acétate d'éthyle, on obtient 6,1 g (Rdt = 58,4 %) d'un solide blanc. F = 138-40 °C.
I.R. (KBr) : $\nu$ (NH) : 3240 $cm^{-1}$ ; (C=O) : 1700 $cm^{-1}$ ; ($SO_2$) = 1340 $cm^{-1}$ ; ($SO_2$) = 1150 $cm^{-1}$.
R.M.N. ($CDCl_3$) : $\delta$ = 1,0-1,9 (8H,m) ; 2,6 (2H,s) ; 2,7 (2H,d, J = 6,75 Hz, se transforme en singulet avec $CF_3COOD$) ; 3,55 (2H,s) ; 3,7 (3H,s) ; 4,5 (1H,t, J = 6,75 Hz, échangeable avec $CF_3COOD$) ; 6,95-7,35 (6H,m) ; 7,65-8,0 (2H,m).

d) Acide 4-[[1-[[[(4-fluorophényl)sulfonyl]amino]méthyl]cyclopentyl]méthyl]benzèneacétique

Obtenu en opérant comme dans l'exemple 5f à partir de 6,1 g (14,5 mmoles) du composé préparé dans l'exemple 16c dans 61 ml d'éthanol et de 1,6 g (29 mmoles) de KOH en pastilles dans 61 ml d'eau. Après deux recristallisations dans un mélange hexane-acétate d'éthyle, on obtient 1,9 g (Rdt = 32,0 %) d'un solide blanc. F = 151-4 °C.

| Analyse centésimale : $C_{21}H_{24}FNO_4S$ (M = 405,484) | | | | | |
|---|---|---|---|---|---|
| | C % | H % | F % | N % | S % |
| Calculé | 62,20 | 5,97 | 4,69 | 3,45 | 7,91 |
| Trouvé | 61,94 | 5,96 | 4,55 | 3,44 | 7,71 |

I.R. (KBr) : $\nu$ (NH) = 3260 $cm^{-1}$ ; (C=O) = 1685 $cm^{-1}$ ; ($SO_2$) = 1310 $cm^{-1}$ ; ($SO_2$) = 1140 $cm^{-1}$.
R.M.N. (acétone $d_6$) : $\delta$ = 1,1-1,8 (8H,m) ; 2,65 (2H,s) ; 2,75 (2H,d, J = 6,75 Hz, se transforme en singulet avec $CF_3COOD$) ; 3,6 (2H,s) ; 6,3 (1H,t, J = 6,75 Hz, échangeable avec $CF_3COOD$) ; 7,15 (4H,s) ; 7,25-7,55 (2H,m) ; 7,7-8,15 (2H,m) ; 10,55 (1H,s large, échangeable avec $CF_3COOD$).

**Exemple 17**

**N-[[1-[(4-Acétylphényl)méthyl]cyclopentyl]méthyl]-4-chlorobenzènesulfonamide**

a) 4-Chloro-N-[[1-[(phényl)méthyl]cyclopentyl]méthyl]-benzènesulfonamide

A une solution composée de 268,2 g (1,416 mole) de l'amine préparée dans l'exemple 15a, de 237 ml (1,700 mole) de triéthylamine et de 2500 ml de dichlorométhane sec, maintenue à 0 °C, sont ajoutés par portions, 298,8 g (1,416 mole) de chlorure de 4-chlorobenzènesulfonyle. Le milieu réactionnel est agité 2,5 jours à température ambiante avant d'être jeté dans 2,5 l d'eau contenant 1,5 mole d'HCl. La phase organique est décantée, lavée à l'eau, séchée sur $Na_2SO_4$ et concentrée sous pression réduite. Après recristallisation dans l'acétate d'éthyle on obtient 467,2 g (Rdt = 90,7 %) d'une solide blanc. F = 121-3 °C.

| Analyse centésimale : $C_{19}H_{22}ClNO_2S$ (M = 363,90) | | | | | |
|---|---|---|---|---|---|
| | C % | H % | Cl % | N % | S % |
| Calculé | 62,71 | 6,09 | 9,74 | 3,85 | 8,81 |
| Trouvé | 62,54 | 6,08 | 9,72 | 3,85 | 8,94 |

I.R. (KBr : $\nu$ (NH) = 3250 $cm^{-1}$ ; ($SO_2$) = 1315 $cm^{-1}$ ; ($SO_2$) = 1170 $cm^{-1}$.
R.M.N. ($CDCl_3$) : $\delta$ = 1,0-1,9 (8H,m) ; 2,6 (2H,s) ; 2,7 (2H,d, J = 6,75 Hz, se transforme en singulet avec $CF_3COOD$) ; 5,0 (1H,t, J = 6,75 Hz, échangeable avec $CF_3COOD$) ; 6,8-7,3 (5H,m) ; 7,3-7,5 (2H,m) ; 7,6-7,9 (2H,m).

b) N-[[1-[(4-Acétylphényl)méthyl]cyclopentyl]méthyl]-4-chlorobenzènesulfonamide

Le composé obtenu dans l'exemple 17a (185,9 g ; 511 mmoles) dissous dans 3000 ml de 1,2-dichloroéthane anhydre est maintenu sous agitation à -20 °C. On coule goutte à goutte 47,2 ml (664 mmoles) de chlorure d'acétyle, puis par portions 340,7 g (2555 mmoles) de chlorure d'aluminium. On laisse remonter la température à -12 °C. Après 8 heures à cette température, on abandonne le mélange 16 heures à -25 °C. Le milieu réactionnel est ensuite jeté directement sur un mélange de 5000 ml d'eau et de 2000 ml HCl concentré. Après décantation de la phase organique et extraction par 4 x 500 ml de dichlorométhane, les phases organiques réunies sont lavées par l'eau (1000 ml), la soude 1N (2 x 1000 ml) puis à l'eau jusqu'à neutralité. Après séchage sur $Na_2SO_4$ et concentration on obtient une huile brune qui est triturée dans l'hexane jusqu'à cristallisation. Le solide rose obtenu, essoré et séché (153,9 g) est utilisé sans autre purification (Rdt = 74,2 %). F = 97-102 °C. Une fraction purifiée par chromatographie sur colonne de silice dans le dichlorométhane puis par recristallisation dans un mélange hexane-acétate d'éthyle fournit un solide blanc. F = 110-3 °C.

| Analyse centésimale : $C_{21}H_{24}ClNO_3S$ (M = 405,94) | | | | | |
|---|---|---|---|---|---|
| | C % | H % | Cl % | N % | S % |
| Calculé | 62,14 | 5,96 | 8,73 | 3,45 | 7,90 |
| Trouvé | 62,13 | 5,90 | 8,81 | 3,45 | 8,17 |

I.R. (KBr) : $\nu$ (NH) = 3200 $cm^{-1}$ ; (C=O) = 1660 $cm^{-1}$ ; ($SO_2$) = 1315 $cm^{-1}$ ; ($SO_2$) = 1150 $cm^{-1}$.
R.M.N. ($CDCl_3$) : $\delta$ = 1,0-2,0 (8H,m) ; 2,6 (3H,s) ; 2,7 (2H,s) ; 2,75 (2H,d, J = 6,75 Hz, se transforme en singulet avec $CF_3COOD$) ; 5,25 (1H,t, J= 6,75 Hz, échangeable avec $CF_3COOD$) ; 6,6-8,0 (8H,m).

**Exemple 18**

**4-[[1-[[[(4-Chlorophényl)sulfonyl]amino]méthyl]cyclopentyl]méthyl]benzèneacétate de méthyle**

Obtenu en opérant comme dans l'exemple 5e à partir de 30,8 g (75,8 mmoles) du dérivé préparé dans l'exemple 17b, de 34 ml de méthanol dans 170 ml de dichlorométhane, de 55,9 ml (455 mmoles) d'éthérate de trifluorure de bore et de 50,4 g (114 mmoles) de tétraacétate de plomb dans 200 ml de dichlorométhane. Le produit est purifié par recristallisation dans l'acétate d'éthyle pour fournir 21,2 g (Rdt = 64,2 %) d'un solide blanc. F = 154-6 °C.

| Analyse centésimale : $C_{22}H_{26}ClNO_4S$ (M = 435,97) | | | | | |
|---|---|---|---|---|---|
| | C % | H % | Cl % | N % | S % |
| Calculé | 60,61 | 6,01 | 8,15 | 3,21 | 7,35 |
| Trouvé | 60,79 | 6,24 | 8,21 | 3,42 | 7,37 |

I.R. (KBr) : $\nu$ (NH) = 3230 $cm^{-1}$ ; (C=O) = 1700 $cm^{-1}$ ; ($SO_2$) = 1330 $cm^{-1}$ ; ($SO_2$) = 1150 $cm^{-1}$.
R.M.N. ($CDCl_3$) : $\delta$ = 1,0-2,0 (8H,m) ; 2,6 (2H,s) ; 2,7 (2H,d, J = 6,75 Hz, se transforme en singulet avec $CF_3COOD$) ; 3,55 (2H,s) ; 3,7 (3H,s) ; 4,4 (1H,t, J = 6,75 Hz, échangeable avec $CF_3COOD$) ; 6,6-7,3 (4H,m)

; 7,3-8,0 (4H,m).

## Exemple 19

### Acide 4-[[1-[[[(4-chlorophényl)sulfonyl]amino]méthyl]cyclopentyl]méthyl]benzèneacétique

Un mélange de 16,1 g (36,9 mmoles) du composé préparé dans l'exemple 18, de 4,1 g (73,8 mmoles) de KOH en pastilles, de 236 ml de méthanol et de 236 ml d'eau est porté à reflux 2 heures. Après élimination du méthanol sous pression réduite, on reprend par l'eau, on lave par l'éther éthylique, avant d'acidifier par HCl dilué. Le précipité formé est essoré, lavé à l'eau et séché à 50 °C. On obtient 14,9 g (Rdt = 96,1 %) d'un solide blanc. F = 151-4 °C.

| Analyse centésimale : $C_{21}H_{24}ClNO_4S$ (M = 421,939) | | | | | |
|---|---|---|---|---|---|
| | C % | H % | Cl % | N % | S % |
| Calculé<br>Trouvé | 59,78<br>59,85 | 5,73<br>5,95 | 8,40<br>8,72 | 3,32<br>3,21 | 7,60<br>7,96 |

I.R. (KBr : $\nu$ (NH) = 3270 $cm^{-1}$ ; (C=O) = 1690 $cm^{-1}$ ; ($SO_2$) = 1315 $cm^{-1}$ ; ($SO_2$) = 1155 $cm^{-1}$.
R.M.N. (acétone $d_6$) : $\delta$ = 1,0-2,0 (8H,m) ; 2,65 (2H,s) ; 2,7 (2H,d, J = 6,75 Hz, se transforme en singulet avec $CF_3COOD$) ; 3,55 (2H,s) ; 6,3 (1H,t, J = 6,75 Hz, échangeable avec $CF_3COOD$) ; 7,1 (4H,s) ; 7,4-7,7 (2H,m) ; 7,7-8,0 (2H,m) ; 9,2 (1H,s large, échangeable avec $CF_3COOD$).

## Exemple 20

### 4-[[1-[[[(4-Chlorophényl)sulfonyl]amino]méthyl]cyclopentyl]méthyl]benzèneacétate de sodium

Un mélange de 0,4 g (0,95 mmole) de l'acide préparé dans l'exemple 19 et de 9,35 ml d'une solution de NaOH 0,1 N est chauffé à 50 °C quelques minutes. Après retour à température ambiante, puis filtration, on concentre à sec sous pression réduite. Le résidu est recristallisé dans un mélange d'éthanol et d'éther pour donner 0,3 g (Rdt = 71,3 %) d'un solide blanc. F = 218-22 °C.

| Analyse centésimale : $C_{21}H_{23}ClNNaO_4S$ (M = 443,92) | | | | | | |
|---|---|---|---|---|---|---|
| | C % | H % | Cl % | N % | Na% | S % |
| Calculé<br>Trouvé | 56,82<br>57,10 | 5,22<br>5,30 | 7,99<br>8,16 | 3,16<br>3,18 | 5,18<br>5,16 | 7,22<br>7,10 |

I.R. (KBr) : $\nu$ (NH) = 3060 $cm^{-1}$ ; (C=O) = 1570 $cm^{-1}$ ; ($SO_2$) = 1370 $cm^{-1}$ ; ($SO_2$) = 1140 $cm^{-1}$.
R.M.N. (DMSO $d_6$) : $\delta$ = 1,1-1,75 (8H,m) ; 2,3-2,8 (5H,m, dont 1H échangeable avec $CF_3COOD$) ; 3,2 (2H,s) ; 6,75-7,25 (4H,m) ; 7,4-8,5 (4H,m).

## Exemple 21

### Acide 4-[[1-[[[(4-chlorophényl)sulfonyl]amino]méthyl]cyclopentyl]méthyl]benzèneacétique

a) 4-Chloro-N-[[1-[[4-[2-(morpholin-4-yl)-2-thioxoéthyl]phényl]méthyl]cyclopentyl]méthyl]-benzènesulfonamide

Un mélange de 12,4 g (30,5 mmoles) du composé obtenu dans l'exemple 17b, de 1,6 g (48,8 mmoles) de soufre et de 200 ml de morpholine, est porté à reflux 30 heures, avant d'être jeté sur un mélange glace-eau. Le précipité obtenu est purifié par chromatographie sur colonne de silice par un mélange hexane-acétate d'éthyle 4/1, puis 2/1, avant d'être recristallisé dans un mélange hexane-acétate d'éthyle, pour donner 1,3 g (Rdt = 8,4 %) d'un solide blanc. F = 141-4 °C. On récupère 2,7 g de produit à partir des eaux-mères (Rdt global = 25,6 %).
I.R. (KBr) : $\nu$ (NH) = 3250 $cm^{-1}$.

R.M.N. ($CDCl_3$) : δ = 1,0-1,75 (8H,m) ; 2,3-2,9 (6H,m) ; 3,0-3,4 (4H,m) ; 3,55-3,9 (4H,m) ; 5,1 (1H,t, J = 6,75 Hz, échangeable avec $CF_3COOD$) ; 6,5-7,3 (4H,m) ; 7,35-7,9 (4H,m).

b) Acide 4-[[1-[[[(4-chlorophényl)sulfonyl]amino]méthyl]cyclopentyl]méthyl]benzèneacétique

Un mélange composé de 2,7 g (5,3 mmoles) du produit préparé dans l'exemple 21a, de 1,3 g (33 mmoles) de NaOH en pastilles et de 33 ml d'eau, est porté à reflux 24 h. Après refroidissement le milieu réactionnel dilué à l'eau est lavé à l'éther, filtré et acidifié par HCl concentré à froid. Le précipité formé est lavé à l'eau, puis séché sous vide à 80 °C, avant d'être recristallisé dans un mélange hexane-acétate d'éthyle pour fournir 0,4 g (Rdt = 17,9 %) d'un produit blanc possédant toutes les caractéristiques physiques, spectrales et chromatographiques du composé obtenu dans l'exemple 19.

**Exemple 22**

**Complexe *trans*-4-[[2-[[(4-chlorophényl)sulfonyl]amino]lcyclopentyl]méthyl]benzèneacétate de sodium et β-cyclodextrine (1:1)**

A une solution composée de 0,62 g (1,45 mmole) de *trans*-4-[[2-[[(4-chlorophényl)sulfonyl]amino]-cyclopentyl]méthyl]benzèneacétate de sodium préparé dans l'exemple 2 et de 60 ml d'eau distillée, est ajouté un mélange tiédi de 1,65 g (1,45 mmole) de β-cyclodextrine et de 60 ml d'eau distillée. Après agitation 20 heures à 20 °C, le solvant est éliminé sous pression réduite. L'eau résiduelle est chassée par distillation azéotropique avec du toluène à pression normale. Le toluène est concentré sous pression réduite et le résidu séché 6 h sous vide. On obtient 2 g (Rdt = 90,9 %) d'un solide blanc cassé. F = 265 °C (coloration à partir de 210 °C).

| Analyse centésimale : $C_{62}H_{91}ClNNaO_{39}S + H_2O$ (M = 1582,881) | | | | | | |
|---|---|---|---|---|---|---|
| | C % | H % | Cl % | N % | Na % | S % |
| Calculé | 47,05 | 5,92 | 2,24 | 0,88 | 1,45 | 2,03 |
| Trouvé | 47,05 | 5,97 | 2,10 | 0,83 | 1,65 | 1,86 |

I.R. (KBr) : ν (OH) = 3360 $cm^{-1}$ ; (C=O) = 1570 $cm^{-1}$ ; ($SO_2$) = 1320 $cm^{-1}$ ; ($SO_2$) = 1155 $cm^{-1}$.
R.M.N. (DMSO $d_6$) : δ = 0,9-2,25 (9H,m) ; 2,6-4,05 (48H,m, dont 3H échangeables par $CF_3COOD$ ; 4,1-4,6 (7H,m, échangeables avec $CF_3COOD$) ; 4,7-5,0 (7H,m) ; 5,65-6,3 (14H,m, échangeables avec $CF_3COOD$) ; 6,7-7,1 (4H,m) ; 7,4-7,9 (4H,m).

**Exemple 23**

**Complexe 4-[[1-[[[(4-chlorophényl)sulfonyl]amino]méthyl]cylopentyl]méthyl]benzèneacétate de sodium et β-cyclodextrine (1:1)**

Obtenu en opérant comme dans l'exemple 22 à partir de 0,5 g (1,12 mmole) de 4-[[1-[[[(4-chlorophényl)sulfonyl]amino]méthyl]cyclopentyl]méthyl]benzèneacétate de sodium préparé dans l'exemple 20, dans 50 ml d'eau distillée et de 1,28 g (1,12 mmole) de β-cyclodextrine dissoute dans 50 ml d'eau distillée. On obtient 1,5 g (Rdt = 83 %) d'un solide blanc cassé. F = 240-270 °C.

| Analyse centésimale : $C_{63}H_{93}ClNNaO_{39}S + 0,75\ C_7H_8$ (toluène) (M = 1648,013) | | | | | | |
|---|---|---|---|---|---|---|
| | C % | H % | Cl % | N % | Na % | S % |
| Calculé | 49,74 | 6,05 | 2,15 | 0,85 | 1,39 | 1,94 |
| Trouvé | 49,50 | 6,07 | 2,25 | 1,07 | 1,30 | 2,29 |

I.R. (KBr) : ν (OH) = 3360 $cm^{-1}$ ; (C=O) = 1570 $cm^{-1}$ ; ($SO_2$) = 1320 $cm^{-1}$ ; ($SO_2$) = 1150 $cm^{-1}$.
R.M.N. (DMSO $d_6$) ; δ = 1,0-1,6 (8H,m) ; 2,3 (2H,s) ; 2,5 (2H,s) ; 2,75-4,0 (44H,m) ; 4,2-4,6 (7H,m, échangeables avec $CF_3COOD$) ; 4,7-5,0 (7H,m) ; 5,75-6,4 (14H,m, échangeables avec $CF_3COOD$) ; 6,75-7,25 (5H, m, dont 1H échangeable par $CF_3COOD$); 7,4-7,9 (4H,m).

## Exemple 24

## Acide 4-[[1-[[[(4-méthylphényl)sulfonyl]amino]méthyl]cyclopentyl]méthyl]benzèneacétique

a) 4-Méthyl-N-[[1-[(phényl)méthyl]cyclopentyl]méthyl]-benzènesulfonamide

Obtenu en opérant comme dans l'exemple 1c, à partir de 9,4 g (48,3 mmoles) de l'amine préparée dans l'exemple 15a, de 5,9 g (57,9 mmoles) de triéthylamine dans 200 ml de dichlorométhane et de 9,2 g (48,3 mmoles) de chlorure de 4-méthylbenzènesulfonyle dans 60 ml de dichlorométhane. Après agitation 16 heures à température ambiante et traitement, on obtient 16,0 g (96,4 %) d'un solide beige, utilisé sans autre purification. F = 124-6 ° C.
I.R. (KBr) : $\nu$ (NH) = 3280 $cm^{-1}$ ; ($SO_2$) = 1315 $cm^{-1}$ ; ($SO_2$) = 1160 $cm^{-1}$.
R.M.N. ($CDCl_3$) : $\delta$ = 1,0-2,0 (8H,m) ; 2,4 (3H,s) ; 2,6 (2H,s) ; 2,7 (2H,d) ; 4,5 (1H,m, échangeable avec $CF_3COOD$) ; 6,7-7,45 (7H,m) ; 7,5-7,9 (2H,m).

b) N-[[1-[(4-Acétylphényl)méthyl]cyclopentyl]méthyl]-4-méthylbenzènesulfonamide

Obtenu en opérant comme dans l'exemple 16 b, à partir de 16,0 g (46,5 mmoles) du composé préparé dans l'exemple 24a, de 4,3 ml (60,4 mmoles) de chlorure d'acétyle dans 280 ml de dichlorométhane et de 20,5 g (153,7 mmoles) de chlorure d'aluminium. Après purification par chromatographie sur colonne de silice avec un mélange hexane-acétate d'éthyle 2/1 puis 1/1 on obtient 10,7 g (Rdt = 59,7 %) d'un solide blanc cassé. F = 80-4 ° C.
I.R. (KBr) : $\nu$ (NH) = 3220 $cm^{-1}$ ; (C=O) = 1650 $cm^{-1}$ ; ($SO_2$) = 1310 $cm^{-1}$ ; ($SO_2$) = 1140 $cm^{-1}$.
R.M.N. ($CDCl_3$) : $\delta$ = 1,25-1,8 (8H,m) ; 2,4 (3H,s) ; 2,5-2,85 (7H,m, dont un doublet se transformant en singulet avec $CF_3COOD$) ; 4,9 (1H,m, échangeable avec $CF_3COOD$) ; 6,9-8,0 (8H,m).

c) 4-[[1-[[[(4-Méthylphényl)sulfonyl]amino]méthyl]cyclopentyl]méthyl]benzèneacétate de méthyle

Obtenu en opérant comme dans l'exemple 5e à partir de 10,7 g (27,7 mmoles) du composé préparé dans l'exemple 24b, de 12 ml de méthanol, de 13,6 ml (111 mmoles) d'éthérate de trifluorure de bore dans 60 ml de dichlorométhane et de 14,7 g (33,2 mmoles) de tétraacétate de plomb dans 75 ml de benzène. Après recristallisation dans l'acétate d'éthyle on obtient 2,5 g (Rdt - 21,7 %) d'un solide blanc cassé. F = 148-51 ° C.
I.R. (KBr) : $\nu$ (NH) = 3250 $cm^{-1}$ ; (C=O) = 1710 $cm^{-1}$ ; ($SO_2$) = 1315 $cm^{-1}$ ; ($SO_2$) = 1155 $cm^{-1}$.
R.M.N. ($CDCl_3$) : $\delta$ = 1,0-1,9 (8H,m) ; 2,4 (3H,s) ; 2,55 (2H,s) ; 2,7 (2H,d, J = 6,75 Hz, se transforme en singulet avec $CF_3COOD$) ; 3,5 (2H,s) ; 3,65 (3H,s) ; 4,5 (1H,t, J = 6,75 Hz, échangeable avec $CF_3COOD$) ; 6,85-7,4 (6H,m) ; 7,5-7,8 (2H,m).

d) Acide 4-[[1-[[[(4-méthylphényl)sulfonyl]amino]méthyl]cyclopentyl]méthyl]benzèneacétique

Obtenu en opérant comme dans l'exemple 5f à partir de 2,5 g (6 mmoles) de l'ester préparé dans l'exemple 24c, de 0,67 g (12 mmoles) de KOH en pastilles, de 25 ml d'éthanol et de 25 ml d'eau. Après deux recristallisations dans un mélange hexane-acétate d'éthyle, on obtient 1,2 g (Rdt = 50,0 %) d'un solide blanc cassé. F = 164-6 ° C.

| Analyse centésimale : $C_{22}H_{27}NO_4S$ (M = 401,521) | | | | |
|---|---|---|---|---|
| | C % | H % | N % | S % |
| Calculé | 65,81 | 6,78 | 3,49 | 7,98 |
| Trouvé | 65,97 | 6,88 | 3,46 | 8,25 |

I.R. (KBr) : $\nu$ (NH) = 3250 $cm^{-1}$ ; (C=O) = 1690 $cm^{-1}$ ; ($SO_2$) = 1315 $cm^{-1}$ ; ($SO_2$) = 1150 $cm^{-1}$.
R.M.N. (DMSO $d_6$) : $\delta$ = 1,0-1,75 (8H,m) ; 2,4 (3H,s) ; 2,4-2,8 (4H,m) ; 3,5 (2H,s) ; 7,05 (4H,s) ; 7,2-8,0 (5H,m, dont 1H échangeable avec $CF_3COOD$ ; 12,2 (1H,s, échangeable avec $CF_3COOD$).

**Exemple 25**

**Acide 4-[[1-[[[(4-chlorophényl)sulfonyl]amino]méthyl(cyclopentyl]méthyl]benzoïque**

a) 4-(Chlorométhyl)benzèneméthanol

Un mélange de 50 g (293 mmoles) d'acide 4-chlorométhylbenzoïque et de 400 ml de tétrahydrofurane est ajouté goutte à goutte à une solution maintenue à 15 °C, du complexe d'hydrure de bore-diméthylsulfure (320 mmoles) dans 410 ml de tétrahydrofurane. On porte à reflux pendant 6 heures. Après refroidissement on ajoute 400 ml d'eau, et on sature le mélange par le carbonate de potassium avant d'extraire par l'acétate d'éthyle. La phase organique lavée à l'eau jusqu'à neutralité est séchée sur $Na_2SO_4$, puis concentrée sous pression réduite avant d'être purifiée par distillation pour donner 35,95 g (Rdt = 78,3 %) d'un liquide jaune pâle, qui cristallise rapidement.
F = 49-51 °C ; $Eb_{0,6}$ = 121 °C.
I.R. (KBr) : $\nu$ (OH) = 3340 $cm^{-1}$.
R.M.N. ($CDCl_3$) : $\delta$ = 2,25 (1H,s, échangeable avec $CF_3COOD$) ; 4,55 (2H,s) ; 4,6 (2H,s) ; 7,3 (4H,s).

b) 1-(Chlorométhyl)-4-[(triméthylsilyloxy)méthyl]benzène

A une solution de 35,95 g (230 mmoles) du composé préparé dans l'exemple 25a et de 370 ml de dichlorométhane anhydre maintenue à 5 °C, on coule goutte à goutte 36,9 g (230 mmoles) de 1,1,1,3,3,3-hexaméthyldisilazane,puis 23,3 g (230 mmoles) de triéthylamine puis enfin 24,6 g (230 mmoles) de chlorure de triméthylsilyle. Le milieu réactionnel est maintenu 21 heures à 5 °C puis 7 heures à 20 °C. Le précipité blanc formé est filtré sur verre fritté et rincé par le dichlorométhane. Le filtrat concentré à sec sous vide réduit à 30 °C est repris par l'hexane. Le nouveau précipité formé est filtré sur verre fritté et rincé à l'hexane. Le filtrat, après concentration est distillé pour donner 42,9 g (Rdt = 81,5 %) d'un liquide incolore.
$Eb_{0,45}$ = 85-8 °C.
R.M.N. ($CDCl_3$) : $\delta$ = 0,0 (9H,s) ; 4,4 (2H,s) ; 4,5 (2H,s) ; 7,1 (4H,s).

c) 1-[[4-[(Triméthylsilyloxy)méthyl]phényl]méthyl]cyclopentanecarbonitrile

A un mélange de 13,8 g (136 mmoles) de diisopropylamine et de 157 ml de tétrahydrofurane refroidit à -75 °C on coule goutte à goutte en 40 minutes, 65,6 ml de n-butyllithium en solution 1,6 M dans l'hexane, puis 10 g (105 mmoles) de cyclopentanecarbonitrile et enfin 26,4 g (115 mmoles) du composé préparé dans l'exemple 25b. Le milieu réactionnel est ensuite agité 2 heures à -70 °C puis 16 heures à 20 °C. On ajoute alors 100 ml d'eau et on décante la phase organique, qui est séchée sur $Na_2SO_4$ et concentrée à sec sous vide. Le produit est purifié par distillation pour donner 14,1 g (Rdt = 46,8 %) d'une huile épaisse jaune pâle. $Eb_{1,3}$ = 170 °C.
I.R. (film) : $\nu$ (C≡N) = 2230 $cm^{-1}$.
R.M.N. ($CDCl_3$) : $\delta$ = 0,0 (9H,s) ; 1,3-2,25 (8H,m) ; 2,7 (2H,s) ; 4,5 (2H,s) ; 7,1 (4H,s).

d) 1-[[4-(Hydroxyméthyl)phényl]méthyl]cyclopentanecarbonitrile

A une solution de 13,2 g (45 mmoles) du composé préparé dans l'exemple 25c et de 50 ml de tétrahydrofurane, on ajoute 53,7 ml (53 mmoles) d'une solution 1M de fluorure de tétrabutylammonium dans le tétrahydrofurane. Après avoir agité 15 minutes, on jette sur 800 ml d'eau, on extrait par l'acétate d'éthyle que l'on sèche ensuite sur $Na_2SO_4$. L'huile brune fluide obtenue (Rdt = quantitatif) est utilisée sans autre purification.
I.R. (film) : $\nu$ (OH) = 3370 $cm^{-1}$ ; (C≡N) = 2200 $cm^{-1}$.
R.M.N. ($CDCl_3$) : $\delta$ = 1,5-2,25 (8H,m) ; 2,4 (1H,s, échangeable avec $D_2O$) ; 2,8 (2H,s) ; 4,55 (2H,s) ; 7,2 (4H,s).

e) 4-[[1-(Aminométhyl)cyclopentyl]méthyl]benzèneméthanol

Un mélange de 6,4 g (29,7 mmoles) du composé préparé dans l'exemple 25d et de 12,6 ml d'éther est coulé goutte à goutte, à température ambiante sur 1,35 g (35,6 mmoles) de $LiAlH_4$ en suspension dans 35 ml d'éther. La vitesse d'addition est réglée pour maintenir le solvant à reflux. Après addition, le milieu réactionnel est agité 18 heures à température ambiante puis 5 heures à reflux. L'excès d'hydrure est détruit

par 6,75 ml d'eau. Les sels minéraux formés sont filtrés sur $Na_2SO_4$ et lavés par l'éther. Le filtrat concentré sous pression réduite donne 5 g (Rdt = 76,7 %), d'une huile jaune utilisée sans autre purification.
I.R. (film) : $\nu$ ($NH_2$) = 3350 $cm^{-1}$ ; (OH) = 3300 $cm^{-1}$.
R.M.N. ($CDCl_3$) : $\delta$ = 0,9-2,1 (8H,m) ; 2,3 (2H,s) ; 2,5 (2H,s) ; 2,6 (3H,s large, échangeables avec $CF_3COOD$) ; 4,5 (2H,s) ; 6,6-7,5 (4H,m).

f) 4-Chloro-N-[[1-4-(hydroxyméthyl)phényl]méthyl]cyclopentyl]méthyl]benzènesulfonamide

Un mélange de 1 g (4,6 mmoles) du composé préparé dans l'exemple 25e, de 0,55 g (5,5 mmoles) de triéthylamine et de 8 ml de dichlorométhane sec est maintenu à -20 °C. Une solution de 0,96 g (4,6 mmoles) de chlorure de 4-chlorobenzènesulfonyle dans 4 ml d'éther est coulée en 15 mn. On agite pendant 2,5 heures à une température comprise entre -20 ° et -10 °C avant de jeter sur 50 ml d'eau additionnée d'1 ml d'HCl concentré. On extrait par le dichlorométhane, qui est lavé jusqu'à neutralité, séché sur $Na_2SO_4$ et concentré sous pression réduite pour donner un solide beige. On purifie le produit par chromatographie sur colonne de silice dans un mélange dichlorométhane-méthanol 95:5. On obtient 0,8 g (Rdt = 44,6 %) d'un solide pâteux blanc.
I.R. (film) : $\nu$ (OH) = 3460 $cm^{-1}$ ; (NH) = 3270 $cm^{-1}$ ; ($SO_2$) = 1315 $cm^{-1}$ ; ($SO_2$) = 1150 $cm^{-1}$.
R.M.N. ($CDCl_3$) : $\delta$ = 1,25-1,75 (8H,m) ; 1,9 (1H,s large, échangeable avec $CF_3COOD$) ; 2,6 (2H,s) ; 2,7 (2H,d, J = 6,75 Hz, se transforme en singulet avec $CF_3COOD$) ; 4,6 (2H,s) ; 4,85 (1H, t, J = 6,75 Hz, échangeable avec $CF_3COOD$); 7,0-7,3 (4H,m) ; 7,3-7,6 (2H,m) ; 7,6-7,9 (2H,m).

g) Acide 4-[[1-[[[(4-chlorophényl)sulfonyl]amino]méthyl]cyclopentyl]méthyl]benzoïque

A une solution maintenue à 0 °C de 0,8 g (2,0 mmoles) du composé préparé dans l'exemple 25f dans 17 ml d'acétone, on ajoute 1,2 ml de réactif de Jones (préparé par addition à 0 °C d'un mélange de 0,65 ml d'acide sulfurique concentré et de 0,52 ml d'eau sur 0,41 g (4,1 mmoles) d'oxyde de chrome (VI) dissous dans 0,65 ml d'eau). Après avoir agité 4 h à température ambiante, on filtre les sels formés que l'on lave à l'acétone. Le filtrat concentré à sec sous pression réduite est repris par l'eau et extrait par l'éther. Cette phase organique est ensuite extraite par la soude 1N. L'acidification de cette phase aqueuse par HCl dilué permet d'obtenir un précipité blanc purifié par recristallisation dans un mélange hexane-acétate d'éthyle pour donner 0,35 g (Rdt = 42,3 %) d'un solide blanc. F = 184-9 °C.

| Analyse centésimale : $C_{20}H_{22}ClNO_4S$ (M = 407,912) | | | | | |
|---|---|---|---|---|---|
| | C % | H % | Cl % | N % | S % |
| Calculé | 58,89 | 5,44 | 8,69 | 3,43 | 7,86 |
| Trouvé | 59,02 | 5,34 | 8,75 | 3,43 | 7,77 |

I.R. (KBr) : $\nu$ (NH) = 3240 $cm^{-1}$ ; (C=O) = 1675 $cm^{-1}$ ; ($SO_2$) = 1320 $cm^{-1}$ ; ($SO_2$) = 1150 $cm^{-1}$.
R.M.N. (acétone $d_6$) : $\delta$ = 1,4-1,8 (8H,m) ; 2,8 (2H, d, J = 6,75 Hz, se transforme en singulet avec $CF_3COOD$) ; 2,9 (2H,s) ; 6,5 (1H,t,J = 6,75 Hz, échangeable avec $CF_3COOD$) ; 7,2-8,2 (9H,m, dont 1H échangeable avec $CF_3COOD$).

**Exemple 26**

**Acide 4-[[1-[[[(3,4-dichlorophényl)sulfonyl]amino]méthy]cyclopentyl]méthyl]benzèneacétique**

a) 1-(Bromométhyl)-4-[2-(triméthylsilyloxy)éthyl]benzène

A une solution de 20,9 g (97,2 mmoles) de 4-bromométhyl)benzèneéthanol (obtenu selon Plaue S. et Heissler D., Tetrahedron Lett. (1987) 28, 1401-4), dans 150 ml de tétrahydrofurane maintenue à 5 °C, on coule goutte à goutte 20,5 ml (97,2 mmoles) de 1,1,1,3,3,3-hexaméthyldisilazane, puis 13,55 ml (97,2 mmoles) de triéthylamine, puis enfin 12,3 ml (97,2 mmoles) de chlorure de triméthylsilyle. Le milieu réactionnel est agité 1 heure à la même température, avant de filtrer le précipité formé et de le rincer par l'hexane. Le filtrat concentré à sec sous pression réduite est purifié par distillation pour donner 16,8 g (Rdt = 60,0 %) d'un liquide incolore. $Eb_{0,35}$ = 98-108 °C.
R.M.N. ($CDCl_3$) : $\delta$ = 0,0 (9H,s) ; 2,7 (2H,t, J = 6,75 Hz) ; 3,7 (2H,t, J = 6,75 Hz) ; 4,3 (2H,s) ; 6,8-7,5

(4H,m).

b) 1-[[4-(2-Hydroxyéthyl)phényl]méthyl]cyclopentanecarbonitrile

A un mélange de 38,9 ml (278 mmoles) de diisopropylamine et de 320 ml de tétrahydrofurane, refroidi à -60 °C, on coule successivement goutte à goutte 174 ml d'une solution 1,6 M de n-butyllithium dans l'hexane additionnée de 50 ml de tétrahydrofurane, un mélange de 24 g (252 mmoles) de cyclopentanecarbonitrile et de 50 ml de tétrahydrofurane, 65 ml de 1,3-diméthylimidazolidinone et enfin 73,95 g (257 mmoles) du composé préparé dans l'exemple 26a. On laisse remonter la température à 20 °C. Après 16 heures à 20 °C on porte à reflux durant 13 heures. Après refroidissement on ajoute 1000 ml d'eau et on agite une heure à température ambiante avant d'ajouter 35 ml d'HCl concentré et d'agiter encore une heure. On extrait par l'acétate d'éthyle qui est ensuite lavé par l'eau, séché sur $Na_2SO_4$, avant d'être concentré sous pression réduite. La purification est réalisée par distillation qui donne 41,1 g (Rdt = 71,1 %) d'une huile jaune. $Eb_{0,3}$ = 175-85 °C.
I.R. (film) : $\nu$ (OH) = 3410 $cm^{-1}$ ; (C≡N) = 2240 $cm^{-1}$.
R.M.N. ($CDCl_3$) : $\delta$ = 1,25-2,25 (8H,m) ; 2,4 (1H,s, échangeable avec $CF_3COOD$) ; 2,8 (2H,s) ; 2,8 (2H,t, J = 6,75 Hz) ; 3,75 (2H,t, J = 6,75 Hz) ; 6,65-7,5 (4H,m).

c)4-[[11-(Aminométhyl)cyclopentyl]méthyl]benzèneéthanol

A température ambiante, on coule goutte à goutte 7 g (30,5 mmoles) du composé préparé dans l'exemple 26b en solution dans 17 ml d'éther, sur 7,7 ml d'une solution commerciale à 13 % de $LiAlH_4$ (1,3 g, 33,55 mmoles) dans un mélange de toluène et de tétrahydrofurane, additionnée de 7 ml d'éther. Après agitation pendant 14 heures, on ajoute avec précaution 120 ml d'eau puis 100 ml d'éther. Les sels minéraux insolubles sont filtrés. L'éther est décanté et la phase aqueuse à nouveau extraite par l'éther. Les phases organiques réunies sont lavées à l'eau, séchées sur $Na_2SO_4$, puis concentrées sous pression réduite, pour donner quantitativement l'amine attendue, utilisée sans autre purification.
I.R. (film) : $\nu$ ($NH_2$) = 3360 $cm^{-1}$ ; (OH) = 3300 $cm^{-1}$.
R.M.N. ($CDCl_3$) : $\delta$ = 1,0-1,8 (8H,m) ; 1,9 (3H,s, échangeables avec $D_2O$) ; 2,4 (2H,s) ; 2,6 (2H,s) ; 2,8 (2H,t, J = 6,75 Hz) ; 3,8 (2H,t, J = 6,75 Hz) ; 6,7-7,4 (4H,m).

d) 3,4-Dichloro-N-[[1-[[4-[(2-hydroxyéthyl)phényl]méthyl]cyclopentyl]méthyl]-benzènesulfonamide

Obtenu en opérant comme dans l'exemple 1c, à partir de 3 g (12,8 mmoles) du composé obtenu dans l'exemple 26c dans 50 ml de dichlorométhane, de 2,15 ml (15,4 mmoles) de triéthylamine et de 3,15 g (12,8 mmoles) de chlorure de 3,4-dichlorobenzènesulfonyle, dans 10 ml de dichlorométhane. Après agitation 16 heures à température ambiante et traitement usuel, on purifie le produit par chromatographie sur colonne de silice avec un mélange hexane-acétate d'éthyle 4:1 pour obtenir 0,95 g (Rdt = 16,7 %) d'une huile jaune qui cristallise.
I.R. (film) : $\nu$ (OH) = 3460 $cm^{-1}$ ; (NH) = 3270 $cm^{-1}$ ; ($SO_2$) = 1330 $cm^{-1}$ ; ($SO_2$) = 1160 $cm^{-1}$.
R.M.N. ($CDCl_3$) : $\delta$ = 1,0-2,0 (9H,m, dont 1H échangeable avec $CF_3COOD$) ; 2,5 (2H,s) ; 2,7 (2H,d, J = 6,75 Hz, se transforme en singulet avec $CF_3COOD$) ; 2,75 (2H,t, J = 6,75 Hz) ; 3,8 (2H,t, J = 6,75 Hz) ; 4, 5 (1H,t, J = 6,75 Hz, échangeable avec $CF_3COOD$) ; 6,9 (4H,s) ; 7,5 (2H,m) ; 7,8 (1H,m).

e) Acide 4-[[1-[[[(3,4-dichlorophényl)sulfonyl]amino]méthyl]cyclopentyl]méthyl]benzèneacétique

Obtenu en opérant comme dans l'exemple 25g, à partir de 0,9 g (2 mmoles) du composé préparé dans l'exemple 26d dans 20 ml d'acétone et de 1,85 ml de réactif de Jones (4 mmoles). Après deux recristallisations dans un mélange hexane-acétate d'éthyle on obtient 0,3 g (Rdt = 32,2 %) d'un solide blanc. F = 134-5 °C.

| Analyse centésimale : $C_{21}H_{23}Cl_2NO_4S$ (M = 456,384) | | | | | |
|---|---|---|---|---|---|
| | C % | H % | Cl % | N % | S % |
| Calculé | 55,27 | 5,08 | 15,54 | 3,07 | 7,02 |
| Trouvé | 55,40 | 4,86 | 15,26 | 3,05 | 6,92 |

I.R. (KBr) : ν (NH) = 3210 $cm^{-1}$ ; (C=O) = 1680 $cm^{-1}$ ; ($SO_2$) = 1300 $cm^{-1}$ ; ($SO_2$) = 1140 $cm^{-1}$.
R.M.N. (acétone $d_6$) : δ = 1,25-1,8 (8H,m) ; 2,65 (2H,s) ; 2,75 (2H,d, J = 6,75 Hz, se transforme en singulet avec $CF_3COOD$) ; 3,5 (2H,s) ; 6,2-6,8 (2H, m dont 1 triplet, J = 6,75 Hz, échangeables avec $CF_3COOD$) ; 7,1 (4H,s) ; 7,75 (2H,m) ; 7,9 (1H,m).

**Exemple 27**

**Acide 4-[[1-[[[(4-chlorophényl)sulfonyl]amino]méthyl]cyclobutyl]méthyl]benzèneacétique**

a) Cyclobutanecarbonitrile

Une solution de 80 g (799 mmoles) d'acide cyolobutanecarboxylique dans 250 ml de dichlorométhane est portée à reflux. On coule goutte à goutte, lentement, 115,6 g (819 mmoles) de chlorosulfonylisocyanate. Le reflux est poursuivi une heure après la fin de l'addition, jusqu'à évolution complète du $CO_2$. Le milieu réactionnel est alors refroidi à 10 °C. On y coule goutte à goutte, en 15 mn, 119,6 g (1638 mmoles) de N,N-diméthylformamide, avant de laisser l'agitation 1/4 d'heure à température ambiante. Puis on jette sur glace-eau, avant d'extraire par le dichlorométhane. La phase organique lavée par l'eau, est séchée sur $Na_2SO_4$ et concentrée sous vide. La distillation du résidu fournit 43,5 g (Rdt = 66,5 %) d'un liquide incolore. $Eb_{15}$ = 50 °C.
I.R. (film) : ν (C≡N) = 2250 $cm^{-1}$.
R.M.N. ($CDCl_3$) : δ = 1,5-2,7 (6H,m) ; 2,7-3,4 (1H,m).

b) 1-[(Phényl)méthyl]cyclobutanecarbonitrile

A une solution sous courant d'azote et maintenue à -30C °, de 82,3 ml (583 mmoles) de N,N-diisopropylamine dans 600 ml de tétrahydrofurane sec, on coule goutte à goutte 364,4 ml (583 mmoles) d'une solution 1,6 M dans l'hexane de n-butyllithium, puis 83,4 ml de 1,3-diméthylimidazolidinone. On agite 1/4 heure à -60 °C avant de descendre à -75 °C pour ajouter goutte à goutte un mélange de 43,5 g (530 mmoles) du composé préparé dans l'exemple 27a et de 500 ml de tétrahydrofurane sec. On agite 1 heure à -75 °C puis on ajoute goutte à goutte 67,1 g (530 mmoles) de chlorure de benzyle. On agite 1 heure à -75 °C avant de jeter le milieu réactionnel sur un mélange glace-HCl concentré. On extrait par l'éther, que l'on lave ensuite par l'eau jusqu'à neutralité, avant de le sécher sur $Na_2SO_4$. Après élimination du solvant sous pression réduite et distillation du résidu on obtient 68,1 g (Rdt = 75,9 %) d'un liquide incolore, gui cristallise vers -20 °C. $Eb_{15}$ = 140 °C. ($Eb_{0,1}$ = 118 ° selon Mousseron M., Jacquier R. et Fraisse R., Compt. Rend. Acad. Sci., Paris (1955), 241, 602-4).
I.R. (film) : ν (C≡N) = 2220 $cm^{-1}$.
R.M.N. : δ = 1,75-2,6 (6H,m) ; 2,9 (2H,s) ; 7,2 (5H,s).

c) [1-[(Phényl)méthyl]cyclobutyl]méthanamine

Obtenue en opérant comme dans l'exemple 15a, à partir de 18,3 g (482 mmoles) de $LiAlH_4$ dans 450 ml d'éther et de 68,1 g (402 mmoles) du composé préparé dans l'exemple 27b dans 170 ml d'éther. Après agitation 2 heures à température ambiante et traitement usuel, on obtient 68,4 g (Rdt = 97,1 %) d'une huile jaune, utilisée sans autre purification.
I.R. (film) : ν ($NH_2$) = 3360 $cm^{-1}$.
R.M.N. ($CDCl_3$) : δ = 1,25 (2H,s, échangeables avec $CF_3COOD$) ; 1,8 (6H,m) ; 2,6 (2H,s) ; 2,7 (2H,s) ; 7,1 (5H,s).

d) 4-Chloro-N-[[1-[(phényl)méthyl]cyclobutyl]méthyl]-benzènesulfonamide

Obtenu en opérant comme dans l'exemple 1c, à partir de 10 g (57 mmoles) de l'amine préparée dans l'exemple 27c, dans 100 ml de dichlorométhane, avec 7 g (68,4 mmoles) de triéthylamine et 12,4 g (59,3 mmoles) de chlorure de 4-chlorobenzènesulfonyle. Le solide beige obtenu est utilisé sans purification (16,3 g ; Rdt = 81,9 %). Une fraction recristallisée dans l'acétate d'éthyle fournit un solide blanc. F = 144-8 °C.

| Analyse centésimale : $C_{18}H_{20}ClNO_2S$ (M = 349,875) | | | | | |
|---|---|---|---|---|---|
| | C % | H % | Cl % | N % | S % |
| Calculé | 61,79 | 5,76 | 10,13 | 4,00 | 9,16 |
| Trouvé | 61,52 | 6,01 | 10,34 | 4,22 | 9,30 |

I.R. (KBr) : ν (NH) = 3250 $cm^{-1}$ ; ($SO_2$) = 1315 $cm^{-1}$ ; ($SO_2$) = 1150 $cm^{-1}$.
R.M.N. ($CDCl_3$) : δ = 1,5-2,2 (6H,m) ; 2,7 (2H,s) ; 2,8 (2H,d J = 6,75 Hz, se transforme en singulet avec $CF_3COOD$) ; 4,7 (1H,t, J = 6,75 Hz, échangeable avec $CF_3COOD$) ; 6,8-7,25 (5H,m) ; 7,25-7,5 (2H,m) ; 7,6-7,9 (2H,m).

e) N-[[1-[(4-Acétylphényl)méthyl]cyclobutyl]méthyl]-4-chlorobenzènesulfonamide

Obtenu en opérant comme dans l'exemple 16b, à partir de 4 g (11,4 mmoles) du composé préparé dans l'exemple 27d dans 160 ml de dichlorométhane, avec 1,2 g (14,8 mmoles) de chlorure d'acétyle, et 5 g (37,6 mmoles) de chlorure d'aluminium. La purification réalisée par chromatographie sur colonne de silice par un mélange hexane-acétate d'éthyle fournit 2,9 g (Rdt = 65,0 %) d'un solide beige. F = 158-60 °C. Une fraction recristallisée dans acétate d'éthyle-heptane donne un produit blanc. F = 158-60 °C.

| Analyse centésimale : $C_{20}H_{22}ClNO_3S$ + 0,6 $H_2O$ (M = 402,722) | | | | | |
|---|---|---|---|---|---|
| | C % | H % | Cl % | N % | S % |
| Calculé | 59,65 | 5,81 | 8,80 | 3,48 | 7,96 |
| Trouvé | 59,60 | 5,46 | 8,88 | 3,58 | 8,26 |

I.R. (KBr) : ν (NH) = 3270 $cm^{-1}$ ; (C=O) = 1670 $cm^{-1}$ ; ($SO_2$) = 1320 $cm^{-1}$ ; ($SO_2$) = 1155 $cm^{-1}$.
R.M.N. ($CDCl_3$) : δ = 1,35-2,2 (6H,m) ; 2,55 (3H,s) ; 2,8 (2H,s) ; 2,9 (2H,d, J = 6,75 Hz, se transforme en singulet avec $CF_3COOD$) ; 5,05 (1H,t J = 6,75 Hz, échangeable avec $CF_3COOD$) ; 7,0-8,0 (8H,m).

f) 4-[[1-[[[(4-Chlorophényl)sulfonyl]amino]méthyl]cyclobutyl]méthyl]benzèneacétate de méthyle

Obtenu en opérant comme dans l'exemple 5e, à partir de 2,1 g (5,3 mmoles) du composé préparé dans l'exemple 27e, de 2,5 ml (58,3 mmoles) de méthanol dans 50 ml de dichlorométhane, avec 5,2 ml (42,4 mmoles) d'éthérate de trifluorure de bore et 2,6 g (5,6 mmoles) de tétraacétate de plomb dans 40 ml de toluène. La purification par recristallisation dans l'acétate d'éthyle fournit 0,9 g (Rdt = 42,8 %) d'un solide blanc.
F = 135-7 °C.
I.R. (KBr) : ν (NH) = 3250 $cm^{-1}$ ; (C=O) = 1715 $cm^{-1}$ ; (($SO_2$) = 1320 $cm^{-1}$ ; ($SO_2$) = 1160 $cm^{-1}$.
R.M.N. ($CDCl_3$) : δ = 1,7-2,0 (6H,m) ; 2,7 (2H,s) ; 2,9 (2H,d, J = 6,75 Hz, se transforme en singulet avec $CF_3COOD$) ; 3,6 (2H,s) ; 3,7 (3H,s) ; 4,4 (1H,t J = 6,75 Hz, échangeable avec $CF_3COOD$) ; 6,9-7,9 (8H,m).

g) Acide 4-[[1-[[[(4-chlorophényl)sulfonyl]amino]méthyl]cyclobutyl]méthyl]benzèneacétique

Obtenu en opérant comme dans l'exemple 5f, à partir de 0,9 g (2,1 mmoles) de l'ester préparé dans l'exemple 27f, de 40 ml d'éthanol et de 0,23 g (4,2 mmoles) de potasse en pastilles dissoute dans 10 ml d'eau. Après recristallisation dans un mélange acétate d'éthyle-heptane, on isole 0,3 g (Rdt = 34,9 %) d'un solide blanc. F = 168-70 °C.

| Analyse centésimale : $C_{20}H_{22}ClNO_4S$ (M = 407,912) | | | | | |
|---|---|---|---|---|---|
| | C % | H % | Cl % | N % | S % |
| Calculé | 58,89 | 5,43 | 8,69 | 3,43 | 7,86 |
| Trouvé | 59,01 | 5,36 | 8,65 | 3,45 | 8,01 |

I.R. (KBr) : ν (NH) = 3230 $cm^{-1}$ ; (C=O) = 1720 $cm^{-1}$ ; ($SO_2$) = 1315 $cm^{-1}$ ; ($SO_2$) = 1160 $cm^{-1}$.
R.M.N. (DMSO $d_6$) : δ = 1,3-2,1 (6H,m) ; 2,6-2,8 (4H,m) ; 3,45 (2H,s) ; 7,05 (4H,s) ; 7,5-7,95 (5H,m, dont 1H échangeable avec $CF_3COOD$) ; 11,9 (1H,s large, échangeable avec $CF_3COOD$).

**Exemple 28**

**4-Chloro-N-[[1-[[4-(2-hydroxyéthyl)phényl]méthyl]cyclopentyl]méthyl]benzènesulfonamide**

Obtenu en opérant comme dans l'exemple 25f à partir de 2,5 g (10,7 mmoles) de 4-[[1-(aminométhyl)-cyclopentyl]méthyl]benzèneéthanol préparé dans l'exemple 26c, de 1,3 g (12,8 mmoles) de triéthylamine dans 20 ml de dichlorométhane et de 2,1 g (9,8 mmoles) de chlorure de 4-chlorobenzènesulfonyle dans 5 ml d'éther. Après traitement, le résidu est purifié par chromatographie sur colonne de silice dans un mélange hexane-acétate d'éthyle 2/1, suivie de recristallisations dans hexane-acétate d'éthyle pour donner 0,4 g (Rdt = 10 %) d'un solide blanc. F = 131-2 °C.

| Analyse centésimale : $C_{21}H_{26}ClNO_3S$ (M = 407,954) | | | | | |
|---|---|---|---|---|---|
| | C % | H % | Cl % | N % | S % |
| Calculé | 61,83 | 6,42 | 8,69 | 3,43 | 7,86 |
| Trouvé | 61,91 | 6,44 | 8,79 | 3,53 | 8,03 |

I.R. (KBr) : ν (OH) = 3530 $cm^{-1}$ ; (NH) = 3250 $cm^{-1}$ ; ($SO_2$) = 1315 $cm^{-1}$ ; ($SO_2$) = 1155 $cm^{-1}$.
R.M.N.($CDCl_3$) : δ = 1,2 - 1,8 (9H, m, dont 1H échangeable avec $CF_3COOD$) ; 2,6 (2H, s) ; 2,7 (2H, d, J = 6,75 Hz, se transforme en singulet avec $CF_3COOD$) ; 2,8 (2H, t, J = 6,4 Hz) ; 3,8 = 6,4 Hz) ; 4,4 (1H, t, J = 6,75 Hz, échangeable avec $CF_3COOD$) ; 7,0 (4H, s) ; 7,25 - 7,55 (2H, m) ; 7,55 - 7,9 (2H, m).

**Exemple 29**

**Acide 4-[1-[[[(4-chlorophényl)sulfonyl]amino]méthyl]cyclohexyl]benzèneacétique**

a) 2-[2-[4-(Bromométhyl)phényl]éthoxy]-3,4,5,6-tétrahydro-2H-pyrane

Un mélange de 16,9 g (78,5 mmoles) de 4-(bromométhyl)benzèneéthanol (préparé selon Plaue S. et Heissler D., Tetrahedron Lett. (1987) 28, 1401-4), de 10,6 g (125 mmoles) de 3,4-dihydro-2H-pyrane et de 0,16 g d'acide para-toluènesulfoniquedans 160 ml d'éther éthylique sec, est agité 16 heures à température ambiante. Le milieu réactionnel est ensuite lavé par une solution saturée en bicarbonate de sodium, puis à l'eau, séché sur $Na_2SO_4$ et concentré, pour donner 21,5 g (Rdt = 91,5 %) d'une huile jaune utilisée sans autre purification.
R.M.N. ($CDCl_3$) : δ = 1,25-2,0 (6H, m) ; 2,9 (2H, t, J = 6,75 Hz) ; 3,25-4,15 (4 H, m) ; 4,45 (2H,s) ; 4,55 (1H, m) ; 7,2 (4H, s).

b) 4-[2-[(3,4,5,6-Tétrahydro-2H-pyran-2-yl)oxy]éthyl]benzèneacétonitrile

On ajoute 21,5 g (71,8 mmoles) du composé préparé dans l'exemple 29a, à une solution de 3,9 g (79 mmoles) de cyanure de sodium dans 50 ml de diméthylsulfoxyde portée à 115 °C. On maintient cette température durant 5 heures. Après refroidissement, on jette sur $H_2O$ avant d'extraire par l'éther. La phase organique lavée par l'eau, séchée par $Na_2SO_4$ et concentrée, est purifiée par chromatographie sur colonne de silice avec un mélange hexane-acétate d'éthyle (4:1) pour donner 9.3 g (Rdt = 52,8 %) d'une huile incolore.
I.R. (film) : ν (C≡N) = 2255 $cm^{-1}$
R.M.N ($CDCl_3$) : δ = 1,25-2,0 (6H, m), 2.9 (2H, t, J=6,75 Hz) ; 3,25-4,25 (4H, m) ; 3,7 (2H, s) ; 4,5 (1H, m) ; 7,2 (4H, s).

c) 1-[4-[2-[(3,4,5,6-Tétrahydro-2H-pyran-2-yl)oxy]éthyl]phényl]cyclohexanecarbonitrile

Sur une suspension sous azote de 3,8 g (94,7 mmoles) de NaH (à 60 % dans l'huile minérale) dans 125 ml de diméthylsulfoxyde sec, maintenue à 20 °C par un bain de glace, on coule lentement une solution

de 9,3 g (37,9 mmoles) du composé préparé dans l'exemple 29 b, dans 20 ml de diméthylsulfoxyde sec. Après avoir agité 1 heure à température ambiante, on coule un mélange de 13,1 g (56,8 mmoles) de 1,5-dibromopentane et de 20 ml de diméthylsulfoxyde sec. Le milieu réactionnel est agité 40 heures à température ambiante avant d'être jeté sur de l'eau glacée et extrait à l'éther. La phase organique lavée à l'eau, et séchée sur $Na_2SO_4$ est purifiée par chromatographie sur colonne de silice par un mélange hexane-acétate d'éthyle (1:1) pour donner 10,2 g (Rdt = 85,7 %) d'une huile jaune orangé.

I.R. (film) : $\nu$ (C≡N) = 2250 $cm^{-1}$.

R.M.N. ($CDCl_3$) : $\delta$ = 0,75-2,5 (16H, m) ; 2,85 (2H, t, J = 6,75 Hz) ; 3,2-4,1 (4H, m) ; 4,5 (1H, m) ; 7,0 - 7,5 (4H, m).

d) 4-[1-(Aminométhyl)cyclohexyl]benzèneéthanol

Une solution de 10,2 g (32,5 mmoles) du composé préparé dans l'exemple 29 c dans 20 ml d'éther sec est ajouté à une suspension sous azote de 1,9 g (48,8 mmoles) de $LiAlH_4$ dans 100 ml d'éther sec. Le milieu réactionnel est porté 5 heures à reflux. Après refroidissement, on ajoute avec précaution 9,5 ml d'eau, puis 100 ml d'éther. Le milieu réactionnel est extrait par HCl N. Cette phase aqueuse est lavée à l'éther, basifiée par une solution de NaOH concentrée, puis extraite par l'éther. La phase éthérée est lavée par l'eau, séchée sur $Na_2SO_4$ et concentrée pour fournir 5,9 g (Rdt = 77,8 %) d'une huile jaune utilisée sans autre purification.

I.R. (film) : $\nu$ ($NH_2$) = 3350 $cm^{-1}$ ; (OH) = 3320 $cm^{-1}$.

R.M.N.($CDCl_3$) : $\delta$ = 0,75-2,35 (13H, m, dont 3H échangeables par $D_2O$) ; 2,6 (2H, s) ; 2,8 (2H, t, J = 6,75 Hz) ; 3,8 (2H, t, J = 6,75 Hz) ; 7,15 (4H, s).

e) 4-Chloro-N-[[1-[4-(2-hydroxyéthyl)phényl]cyclohexyl]méthyl]-benzènesulfonamide

A une solution de 5,9 g (25,2 mmoles) du composé préparé dans l'exemple 29 d, de 3,0 g (30,2 mmoles) de triéthylamine dans 60 ml de $CH_2Cl_2$ sec, maintenue à - 20 °C, on ajoute 5,2 g (24,7 mmoles) de chlorure de 4-chlorobenzènesulfonyle. Après 3 heures à - 20 °C, on jette le milieu réactionnel sur une solution d'HCl diluée, avant d'extraire par le dichlorométhane. La phase organique, lavée, séchée sur $Na_2SO_4$ et concentrée donne une huile qui est purifiée par chromatographie sur colonne de silice par un mélange hexane-acétate d'éthyle (1:1). On obtient 1,2 g (Rdt = 11,6 %) d'un solide blanc cassé. F = 110-1 °C.

I.R. (KBr): $\nu$ (OH) = 3450 $cm^{-1}$ ; (NH) = 3080 $cm^{-1}$ ; ($SO_2$) = 1310 $cm^{-1}$ ; ($SO_2$) = 1140 $cm^{-1}$.

R.M.N.($CDCl_3$) : $\delta$ = 0,75-2,25 (11H, m, dont 1H échangeable par $CF_3COOD$) ; 2,8 (2H, t, J = 6,75 Hz) ; 2,9 (2H, d, J = 6,75 Hz, se transforme en singulet avec $CF_3COOD$) ; 3,8 (2H, t, J = 6,75 Hz) ; 4,1 (1H, t, J = 6,75 Hz, échangeable avec $CF_3COOD$) ; 7,1 (4H, s) ; 7,3 (2H, m) ; 7,55 (2H, m).

f) Acide 4-[1-[[[(4-chlorophényl)sulfonyl]amino]méthyl]cyclohexyl]benzèneacétique

Obtenu en opérant comme dans l'exemple 25 g, à partir de 1,2 g (2,9 mmoles) du composé préparé dans l'exemple 29e dans 120 ml d'acétone et 2,8 ml (5,9 mmoles) de réactif de Jones. Le milieu réactionnel est agité 24 heures à température ambiante. Après 2 recristallisations dans le toluène, on obtient 0,3 g (Rdt = 25,0 %) d'un solide blanc. F = 147 - 51 °C.

| Analyse centésimale : $C_{21}H_{24}ClNO_4S$ (M = 421,939) | | | | | |
|---|---|---|---|---|---|
| | C % | H % | Cl % | N % | S % |
| Calculé | 59,78 | 5,73 | 8,40 | 3,32 | 7,60 |
| Trouvé | 59,52 | 5,70 | 8,34 | 3,22 | 7,57 |

I.R. (KBr) : $\nu$ (NH) = 3290 $cm^{-1}$ ; (C = 0) = 1700 $cm^{-1}$ ; ($SO_2$) = 1325 $cm^{-1}$ ; ($SO_2$) = 1160 $cm^{-1}$.

R.M.N.(DMSO $d_6$) : $\delta$ = 1,0 - 2,2 (10H, m) ; 2,7 (2H, d, J = 6,75 Hz, se transforme en singulet avec $CF_3COOD$) ; 3,4 (1H, m, échangeable avec $CF_3COOD$) ; 3,5 (2H, s) ; 6,9 - 7,4 (4H, m) ; 7,4 - 7,8 (4H, m) ; 12,2 (1H, s large, échangeable avec $CF_3COOD$).

**Exemple 30**

**Acide 4-[4-[[1-[[[(4-chlorophényl)sulfonyl]amino]méthyl]cyclopentyl]méthyl]phényl]-4-oxobutanoïque**

a) 4-[4-[[1-[[[(4-Chlorophényl)sulfonyl]amino]méthyl]cyclopentyl]méthyl]phényl]-4-oxobutanoate d'éthyle

A un mélange de 25,1 g (69 mmoles) du composé préparé dans l'exemple 17 a, de 12,5 g (75,9 mmoles) de 3-chloroformylpropanoate d'éthyle commercial et de 130 ml de dichlorométhane, maintenu à 0 °C, on ajoute par portions, 27,6 g (207,0 mmoles) de chlorure d'aluminium anhydre. Le milieu réactionnel est agité 2 heures à 0 °C puis 1 heure à température ambiante, avant d'être versé sur un mélange glace-acide chlorhydrique concentré. Après avoir récupéré la phase organique, la phase aqueuse est extraite par $CH_2Cl_2$. Les phases organiques réunies sont lavées par $H_2O$, séchées sur $Na_2SO_4$ et concentrées pour donner 30,9 g (Rdt = 90,9 %) d'un solide crème, utilisé sans autre purification. F = 100-4 °C.
I.R. (KBr) : $\nu$ (NH) = 3250 $cm^{-1}$ ; (C = 0) = 1710 $cm^{-1}$ ; (C = O) = 1690 $cm^{-1}$ ; ($SO_2$) = 1355 $cm^{-1}$ ; ($SO_2$) = 1170 $cm^{-1}$.
R.M.N.($CDCl_3$) : $\delta$ = 1,25 (3H, t, J = 6,75 Hz) ; 1,1 - 1,8 (8H, m) ; 2,5 - 2,9 (6H, m, dont un triplet, J = 6 Hz) ; 3,25 (2H,t, J = 6 Hz) ; 4,1 (2H, q, J = 6,75 Hz) ; 4,9 (1H, m, échangeable avec $CF_3COOD$) ; 7,0 - 7,25 (2H, m) ; 7,3 - 7,6 (2H, m) ; 7,6 - 7,9 (4H, m).

b) Acide 4-[4-[[1-[[[(4-chlorophényl)sulfonyl]amino]méthyl]cyclopentyl]méthyl]phényl]-4-oxobutanoïque

A une solution de 10 g (20,2 mmoles) du composé préparé dans l'exemple 30 a et de 200 ml d'éthanol 96 °, on ajoute 2,2 g (39,2 mmoles) d'hydroxyde de potassium dissous dans 100 ml d'eau. Le mélange est agité 4 heures à 60 °C, avant d'être concentré à sec sous pression réduite. Le résidu solide obtenu est repris par l'eau, lavé par l'acétate d'éthyle, puis coulé goutte à goutte sur une solution d'acide chlorhydrique dilué. Le précipité blanc formé est filtré, essoré et séché sous vide. Le produit est purifié par chromatographie sur colonne de silice dans un mélange dichlorométhane-méthanol (19:1) puis par 2 recristallisations dans le toluène. On obtient 5,2 g (Rdt = 55,9 %) d'un solide blanc. F = 139 - 42 °C.

| Analyse centésimale : $C_{23}H_{26}ClNO_5S$ (M = 463,975) | | | | | |
|---|---|---|---|---|---|
| | C % | H % | Cl % | N % | S % |
| Calculé | 59,54 | 5,65 | 7,64 | 3,02 | 6,91 |
| Trouvé | 59,39 | 5,81 | 7,62 | 2,93 | 7,17 |

I.R. (KBr) : $\nu$ (NH) = 3300 $cm^{-1}$ ; (C = 0) = 1690 $cm^{-1}$ ; (C = 0) = 1670 $cm^{-1}$ ; ($SO_2$) = 1330 $cm^{-1}$ ; ($SO_2$) = 1150 $cm^{-1}$.
R.M.N.(acétone $d_6$) : $\delta$ = 1,25 - 1,7 (8H, m) ; 2,4 - 2,95 (4H, m) ; 2,75 (2H, d, J = 6,75 Hz, se transforme en singulet avec $CF_3COOD$) ; 3,3 (2H, t, J = 6 Hz) ; 6,4 (1H, t, J = 6,75 Hz, échangeable avec $CF_3COOD$) ; 7,0 - 7,95 (8H, m) ; 10,25 (1H, s large, échangeable avec $CF_3COOD$).

**Exemple 31**

**4-Chloro-N-[[1-[[4-(4,5-dihydro-3-oxo-2H-pyridazin-6-yl)phényl]méthyl]cyclopentyl]méthyl]-benzènesulfonamide**

A une solution de 3 g (6,5 mmoles) du composé préparé dans l'exemple 30 b et de 40 ml d'acide acétique, on ajoute 1,8 g (35,9 mmoles) d'hydrate d'hydrazine. Le mélange est agité à reflux pendant 6 heures. Après refroidissement, le milieu réactionnel est dilué par l'eau, puis extrait par le dichlorométhane. La phase organique est lavée par la soude, puis par l'eau, séchée sur $Na_2SO_4$ et concentrée à sec, pour donner un solide beige. On purifie le produit par recristallisation dans le chloroforme, puis dans un mélange chloroform-heptane pour obtenir 1,1 g (Rdt = 37,9 %) d'un solide blanc. F = 187 - 8 °C.

| Analyse centésimale : $C_{23}H_{26}ClN_3O_3S$ + 0,5 $CHCl_3$ (M = 465,965) | | | | | |
|---|---|---|---|---|---|
| | C % | H % | Cl % | N % | S % |
| Calculé | 59,41 | 5,63 | 8,75 | 9,02 | 6,88 |
| Trouvé | 59,49 | 5,59 | 8,49 | 9,30 | 6,93 |

I.R. (KBr) : $\nu$ (NH) = 3270 $cm^{-1}$ ; (C = 0) = 1670 $cm^{-1}$ : (C = N) = 1645 $cm^{-1}$ ; ($SO_2$) = 1335 $cm^{-1}$ ; ($SO_2$) = 1140 $cm^{-1}$.
R.M.N.(acétone $d_6$ + DMSO $d_6$) : $\delta$ = 1,25 - 1,8 (8H, m) ; 2,25 - 3,25 (9H, m, dont 1H échangeable avec $CF_3COOD$) ; 7,0 - 7,3 (2H, m) ; 7,4 - 7,7 (4H, m) ; 7,7 - 8,0 (2H, m) ; 10,3 (1H, s large, échangeable avec $CF_3COOD$).

**Exemple 32**

**Acide 4-[[1-[[[(2-chlorophényl)sulfonyl]amino]méthyl]cyclopentyl]méthyl]benzèneacétique**

a) 1-[(4-Acétylphényl)méthyl]cyclopentanecarbonitrile

A une solution de 92,6 g (0,5 mole) de 1-(phénylméthyl)cyclopentanecarbonitrile(préparé selon Campaigne E. et Forsch R.A., J. Org. Chem. (1978) 43, 1044-50) dans 2000 ml de dichlorométhane maintenue à -5°C, on ajoute 78,5 g (1 mole) de chlorure d'acétyle, puis par portions 200 g (1,5 mole) de chlorure d'aluminium. On maintient la température à -5°C pendant 2 heures, puis on laisse remonter à température ambiante pour agiter 16 heures. Le milieu réactionnel est versé dans un mélange glace + eau + acide chlorhydrique et extrait par le dichlorométhane. La phase organique est lavée jusqu'à neutralité, séchée sur $Na_2SO_4$ et concentrée pour donner une huile rouge dans laquelle il reste environ 60 % de produit de départ. Cette huile est alors retraitée dans 2000 ml de dichlorométhane par 78,5 g (1 mole) de chlorure d'acétyle et 200 g (1,5 mole) de chlorure d'aluminium à reflux durant 4 heures. Après refroidissement, le milieu réactionnel est versé dans un mélange glace + eau + acide chlorhydrique, avant d'être extrait par le dichlorométhane. La phase organique séchée sur $Na_2SO_4$ est concentrée pour donner une huile rouge foncée, purifiée par distillation. On obtient 76,5 g (Rdt = 67,6 %) d'une huile jaune qui cristallise. $Eb_{0,4}$ = 120 - 60°C.
Une fraction de ce produit est recristallisée dans un mélange acétate d'éthyle-hexane pour donner un solide blanc. F = 46 - 8°C.

| Analyse centésimale : $C_{15}H_{17}NO$ (M = 227, 305) | | | |
|---|---|---|---|
| | C % | H % | N % |
| Calculé | 79,26 | 7,54 | 6,16 |
| Trouvé | 79,59 | 7,61 | 6,14 |

I.R. (KBr) : $\nu$ (C ≡ N) = 2200 $cm^{-1}$ ; (C = 0) = 1650 $cm^{-1}$.
R.M.N.($CDCl_3$) : $\delta$ = 1,5 - 2,4 (8H, m) ; 2,6 (3H, s) ; 2,9 (2H, s) ; 7,25 - 7,5 (2H, m) ; 7,75 - 8,0 (2H, m).

b) 4-[(1-Cyanocyclopentyl)méthyl]benzèneacétate de méthyle

Obtenu en opérant comme dans l'exemple 5e à partir de 39,9 g (175,5 mmoles) du composé préparé dans l'exemple 32 a, de 126 ml de méthanol dans 630 ml de dichlorométhane, de 99,6 g (702 mmoles) d'éthérate de trifluorure de bore et de 81,6 g (184,2 mmoles) de tétraacétate de plomb en solution dans 200 ml de dichlorométhane. On obtient 44,8 g (Rdt = 99,2 %) d'une huile jaune utilisée sans autre purification.
I.R. (film) : $\nu$ (C ≡ N) = 2260 $cm^{-1}$ ; (C = 0) = 1715 $cm^{-1}$.
R.M.N.($CDCl_3$) : $\delta$ = 1,5 - 2,25 (8H, m) ; 2,8 (2H, s) ; 3,6 (2H, s) ; 3,65 (3H, s) ; 7,15 (4H, s).

c) 4-[[1-(Aminométhyl)cyclopentyl]méthyl]benzèneéthanol

A une suspension à 35°C sous courant d'azote, de 12,5 g (332,9 mmoles) de $LiAlH_4$ dans 242 ml de tétrahydrofurane, on ajoute 25,2 g (97,9 mmoles) du composé préparé dans l'exemple 32 b en solution

dans 121 ml de tétrahydrofurane. Le milieu réactionnel est porté à reflux 6,5 heures. Après refroidissement, on coule avec précaution 68 ml d'eau, puis on dilue par l'éther éthylique avant de filtrer le précipité formé. Le filtrat est dilué par l'eau avant d'être extrait par l'éther. La phase organique, lavée à l'eau, est séchée sur $Na_2SO_4$ et concentrée. L'huile obtenue est reprise par une solution d'HCl diluée, lavée par l'éther. Cette phase aqueuse est basifiée par $Na_2CO_3$, extraite par le dichlorométhane qui est ensuite lavé par l'eau et séché sur $Na_2SO_4$, avant d'être concentré. On obtient 20,6 g (Rdt = 90,1 %) d'une huile jaune utilisée sans autre purification.

Une portion de cette huile est purifiée par chromatographie sur silice dans un mélange chloroforme-méthanol (9:1), puis par distillation pour donner une huile jaune pâle. $Eb_{0,3}$ = 150 - 3°C.

| Analyse centésimale : $C_{15}H_{23}NO$ (233, 355) | | | |
|---|---|---|---|
| | C % | H % | N % |
| Calculé | 77,21 | 9,94 | 6,00 |
| Trouvé | 77,13 | 9,99 | 6,17 |

Les spectres I.R. et R.M.N. sont identiques à ceux obtenus dans l'exemple 26 c.

d) 2-Chloro-N-[[1-[[4-(2-hydroxyéthyl)phényl]méthyl]cyclopentyl]méthyl]benzènesulfonamide

A un mélange de 3 g (12,9 mmoles) du composé préparé dans l'exemple 32 c et de 1,6 g (15,8 mmoles) de triéthylamine dans 30 ml de dichlorométhane sec, maintenu à - 15°C, on ajoute 2,6 g (12,3 mmoles) de chlorure de 2-chlorobenzènesulfonyle commercial dissous dans 30 ml de dichlorométhane. Après avoir agité le milieu réactionnel 3 heures à - 15°C, on le verse sur un mélange glace-eau, avant de l'extraire par le dichlorométhane. La phase organique est lavée par HCl N, rincée à l'eau, séchée sur $Na_2SO_4$ et concentrée. Le solide blanc résiduel, 4,7 g (Rdt = 90,4 %) est utilisé sans autre purification. F = 126 - 8°C. Une fraction purifiée par recristallisations dans un mélange acétate d'éthyle-heptane donne une poudre blanche. F = 127 - 9°C.

| Analyse centésimale : $C_{21}H_{26}ClNO_3S$ (M = 407, 954) | | | | | |
|---|---|---|---|---|---|
| | C % | H % | Cl % | N % | S % |
| Calculé | 61,83 | 6,42 | 8,69 | 3,43 | 7,86 |
| Trouvé | 61,80 | 6,30 | 9,10 | 3,48 | 7,89 |

I.R. (KBr) : $\nu$ (OH) = 3570 $cm^{-1}$ : (NH) = 3300 $cm^{-1}$ ; ($SO_2$) = 1320 $cm^{-1}$ ; ($SO_2$) = 1160 $cm^{-1}$.
R.M.N.($CDCl_3$) : $\delta$ = 1,25 - 1,9 (9H, m, dont 1H échangeable avec $CF_3COOD$) ; 2,6 (2H, s) ; 2,6 (2H, d, J = 6,75 Hz, se transforme en singulet avec $CF_3COOD$) ; 2,8 (2H, t, J = 6,75 Hz) ; 3,8 (2H, t,J = 6,75 Hz); 4,8 (1H, m, échangeable avec $CF_3COOD$) ; 7,1 (4H, s) ; 7,25 - 7,6 (3H, m) ; 7,8 - 8,2 (1H, m).

e) Acide 4-[[1-[[[(2-chlorophényl)sulfonyl]amino]méthyl]cyclopentyl]méthyl]benzèneacétique

Obtenu en opérant comme dans l'exemple 25 g, à partir de 4,2 g (10,3 mmoles) du composé préparé dans l'exemple 32 d, dissous dans 140 ml d'acétone et de 9,6 ml de réactif de Jones (20,5 mmoles). Après deux recristallisations dans un mélange acétate d'éthyle-heptane, on obtient 0,9 g (Rdt = 20,9 %) d'une poudre blanche. F = 147 - 9°C.

| Analyse centésimale : $C_{21}H_{24}ClNO_4S$ (M = 421, 940) | | | | | |
|---|---|---|---|---|---|
| | C % | H % | Cl % | N % | S % |
| Calculé | 59,78 | 5,73 | 8,40 | 3,32 | 7,60 |
| Trouvé | 59,83 | 5,81 | 8,17 | 3,58 | 7,73 |

I.R. (KBr): $\nu$ (NH) = 3280 $cm^{-1}$ ; (C=O) = 1690 $cm^{-1}$ ; ($SO_2$) = 1320 $cm^{-1}$ ; ($SO_2$) = 1155 $cm^{-1}$.
R.M.N.($CDCl_3$) : $\delta$ = 1,1 - 1,8 (8H, m) ; 2,6 (2H, d, J = 6,75 Hz, se transforme en singulet avec $CF_3COOD$)

; 2,6 (2H, s) ; 3,6 (2H, s) ; 5,0 (1H, t, J = 6,75 Hz, échangeable avec $CF_3COOD$) ; 6,5 (1H, s large échangeable avec $CF_3COOD$) ; 7,1 (4H, s); 7,4 - 7,6 (3H, m) ; 7,85 - 8,1 (1H, m).

**Exemples 33 à 51**

Les composés des exemples 33 à 51 :
3-Chloro-N-[[1-[[4-(2-hydroxyéthyl)phényl]méthyl]cyclopentyl]méthyl]-benzènesulfonamide
4-Bromo-N-[[1-[[4-(2-hydroxyéthyl)phényl]méthyl]cyclopentyl]méthyl]-benzènesulfonamide
N-[[1-[[4-(2-Hydroxyéthyl)phényl]méthyl]cyclopentyl]méthyl]-4-iodobenzènesulfonamide
N-[[1-[[4-(2-Hydroxyéthyl)phényl]méthyl]cyclopentyl]méthyl]-4-trifluorométhylbenzènesulfonamide
N-[[1-[[4-(2-Hydroxyéthyl)phényl]méthyl]cyclopentyl]méthyl]-3-trifluorométhylbenzènesulfonamide
4-Cyano-N-[[1-[[4-(2-hydroxyéthyl)phényl]méthyl]cyclopentyl]méthyl]-benzènesulfonamide
N-[[1-[[4-(2-Hydroxyéthyl)phényl]méthyl]cyclopentyl]méthyl]-3-nitrobenzènesulfonamide
2,4-Dichloro-N-[[1-[[4-(2-hydroxyéthyl)phényl]méthyl]cyclopentyl]méthyl]benzènesulfonamide
N-[[1-[[4-(2-Hydroxyéthyl)phényl]méthyl]cyclopentyl]méthyl]-4-(1-méthyléthyl)benzènesulfonamide
4-(1,1-Diméthyléthyl)-N-[[1-[[4-(2-hydroxyéthyl)phényl]méthyl]cyclopentyl]méthyl]-benzènesulfonamide
4-Acétyl-N-[[1-[[4-(2-hydroxyéthyl)phényl]méthyl]cyclopentyl]méthyl]-benzènesulfonamide
N-[[1-[[4-(2-Hydroxyéthyl)phényl]méthyl]cyclopentyl]méthyl]-4-méthylsulfonylbenzènesulfonamide
N-[[1-[[4-(2-Hydroxyéthyl)phényl]méthyl]cyclopentyl]méthyl]-4-trifluorométhoxybenzènesulfonamide
N-[[1-[[4-(2-Hydroxyéthyl)phényl]méthyl]cyclopentyl]méthyl]-4-méthoxybenzènesulfonamide
N-[[1-[[4-(2-Hydroxyéthyl)phényl]méthyl]cyclopentyl]méthyl]-3-méthylquinol-8-ylsulfonamide
N-[[1-[[4-(2-Hydroxyéthyl)phényl]méthyl]cyclopentyl]méthyl]-thien-2-ylsulfonamide
5-Chloro-N-[[1-[[4-(2-hydroxyéthyl)phényl]méthyl]cyclopentyl]méthyl]-thien-2-ylsulfonamide
N-[[1-[[4-(1-[[2-Hydroxyéthyl)phényl]méthyl]cyclopentyl]méthyl]-imidazol-4-ylsulfonamide
N-[[1-[[4-(2-Hydroxyéthyl)phényl]méthyl]cyclopentyl]méthyl]-fur-2-ylsulfonamide
ont été préparés selon l'exemple 32 d, et leurs caractéristiques sont données dans les tableaux Ia à Ie :

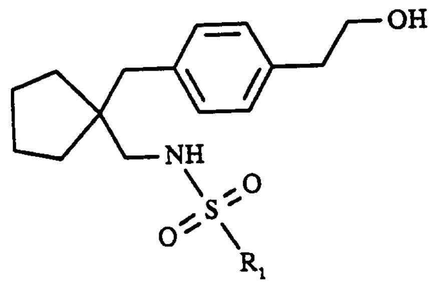

Tableau Ia

| Ex. N° | $R_1$ | Formule brute[a] | F (°C) | RMN ($\delta$,$CDCl_3$) |
|---|---|---|---|---|
| 33 | 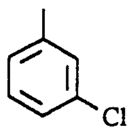 | $C_{21}H_{26}ClNO_3S$ (407, 956) | 94,3 - 95,4 (acétate d'éthyle-hexane) | 1,3 - 1,9 (9H, m, dont 1H échangeable avec $CF_3COOD$) ; 2,6 (2H, s) ; 2,7 (2H, d, J = 6,75 Hz, se transforme en s avec $CF_3COOD$) ; 2,8 (2H, t, J = 6,75 Hz) ; 3,8 (2H, t, J = 6,75 Hz) ; 4,35 (1H, t, J = 6,75Hz, échangeable avec $CF_3COOD$) ; 7,0 (4H, s) ; 7,2 - 7,9 (4H, m) |
| 34 | 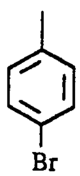 | $C_{21}H_{26}BrNO_3S$ (452, 407) | 138 - 142 (acétate d'éthyle-heptane) | 1,25 - 1,75 (9H, m, dont 1H échangeable avec $CF_3COOD$) ; 2,6 (2H, s) ; 2,7 (2H, d, J = 6,75 Hz, se transforme en s avec $CF_3COOD$) ; 2,8 (2H, t, J = 6,75 Hz) ; 3,8 (2H, t, J = 6,75 Hz) ; 4,4 (1H, t, J = 6,75 Hz, échangeable avec CF3COOD ; 7,0 (4H, s) ; 7,6 (4H, s) |
| 35 | 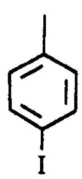 | $C_{21}H_{26}INO_3S$ (499, 407) | 146 - 148 (acétate d'éthyle-heptane) | 1,0 - 1,7 (8H, m) ; 2,6 (4H, m) ; 2,75 (2H, t, J = 6,75 Hz) ; 3,6 (1H, s, échangeable avec $CF_3COOD$) ; 3,7 (2H, t, J = 6,75 Hz) ; 6,45 (1H, m, échangeable avec $CF_3COOD$) ; 7,0 (4H, s); 7,4 - 7,65 (2H, m) ; 7,7 - 7,9 (2H, m) |
| 36 | 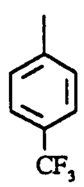 | | 102-103 | 1,25 - 1,85 (8H, m) ; 1,5 (1H, s, échangeable avec $CF_3COOD$) ; 2,6 (2H, s) ; 2,75 (2H, d, J = 6,75 Hz, se transforme en s avec $CF_3COOD$) ; 2,8 (2H, t, J = 6,75 Hz) ; 3,8 (2H, t, J = 6,75 Hz) ; 4,5 (1H, t, J=6,75 Hz, échangeable avec $CF_3COOD$) ; 6,75 - 7,3 (4H, m) ; 7,6 - 8,0 (4H, m) |

[a] Analyse centésimale : C, H, Cl, Br, N, S ± 0,29 sauf exemple 35, S = + 0,6

Tableau Ib

| Ex. N° | $R_1$ | Formule brute[a] | F (°C) | RMN ($\delta$,$CDCl_3$) |
|---|---|---|---|---|
| 37 | 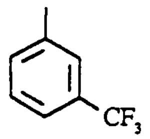 | | 102-103 | 1,25 - 1,8 (8H, m) ; 1,6 (1H, s, échangeable avec $CF_3COOD$) ; 2,6 (2H, s) ; 2,75 (2H, d, J = 6,75 Hz, se transforme en s avec $CF_3COOD$) ; 2,8 (2H, t, J = 6,75 Hz) ; 3,8 (2H, t, J = 6,75 Hz) ; 4,6 (1H, t, J = 6,75 Hz échangeable avec $CF_3COOD$) ; 7,0 (4H, s) ; 7,5 - 8,15 (4H, m) |
| 38 | 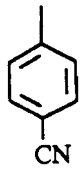 | $C_{22}H_{26}N_2O_3S$ (398,521) | 103 - 104 (acétate d'éthyle -hexane) | 1,3 - 1,8 (8H, m) ; 1,5 (1H, s, échangeable avec $CF_3COOD$) ; 2,55 (2H, s) ; 2,65 (2H, d, J = 6,75 Hz, se transforme en s avec $CF_3COOD$) ; 2,75 (2H, t, J = 6,75 Hz) ; 3,8 (2H, t, J = 6,75 Hz) ; 4,3 (1H, t, J = 6,75 Hz, échangeable avec $CF_3COOD$) ; 7,0 (4H, s) ; 7,55 - 8,0 (4H, m) |
| 39 | 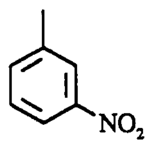 | | huile | 1,25 - 1,8 (8H, m) ; 2,0 (1H, s, échangeable avec $CF_3COOD$) ; 2,6 (2H, s) ; 2,7 (2H, d, J = 6,75 Hz, se transforme en s avec $CF_3COOD$) ; 2,8 (2H, t, J = 6,75 Hz) ; 3,8 (2H, t, J = 6,75 Hz) ; 5,0 (1H, t, J = 6,75 Hz, échangeable avec $CF_3COOD$) ; 7,0 (4H, s) ; 7,45 - 8,65 (4H, m) |
| 40 | 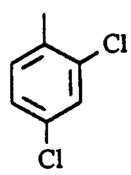 | | 100 | |

[a] Analyse centésimale : C, H, N, S ± 0,11

Tableau Ic

| Ex. N° | $R_1$ | Formule brute[a] | F (°C) | RMN ($\delta$,$CDCl_3$) |
|---|---|---|---|---|
| 41 | 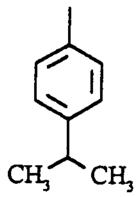 | | huile | 1,05 - 1,75 (8H, m) ; 1,2 (6H, d, J = 6,75 Hz) ; 1,8 (1H, s, échangeable avec $CF_3COOD$) ; 2,55 (2H, s) ; 2,7 (2H, d, J = 6,75 Hz, se transforme en s avec $CF_3COOD$) ; 2,8 (2H, t, J = 6,75 Hz) ; 2,95 (1H, m) ; 3,8 (2H, t, J = 6,75 Hz) ; 4,7 (1H, t, J = 6,75 Hz, avec échangeable avec $CF_3COOD$) ; 6,95 (4H, s) ; 7,15 - 7,4 (2H, m) ; 7,6 - 7,9 (2H, m) |
| 42 | 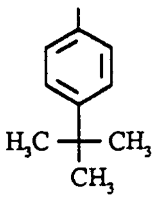 | | 108-111 (acétate d'éthyle-hexane) | 1,25 - 1,75 (8H, m) ; 1,3 (9H, s) ; 1,6 (1H, s, échangeable avec $CF_3COOD$) ; 2,6 (2H, s) ; 2,7 (2H, d, J = 6,75 Hz, se transforme en s avec $CF_3COOD$) ; 2,8 (2H, t, J = 6,75 Hz) ; 3,8 (2H, t, J = 6,75 Hz) ; 4,4 (1H, t, J = 6,75 Hz, échangeable avec $CF_3COOD$) ; 7,0 (4H, s) ; 7,35 - 7,6 (2H, m) ; 7,6 - 7,9 (2H, m) |
| 43 | 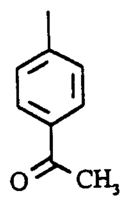 | $C_{23}H_{29}NO_4S$ (415, 548) | 68 - 69,5 (acétate d'éthyle -hexane) | 1,25 - 1,8 (8H, m) ; 1,9 (1H, s, échangeable avec $CF_3COOD$) ; 2,6 (7H, m) ; 2,8 (2H, t, J = 6,75 Hz) ; 3,8 (2H, t, J = 6,75 Hz) ; 4,8 (1H, t, J = 6,75 Hz, échangeable avec $CF_3COOD$) ; 7,0 (4H, s) ; 7,7 - 8,2 (4H, m) |
| 44 | 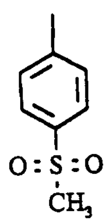 | $C_{22}H_{29}NO_5S_2$ + 0,2 $H_2O$ (455, 207) | 115,5-116,5 (acétate d'éthyle) | 1,25 - 1,8 (8H, m) ; 1,75 (1H, s, échangeable avec $CF_3COOD$) ; 2,6 (2H, s) ; 2,7 (2H, d, J = 6,75 Hz, se transforme en s avec $CF_3COOD$) ; 2,8 (2H, t, J = 6,75 Hz) ; 3,1 (3H, s) ; 3,8 (2H, t, J = 6,75 Hz) ; 4,5 (1H, t, J = 6,75 Hz, échangeable avec $CF_3COOD$) ; 7,0 (4H, m) ; 8,0 (4H, m) |

[a] Analyse centésimale : C, H, N, S ± 0,30

Tableau Id

| Ex. N° | $R_1$ | Formule brute[a] | F (°C) | RMN ($\delta$,$CDCl_3$) |
|---|---|---|---|---|
| 45 | 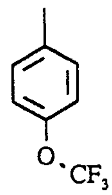 | $C_{22}H_{26}F_3NO_4S$ (457, 507) | 71-73 (acétate d'éthyle--hexane) | 1,25 - 1,75 (8H, m) ; 1,6 (1H, s, échangeable avec $CF_3COOD$) ; 2,6 (2H, s) ; 2,7 (2H, d, J = 6,75 Hz, se transforme en s avec $CF_3COOD$) ; 2,8 (2H, t, J = 6,75 Hz) ; 3,8 (2H, t, J = 6,75 Hz) ; 4,55 (1H, t, J = 6,75 Hz, échangeable avec $CF_3COOD$) ; 7,05 (4H, s) ; 7,1 - 7,4 (2H, m) ; 7,7 - 8,0 (2H, m) |
| 46 | 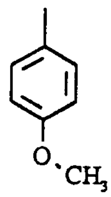 | $C_{22}H_{29}NO_4S$ (403, 537) | 139,5-141,6 (acétate d'éthyle) | 1,25 - 1,75 (8H, m) ; 1,55 (1H, s, échangeable avec $CF_3COOD$) ; 2,5 (2H, s) ; 2,6 (2H, d, J = 6,75 Hz, se transforme en s avec $CF_3COOD$) ; 2,75 (2H, t, J = 6,75 Hz) ; 3,75 (2H, t, J = 6,75 Hz) ; 3,8 (3H, s) ; 4,3 (1H, m, échangeable avec $CF_3COOD$) ; 6,75 - 7,1 (2H, m) ; 7,0 (4H, s) ; 7,55 - 7,8 (2H, m) |
| 47 | 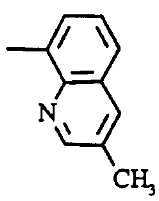 | $C_{25}H_{30}N_2O_3S$ (438, 586) | 140 - 142 (acétate d'éthyle -hexane) | 1,25 - 1,75 (8H, m) ; 1,5 (1H, s, échangeable avec $CF_3COOD$) ; 2,45 - 3,0 (9H, m) ; 3,8 (2H, t, J = 6,75 Hz) ; 6,3 (1H, m, échangeable avec $CF_3COOD$) ; 7,0 (4H, m); 7,3 - 8,4 (4H, m) ; 8,8 (1H, m) |
| 48 | 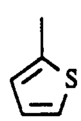 | $C_{19}H_{25}NO_3S_2$ (379, 533) | 119-121 (acétate d'éthyle -hexane) | 1,25 - 1,75 (8H, m) ; 1,55 (1H, s, échangeable avec $CF_3COOD$) ; 2,6 (2H, s) ; 2,7 (2H, d, J = 6,75 Hz, se transforme en s avec $CF_3COOD$) ; 2,8 (2H, t, J = 6,75 Hz) ; 3,8 (2H, t, J = 6,75 Hz) ; 4,5 (1H, t, J = 6,75 Hz, échangeable avec $CF_3COOD$) ; 6,8 - 7,25 (5H, m) ; 7,4 - 7,65 (2H, m) |

[a] Analyse centésimale : C, H, F, N, S ± 0,19 sauf exemple 48, S ± 0,5

Tableau Ie

| Ex. N° | $R_1$ | Formule brute[a] | F (°C) | RMN ($\delta$,$CDCl_3$) |
|---|---|---|---|---|
| 49 | 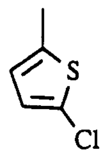 | $C_{19}H_{24}ClNO_3S_2$ (413, 978) | 98 - 100 (acétate d'éthyle -hexane) | 1,25 - 1,75 (8H, m) ; 1,5 (1H, s, échangeable avec $D_2O$) ; 2,6 (2H, s) ; 2,75 (2H, d, J = 6,75 Hz) ; 2,8 (2H, t, J = 6,75 Hz) ; 3,8 (2H, t, J = 6,75 Hz) ; 4,4 (1H, t, J = 6,75 Hz) ; 6,75 - 6,9 (1H, m) ; 7,0 (4H, m) ; 7,1 - 7,35 (1H, m) |
| 50 | 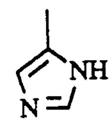 | $C_{18}H_{25}N_3O_3S$ (363,476) | 182 - 184 (éthanol-eau) | (DMSO $d_6$) : 1,1 - 1,75 (8H, m) ; 2,4 - 2,85 (6H, m) ; 3,25 - 3,75 (2H, t, J = 6,75 Hz) ; 4,3-4,7 (1H, m, échangeable avec $CF_3COOD$); 6,9 (4H, s) ; 7,3 (1H, t, J = 6,75 Hz, échangeable avec $CF_3COOD$) ; 7,55 (1H, s) ; 7,8 (1H, s) ; 12,5 (1H, m, échangeable avec $CF_3COOD$) |
| 51 | 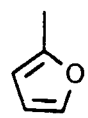 | | huile | 1,25 - 1,8 (9H, m, dont 1H échangeable avec $D_2O$) ; 2,6 (2H, s) ; 2,65 - 3,0 (4H, m) ; 3,8 (2H, t, J = 6,75 Hz) ; 4,6 (1H, m) ; 6,3 - 6,5 (1H, m) ; 6,8 - 7,25 (5H, m) ; 7,4 - 7,65 (1H, m) |

[a] Analyse centésimale : C, H, Cl, N, S ± 0,26 sauf exemple 50, C = - 0,44

## Exemples 52 à 70

Les composés des exemples 52 à 70 :

Acide 4-[[1-[[[(3-chlorophényl)sulfonyl]amino]méthyl]cyclopentyl]méthyl]benzèneacétique
Acide 4-[[1-[[[(4-bromophényl)sulfonyl]amino]méthyl]cyclopentyl]méthyl]benzèneacétique
Acide 4-[[1-[[[(4-iodophényl)sulfonyl]amino]méthyl]cyclopentyl]méthyl]benzèneacétique
Acide 4-[[1-[[[(4-trifluorométhylphényl)sulfonyl]amino]méthyl]cyclopentyl]méthyl]benzèneacétique
Acide 4-[[1-[[[(3-trifluorométhylphényl)sulfonyl]amino]méthyl]cyclopentyl]méthyl]benzèneacétique
Acide 4-[[1-[[[(4-cyanophényl)sulfonyl]amino]méthyl]cyclopentyl]méthyl]benzèneacétique
Acide 4-[[1-[[[(3-nitrophényl)sulfonyl]amino]méthyl]cyclopentyl]méthyl]benzèneacétique
Acide 4-[[1-[[[(2,4-dichlorophényl)sulfonyl]amino]méthyl]cyclopentyl]méthyl]benzèneacétique
Acide 4-[[1-[[[[4-(1-méthyléthyl)phényl]sulfonyl]amino]méthyl]cyclopentyl]méthyl]benzèneacétique
Acide 4-[[1-[[[[4-(1,1-diméthyléthyl)phényl]sulfonyl]amino]méthyl]cyclopentyl]méthyl]benzèneacétique
Acide 4-[[1-[[[(4-acétylphényl)sulfonyl]amino]méthyl]cyclopentyl]méthyl]benzèneacétique
Acide 4-[[1-[[[(4-méthylsulfonylphényl)sulfonyl]amino]méthyl]cyclopentyl]méthyl]benzèneacétique
Acide 4-[[1-[[[(4-trifluorométhoxyphényl)sulfonyl]amino]méthyl]cyclopentyl]méthyl]benzèneacétique
Acide 4-[[1-[[[(4-méthoxyphényl)sulfonyl]amino]méthyl]cyclopentyl]méthyl]benzèneacétique
Acide 4-[[1-[[[(3-méthylquinol-8-yl)sulfonyl]amino]méthyl]cyclopentyl]méthyl]benzèneacétique

Acide 4-[[1-[[[(thien-2-yl)sulfonyl]amino]méthyl]cyclopentyl]méthyl]benzèneacétique
Acide 4-[[1-[[[(5-chlorothien-2-yl)sulfonyl]amino]méthyl]cyclopentyl]méthyl]benzèneacétique
Acide 4-[[1-[[[(imidazol-4-yl)sulfonyl]amino]méthyl]cyclopentyl]méthyl]benzèneacétique
Acide 4-[[1-[[[(fur-2-yl)sulfonyl]amino]méthyl]cyclopentyl]méthyl]benzèneacétique
ont été préparés selon l'exemple 25 g, et leurs caractéristiques sont données dans les tableaux IIa à IIe :

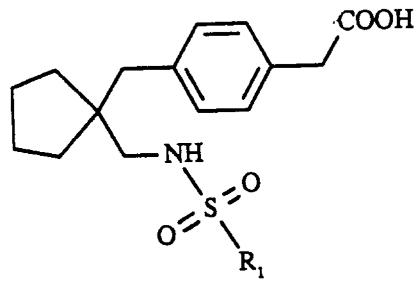

**Tableau IIa**

| Ex. N° | $R_1$ | Formule brute[a] | F (°C) | RMN (δ,acétone $d_6$) |
|---|---|---|---|---|
| 52 | 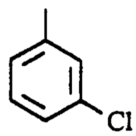 | $C_{21}H_{24}ClNO_4S$ (421, 939) | 121,8-121,9 (acétate d'éthyle-hexane) | 1,35 - 1,75 (8H, m) ; 2,7 (2H, s) ; 2,8 (2H, d, J = 6,75 Hz, se transforme en s avec $CF_3COOD$) ; 3,6 (2H, s) ; 6,45 (1H, t, J = 6,75 Hz, échangeable avec $CF_3COOD$) ; 7,1 (4H, s) ; 7,5 - 7,9 (4H, m) ; 10,45 (1H, s large, échangeable avec $CF_3COOD$) |
| 53 | 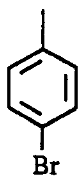 | $C_{21}H_{24}BrNO_4S$ (466, 39) | 149-150 (acétate d'éthyle -heptane) | 1,35 - 1,75 (8H, m) ; 2,6 (2H, s) ; 2,7 (2H, d, J = 6,75 Hz, se transforme en s avec $CF_3COOD$) ; 3,5 (2H, s) ; 6,35 (1H, t, J = 6,75 Hz, échangeable avec $CF_3COOD$) ; 7,05 (4H, s) ; 7,7 (4H, s) ; 10,4 (1H, s large, échangeable avec $CF_3COOD$) |
| 54 | 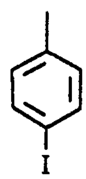 | $C_{21}H_{24}INO_4S$ (513, 40) | 148 - 151 (toluène) | (DMSO $d_6$) : 1,15 - 1,7 (8H, m) ; 2,3 - 2,7 (4H, m) ; 3,45 (2H, s) ; 4,05 (1H, m, échangeable avec $CF_3COOD$) ; 7,0 (4H, s) ; 7,4 - 7,7 (3H, m, dont 1H échangeable avec $CF_3COOD$) ; 7,8 - 8,0 (2H, m) |
| 55 | 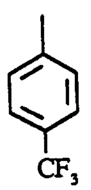 | $C_{22}H_{24}F_3NO_4S$ (455, 491) | 138-140 (acétate d'éthyle -heptane) | 1,25 - 1,85 (8H, m) ; 2,7 (2H, s) ; 2,8 (2H, d, J = 6,75 Hz, se transforme en s avec $CF_3COOD$) ; 3,55 (2H, s) ; 6,5 (1H, t, J = 6,75 Hz, échangeable avec $CF_3COOD$) ; 7,1 (4H, s) ; 7,5 - 8,25 (5H, m, dont 1H échangeable avec $CF_3COOD$) |

[a] Analyse centésimale : C. H. Br. Cl. F. I. N. S ± 0,35 sauf exemples 52 et 53, S = + 0,39 et S = + 0,54

**Tableau IIb**

| Ex. N° | $R_1$ | Formule brute[a] | F (°C) | RMN (δ, acétone $d_6$) |
|---|---|---|---|---|
| 56 | 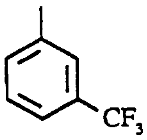 | $C_{22}H_{24}F_3NO_4S$<br>(455, 491) | 100 - 101<br>(acétate d'éthyle-heptane) | 1,35 - 1,75 (8H, m) ; 2,7 (2H, s) ; 2,8 (2H, d, J = 6,75 Hz, se transforme en s avec $CF_3COOD$) ; 3,55 (2H, s) ; 6,5 (1H, t, J = 6,75 Hz, échangeable avec $CF_3COOD$) ; 7,1 (4H, s) ; 7,75 - 8,25 (4H, m) ; 10,0 (1H, s large, échangeable avec $CF_3COOD$) |
| 57 | 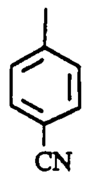 | $C_{22}H_{24}N_2O_4S$<br>+ 1/4 $H_2O$<br>(417, 012) | 155 - 158<br>(acétate d'éthyle -hexane) | ($CDCl_3$ + DMSO $d_6$) : 1,1 - 1,75 (8H, m) ; 2,6 (2H, s); 2,6 (2H, d, J = 6,75 Hz, se transforme en s avec $CF_3COOD$) ; 3,5 (2H, s) ; 6,9 - 7,4 (5H, m, dont 1H échangeable avec $CF_3COOD$) ; 7,5 - 8,2 (4H, m) ; 10,65 (1H, s large, échangeable avec $CF_3COOD$) |
| 58 | 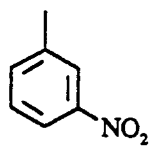 | $C_{21}H_{24}N_2O_6S$<br>+ 1/2 $H_2O$<br>(441,50) | 123 - 128<br>(toluène) | (DMSO $d_6$) : 1,1 - 1,7 (8H, m) ; 2,4 - 2,7 (5H, m, dont 1H échangeable avec $CF_3COOD$) ; 3,45 (2H, s) ; 7,0 (4H, s) ; 7,5 - 8,7 (6H, m, dont 2H échangeables avec $CF_3COOD$) |
| 59 | 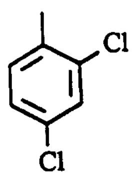 | $C_{21}H_{23}Cl_2NO_4S$<br>(456, 384) | 138 - 140<br>(acétate d'éthyle) | ($CDCl_3$) : 1,0 - 1,75 (8H, m) ; 2,5 (2H, s) ; 2,5 (2H, d, J = 6,75 Hz, se transforme en s avec $CF_3COOD$) ; 3,5 (2H, s) ; 4,95 (1H, t, J = 6,75 Hz, échangeable avec $CF_3COOD$) ; 7,0 (4H, s) ; 7,1 - 7,5 (2H, m) ; 7,65 - 7,95 (1H, m) ; 8,9 (1H, s large, échangeable avec $CF_3COOD$) |

[a] Analyse centésimale : C, H, Cl, F, N, S ± 0,27 sauf exemple 58, S = - 0,43

Tableau IIc

| Ex. N° | $R_1$ | Formule brute[a] | F (°C) | RMN (δ,acétone $d_6$) |
|---|---|---|---|---|
| 60 | 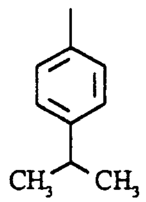 | $C_{24}H_{31}NO_4S$ (429,575) | 141-144 (toluène) | ($DMSO\ d_6$) : 1,1 (6H, d, J = 6,75 Hz) ; 1,0 - 1,65 (8H, m) ; 2,3 - 2,7 (4H, m) ; 2,9 (1H, dq, J = 6,75 Hz) ; 3,4 (2H, s) ; 4,2 (1H, m, échangeable avec $CF_3COOD$) ; 7,0 (4H, s) ; 7,3 - 7,55 (2H, m) ; 7,6 - 7,9 (2H, m) ; 12,2 (1H, s large, échangeable avec $CF_3COOD$) |
| 61 | 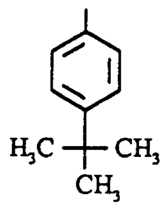 | $C_{25}H_{33}NO_4S$ (443, 602) | 141-143 (acétate d'éthyle -hexane) | ($CDCl_3$) : 1,3 (9H, s) ; 1,0 - 1,75 (8H, m) ; 2,35 - 2,8 (4H, m) ; 3,4 (2H, s) ; 7,0 (4H, s) 7,3 - 7,8 (5H, m, dont 1 H échangeable avec $CF_3COOD$) ; 12,1 (1H, s large, échangeable avec $CF_3COOD$) |
| 62 | 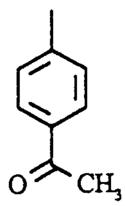 | $C_{23}H_{27}NO_5S$ + 0,3 $H_2O$ (434,94) | 157,5 - 158 (acétate d'éthyle -hexane) | ($CDCl_3$ + $DMSO\ d_6$) : 1,0 - 1,7 (8H, m) ; 2,4 - 2,85 (7H, m) ; 3,5 (2H, s) ; 6,6 (1H, m, échangeable avec $CF_3COOD$) ; 7,0 (4H, s) ; 7,7 - 8,2 (4H, m) ; 8,9 (1H, s large, échangeable avec $CF_3COOD$) |
| 63 | 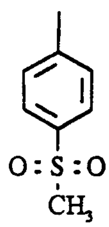 | $C_{22}H_{27}NO_6S_2$ (465, 59) | 176 - 177,5 (acétate d'éthyle) | ($CDCl_3$ + $DMSO\ d_6$) : 1,1 - 1,7 (8H, m) ; 2,4 - 2,75 (5H, m, dont 1H échangeable avec $CF_3COOD$) ; 3,1 (3H, s) ; 3,45 (2H, s) ; 7,0 (4H, s) ; 7,3 (1H, s large, échangeable avec $CF_3COOD$) ; 8,0 (4H, s) |

[a] Analyse centésimale : C, H, N, S ± 0,29

Tableau IId

| Ex. N° | $R_1$ | Formule brute[a] | F (°C) | RMN (δ, acétone $d_6$) |
|---|---|---|---|---|
| 64 | 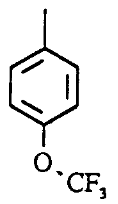 | $C_{22}H_{24}F_3NO_5S$ (471, 49) | 109 - 113 (acétate d'éthyle -pentane) | 1,25-1,75 (8H, m) ; 2,65 (2H, s) ; 2,8 (2H, d, J = 6,75 Hz, se transforme en s avec $CF_3COOD$) ; 3,55 (2H, s) ; 6,4 (1H, t, J = 6,75 Hz, échangeable avec $CF_3COOD$) ; 7,1 (4H, s) ; 7,3 - 7,6 (2H, m) ; 7,8 - 8,1 (2H, m) ; 9,95 (1H, s large, échangeable $CF_3COOD$) |
| 65 | 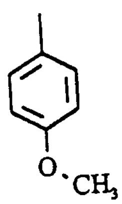 | $C_{22}H_{27}NO_5S$ (417, 53) | 149-152 (acétate d'éthyle -hexane) | 1,25 - 1,7 (8H, m) ; 2,6 (2H, s) ; 2,7 (2H, d, J = 6,75 Hz, se transforme en s avec $CF_3COOD$) ; 3,5 (2H, s) ; 3,8 (3H, s) ; 6,1 (1H, t, J = 6,75 Hz, échangeable avec $CF_3COOD$) ; 6,8 - 7,2 (2H, m) ; 7,0 (4H, s) ; 7,5 - 7,9 (2H, m) ; 9,5 (1H, s large, échangeable avec $CF_3COOD$) |
| 66 | 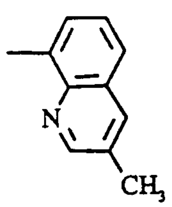 | $C_{25}H_{28}N_2O_4S$ + 1/4 $H_2O$ (457,07) | 219 - 221 (DMF-éthanol) | (DMSO $d_6$) : 1,2 - 1,7 (8H, m) ; 2,4 - 2,8 (7H, m) ; 3,5 (2H, s) ; 7,0 (4H, s) ; 7,4 - 8,4 (5H, m, dont 1 H échangeable avec $CF_3COOD$) ; 8,95 (1H, m) ; 12,2 (1H, s large, échangeable avec $CF_3COOD$) |
| 67 | 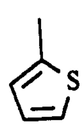 | $C_{19}H_{23}NO_4S_2$ (393, 516) | 138 - 140 (acétate d'éthyle hexane) | (DMSO $d_6$) : 1,0-1,7 (8H, m) ; 2,4 - 2,85 (5H, m, dont 1 H échangeable avec $CF_3COOD$) ; 3,45 (2H, s) ; 6,75 - 7,2 (4H, m) ; 7,4 - 8,0 (3H, m) ; 12,2 (1H, s large, échangeable avec $CF_3COOD$) |

[a] Analyse centésimale : C, H, F, N, S ± 0,32

Tableau IIe

| Ex. N° | $R_1$ | Formule brute[a] | F (°C) | RMN (δ, acétone $d_6$) |
|---|---|---|---|---|
| 68 | 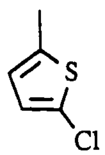 | $C_{19}H_{22}ClNO_4S_2$ (427, 961) | 136 - 138 (acétate d'éthyle -hexane) | (DMSO $d_6$) : 1,25 - 1,75 (8H, m) ; 2,5 - 2,75 (4H, m) ; 3,45 (2H, s) ; 7,0 (4H, s) ; 7,15 (1H, d, J = 4,1 Hz) ; 7,4 (1H, d, J = 4,1 Hz) ; 7,9 (1H, t, J = 6,75 Hz, échangeable avec $CF_3COOD$) ; 12,05 (1H, s, échangeable avec $CF_3COOD$) |
| 69 | 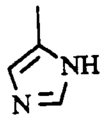 | $C_{18}H_{23}N_3O_4S$ (377, 459) | 176-178 (acétate d'éthyle -hexane) | (DMSO $d_6$) : 1,2 - 1,6 (8H, m) ; 2,4 - 2,7 (4H, m) ; 3,1 - 3,4 (1H, m, échangeable avec $CF_3COOD$) ; 3,45 (2H, s) ; 7,0 (4H, s) ; 7,45 - 7,9 (3H, m, dont 1H échangeable avec $CF_3COOD$) ; 12,2 (1H, s large, échangeable avec $CF_3COOD$) |
| 70 | 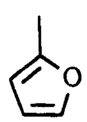 | $C_{19}H_{23}NO_5S$ (377, 455) | 116 (acétate d'éthyle -hexane) | (DMSO $d_6$) : 1,0 - 1,75 (8H, m) ; 2,3 - 2,85 (5H, m, dont 1H échangeable avec $CF_3COOD$) ; 3,5 (2H, s) ; 6,5 - 6,7 (1H, m) ; 7,0 (4H, s) ; 7,75 - 8,1 (2H, m) ; 12,05 (1H, s large, échangeable avec $CF_3COOD$) |

[a] Analyse centésimale : C, H, Cl, N, S ± 0,26

## Exemple 71

### Acide 4-[[1-[[[(4-hydroxyphényl)sulfonyl]amino]méthyl]cyclopentyl]méthylbenzèneacétique

Une solution de 3,6 g (8,6 mmoles) du composé préparé dans l'exemple 65, dans 40 ml de 1,2-dichloroéthane est ajoutée goutte à goutte à un mélange sous azote, de 10,7 g (34,2 mmoles) du complexe tribromure de bore-diméthysulfure et de 40 ml de 1,2-dichloroéthane. Le milieu réactionnel est porté à reflux durant 10 heures. On rajoute 10 g (32 mmoles) du complexe tribomure de borediméthylsulfure, et on porte à nouveau à reflux durant 7 heures. Après refroidissement, le milieu réactionnel est jeté sur glace + eau, et extrait par le dichlorométhane. La phase organique est lavée par une solution de $NaHCO_3$, qui est ensuite acidifiée par HCl, puis extraite par $CH_2Cl_2$. La phase organique est séchée sur $Na_2SO_4$ et concentrée. Le résidu est purifié par chromatographie sur colonne de silice, dans un mélange dichlorométhane-méthanol (98:2 à 95:5), puis par recristallisation dans un mélange acétate d'éthyle-toluène puis dans acétate d'éthyle pour donner 0,15 g (Rdt = 4,3 %) d'une poudre blanc cassé. F = 132 - 5 °C.

| Analyse centésimale : $C_{21}H_{25}NO_5S$ (M = 403,492) | | | | |
|---|---|---|---|---|
| | C % | H % | N % | S % |
| Calculé | 62,51 | 6,25 | 3,47 | 7,95 |
| Trouvé | 62,47 | 6,20 | 3,38 | 8,05 |

I.R. (KBr) : $\nu$ (NH) = 3280 $cm^{-1}$ ; (C = 0) = 1680 $cm^{-1}$ ; ($SO_2$) - 1305 $cm^{-1}$ ; ($SO_2$) = 1145 $cm^{-1}$.
R.M.N.(DMSO $d_6$) : $\delta$ = 1,1 - 1,6 (8H, m) ; 2,3 - 2,7 (4H, m) ; 3,1 (1H, m, échangeable avec $CF_3COOD$) ; 3,45 (2H, s) ; 4,0 (1H, s large, échangeable avec $CF_3COOD$) ; 6,7 - 7,2 (4H, m) ; 7,4 - 7,65 (4H, m) ; 11,0 (1H, s large, échangeable avec $CF_3COOD$).

**Exemple 72**

**Acide 4-[[1-[[[4-chlorophényl)sulfonyl]amino]méthyl]cyclopropyl]méthyl]benzèneacétique**

a) 1-(Phénylméthyl)cyclopropanecarbonitrile

A un mélange sous azote de 26,3 g (260 mmoles) de diisopropylamine et de 300 ml de tétrahydrofurane refroidit à - 75 ° C, on ajoute goutte à goutte 125 ml (200 mmoles) de n-butyllithium en solution 1,6 M dans l'hexane, puis 13,4 g (200 mmoles) de cyclopropanecarbonitrile commercial et enfin 25,3 g (200 mmoles) de chlorure de benzyle. Le milieu réactionnel est agité 2 heures à - 70 ° C puis 2 jours à 20 ° C. On ajoute 4 ml d'eau, avant de laver le milieu réactionnel à l'eau saturée par NaCl. La phase organique séchée sur $Na_2SO_4$ est concentrée pour donner un liquide brun, purifié par distillation. On obtient 16,8 g (Rdt = 53,5 %) d'un liquide incolore. $Eb_{13}$ = 140 - 8 ° C.
Une nouvelle distillation donne un liquide incolore. $Eb_{0,3}$ = 64-8 ° C.

| Analyse centésimale : $C_{11}H_{11}N$ (M = 157,215) | | | |
|---|---|---|---|
| | C % | H % | N % |
| Calculé | 84,04 | 7,05 | 8,91 |
| Trouvé | 83,98 | 7,04 | 8,86 |

I.R. (film) : $\nu$ (C≡N) = 2230 $cm^{-1}$.
R.M.N.($CDCl_3$) : $\delta$ = 0,7 - 1,0 (2H, m) ; 1,1 - 1,4 (2H, m) 2,7 (2H, s) 7,2 (5H, s).

b) [1(Phénylméthyl)cyclopropyl]méthanamine

Obtenu en opérant comme dans l'exemple 15a à partir de 98 g (623 mmoles) du composé préparé dans l'exemple 72a, de 28,4 g (740 mmoles) de $LiAlH_4$ dans 800 ml d'éther. Après distillation, on obtient 82,3 g (Rdt = 81,9 %) d'un liquide incolore. $Eb_{0,3}$ = 75 ° C ($Eb_{0,3}$ = 62 ° C, selon Bumgardner C.L., J. Org. Chem. (1964) 29, 767-8).

| Analyse centésimale : $C_{11}H_{15}N$ (M = 161,246) | | | |
|---|---|---|---|
| | C % | H % | N % |
| Calculé | 81,94 | 9,38 | 8,69 |
| Trouvé | 82,05 | 9,61 | 8,45 |

I.R. (film) : $\nu$ ($NH_2$) = 3360 $cm^{-1}$.
R.M.N.($CDCl_3$) : $\delta$ = 0,25 - 0,5 (4H, m) ; 1,0 (2H, s, échangeable avec $D_2O$) ; 2,4 (2H, s) ; 2,65 (2H, s) ; 7,2 (5H, s).

c) 4-Chloro-N-[[1-(phénylméthyl)cyclopropyl]méthyl]benzènesulfonamide

Obtenu en opérant comme dans l'exemple 1c, à partir de 80,3 g (498 mmoles) du composé préparé dans l'exemple 72 b, de 60,5 g (598 mmoles) de triéthylamine, de 105 g (497 mmoles) de chlorure de 4-chlorobenzènesulfonyle dans 875 ml de dichlorométhane. Après recristallisation dans un mélange acétate d'éthyle-hexane, on obtient 145,8 g (Rdt = 87,2 %) d'une poudre blanche. F = 105,7 - 6,4 °C.

| Analyse centésimale : $C_{17}H_{18}ClNO_2S$ (M = 335,848) | | | | | |
|---|---|---|---|---|---|
| | C % | H % | Cl % | N % | S % |
| Calculé | 60,80 | 5,40 | 10,56 | 4,17 | 9,55 |
| Trouvé | 61,10 | 5,53 | 10,72 | 4,34 | 9,36 |

I.R. (KBr): $\nu$ (NH) = 3220 $cm^{-1}$ ; ($SO_2$) = 1315 $cm^{-1}$ ; ($SO_2$) = 1145 $cm^{-1}$.
R.M.N.($CDCl_3$) : $\delta$ = 0,3 - 0,6 (4H, m) ; 2,6 (2H, s) ; 2,7 (2H, d, J = 6 Hz, se transforme en singulet avec $CF_3COOD$) ; 4,9 (1H, t, J = 6 Hz, échangeable avec $CF_3COOD$) ; 6,9 - 7,3 (5H, m) ; 7,3 - 7,5 (2H, m) ; 7,6 - 7,9 (2H, m).

d) N-[[1-[(4-Acétylphényl)méthyl]cyclopropyl]méthyl]-4-chlorobenzènesulfonamide

Obtenu en opérant comme dans l'exemple 17b, à partir de 10 g (29,7 mmoles) du composé préparé dans l'exemple 72c, de 3,0 g de chlorure d'acétyle (38,4 mmoles) et de 19,8 g (148,5 mmoles) de chlorure d'aluminium dans 200 ml de 1,2-dichloroéthane. Après purification par chromatographie sur colonne de silice avec un mélange acétate d'éthyl-hexane, on obtient 3 g (Rdt = 23,2 %) d'un solide pâteux.
I.R. (KBr) : $\nu$ (NH) = 3280 $cm^{-1}$ ; (C=0) = 1870 $cm^{-1}$ ; ($SO_2$) = 1155 $cm^{-1}$.
R.M.N.($CDCl_3$) : $\delta$ = 0 - 0,1 (4H, m) ; 2,6 (3H, s) ; 2,65 - 2,8 (4H, m) ; 4,7 (1H, t, J = 6,75 Hz, échargeable avec $CF_3COOD$) ; 6,7 - 8,0 (8H, m).

e) 4-[[1-[[[(4-Chlorophényl)sulfonyl]amino]méthyl]cyclopropyl]méthyl]benzèneacétate de méthyle

Obtenu en opérant comme dans l'exemple 5e, à partir de 3 g (7,9 mmoles) du composé préparé dans l'exemple 72 d, de 3,6 ml de méthanol, de 6,7 g (47,6 mmoles) d'éthérate de trifluorure de bore, de 5,3 g (11,9 mmoles) de tétraacétate de plomb dans 52 ml de dichlorométhane. Après purification par chromatographie sur colonne de silice dans un mélange hexane-acétate d'éthyle, on obtient 1,5 g (Rdt = 43,7 %) d'un solide blanc. F = 97,2 - 9,6 °C.
I.R. (KBr) : $\nu$ (NH) = 3260 $cm^{-1}$ ; (C=0) = 1710 $cm^{-1}$ ; ($SO_2$) = 1315 $cm^{-1}$ ; ($SO_2$) = 1155 $cm^{-1}$.
R.M.N. ($CDCl_3$) : $\delta$ = 0,25 - 0,5 (4H, m) ; 2,5 (2H, s) ; 2,7 (2H, d, J = 6 Hz, se transforme en singulet avec $CF_3COOD$) ; 3,55 (2H, s) ; 3,65 (3H, s) ; 4,65 (1H, t, J = 6 Hz, échangeable avec $CF_3COOD$) ; 6,75 - 7,2 (4H, m) ; 7,25 - 7,5 (2H, m) ; 7,5 - 7,8 (2H, m).

f) Acide 4-[[1-[[[(4-chlorophényl)sulfonyl]amino]méthyl]cyclopropyl]méthyl]benzèneacétique

Un mélange de 1,4 g (3,4 mmoles) de l'ester préparé dans l'exemple 72 e, de 22 ml de méthanol, de 0,38 g (9,5 mmoles) de soude en pastilles et de 22 ml d'eau, est porté à reflux 1,5 heure. Après refroidissement, concentration à sec et reprise à l'eau, on lave par l'éther et on acidifie la phase aqueuse par HCl. Le précipité blanc formé est lavé à l'eau, puis séché sous vide à 80 °C. On purifie le solide obtenu par recristallisation dans un mélange acétate d'éthyle-hexane pour obtenir 0,7 g (Rdt = 51,8 %) d'une poudre blanche. F = 160,3 - 2,4 °C.

| Analyse centésimale : $C_{19}H_{20}ClNO_4S$ (M = 393,855) | | | | | |
|---|---|---|---|---|---|
| | C % | H % | Cl % | N % | S % |
| Calculé | 57,94 | 5,12 | 9,00 | 3,56 | 8,14 |
| Trouvé | 58,07 | 4,96 | 8,74 | 3,69 | 7,74 |

I.R. (KBr) : ν (NH) = 3250 $cm^{-1}$ ; (C = 0) = 1695 $cm^{-1}$ ; ($SO_2$) = 1315 $cm^{-1}$ ; ($SO_2$) = 1145 $cm^{-1}$.
R.M.N.(acétone $d_6$) : δ = 0,4 - 0,6 (4H, m) ; 2,65 (2H, s) ; 2,75 (2H, d, J = 6 Hz, se transforme en singulet avec $CF_3COOD$) ; 3,6 (2H, s) ; 6,55 (1H, t, J = 6 Hz, échangeable avec $CF_3COOD$) ; 6,9 - 7,2 (4H, m) ; 7,4 - 7,9 (4H, m) ; 10,5 (1H, s large, échangeable avec $CF_3COOD$).

## Exemple 73

### Acide 4-[[1-[[[(4-chlorophényl)sulfonyl]amino]méthyl]cycloheptyl]méthyl]benzèneacétique

a) 1-(Phénylméthyl)cycloheptanecarbonitrile

Obtenu en opérant comme dans l'exemple 27 b, à partir de 195 mmoles de diisopropylamine lithiée (25,6 g (252,9 mmoles) de diisopropylamine traités par 122 ml (195 mmoles) de n-butyllithium 1,6 M dans l'hexane) dans 300 ml de tétrahydrofurane sec, de 24 g (195 mmoles) de cycloheptanecarbonitrile commercial et de 24,6 g (195 mmoles) de chlorure de benzyle. Après distillation, on obtient 23,2 g (Rdt = 55,8 %) d'un liquide visqueux jaune. $Eb_{16}$ = 190 - 7 °C.
I.R. (film) : ν (C≡N) = 2225 $cm^{-1}$.
R.M.N.($CDCl_3$) : δ = 1,25 - 2,25 (12H, m) ; 2,75 (2H, s) ; 7,2 (5H, s).

b) 1-[(4-Acétylphényl)méthyl]cycloheptanecarbonitrile

Obtenu en opérant comme dans l'exemple 32a, à partir de 23 g (107,8 mmoles) du composé préparé dans l'exemple 73a, de 17 g (216,5 mmoles) de chlorure d'acétyle et de 43,1 g (323,2 mmoles) de chlorure d'aluminium, dans 500 ml de dichlorométhane. Après distillation, on obtient 17,5 g (Rdt = 63,6 %) d'une huile très épaisse jaune pâle. $Eb_{0,55}$ = 178 °C.
I.R. (film) : ν (C≡N) = 2220 $cm^{-1}$ ; (C=0) = 1670 $cm^{-1}$.
R.M.N.($CDCl_3$) : δ = 1,25 - 2,25 (12H, m) ; 2,5 (3H, s) ; 2,8 (2H, s) ; 7,15 - 7,5 (2H, m) ; 7,7 - 8,0 (2H, m).

c) 1-[[4-(2,5,5-Triméthyl-1,3-dioxan-2-yl)phényl]méthyl]cycloheptanecarbonitrile

Un mélange de 5 g (19,6 mmoles) du composé préparé dans l'exemple 73 b, de 3,3 g (31,7 mmoles) de 2,2-diméthyl-1,3-propanediol de 0,1 g (0,6 mmoles) d'acide para-toluènesulfonique et de 20 ml de toluène, est porté à reflux 8 heures dans un ballon surmonté d'un séparateur dit de "Dean-Stark" permettant d'éliminer l'eau formée au cours de la réaction. Après concentration à sec, le milieu réactionnel est repris par $CH_2Cl_2$, lavé par $H_2O$ et séché sur $Na_2SO_4$. Après concentration, on obtient 5,6 g (Rdt = 83,8 %) d'une huile brun clair, qui cristallise lentement, et que l'on utilise sans autre purification.
I.R. (film) : ν (C≡N) = 2225 $cm^{-1}$.
R.M.N.($CDCl_3$) : δ = 0,6 (3H, s) ; 1,25 (3H, s) ; 1,5 (3H, s) ; 1,4 - 2,4 (12H, m) ; 2,8 (2H, s) ; 3,4 (4H, s) ; 7,3 (4H, s).

d) 1-[[4-(2,5,5-Triméthyl-1,3-dioxan-2-yl)phényl]méthyl]cycloheptaneméthanamine

Obtenu en opérant comme dans l'exemple 15a, à partir de 5,5 g (16,1 mmoles) du composé préparé dans l'exemple 73 c, de 0,73 g (19,2 mmoles) de $LiAlH_4$ dans 70 ml d'éther anhydre. On obtient 4,9 g (Rdt = 88,1 %) d'huile orange utilisée sans autre purification.
I.R. (film) : ν ($NH_2$) = 3360 $cm^{-1}$.
R.M.N.($CDCl_3$) : δ = 0,6 (3H, s) ; 1,3 (3H, s) ; 1,2 - 1,9 (17H, m, dont 2H échangeables avec $D_2O$) ; 2,4 (2H, s) ; 2,6 (2H,s) ; 3,4 (4H, s) ; 6,9 - 7,6 (4H, m).

e) 4-Chloro-N-[[1-[[4-(2,5,5-triméthyl-1,3-dioxan-2-yl)phényl]méthyl]cycloheptyl]méthyl]-benzènesulfonamide

Obtenu en opérant comme dans l'exemple 1c, à partir de 4,8 g (13,9 mmoles) du composé préparé dans l'exemple 73d, de 1,7 g (16,8 mmoles) de triéthylamine, de 2,9 g (13,7 mmoles) de chlorure de 4-chlorobenzènesulfonyle dans 25 ml de dichlorométhane sec. Après agitation 23 heures à température ambiante, traitement usuel et purification par chromatographie sur colonne de silice avec un mélange acétate d'éthyle-hexane (4:1), on obtient 4,4 g (Rdt = 61,7 %) d'un solide pâteux.
I.R. (KBr) : ν (NH) = 3270 $cm^{-1}$ ; ($SO_2$) = 1320 $cm^{-1}$ ; ($SO_2$) = 1155 $cm^{-1}$.
R.M.N.($CDCl_3$) : δ = 0,8 (3H, s) ; 1,2 (3H, s) ; 1,1 - 1,8 (15H, m) ; 2,5 (2H, s) ; 2,6 (2H, d, J = 6,75 Hz, se

transforme en singulet avec $CF_3COOD$) ; 3,3 (4H, s) ; 4,3 (1H, t, J = 6,75 Hz, échangeable avec $CF_3COOD$) ; 6,8 - 7,9 (8H, m).

f) N-[[1-[(4-Acétylphényl)méthyl]cycloheptyl]méthyl]-4-chlorobenzènesulfonamide

Un mélange de 1 g (1,9 mmoles) du composé préparé dans l'exemple 73e, de 1,5 ml d'HCl 10,7 N, de 0,9 ml d'eau et de 5 ml d'isopropanol, est porté à reflux durant 4 heures. Après refroidissement et concentration à sec, le résidu est repris par l'eau et extrait par le dichlorométhane. La phase organique, séchée sur $Na_2SO_4$ est concentrée et purifiée par chromatographie sur colonne de silice dans mélange acétate d'éthyle-hexane (5:1) pour donner 0,6 g (Rdt : 72,3 %) d'un solide blanc pâteux.
I.R. (KBr) : $\nu$ (NH) = 3270 $cm^{-1}$ ; (C=0) = 1660 $cm^{-1}$ ; ($SO_2$) = 1315 $cm^{-1}$ ; ($SO_2$) = 1175 $cm^{-1}$.
R.M.N.($CDCl_3$) : $\delta$ = 1,0 - 1,6 (12 H, m) ; 2,5 (3H, s) ; 2,6 (2H,s) ; 2,65 (2H, d, J = 6,75 Hz, se transforme en singulet avec $CF_3COOD$) ; 4,9 (1H, t, J = 6,75 Hz, échangeable avec $CF_3COOD$) ; 7,0 - 7,25 (2H, m) ; 7,3 - 7,6 (2H, m) ; 7,65 - 7,9 (4H, m).

g) 4-[[1-[[[(4-(Chlorophényl)sulfonyl]amino]méthyl]cycloheptyl]méthyl]benzèneacétate de méthyle

Obtenu en Opérant comme dans l'exemple 5e, à partir de 2,2 g (5,1 mmoles) du composé préparé dans l'exemple 73 f, de 2,3 ml de méthanol, de 4,3 g (30 mmoles) d'éthérate de trifluorure de bore, de 3,4 g (7,7 mmoles) de tétraacétate de plomb dans 50 ml de dichlorométhane sec. On obtient 2,2 g (Rdt = 93,6 %) d'un solide blanc utilisé sans autre purification. F = 158,1 - 60,9 ° C.
I.R. (KBr) : $\nu$ (NH) = 3250 $cm^{-1}$ ; (C=0) = 1705 $cm^{-1}$ ; ($SO_2$) = 1345 $cm^{-1}$ ; ($SO_2$) = 1155 $cm^{-1}$.
R.M.N.($CDCl_3$) : $\delta$ = 1,2 - 1,6 (12 H, m) ; 2,5 (2H, s) ; 2,7 (2H, d, J = 6,75 Hz, se transforme en singulet avec $CF_3COOD$) ; 3,6 (2H, s) ; 3,7 (3H, s) ; 4,45 (1H, t, J = 6,75 Hz, échangeable avec $CF_3COOD$) ; 6,8 - 7,3 (4H, m) ; 7,35 - 7,6 (2H, m) ; 7,65 - 7,9 (2H, m).

h) Acide 4-[[1-[[[(4-chlorophényl)sulfonyl]amino]méthyl]cycloheptyl]méthyl]benzèneacétique

Obtenu en opérant comme dans l'exemple 72 f, à partir de 2 g (4,3 mmoles) du composé préparé dans l'exemple 73 g, de 28 ml de méthanol, de 0,48 g (12 mmoles) de soude en pastilles et de 28 ml d'eau. Après 2 recristallisations dans un mélange acétate d'éthyle-hexane, on obtient 0,3 g (Rdt = 15,5 %) d'une poudre blanche. F = 127,8 - 9,6 ° C.

| Analyse centésimale : $C_{23}H_{28}ClNO_4S$ (M = 449,992) | | | | | |
|---|---|---|---|---|---|
| | C % | H % | Cl % | N % | S % |
| Calculé | 61,39 | 6,27 | 7,88 | 3,11 | 7,12 |
| Trouvé | 61,70 | 6,27 | 7,94 | 3,16 | 6,98 |

I.R. (KBr) : $\nu$ (NH) = 3210 $cm^{-1}$ ; (C = 0) = 1690 $cm^{-1}$ ; ($SO_2$) = 1315 $cm^{-1}$ ; ($SO_2$) = 1150 $cm^{-1}$.
R.M.N.(acétone $d_6$) : $\delta$ = 1,25 - 1,6 (12H, m) ; 2,6 (2H, s) ; 2,7 (2H, d, J = 6,75 Hz, se transforme en singulet avec $CF_3COOD$) ; 3,55 (2H, s) ; 6,3 (1H, t, J = 6,75 Hz, échangeable avec $CF_3COOD$) ; 7,1 (4H, s) ; 7,4 - 7,9 (4H, m) ; 10,25 (1H, s large, échangeable avec $CF_3COOD$).

**Exemple 74**

**4-Chloro-N-[[1-[[4-[2-(morpholin-4-yl)-2-oxoéthyl]phényl]méthyl]cyclopentyl]méthyl]-benzènesulfonamide**

a) Chlorure de l'acide 4-[[1-[[[(4-chlorophényl)sulfonyl]amino]méthyl]cyclopentyl]méthyl]benzèneacètique

Un mélange de 17,1 g (40.5 mmoles) du composé préparé dans l'exemple 19 et de 171 ml (2360 mmoles) de chlorure de thionyle est porté à reflux durant 4 heures. Après refroidissement et concentration, le solide jaune obtenu est lavé par l'heptane puis essoré pour donner 17,0 g (Rdt = 95,3 %) d'un solide jaune utilisé sans autre purification. F = 90 - 5 ° C.
I.R. (KBr) : $\nu$ (NH) = 3270 $cm^{-1}$ ; (C=0) = 1775 $cm^{-1}$ ; ($SO_2$) = 1325 $cm^{-1}$ ; ($SO_2$) = 1155 $cm^{-1}$.
R.M.N.($CDCl_3$) : $\delta$ = 1,15 - 1,9 (8H, m) ; 2,6 (2H, s) ; 2,6 - 2,9 (2H, m, se transforme en singulet avec

$CF_3COOD$) ; 4,1 (2H, s) ; 4,8 (1H, m, échangeable avec $CF_3COOD$) ; 7,1 (4H, s) ; 7,3 - 7,6 (2H, m) ; 7,65 - 8,0 (2H, m).

b) 4-Chloro-N-[[1-[[4-[2-(morpholin-4-yl)-2-oxoéthyl]phényl]méthyl]cyclopentyl]méthyl]-benzènesulfonamide

Une solution de 2,7 g (6,1 mmoles) du composé préparé dans l'exemple 74 a dans 27 ml de dichlorométhane est ajoutée goutte à goutte à une solution composée de 1,6 g (18,3 mmoles) de morpholine et de 160 ml de dichlorométhane. Après avoir agité 22 heures à température ambiante, le milieu réactionnel est jeté sur un mélange eau-HCl et extrait par le dichlorométhane. La phase organique, lavée à l'eau et séchée sur $Na_2SO_4$ est concentrée sous vide. Le résidu est recristallisé dans un mélange acétate d'éthyle-hexane pour donner 0,7 g (Rdt = 23,3 %) d'un solide blanc. F = 157 - 9 °C.

| Analyse centésimale : $C_{25}H_{31}ClN_2O_4S$ (M = 491,046) | | | | | |
|---|---|---|---|---|---|
| | C % | H % | Cl % | N % | S % |
| Calculé | 61,15 | 6,36 | 7,22 | 5,70 | 6,53 |
| Trouvé | 61,35 | 6,50 | 7,25 | 5,79 | 6,90 |

I.R. (KBr) : $\nu$ (NH) = 3160 $cm^{-1}$ ; (C=0) = 1630 $cm^{-1}$ ; ($SO_2$) = 1325 $cm^{-1}$ ; ($SO_2$) = 1160 $cm^{-1}$.
R.M.N.($CDCl_3$) : $\delta$ = 1,15 - 1,75 (8H, m) ; 2,55 (2H, s) ; 2,65 (2H, d, J = 6,75 Hz, se transforme en singulet avec $CF_3COOD$); 3,25 - 3,75 (10H, m) ; 4,5 (1H, t, J = 6,75 Hz, échangeable avec $CF_3COOD$) ; 7,0 (4H, s) ; 7,25 - 7,5 (2H, m) ; 7,6 - 7,8 (2H, m).

**Exemple 75**

**4-[[1-[[[(4-Chlorophényl)sulfonyl]amino]méthyl]cyclpentyl]méthyl]benzèneacétamide**

Un mélange de 3,1 g (7,1 mmoles) du chlorure d'acide préparé dans l'exemple 74a et de 70 ml d'une solution aqueuse d'ammoniaque à 22 % (d = 0,91) est agité 3 jours à température ambiante. Le précipité formé est filtré, lavé à l'eau et séché à 50 °C. Après 2 recristallisations dans l'acétate d'éthyle, on obtient 1,0 g (Rdt = 33,3 %) d'un solide blanc. F = 151 - 3 °C.

| Analyse centésimale : $C_{21}H_{25}ClN_2O_3S$ (M = 420,95) | | | | | |
|---|---|---|---|---|---|
| | C % | H % | Cl % | N % | S % |
| Calculé | 59,92 | 5,99 | 8,42 | 6,65 | 7,62 |
| Trouvé | 59,62 | 5,96 | 8,38 | 6,67 | 7,58 |

I.R. (KBr) : $\nu$ ($NH_2$) = 3440 $cm^{-1}$ ; (NH) = 3190 $cm^{-1}$ ; (C=0) = 1660 $cm^{-1}$ ; ($SO_2$) = 1320 $cm^{-1}$ ; ($SO_2$) = 1160 $cm^{-1}$.
R.M.N.($CDCl_3$ + DMSO $d_6$) : $\delta$ = 1,25 - 1,8 (8H, m) ; 2,5 - 2,8 (4H, m) ; 3,45 (2H, s) ; 6,4 (1H, t, J = 6,75 Hz, échangeable par $CF_3COOD$) ; 6,5 - 7,0 (2H, s large, échangeables avec $CF_3COOD$) ; 7,1 (4H, s) ; 7,35 - 7,55 (2H, m) ; 7,6 - 7,9 (2H, m).

**Exemple 76**

**Acide 3-[[2-[4[[1-[[[(4-chlorophényl)sulfonyl]amino]méthyl]cyclopentyl]méthyl]phényl]-1-oxoéthyl]-amino]propanoique**

a) 3-[[2-[4-[[1-[[[(4-Chlorophényl)sulfonyl]amino]méthyl]cyclopentyl]méthyl]phényl]-1-oxoéthyl]amino]-propanoate d'éthyle

Sur un mélange de 2,4 g (15 mmoles) de chlorhydrate de 3-aminopropanoate d'éthyle commercial, de 3,3 g (33 mmoles) de triéthylamine et de 150 ml de dichlorométhane, on coule 6,6 g (15 mmoles) du chlorure d'acide préparé dans l'exemple 74a dissous dans 80 ml de dichlorométhane. Après agitation 24 heures à température ambiante, le milieu réactionnel est jeté sur un mélange eau-HCl. La phase organique

est décantée, lavée par une solution d'HCl dilué, puis par une solution de $NaHCO_3$ et par l'eau avant d'être séchée sur $Na_2SO_4$. Après concentration, le résidu obtenu est purifié par chromatographie sur colonne de silice par un mélange acétate d'éthyle-hexane (2:1) pour donner 3,0 g (Rdt = 38,4 %) d'un solide blanc cassé. F = 128 - 30 °C.

I.R. (KBr) : $\nu$ (NH) = 3375 $cm^{-1}$ ; (NH) = 3200 $cm^{-1}$ ; (C=0) = 1720 $cm^{-1}$ ; (C=0) = 1650 $cm^{-1}$ ; ($SO_2$) = 1320 $cm^{-1}$ ; (SO2) = 1160 $cm^{-1}$.

R.M.N.($CDCl_3$) : $\delta$ = 1,2 (3H, t, J = 6,75 Hz) ; 1,35 - 1,8 (8H, m) ; 2,5 (2H, t, J = 6,75 Hz) ; 2,6 (2H, s) ; 2,75 (2H, d, J = 6,75 Hz, se transforme en singulet avec $CF_3COOD$) ; 3,4 (2H, t, J = 6,75 Hz) ; 3,5 (2H, s) ; 4,1 (2H, q, J = 6,75 Hz) ; 4,7 (1H, t, J = 6,75 Hz, échangeable avec $CF_3COOD$) ; 6,0 (1H, s large, échangeable avec $CF_3COOD$) ; 7,1 (4H, s) ; 7,35 - 7,6 (2H, m) ; 7,7 - 7,9 (2H, m).

b) Acide 3-[[2-[4-[[1-[[[(4-chlorophényl)sulfonyl]amino]méthyl]cyclopentyl]méthyl]phényl]-1-oxoéthyl]amino]-propanoique

Obtenu en opérant comme dans l'exemple 5 f, à partir de 3,0 g (5,75 mmoles) du composé préparé dans l'exemple 76a, de 0,65 g (11,5 mmoles) de KOH en pastilles, de 30 ml d'éthanol et de 30 ml d'eau. Après 2 recristallisations dans l'acétate d'éthyle, on obtient 1,7 g (Rdt = 60,7 %) d'un solide blanc. F = 148 - 50 °C.

| Analyse centésimale : $C_{24}H_{29}ClN_2O_5S$ (M = 493,018) | | | | | |
|---|---|---|---|---|---|
| | C % | H % | Cl % | N % | S % |
| Calculé | 58,47 | 5,93 | 7,19 | 5,68 | 6,50 |
| Trouvé | 58,22 | 5,91 | 7,30 | 5,64 | 6,58 |

I.R. (KBr) : $\nu$ (NH) = 3400 $cm^{-1}$ ; (NH) = 3240 $cm^{-1}$ ; (C=0) = 1705 $cm^{-1}$ ; (C=0) = 1610 $cm^{-1}$ ; ($SO_2$) = 1325 $cm^{-1}$ ; ($SO_2$) = 1160 $cm^{-1}$.

R.M.N.(DMSO $d_6$) : $\delta$ = 1,1 - 1,8 (8H, m) ; 2,25 - 2,9 (6H, m) ; 3,2 (2H, t, J = 6,75 Hz) ; 3,3 (2H, s) ; 7,1 (4H, s) ; 7,5 - 8,5 (6H, m, dont 2H échangeables avec $CF_3COOD$) ; 12,2 (1H, s, échangeable avec $CF_3COOD$).

**Exemple 77**

**4-[[1-[[[(4-Chlorophényl)sulfonyl]amino]méthyl]cyclopentyl]méthyl]benzèneacétate d'éthyle**

Un mélange de 4,4 g (10 mmoles) du chlorure d'acide préparé dans l'exemple 74a, de 1,2 g (12 mmoles) de triéthylamine dans 50 ml d'éthanol absolu, est agité 16 heures à température ambiante, avant d'être concentré à sec, repris à l'eau et extrait à l'éther. La phase organique est lavée à l'eau, puis par une solution de $NaHCO_3$, avant d'être séchée sur $Na_2SO_4$ et concentrée. Le résidu est purifié par chromatographie sur colonne de silice par un mélange hexane-acétate d'éthyle (1:1), puis recristallisé dans un mélange hexane-acétate d'éthyle pour donner 0,1 g (Rdt = 2,2 %) d'un solide blanc. F = 117 - 9 °C.

| Analyse centésimale : $C_{23}H_{28}ClNO_4S$ (M = 449,993) | | | | | |
|---|---|---|---|---|---|
| | C % | H % | Cl % | N % | S % |
| Calculé | 61,39 | 6,27 | 7,88 | 3,11 | 7,12 |
| Trouvé | 61,30 | 6,27 | 7,90 | 3,27 | 7,16 |

I.R. (KBr) : $\nu$ (NH) = 3250 $cm^{-1}$ ; (C=0) = 1700 $cm^{-1}$ ; ($SO_2$) = 1320 $cm^{-1}$ ; ($SO_2$) = 1155 $cm^{-1}$.

R.M.N.($CDCl_3$) : $\delta$ = 1,25 (3H, t, J = 6,75 Hz) ; 1,3 - 1,8 (8H, m) ; 2,55 (2H, s) ; 2,7 (2H, d, J = 6,75 Hz, se transforme en singulet avec $CF_3COOD$) ; 3,55 (2H, s) ; 4,15 (2H, q, J = 6,75 Hz) ; 4,7 (1H, t, J = 6,75 Hz, échangeable avec $CF_3COOD$) ; 6,8 - 7,25 (4H, m) ; 7,3 - 7,5 (2H, m) ; 7,6 - 7,85 (2H, m).

**Exemple 78**

**4-[[1-[[[(4-Chlorophényl)sulfonyl]amino]méthyl]cyclopentyl]méthyl]benzèneacétate d'éthyle**

Une solution de 2,1 g (5 mmoles) de l'acide préparé dans l'exemple 19, dans 210 ml d'éthanol absolu, est saturé par HCl gaz, puis porté à reflux 5 heures. Après refroidissement et concentration à sec, le solide blanc obtenu est repris par l'éther, lavé à l'eau, séché sur $Na_2SO_4$ et concentré. Après 2 recristallisations dans un mélange hexane-acétate d'éthyle, on obtient 1,4 g (Rdt = 62,2 %) d'un solide blanc, dont les propriétés spectrales sont identiques à celles du produit obtenu dans l'exemple 77. F = 117 - 9 ° C.

**Exemple 79**

**4-[[1-[[[(4-Chlorophényl)sulfonyl]amino]méthyl]cyclopentyl]méthyl]benzèneacétate de 2-(diéthylamino)éthyle**

A une suspension composée de 4,4 g (10 mmoles) du composé préparé dans l'exemple 20, dans 200 ml d'isopropanol, on ajoute 1,4 g (10,3 mmoles) de (2-chloroéthyl)-diéthylamine commerciale. On porte à reflux 4 heures. Après refroidissement et filtration d'un insoluble, on concentre le milieu réactionnel. Le résidu repris à l'éther est lavé à l'eau, séché par $Na_2SO_4$ et concentré. L'huile obtenue est recristallisée dans l'hexane pour donner 0,4 g (Rdt = 7,7 %) d'un solide blanc. F = 80 - 1 ° C.

| Analyse centésimale : $C_{27}H_{37}ClN_2O_4S$ (M = 521,116) | | | | | |
|---|---|---|---|---|---|
| | C % | H % | Cl % | N % | S % |
| Calculé | 62,23 | 7,16 | 6,80 | 5,38 | 6,15 |
| Trouvé | 62,08 | 7,32 | 6,80 | 5,27 | 5,84 |

I.R. (KBr) : $\nu$ (NH) = 3250 $cm^{-1}$ ; (C=0) = 1690 $cm^{-1}$ ; ($SO_2$) = 1335 $cm^{-1}$ ; ($SO_2$) = 1150 $cm^{-1}$.
R.M.N.($CDCl_3$) : $\delta$ = 0,95 (6H, t, J = 7,5 Hz) ; 1,2 - 1,75 (8H, m) ; 2,5 (2H, s) ; 2,6 (2H, d, J = 6,75 Hz, se transforme en singulet avec $CF_3COOD$) ; 2,6 (6H, m) ; 3,55 (2H, s) ; 4,1 (2H, t, J = 6,0 Hz) ; 4,75 (1H, t, J = 6,75 Hz, échangeable avec $CF_3COOD$) ; 6,8 - 7,3 (4H, m) ; 7,35 - 7,55 (2H, m) ; 7,6 - 7,9 (2H, m).

**Exemple 80**

**Acide 4-[[1-[[[(4-chlorophényl)sulfonyl]amino]méthyl]cyclopentyl]méthyl]-benzèneméthanesulfonique**

a) 4-Chloro-N-[[1-[[4-(chlorométhyl)phényl)méthyl]cyclopentyl]méthyl]benzènesulfonamide

Sur un mélange maintenu sous azote, contenant 46,7 g (118,5 mmoles) de l'alcool préparé dans l'exemple 25 f, de 9,1 g (115,5 mmoles) de pyridine séchée sur KOH, et de 467 ml de dichlorométhane sec, on ajoute goutte à goutte en 3 heures, une solution de 33 ml (452,4 mmoles) de chlorure de thionyle et de 230 ml de dichlorométhane sec. Après 2 heures d'agitation à température ambiante, le milieu réactionnel est versé lentement dans une solution aqueuse de $NaHCO_3$ sous forte agitation. L'extraction est réalisée par le dichlorométhane, qui est ensuite lavé par une solution d'HCl dilué, puis par l'eau et séché sur $Na_2SO_4$, avant d'être concentré. Le résidu est purifié par chromatographie sur colonne de silice puis par recristallisation dans un mélange acétate d'éthyle-hexane pour donner 33,5 g (Rdt = 68,7 %) d'une poudre blanche. F = 124-6 ° C.
I.R. (KBr) : $\nu$ (NH) = 3270 $cm^{-1}$ ; ($SO_2$) = 1315 $cm^{-1}$ ; ($SO_2$) = 1155 $cm^{-1}$.
R.M.N.($CDCl_3$) : $\delta$ = 1,25 - 1,8 (8H, m) ; 2,6 (2H, s) ; 2,7 (2H, d, J = 6,75 Hz, se transforme en singulet avec $CF_3COOD$) ; 4,5 (3H, m dont 1H échangeable avec $CF_3COOD$) ; 6,9 - 7,3 (4H, m) ; 7,3 - 7,55 (2H, m) ; 7,6 - 7,9 (2H, m).

b) Acide 4-[[1-[[[(4-chlorophényl)sulfonylamino]méthyl]cyclopentyl]méthyl]benzèneméthanesulfonique

A une solution de 5 g (12 mmoles) du composé préparé dans l'exemple 80 a, de 45 ml d'eau et de 125 ml d'acétone, portée à reflux, on ajoute goutte à goutte une solution composée de 1,5 g de sulfite de

sodium et de 45 ml d'eau. On maintient le reflux 7 heures après la fin de l'addition. Après refroidissement, le milieu réactionnel est acidifié par 70 ml d'HCl 4N et porté à reflux 1 1/4 heure. Après refroidissement et dilution par 100 ml d'eau, on élimine un insoluble. Le filtrat concentré à sec est repris par l'éthanol pour éliminer un insoluble. Le filtrat éthanolique est concentré et purifié par chromatographie sur colonne de silice par un mélange dichlorométhane-méthanol (95:5), puis par recristallisation dans l'isopropanol, pour donner 1,1 g (Rdt = 20,0 %) de poudre blanche. F = 200 - 30 °C.

| Analyse centésimale : $C_{20}H_{24}ClNO_5S$ + 1,1 $H_2O$ (M = 477,812) | | | | | |
|---|---|---|---|---|---|
| | C % | H % | Cl % | N % | S % |
| Calculé | 50,27 | 5,53 | 7,42 | 2,93 | 13,42 |
| Trouvé | 50,63 | 5,15 | 7,17 | 2,73 | 12,98 |

I.R. (KBr) : $\nu$ (NH) = 3280 $cm^{-1}$ ; ($SO_2$) = 1330 $cm^{-1}$ ; ($SO_2$) = 1150 $cm^{-1}$.
R.M.N.($CDCl_3$ + DMSO $d_6$) : $\delta$ = 1,25 - 1,75 (8H, m) ; 2,3 - 2,8 (4H, m) ; 3,9 (2H, s) ; 6,8 - 7,6 (8H, m, dont 2H échangeables avec $CF_3COOD$) ; 7,6 - 7,8 (2H, m).

## Exemple 81

### 4-Chloro-N-[1-[[[4-[(1H-tétrazol-5-yl)méthyl]phényl]méthyl]cyclopentyl]méthyl]benzènesulfonamide

a) 4-Chloro-N-[[1-[[4-(cyanométhyl)phényl]méthyl]cyclopentyl]méthyl]benzènesulfonamide

Un mélange maintenu sous azote, composé de 8,0 g (19,4 mmoles) du composé préparé dans l'exemple 80 a, de 1,45 g (29 mmoles) de cyanure de sodium et de 50 ml d'éthanol 96 °, est porté à reflux 3 1/4 heures. Après concentration à sec, et reprise par l'eau, on extrait par $CH_2Cl_2$. La phase organique est lavée à l'eau saturée par NaCl, séchée sur $Na_2SO_4$ et concentrée. Le résidu est recristallisé dans un mélange acétate d'éthyle-hexane pour donner 4,2 g (Rdt = 53,8 %) d'un solide blanc cassé. F = 128 - 31 °C.
Une portion de ce solide purifiée par chromatographie sur colonne de silice dans un mélange acétate d'éthyle-hexane (1:3) fournit une poudre blanche. F = 134,2 - 4,9 °C.

| Analyse centésimale : $C_{21}H_{23}ClN_2O_2S$ (M = 402,94) | | | | | |
|---|---|---|---|---|---|
| | C % | H % | Cl % | N % | S % |
| Calculê | 62,60 | 5,75 | 8,80 | 6,95 | 7,96 |
| Trouvé | 62,55 | 5,71 | 8,86 | 7,05 | 7,90 |

I.R. (KBr) : $\nu$ (NH) = 3260 $cm^{-1}$ ; (C≡N) = 2260 $cm^{-1}$ ; ($SO_2$) = 1320 $cm^{-1}$ ; ($SO_2$) = 1150 $cm^{-1}$.
R.M.N.($CDCl_3$) : $\delta$ = 1,0 - 1,9 (8H, m) ; 2,6 (2H, s) ; 2,7 (2H, d, J = 6,0 Hz, se transforme en singulet avec $CF_3COOD$) ; 3,65 (2H, s) ; 5,0 (1H, t, J = 6,0 Hz, échangeable avec $CF_3COOD$) ; 7,0 (4H, s) ; 7,25 - 7,55 (2H, m) ; 7,6-7,9 (2H, m).

b) 4-Chloro-N-[1-[[[4-[(1H-tétrazol-5-yl)méthyl]phényl]méthyl]cyclopentyl]méthyl]benzènesulfonamide

Un mélange sous azote, composé de 4,1 g (10,1 mmoles) du composé préparé dans l'exemple 81a, de 2,0 g (30,8 mmoles) d'azoture de sodium, de 2,15 g (15,6 mmoles) de chlorhydrate de triéthylamine et de 100 ml de 1-méthylpyrrolidin-2-one séchée sur tamis, est chauffé 8 heures à 150 °C. Après refroidissement, le milieu réactionnel est concentré à sec, repris par une solution de NaOH 2H, lavé par l'éther avant d'être acidifié par HCl 5N. Le précipité pâteux formé est repris par l'acétate d'éthyle, lavé par l'eau saturée par NaCl et séché sur $Na_2SO_4$. Après concentration, le résidu obtenu est purifiê par chromatographie sur colonne de silice dans un mélange dichlorométhane-méthanol (98:2). Les fractions de tête fournissent un solide beige, qui après 2 recristallisations dans l'éthanol donne 1,0 g (Rdt = 22,2 %) d'une poudre blanche. F = 183 - 5 °C.

| Analyse centésimale : $C_{21}H_{24}ClN_5O_2S$ + 1/4 $H_2O$ (M = 450,47) | | | | | |
|---|---|---|---|---|---|
| | C % | H % | Cl % | N % | S % |
| Calculé | 55,99 | 5,48 | 7,87 | 15,55 | 7,11 |
| Trouvé | 56,03 | 5,67 | 7,91 | 15,52 | 7,26 |

I.R. (KBr) : $\nu$ (NH) = 3305 $cm^{-1}$ ; ($SO_2$) = 1300 $cm^{-1}$ ; ($SO_2$) = 1145 $cm^{-1}$.
R.M.N.(DMSO $d_6$) : $\delta$ = 1,25 - 1,65 (8H, m) ; 2,4 - 2,7 (5H, m, dont 1H échangeable avec $CF_3COOD$) ; 4,2 (2H, s) ; 7,0 (4H, s) ; 7,3 - 7,95 (5H, m, dont 1H échangeable avec $CF_3COOD$).

**Exemple 82**

**4-Amino-N-[1-[[[4-[(1H-tétrazol-5-yl)méthyl]phényl]méthyl]cyclopentyl]méthyl]benzènesulfonamide**

Formé lors de la réaction pour obtenir le composé de l'exemple 81b et isolé lors de la purification chromatographique de ce composé. Une seconde chromatographie sur colonne de silice dans un mélange dichlorométhaneméthanol (98:2) suivie d'une recristallisation dans l'éthanol fournit 0,1 g (Rdt = 2,3 %) d'une poudre blanche. F = 204 - 5 °C.

| Analyse centésimale : $C_{21}H_{26}N_6O_2S$ (M = 426,539) | | | | |
|---|---|---|---|---|
| | C % | H % | N % | S % |
| Calculé | 59,13 | 6,14 | 19,70 | 7,52 |
| Trouvé | 59,18 | 6,04 | 19,75 | 7,35 |

I.R. (KBr) : $\nu$ ($NH_2$) = 3460 $cm^{-1}$ ; ($NH_2$) = 3360 $cm^{-1}$ ;(NH) = 3260 $cm^{-1}$ ; ($SO_2$) = 1300 $cm^{-1}$ ; ($SO_2$) = 1145 $cm^{-1}$.
R.M.N.(acétone $d_6$ + DMSO $d_6$) : $\delta$ = 1,3 - 1,6 (8H, m) ; 2,4 - 2,7 (5H, m, dont 1H échangeable avec $CF_3COOD$) ; 4,2 (2H, s) ; 6,45 - 6,8 (4H, m, dont 2H échangeables avec $CF_3COOD$) ; 7,1 (4H, s) ; 7,25 - 7,65 (3H, dont 1H échangeable avec $CF_3COOD$).

**Exemple 83**

**Acide [[4-[[1-[[[(4-chlorophényl)sulfonyl]amino]méthyl]cyclopentyl]méthyl]phényl]méthyl]-phosphonique**

a) [[4-[[1-[[[(4-Chlorophényl)sulfonyl]amino]méthyl]cyclopentyl]méthyl]phényl]méthyl]phosphonate de diéthyle

Un mélange sous azote de 5 g (12,1 mmoles) du composé préparé dans l'exemple 80a et de 32 ml de triéthylphosphite est chauffé 5 heures à 140 °C. Après refroidissement, on filtre le précipité formé, qui après lavage à l'éther et séchage à l'air donne 4,8 g (Rdt = 77,2 %) d'une poudre blanche utilisée sans autre purification. F = 127 - 32 °C.
I.R. (KBr) : $\nu$ (NH) = 3140 $cm^{-1}$ ; ($SO_2$) = 1330 $cm^{-1}$ ; ($SO_2$) = 1165 $cm^{-1}$.
R.M.N.($CDCl_3$) : $\delta$ = 1,25 (6H, t, J = 6,75 Hz) ; 1,3 - 1,75 (8H, m) ; 2,6 (2H, s) ; 2,7 (2H, d, J = 6,75 Hz, se transforme en singulet avec $CF_3COOD$) ; 3,1 (2H, d, J = 21,75 Hz) ; 4,0 (4H, dq, $J_1$ = 6,75 Hz, $J_2$ = 6,75 Hz) ; 5,2 (1H, t, J = 6,75 Hz, échangeable avec $CF_3COOD$) ; 6,8 - 7,3 (4H, m) ; 7,3 - 7,6 (2H, m) ; 7,65 - 7,95 (2H, m).

b) Acide [[4-[[1-[[[(4-chlorophényl)sulfonyl]amino]méthyl]cyclopentyl]méthyl]phényl]méthyl]phosphonique

Un mélange de 4,8 g (9,3 mmoles) du composé préparé dans l'exemple 83 a, de 9 ml d'HCl 10,7 N et de 6 ml d'acide acétique, est porté à reflux 2 heures. Après refroidissement, on dilue par 25 ml d'eau, avant d'extraire par le dichlorométhane. La phase organique séchée sur $Na_2SO_4$ et concentrée, fournit un résidu pâteux, purifié par chromatographie sur colonne dans un mélange dichlorométhane-méthanol (9:1). On obtient essentiellement du composé monoestérifié, qui est retraité dans les mêmes conditions (HCl 10,7 N,

acide acétique), à reflux 10 heures. Après refroidissement, dilution par $H_2O$, extraction par $CH_2Cl_2$, lavage de la phase organique à l'eau, séchage et concentration, on obtient un solide pâteux qui est recristallisé dans toluène-heptane-acétone puis dans acétate d'éthyle-éther isopropylique pour donner 1,2 g (Rdt = 28,2 %) d'une poudre blanc cassé. F = 100 °C (dec).

| Analyse centésimale : $C_{20}H_{25}ClNO_5PS$ (M = 457,909) | | | | | | |
|---|---|---|---|---|---|---|
| | C % | H % | Cl % | N % | P % | S % |
| Calculé | 52,46 | 5,50 | 7,74 | 3,06 | 6,76 | 7,00 |
| Trouvé | 52,17 | 5,67 | 7,47 | 3,01 | 6,70 | 7,16 |

I.R. (KBr) : $\nu$ (NH) = 3280 $cm^{-1}$ ; ($SO_2$) = 1320 $cm^{-1}$ ; ($SO_2$) = 1150 $cm^{-1}$.
R.M.N.($CDCl_3$) : $\delta$ = 1,0 - 1,7 (8H, m) ; 2,25 - 2,6 (4H, m) ; 2,6 - 3,0 (2H, m) ; 5,0 - 5,5 (1H, m, échangeable avec $CF_3COOD$) ; 6,6 - 8,0 (10H, dont 2 H échangeables avec $CF_3COOD$).

**Exemple 84**

**Acide 4-[[1-[[[(4-méthylsulfinylphényl]sulfonyl]amino]méthyl]cyclopentyl]méthyl]benzèneacétique**

a) N-[[1-[[4-(2-hydroxyéthyl)phényl]méthyl]cyclopentyl]méthyl]-4-méthylthiobenzènesulfonamide

Obtenu en opérant comme dans l'exemple 32 d, à partir de 3 g (12,9 mmoles) du composé préparé dans l'exemple 32 c, de 1,6 g (15,8 mmoles) de triéthylamine et de 2,8 g (12,5 mmoles) de chlorure de 4-méthylthiobenzènesulfonyle (préparé selon Burton H. et Hu P.F., J. Chem. Soc. (1948), 604-5) dans 60 ml de dichlorométhane sec. On obtient 4,3 g (Rdt = 80,1 %) d'un solide blanc, utilisé sans autre purification. F = 131 - 4 °C.
Une fraction recristallisée dans l'acétate d'éthyle donne un produit fondant à F = 149,5 - 151,5 °C.
I.R. (KBr) : $\nu$ (OH) = 3480 $cm^{-1}$ ; (NH) = 3180 $cm^{-1}$ ; ($SO_2$) = 1300 $cm^{-1}$ ; ($SO_2$) = 1150 $cm^{-1}$.
R.M.N.($CDCl_3$) : $\delta$ = 1,25 - 1,85 (9H, m, dont 1H échangeable avec $D_2O$) ; 2,5 (3H, s) ; 2,55 (2H, s) ; 2,7 (2H, d, J = 6,75 Hz, se transforme en singulet avec $D_2O$) ; 2,8 (2H, t, J = 6,75 Hz) ; 3,8 (2H, t, J = 6,75 Hz) ; 4,4 (1H, m, échangeable avec $D_2O$) ; 7,0 (4H, s) ; 7,1 - 7,4 (2H, m) ; 7,5 - 7,8 (2H, m).

b) Acide 4-[[1-[[[(4-méthylsulfinylphényl)sulfonyl]amino]méthyl]cyclopentyl]méthyl]benzèneacétique

Obtenu en opérant comme dans l'exemple 25 g, à partir de 2,1 g (5 mmoles) du composé préparé dans l'exemple 84a, dissous dans 70 ml d'acétone et de 4,7 ml (10,0 mmoles) de réactif de Jones. Après purification par chromatographie sur colonne de silice dans un mélange acétate d'éthyl-méthanol (4:1) et recristallisation dans l'acétate d'éthyle, on obtient 0,1 g (Rdt = 4,2 %) d'un solide blanc. F = 135 - 6 °C.

| Analyse centésimale : $C_{22}H_{27}NO_5S_2$ + 1/4 $C_4H_8O_2$ (acétate d'éthyle) (M = 471,614) | | | | |
|---|---|---|---|---|
| | C % | H % | N % | S % |
| Calculé | 58,58 | 6,20 | 2,97 | 13,60 |
| Trouvé | 58,53 | 6,25 | 2,73 | 13,63 |

I.R. (KBr) : $\nu$ (NH) = 3150 $cm^{-1}$ ; (C=O) = 1710 $cm^{-1}$ ; ($SO_2$) = 1325 $cm^{-1}$ ; ($SO_2$) = 1165 $cm^{-1}$.
R.M.N.($CDCl_3$ + DMSO $d_6$) : $\delta$ = 1,25 - 1,6 (8H, m) ; 2,45 - 2,70 (5H, m, dont 1H échangeable avec $CF_3COOD$) ; 2,75 (3H, s) ; 3,5 (2H, s) ; 7,0 (4H, s) ; 7,4 - 8,0 (5H, m, dont 1H échangeable avec $CF_3COOD$).

## Exemple 85

### Acide 4-[[1-[[[(4-acétamidophényl)sulfonyl]amino]méthyl]cyclopentyl]méthyl]benzèneacétique

a) 1-[[4-[2-[(3,4,5,6-Tétrahydro-2H-pyran-2-yl)oxy]éthyl]phényl]méthyl]cyclopentanecarbonitrile

A un mélange composé de 46,1 g (201 mmoles) de l'alcool préparé dans l'exemple 26 b, de 0,1 g d'acide para-toluènesulfonique et de 205 ml d'éther sec, maintenu à 10 ° C, on ajoute 21,2 g (246,1 mmoles) de 3,4-dihydro-2H-pyrane. On laisse ensuite sous agitation 16 heures à température ambiante. Le milieu réactionnel est alors concentré sous vide pour donner une huile brune (Rdt = quantitatif) utilisée sans autre purification.
I.R. (film) : $\nu$ (C≡N) = 2240 $cm^{-1}$.

b) 1-[[4-[2-[(3,4,5,6-Tétrahydro-2H-pyran-2-yl)oxy]éthyl]phényl]méthyl]cyclopentaneméthanamine

A une suspension sous azote et à température ambiante de 17,1 g (450,6 mmoles) de $LiAlH_4$ dans 300 ml de tétrahydrofurane sec, on ajoute goutte à goutte 64,25 g (205 mmoles) du composé préparé dans l'exemple 85a dissous dans 400 ml de tétrahydrofurane sec. On porte ensuite 4 heures à reflux, avant de refroidir à 0 ° C et d'ajouter lentement 85,5 ml d'eau, puis 100 ml d'éther. Le précipité formé est filtré sur $Na_2SO_4$ et rincé à l'éther. Après concentration du filtrat, on obtient 66,8 g (Rdt = quantitatif) d'une huile jaune utilisée sans autre purification.
I.R. (film) : $\nu$ ($NH_2$) = 3390 $cm^{-1}$.
R.M.N.($CDCl_3$) : $\delta$ = 1,15 (2H, s, échangeables avec $CF_3COOD$) ; 1,3 - 2,25 (14H, m) ; 2,4 (2H, s) ; 2,6 (2H, s) ; 2,8 (2H, t, J = 6,75 Hz) ; 3,35 - 4,05 (4H, m) ; 4,55 (1H, m) ; 7,0 (4H, s).

c) 4-Acétamido-N-[[1-[[4-[2-[(3,4,5,6-tétrahydro-2H-pyran-2-yl)oxy]éthyl]phényl]méthyl]cyclopentyl]méthyl]-benzènesulfonamide

Obtenu en opérant comme dans l'exemple 1c, à partir de 10 g (31,5 mmoles) du composé préparé dans l'exemple 85 b dans 190 ml de dichlorométhane, de 3,75 g (37,1 mmoles) de triéthylamine et de 7,25 g (31,0 mmoles) de chlorure de 4-acétamidobenzènesulfonyle dans 110 ml N,N-diméthylformamide. Après agitation 16 heures à température ambiante et traitement usuel, on purifie par chromatographie sur colonne de silice avec un mélange dichlorométhaneméthanol (19:1) pour obtenir 10,7 g (Rdt = 66,0 %) d'une huile beige qui cristallise. F = 100 ° C.
I.R. (KBr) : $\nu$ (NH) = 3310 $cm^{-1}$ ; (NH) = 3190 $cm^{-1}$ ; (C=O) = 1660 $cm^{-1}$ ; ($SO_2$) = 1310 $cm^{-1}$ ; ($SO_2$) = 1145 $cm^{-1}$.
R.M.N.($CDCl_3$) : $\delta$ = 1,2 - 1,8 (14H, m) ; 2,2 (3H, s) ; 2,6 (2H, s) ; 2,5 - 2,8 (4H, m) ; 3,3 - 4,0 (4H, m) ; 4,6 (1H, m) ; 5,1 (1H, t, J = 6,75 Hz, échangeable avec $CF_3COOD$) ; 7,0 (4H, s) ; 7,7 (4H, s) ; 7,8 (1H, s, échangeable avec $CF_3COOD$).

d) 4-Acétamido-N-[[1-[[4-(2-hydroxyéthyl)phényl]méthyl]cyclopentyl]méthyl]benzènesulfonamide

Un mélange de 6,3 g (12,2 mmoles) du composé préparé dans l'exemple 85 c, de 60 ml de méthanol et de 0,7 g de résine Amberlite IR-120 (plus), est agité 16 heures à température ambiante. Après filtration et concentration, on obtient une huile qui après trituration dans l'hexane fournit 3,6 g (Rdt = 69,2 %) d'un solide crème. F = 177 - 80 ° C.
I.R. (KBr) : $\nu$ (OH) = 3560 $cm^{-1}$ ; (NH) = 3270 $cm^{-1}$ ; (C=O) = 1670 $cm^{-1}$ ; ($SO_2$) = 1310 $cm^{-1}$ ; ($SO_2$) = 1150 $cm^{-1}$.

e) Acide 4-[[1-[[[(4-acétamidophényl)sulfonyl]amino]méthyl]cyclopentyl]méthyl]benzèneacétique

Obtenu en opérant comme dans l'exemple 25 g à partir de 3,6 g (8,4 mmoles) du composé préparé dans l'exemple 85 d, dans 150 ml d'acétone et de 8,8 ml (17,6 mmoles) de réactif de Jones. Après purification par chromatographie sur colonne de silice dans un mélange dichlorométhane-méthanol (9:1) et recristallisations dans un mélange acétone-hexane, on obtient 0,5 g (Rdt = 13,5 %) d'un solide blanc cassé. F = 174 - 6 ° C.

| Analyse centésimale : $C_{23}H_{28}N_2O_5S$ (M = 444,546) | | | | |
|---|---|---|---|---|
| | C % | H % | N % | S % |
| Calculé | 62,14 | 6,35 | 6,30 | 7,21 |
| Trouvé | 61,91 | 6,32 | 6,63 | 7,32 |

I.R. (KBr) : ν (NH) = 3340 $cm^{-1}$ ; (NH) = 3220 $cm^{-1}$ ; (C=O) = 1690 $cm^{-1}$ ; (C=O) = 1660 $cm^{-1}$ ; ($SO_2$) = 1310 $cm^{-1}$ ; ($SO_2$) = 1145 $cm^{-1}$.
R.M.N.(acétone $d_6$ + DMSO $d_6$) : δ = 1,2 - 1,7 (8H, m) ; 2,1 (3H, s) ; 2,65 (2H, s) ; 2,65 (2H, d, J = 6Hz, se transforme en singulet avec $CF_3COOD$) ; 3,5 (2H, s) ; 6,7 (1H, t, J = 6 Hz, échangeable avec $CF_3COOD$ ; 7,1 (4H, s) ; 7,8 (4H, s) ; 9,8 (1H, s large, échangeable avec $CF_3COOD$) ; 11,55 (1H, s large, échangeable avec $CF_3COOD$).
Ce produit est obtenu aussi, en opérant comme dans l'exemple 25 g à partir de 10,7 g (20,8 mmoles) du composé préparé dans l'exemple 85 c dans 350 ml d'acétone et de 26 ml (52 mmoles) de réactif de Jones. Après purification par chromatographie sur colonne de silice dans un mélange dichlorométhane-méthanol (9:1), on obtient 3,7 g (Rdt = 40,2 %) d'un solide jaune pâle. F = 172 - 3°C.

**Exemple 86**

**Acide 4-[[1-[[[(4-aminophényl)sulfonyl]amino]méthyl]cyclopentyl]méthyl]benzèneacétique**

Un mélange de 3,7 g (8,3 mmoles) du composé préparé dans l'exemple 85 e, de 11,2 ml (112 mmoles) de soude aqueuse 10 N et de 50 ml d'eau, est porté à reflux 2 heures. Après refroidissement et dilution par 50 ml d'eau, on filtre un insoluble, avant de le laver par 100 ml d'éther. La phase aqueuse acidifiée par HCl dilué jusqu'à pH 6 donne un précipité qui après recristallisation dans éthanol, éthanol-hexane puis éthanol fournit 0,3 g (Rdt = 9,0%) d'un solide beige. F = 175 -6°C.

| Analyse centésimale : $C_{21}H_{26}N_2O_4S$ (M = 402,509) | | | | |
|---|---|---|---|---|
| | C % | H % | N % | S % |
| Calculé | 62,66 | 6,51 | 6,96 | 7,97 |
| Trouvé | 62,43 | 6,76 | 7,26 | 8,05 |

I.R. (KBr) : ν ($NH_2$) = 3450 et 3370 $cm^{-1}$ ; (NH) = 3260 $cm^{-1}$ ; (C=O) = 1685 $cm^{-1}$ ; ($SO_2$) = 1300 $cm^{-1}$ ; ($SO_2$) = 1145 $cm^{-1}$.
R.M.N.(DMSO $d_6$) : δ = 1,2 - 1,7 (8H, m) ; 2,35 - 2,7 (4H, m) ; 3,0 - 3,5 (1H, m, échangeable avec $CF_3COOD$) ; 3,45 (2H, s) ; 5,8 (1H, s large échangeable avec $CF_3COOD$) ; 6,45 - 6,7 (2H, m) ; 6,8 - 7,25 (6H, m, dont 2H échangeables avec $CF_3COOD$) ; 7,3 - 7,55 (2H, m).

**Exemple 87**

**Acide 4-[[1-[[[(4-chlorophényl)sulfonyl]amino]méthyl]cyclohexyl]méthy]benzèneacétique**

a) 1-[[4-(2-Hydroxyéthyl)phényl]méthyl]cyclohexanecarbonitrile

A un mélange maintenu sous azote, de 5,6 g (54,9 mmoles) de diisopropylamine et de 92 ml de tétrahydrofurane sec, refroidit à - 40°C, on ajoute goutte à goutte 34,2 ml (54,7 mmoles) d'une solution de n-butyllithium 1,6 M dans l'hexane, puis 8,2 g de 1,3-diméthylimidazolidin-2-one. On refroidit ensuite à -78°C et on agite 1/4 heure avant d'ajouter 5,45 g (50 mmoles) de cyclohexanecarbonitrile commercial en solution dans 82 ml de tétrahydrofurane sec. Après avoir agité 1 heure, à - 78°C, on ajoute 14,3 g (50 mmoles) du composé préparé dans l'exemple 26a. On maintient encore 3 heures à - 78°C, avant de laisser remonter la température et on agite 19 heures à température ambiante. On ajoute alors de l'eau, on acidifie par HCl et on agite encore 1 heure, avant de diluer à l'eau et d'extraire le milieu réactionnel par l'éther. La phase organique, lavée à l'eau, séchée sur $Na_2SO_4$ est concentrée. Le liquide obtenu est purifié par distillation pour donner 7,5g (Rdt = 61,6 %) d'un liquide jaune. $Eb_{0,5}$ = 130 - 80°C.
I.R. (film) : ν (OH) = 3440 $cm^{-1}$ ; (C≡N) = 2230 $cm^{-1}$.

R.M.N.($CDCl_3$) : $\delta$ = 0,9 - 2,1 (10H, m) ; 1,6 (1H, s, échangeable avec $CF_3COOD$) ; 2,75 (2H, s) ; 2,8 (2H, t, J = 6,75 Hz) ; 3,8 (2H, t, J = 6,75 Hz) ; 7,1 (4H, s).

b) 4[[1-(Aminométhyl)cyclohexyl]méthyl]benzèneéthanol

Obtenu en opérant comme dans l'exemple 32 c à partir 2,5 g (67,8 mmoles) de $LiAlH_4$ dans 50 ml de tétrahydrofurane sec et de 7,5 g (30,8 mmoles) du composé préparé dans l'exemple 87a en solution dans 60 ml de tétrahydrofurane sec. Après purification par chromatographie sur colonne de silice dans le méthanol, on obtient 5,1 g (Rdt = 67,1 %) d'une huile jaune.
I.R. (film) : $\nu$ ($NH_2$) = 3375 et 3300 $cm^{-1}$ ; (OH) = 3300 $cm^{-1}$.
R.M.N.($CDCl_3$) : $\delta$ = 1,0 - 1,75 (13H, m, dont 3H échangeables avec $D_2O$) ; 2,4 (2H, s) ; 2,5 (2H, s) ; 2,8 (2H, t, J = 6,75 Hz) ; 3,8 (2H, t, J = 6,75 Hz) ; 7,0 (4H, s).

c) 4-Chloro-N-[[1-[[4-(2-hydroxyéthyl)phényl]méthyl]cyclohexyl]méthyl]benzènesulfonamide

Obtenu en opérant comme dans l'exemple 32 d, à partir de 5,1 g (20,6 mmoles) du composé préparé dans l'exemple 87 b, dans 71 ml de dichlorométhane sec, de 2,5 g (24,7 mmoles) de triéthylamine et de 4,2 g (19,7 mmoles) de chlorure de 4-chlorobenzènesulfonyle. On obtient 6,3 g (Rdt = 75,9 %) d'un solide blanc utilisé sans autre purification. F = 178 - 82 ° C.
Une fraction purifiée par recristallisation dans un mélange éthanol-DMF, puis éthanol donne un solide blanc. F = 179 - 82 ° C.

| Analyse centésimale : $C_{22}H_{28}ClNO_3S$ (M = 421,983) | | | | | |
|---|---|---|---|---|---|
| | C % | H % | Cl % | N % | S % |
| Calculé | 62,62 | 6,69 | 8,40 | 3,32 | 7,60 |
| Trouvé | 62,71 | 6,59 | 8,39 | 3,22 | 7,40 |

I.R. (KBr) : $\nu$ (OH) = 3515 $cm^{-1}$ ; (NH) = 3220 $cm^{-1}$ ; ($SO_2$) = 1300 $cm^{-1}$ ; ($SO_2$) = 1145 $cm^{-1}$.
R.M.N.(DMSO $d_6$) : $\delta$ = 0,75 - 1,6 (10H, m) ; 2,3 - 2,8 (6H, m) ; 3,3 (1H, s, échangeable avec $CF_3COOD$) ; 3,6 (2H, t, J = 6,75 Hz) ; 4,5 (1H, t, J = 6,75 Hz, échangeable avec $CF_3COOD$) 6,9 (4H, s) ; 7,3 - 8,0 (4H, m).

d) Acide 4-[[1-[[[(4-chlorophényl)sulfonyl]amino]méthyl]cyclohexyl]méthyl]benzèneacétique

Obtenu en opérant comme dans l'exemple 25 g à partir de 5,9 g (14,0 mmoles) du composé préparé dans l'exemple 87 c, dissous dans 190 ml d'acétone et de 12,8 ml (28 mmoles) de réactif de Jones. Après recristallisation dans un mélange acétate d'éthyle-éthanol puis acétate d'éthyle, on obtient 1,5 g (Rdt = 24,6 %) d'un solide blanc. F = 191 - 4 ° C.

| Analyse centésimale : $C_{22}H_{26}ClNO_4S$ (M = 435,966) | | | | | |
|---|---|---|---|---|---|
| | C % | H % | Cl % | N % | S % |
| Calculé | 60,61 | 6,01 | 8,13 | 3,21 | 7,35 |
| Trouvé | 60,63 | 6,17 | 8,21 | 3,31 | 7,55 |

I.R. (KBr) : $\nu$ (NH) = 3270 $cm^{-1}$ ; (C=O) = 1700 $cm^{-1}$ ; ($SO_2$) = 1310 $cm^{-1}$ ; ($SO_2$) = 1155 $cm^{-1}$.
R.M.N.(DMSO $d_6$) : $\delta$ = 0,8 - 1,7 (10H, m) ; 2,3 - 2,75 (4H, m) ; 3,5 (2H, s) ; 7,0 (4H, s) ; 7,3 - 8,0 (5H, m, dont 1H échangeable avec $CF_3COOD$) ; 12,1 (1H, s, échangeable avec $CF_3COOD$).

**Exemple 88**

**Acide 4-[[4-[[[(4-chlorophényl)sulfonyl]amino]méthyl]tétrahydropyran-4-yl]méthyl]benzèneacétique**

a) 4-[[4-(2-Hydroxyéthyl)phényl]méthyl]tétrahydropyran-4-carbonitrile

Obtenu en opérant comme dans l'exemple 87 a, à partir de 6,3 g (62,2 mmoles) de diisopropylamine dans 104 ml de tétrahydrofurane, de 38,7 ml (62,0 mmoles) de n-butyllithium en solution 1,6 M dans l'hexane, de 9,3 g de 1,3-diméthylimidazolidin-2-one de 8,8 g (56,7 mmoles) de 2,3,5,6-tétrahydro-4H-pyran-4-carbonitrile (préparé selon Gibson C.S. et Johnson J.D.A., J. Chem. Soc. (1930), 2525-30) dans 93 ml de tétrahydrofurane et de 17,7 g (62 mmoles) du composé préparé dans l'exemple 26 a. Après purification par chromatographie sur colonne de silice dans un mélange hexane-acétate d'éthyle (1:1), on obtient 10,5 g (Rdt = 75,5 %) d'huile.
I.R. (film) : $\nu$ (OH) = 3400 $cm^{-1}$ ; (C≡N) = 2220 $cm^{-1}$.
R.M.N.($CDCl_3$) : $\delta$ = 1,55 (1H, s, échangeable avec $D_2O$) ; 1,6 - 1,9 (4H, m) ; 2,8 (2H, s) ; 2,8 (2H, t, J = 6,75 Hz) ; 3,4 - 4,2 (6H, m) ; 7,1 (4H, s).

b) 4-[[4-(Aminométhyl)tétrahydropyran-4-yl]méthyl]benzèneéthanol

Obtenu en opérant comme dans l'exemple 32 c, à partir de 3,45 g (94,3 mmoles) de LiAlH4 dans 80 ml de tétrahydrofurane sec et de 10,5 g (42,8 mmoles) du composé préparé dans l'exemple 88a en solution dans 80 ml de tétrahydrofurane sec. On obtient 7,1 g (Rdt = 66,3 %) d'huile, utilisée sans autre purification.
I.R. (film) : $\nu$ ($NH_2$) = 3370 et 3290 $cm^{-1}$ ; (OH) = 3360 $cm^{-1}$.
R.M.N.($CDCl_3$) : $\delta$ = 1,0 - 2,0 (4H, m) ; 1,65 (3H, s, échangeables avec $D_2O$) ; 2,5 (2H, s) ; 2,6 (2H, s) ; 2,8 (2H, t, J = 6,75 Hz) ; 3,45 - 4,0 (6H, m) ; 7,0 (4H, s).

c) 4-Chloro-N-[[4-[[4-(2-hydroxyéthyl)phényl]méthyl]tétrahydropyran-4-yl]méthyl]-benzènesulfonamide

Obtenu en opérant comme dans l'exemple 32 d, à partir de 7,1 g (28,4 mmoles) du composé préparé dans l'exemple 88 b, dans 100 ml de dichlorométhane sec, de 3,4 g (33,9 mmoles) de triéthylamine et de 5,75 g (27,2 mmoles) de chlorure de 4-chlorobenzènesulfonyle. On obtient 5,4 g (Rdt = 46,8 %) d'un solide blanc utilisé sans autre purification. F = 178 - 80 °C.
Une fraction purifiée par recristallisation dans un mélange acétate d'éthyle-éthanol, puis dans l'acétate d'éthyle donne un solide blanc. F = 180 - 1 °C.

| Analyse centésimale : $C_{21}H_{26}ClNO_4S$ (M = 423,955) | | | | | |
|---|---|---|---|---|---|
| | C % | H % | Cl % | N % | S % |
| Calculé | 59,49 | 6,18 | 8,36 | 3,30 | 7,56 |
| Trouvé | 59,56 | 6,22 | 8,50 | 3,26 | 7,29 |

I.R. (KBr) : $\nu$ (OH) = 3540 $cm^{-1}$ ; (NH) = 3260 $cm^{-1}$ ; ($SO_2$) = 1305 $cm^{-1}$ ; ($SO_2$) = 1145 $cm^{-1}$.
R.M.N.(DMSO $d_6$) : $\delta$ = 1,0 - 1,5 (4H, m) ; 2,3 - 2,85 (6H, m) ; 3,25 - 3,8 (6H, m) ; 4,0 (1H, s large, échangeable avec $CF_3COOD$) ; 6,9 (4H, s) ; 7,35 - 7,95 (5H, m, dont 1H échangeable avec $CF_3COOD$).

d) Acide 4-[[4-[[[(4-chlorophényl)sulfonyl]amino]méthyl]tétrahydropyran-4-yl]méthyl]benzèneacétique

Obtenu en opérant comme dans l'exemple 25 g, à partir de 4,9 g (11,5 mmoles) du composé préparé dans l'exemple 88 c, dissous dans 150 ml d'acétone et de 10,5 ml (22,9 mmoles) de réactif de Jones. Après recristallisation dans l'acétate d'éthyle, on obtient 0,5 g (Rdt = 9,9 %) d'un solide blanc. F = 179 - 80 °C.

| Analyse centésimale : $C_{21}H_{24}ClNO_5S$ (M = 437,938) | | | | | |
|---|---|---|---|---|---|
| | C % | H % | Cl % | N % | S % |
| Calculé<br>Trouvé | 57,60<br>57,88 | 5,52<br>5,41 | 8,10<br>8,24 | 3,20<br>3,40 | 7,32<br>7,06 |

I.R. (KBr) : $\nu$ (NH) = 3250 $cm^{-1}$ ; (C=O) = 1685 $cm^{-1}$ ; ($SO_2$) = 1310 $cm^{-1}$ ; ($SO_2$) = 1155 $cm^{-1}$.
R.M.N.(DMSO $d_6$) : $\delta$ = 1,1 - 1,5 (4H, m) ; 2,45 - 2,8 (4H, m) ; 3,3 (1H, échangeable avec $CF_3COOD$) ; 3,4 - 3,75 (6H, m) ; 7,0 (4H, s) ; 7,5 - 8,0 (4H, m) ; 12,1 (1H, s large, échangeable avec $CF_3COOD$).

**Exemple 89**

**Acide 4-[[1-[[[(4-chlorophényl)sulfonyl]amino]méthyl]-3,3-méthylcyclobutyl]méthyl]benzèneacétique**

a) 3,3-Diméthyl-1-[[4-(2-hydroxyphényl]méthyl]cyclobutanecarbonitrile

Obtenu en opérant comme dans l'exemple 72 a, à partir de 3,2 g (32 mmoles) de diisopropylamine dans 34 ml de tétrahydrofurane sec, de 20 ml (38,4 mmoles) de n-butyllithium en solution 1,6 M dans l'hexane, de 2,9 g (26,5 mmoles) de 3,3-diméthylcyclobutanecarbonitrile (préparé selon Brannock K.C. et coll., J. Org. Chem. (1964) 29, 801-12) dans 30 ml de tétrahydrofurane sec et de 11,4 g (39,7 mmoles) du composé préparé dans l'exemple 26a dans 10 ml de tétrahydrofurane sec. Après recristallisation dans un mélange hexane-acétate d'éthyle, on obtient 5,0 g (Rdt = 77,5 %) d'un solide. F = 51 - 4 °C.
I.R. (KBr) : $\nu$ (OH) = 3390 $cm^{-1}$ ; (C≡N) = 2250 $cm^{-1}$.
R.M.N.($CDCl_3$) : $\delta$ = 1,05 (3H, s) ; 1,25 (3H, s) ; 1,7 (1H, s, échangeable par $D_2O$) ; 2,1 (4H, m) ; 2,8 (4H, m) ; 3,8 (2H, t, J = 6 Hz) ; 6,9 - 7,4 (4H, m).

b) 4-[[1-(Aminométhyl)-3,3-diméthylcyclobutyl]méthyl]benzèneéthanol

Obtenu en opérant comme dans l'exemple 25 e, à partir de 5,5 g (145 mmoles) de $LiAlH_4$ et de 28,1 g (97 mmoles) du composé préparé dans l'exemple 89 a dans 300 ml d'éther sec. On obtient 16 g (Rdt = 66,7 %) d'une huile jaune clair, utilisée sans autre purification.
I.R. (film) : $\nu$ (OH) = 3300 $cm^{-1}$ ; ($NH_2$) = 3360 et 3280 $cm^{-1}$.
R.M.N.($CDCl_3$) : $\delta$ = 1,0 (3H, s) ; 1,05 (3H, s) ; 1,5 - 1,85 (7H, m, dont 3H échangeables avec $D_2O$) ; 2,6 (2H, t, J = 6,0 Hz) ; 2,6 - 3,0 (4H, m) ; 3,8 (2H, t, J = 6,0 Hz) ; 6,85 - 7,4 (4H, m).

c) 4-Chloro-N-[[3,3-diméthyl-1-[[4-(2-hydroxyéthyl)phényl]méthyl]cyclobutyl]méthyl]-benzènesulfonamide

Obtenu en opérant comme dans l'exemple 32 d, à partir de 8,0 g (32,3 mmoles) du composé préparé dans l'exemple 89 b, de 3,9 g (38,8 mmoles) de triéthylamine, de 6,7 g (31,6 mmoles) de chlorure de 4-chlorobenzènesulfonyle dans 150 ml de dichlorométhane sec. Après purification par chromatographie sur colonne de silice dans un mélange hexane-acétate d'éthyle (1:1), on obtient 3,8 g (Rdt = 27,8 %) d'un solide blanc. F = 90 - 2 °C.
I.R. (KBr) : $\nu$ (OH) = 3470 $cm^{-1}$ ; (NH) = 3160 $cm^{-1}$ ; ($SO_2$) = 1320 $cm^{-1}$ ; ($SO_2$) = 1145 $cm^{-1}$.
R.M.N.($CDCl_3$) : $\delta$ = 1,0 (6H, s) ; 1,5 - 1,85 (5H, m, dont 1H échangeable avec $CF_3COOD$) ; 2,7 (2H, s) ; 2,8 (2H, t, J = 6 Hz) ; 2,85 (2H, d, J = 6 Hz) ; 3,8 (2H, t, J = 6 Hz) ; 4,65 (1H, t, J = 6 Hz, échangeable avec $CF_3COOD$) ; 7,0 (4H, s) ; 7,3 - 8,0 (4H, m).

d) Acide 4-[[1-[[[(4-chlorophényl)sulfonyl]amino]méthyl]-3,3-diméthylcyclobutyl]méthyl]benzèneacétique

Obtenu en opérant comme dans l'exemple 25 g, à partir de 3,5 g (8,3 mmoles) du composé préparé dans l'exemple 89 c, dissous dans 83 ml d'acétone et de 8,3 ml (16,6 mmoles) de réactif de Jones. Après recristallisation dans le toluène, on obtient 1,3 g (Rdt = 35,9 %) d'un solide blanc. F = 140 - 2 °C.

| Analyse centésimale : $C_{22}H_{26}ClNO_4S$ (M = 435,96) | | | | | |
|---|---|---|---|---|---|
| | C % | H % | Cl % | N % | S % |
| Calculé | 60,61 | 6,01 | 8,13 | 3,21 | 7,35 |
| Trouvé | 60,71 | 6,21 | 8,20 | 3,18 | 7,30 |

I.R. (KBr) : $\nu$ (NH) = 3270 $cm^{-1}$ ; (C=O) = 1695 $cm^{-1}$ ; ($SO_2$) = 1325 $cm^{-1}$ ; ($SO_2$) = 1160 $cm^{-1}$.
R.M.N.(DMSO $d_6$) : $\delta$ = 0,9 (3H, s) ; 1,0 (3H, s) ; 1,6 (4H, s) ; 2,55 - 2,8 (4H, m) ; 3,45 (2H, s) ; 7,0 (4H, s) ; 7,35 - 7,9 (5H, m, dont 1H échangeable avec $CF_3COOD$) ; 12,1 (1H, s large, échangeable avec $CF_3COOD$).

**Exemple 90**

**Acide 4-[[1-[[[(4-chlorophényl)sulfonyl]amino]méthyl]-2,2,3,3-tétraméthylcyclopropyl]méthyl]-benzèneacétique**

a) 1-[[4-(2-Hydroxyéthyl)phényl]méthyl]-2,2,3,3-tétraméthylcyclopropanecarbonitrile

Obtenu en opérant comme dans l'exemple 87 a, à partir de 19,7 g (195 mmoles) de diisopropylamine, de 122 ml (195 mmoles) de n-butyllithium en solution 1,6 M dans l'hexane, de 45 ml de 1,3-diméthylimidazolidin-2-one, de 21,8 g de 2,2,3,3-tétraméthylclopropanecarbonitrile (préparé selon le brevet FR 2, 479, 192), de 51,9 g (180,5 mmoles) du composé préparé dans l'exemple 26 a dans 295 ml de tétrahydrofurane. Après distillation, on obtient 20 g (Rdt = 44,0 %) d'une huile jaune épaisse. $Eb_{0,8}$ = 185 - 205 °C.
I.R. (film) : $\nu$ (OH) = 3420 $cm^{-1}$ ; (C≡N) = 2230 $cm^{-1}$.
R.M.N.($CDCl_3$) : $\delta$ = 1,1 (6H, s) ; 1.3 (6H, s) ; 2,1 (1H, s, échangeable avec $CF_3COOD$) ; 2,75 (2H, t, J = 6,75 Hz), 2,8 (2H, s) ; 3,75 (2H, t, J = 6,75 Hz) ; 7,1 (4H, s).

b) 4-[[1-(Aminométhyl)-2,2,3,3-tétraméthylcyclopropyl]méthyl]benzèneéthanol

Obtenu en opérant comme dans l'exemple 13 a, à partir de 30 ml (101 mmoles) de $LiAlH_4$ en solution commerciale à 13 % dans un mélange toluène-tétrahydrofurane, de 20 g (77,7 mmoles) du nitrile préparé dans l'exemple 90a, et de 130 ml de tétrahydrofurane sec. Après distillation, on obtient 13,2 g (Rdt = 65,0 %) d'un liquide orange trés épais, qui cristallise. $Eb_{0,3}$ = 190 - 220 °C. F = 103 - 6 °C (acétate d'éthyle).
I.R. (KBr) : $\nu$ (OH) = 3380 $cm^{-1}$ ; ($NH_2$) = 3380 $cm^{-1}$.
R.M.N.($CDCl_3$) : $\delta$ = 0,75 - 1,4 (12H, m) ; 1,7 (3H, s, échangeables avec $CF_3COOD$) ; 2,5 - 3,2 (6H, m) ; 3,75 (2H, t, J = 6,75 Hz) ; 6,75 - 7,3 (4H, m).

c) 4-Chloro-N-[[1-[[4-(2-hydroxyéthyl)phényl]méthyl]-2,2,3,3-tétraméthylcyclopropyl]méthyl]-benzènesulfonamide

Obtenu en opérant comme dans l'exemple 32 d, à partir de 3,8 g (14,5 mmoles) du composé préparé dans l'exemple 90 b, de 2,4 ml (17,4 mmoles) de triéthylamine et de 3 g (14,25 mmoles) de chlorure de 4-chlorobenzènesulfonyle dans 65 ml de dichlorométhane sec. Après recristallisation dans l'acétate d'éthyle, on obtient 4,2 g (Rdt = 66,7 %) d'un solide blanc cassé. F = 158 - 60 °C. Une fraction purifiée par filtration sur colonne de silice dans un mélange hexane-acétate d'éthyle puis par recristallisation dans le même mélange, donne un solide blanc. F = 162 - 3 °C.

| Analyse centésimale : $C_{23}H_{30}ClNO_3S$ (M = 436,01) | | | | | |
|---|---|---|---|---|---|
| | C % | H % | Cl % | N % | S % |
| Calculé | 63,36 | 6,94 | 8,13 | 3,21 | 7,35 |
| Trouvé | 63,49 | 6,88 | 8,28 | 3,19 | 7,16 |

I.R. (KBr) : $\nu$ (NH) = 3440 $cm^{-1}$ ; (OH) = 3140 $cm^{-1}$ ; ($SO_2$) = 1310 $cm^{-1}$ ; ($SO_2$) = 1145 $cm^{-1}$.
R.M.N.(acétone $d_6$) : $\delta$ = 0,85 - 1,2 (12H, m) ; 2,55 - 3,0 (6H, m) ; 3,6 (1H, s, échangeable avec $CF_3COOD$) ; 3,7 (2H, t, J = 6,75 Hz) ; 5,95 (1H, t, J = 6,75 Hz, échangeable avec $CF_3COOD$) ; 6,75 - 7,1 (4H, m) ; 7,25 - 7,75 (4H, m).

d) Acide 4-[[1-[[[(4-chlorophényl)sulfonyl]amino]méthyl]-2,2,3,3-tétraméthylcyclopropyl]méthyl]-benzèneacétique

Obtenu en opérant comme dans l'exemple 25 g, à partir de 3,5 g (8 mmoles) du composé préparé dans l'exemple 90 c, de 7,6 ml (16 mmoles) de réactif de Jones dans 80 ml d'acétone. Après purification par chromatographie sur colonne de silice par un mélange toluène-acétate d'éthyle (4:0 à 4:1) suivie d'une recristallisation dans le toluène, on obtient 1,8 g (Rdt = 50,0 %) d'un solide blanc. F = 176 - 8°C.

| Analyse centésimale : $C_{23}H_{28}ClNO_4S$ (M = 449,993) | | | | | |
|---|---|---|---|---|---|
| | C % | H % | Cl % | N % | S % |
| Calculé | 61,39 | 6,27 | 7,88 | 3,11 | 7,12 |
| Trouvé | 61,68 | 6,00 | 8,02 | 3,28 | 7,05 |

I.R. (KBr) : $\nu$ (NH) = 3290 $cm^{-1}$ ; (C=O) = 1700 $cm^{-1}$ ; ($SO_2$) = 1340 $cm^{-1}$ ; ($SO_2$) = 1160 $cm^{-1}$.
R.M.N.(acétone $d_6$) : $\delta$ = 0,9 - 1,25 (12H, m) 2,8 (2H, s) ; 2,9 (2H, d, J = 5,25 Hz, se transforme en singulet avec $CF_3COOD$) ; 3,55 (2H, s) ; 6,0 (1H, t, J = 5,25 Hz, échangeable avec $CF_3COOD$) ; 6,75 - 7,3 (4H, m) ; 7,3 - 7,9 (4H, m) ; 10,5 (1H, s large, échangeable avec $CF_3COOD$).

**Exemple 91**

**Acide 4-[[1-[[[(4-chlorophényl)sulfonyl]amino]méthyl]4-oxocyclohexyl]méthyl]benzèneacétique**

a) 4-[[(1,1-Diméthyléthyl)diphénylsilyl]oxy]cyclohexanecarbonitrile

A une solution maintenue à 10°C, sous azote, de 11 g (87,8 mmoles) de 4-hydroxycyclohexanecarbonitrile (préparé selon Praefcke K. et Schmidt D., Z. Naturforsch (1980) 35b, 1451-4) dans 50 ml de N,N-diméthylformamide, on ajoute goutte à goutte 26,6 g (96,8 mmoles) de chlorure de 1,1-diméthyléthyldiphénylsilyle, puis par portions 13,1 g (190 mmoles) d'imidazole. On laisse sous agitation à température ambiante durant 3 jours, avant de verser le milieu réactionnel dans l'eau saturée par NaCl. On extrait par un mélange hexane-éther (1:1). La phase organique est lavée par une solution d'HCl N, puis à l'eau saturée par NaCl, avant d'être séchée et concentrée. Le résidu est purifié par chromatographie sur colonne de silice dans le dichlorométhane pour donner 26,5 g (Rdt = 85,9 %) d'une huile épaisse incolore.
I.R. (film) : $\nu$ (C≡N) = 2240 $cm^{-1}$.
R.M.N.($CDCl_3$) : $\delta$ = 0,7 (9H, s) ; 0,9 - 1,90 (8H, m) ; 2,1 (1H, m) ; 3,35 (1H, m) ; 6,8 - 7,45 (10H, m).

b) 4-[[(1,1-Diméthyléthyl)diphénylsilyl]oxy]-1-[[4-(2-hydroxyéthyl)phényl]méthyl]cyclohexanecarbonitrile

Obtenu en opérant comme dans l'exemple 87 a, à partir de 17,5 g (173 mmoles) de diisopropylamine, de 88,5 ml (145 mmoles) de n-butyllithium en solution 1,6 M dans l'hexane, de 20,6 g de 1,3-diméthylimidazolidin-2-one, de 42,4 g (120 mmoles) du composé préparé dans l'exemple 91a, dans 38 g (132 mmoles) de l'halogénure préparé dans l'exemple 26a, dans 180 ml de tétrahydrofurane sec. Après purification sur colonne de silice dans un mélange hexane-acétate d'éthyle (7:1 à 4:1), on obtient 12,7 g (Rdt = 22,4 %) d'un solide pâteux blanc.
I.R. (KBr) : $\nu$ (OH) = 3400 $cm^{-1}$ ; (C≡N) = 2240 $cm^{-1}$.
R.M.N.($CDCl_3$) : $\delta$ = 1,0 (9H, s) ; 1,5 (1H, s, échangeable avec $CF_3COOD$) ; 1,5 - 2,0 (8H, m) ; 2,7 (2H, s) ; 2,8 (2H, t, J = 6,75 Hz) ; 3,6 (1H, m) ; 3,8 (2H, t, J = 6,75 Hz) ; 7,0 (4H, s) ; 7,2 - 7,75 (10H, m).

c) 4-[[1-(Aminométhyl)-4-hydroxycyclohexyl]méthyl]benzèneéthanol

Obtenu en opérant comme dans l'exemple 32 c, mais à température ambiante, à partir de 1 g (26,3 mmoles) de $LiAlH_4$, de 5 g (10,6 mmoles) du composé préparé dans l'exemple 91 b, dans 60 ml de tétrahydrofurane sec. Après traitement par HCl aqueux et traitement usuel, on obtient 2,3 g (Rdt = 82,1 %) d'une huile épaisse qui cristallise lentement.
I.R. (film) : $\nu$ (OH) = 3350 $cm^{-1}$.
R.M.N.(DMSO $d_6$) : $\delta$ = 0,9 - 1,8 (10H, m, dont 2H échangeables avec $CF_3COOD$) ; 2,25 - 2,9 (6H, m) ; 3,05 - 3,7 (3H, m, dont 2H échangeables avec $CF_3COOD$) ; 3,55 (2H, t, J = 6,75 Hz) ; 7,0 (4H, s).

d) 4-Chloro-N-[[4-hydroxy-1-[[4-(2-hydroxyéthyl)phényl]méthyl]cyclohexyl]méthyl]-benzènesulfonamide

Obtenu en opérant comme dans l'exemple 32 d, à partir de 1.8 g (6,8 mmoles) du composé préparé dans l'exemple 91 c, de 0,9 g (8,9 mmoles) de triéthylamine, de 1,3 g (6,3 mmoles) de chlorure de 4-chlorobenzènesulfonyle dans 100 ml de dichlorométhane sec et 20 ml d'éther. On obtient 1.1 g (Rdt = 36,7 %) d'une poudre blanche utilisée sans autre purification. F = 171 - 4°C.
I.R. (KBr) : ν (NH) = 3500 $cm^{-1}$ ; (OH) = 3400 $cm^{-1}$ ; ($SO_2$) = 1310 $cm^{-1}$ ; ($SO_2$) 1140 $cm^{-1}$.
R.M.N.(acétone $d_6$ + DMSO $d_6$) : δ = 1,0 - 1,9 (8H, m) ; 2,6 - 2.9 (6H, m) 3,25 - 3,8 (6H, dont 3H échangeables avec $CF_3COOD$) ; 7,0 (4H, s) ; 7,4 - 8,0 (4H, m).

e) Acide 4-[[1-[[[(4-chlorophényl)sulfonyl]amino]méthyl]-4-oxocyclohexyl]méthyl]benzèneacétique

Obtenu en opérant comme dans l'exemple 25 g, à partir de 1,1 g (2,5 mmoles) du composé préparé dans l'exemple 91 d, de 4.7 ml (5 mmoles) de réactif de Jones dans 30 ml d'acétone. Après chromatographie sur colonne de silice dans un mélange dichlorométhane-méthanol (95:5), on obtient 0,3 g (Rdt = 26,5 %) d'une poudre blanc cassé. F = 205 - 10°C (dec.).

| Analyse centésimale : $C_{22}H_{24}ClNO_5S$ (M = 449,949) | | | | | |
|---|---|---|---|---|---|
| | C % | H % | Cl % | N % | S % |
| Calculé | 58,73 | 5,38 | 7,88 | 3,11 | 7,13 |
| Trouvé | 58,60 | 5,71 | 8,09 | 2,98 | 6,93 |

I.R. (KBr) : ν (NH) = 3250 $cm^{-1}$ ; (C=O) = 1680 $cm^{-1}$ ; (C=O) = 1690 $cm^{-1}$ ; ($SO_2$) = 1310 $cm^{-1}$ ; ($SO_2$) = 1160 $cm^{-1}$
R.M.N.(DMSO $d_6$) : δ = 1,5 - 1,9 (4H, m) ; 2,1 - 2,45 (4H, m) ; 2,6 - 2,95 (4H, m) ; 3,3 (1H, m, échangeable avec $CF_3COOD$) ; 3,5 (2H, s) ; 7,0 (4H, s) ; 7,5 - 8,0 (4H, m) ; 12,1 (1H, s large, échangeable avec $CF_3COOD$).

**Exemple 92**

**Acide 2-[[1-[[[(4-chlorophényl)sulfonyl]amino]méthyl]cyclopentyl]méthyl]benzèneacétique**

a) Acide 2-(bromométhyl)benzèneacétique

Une solution de 10 g (66.6 mmoles) d'acide 2-méthylbenzèneacétique commercial dans 100 ml de tétrachlorure de carbone est portée à reflux, sous une lampe UV. On coule goutte à goutte en 2 heures, une solution composée de 13,8 g (86,6 mmoles) de brome et de 33 ml de tétrachlorure de carbone. On maintient le reflux 1 heure après la fin de l'addition et on concentre à sec. Le résidu recristallisé dans le tétrachlorure de carbone puis dans un mélange hexane-acétate d'éthyle fournit 5 g (Rdt = 32,9 %) d'un solide blanc. F = 129 - 32°C (Litt. : 129 - 32°C ; Leroy Chauffe L. J.A. et Keefer R.M., J. Org. Chem. (1966) 31, 3758-68).
I.R. (KBr) : ν (C=O) = 1675 $cm^{-1}$.
R.M.N.($CDCl_3$ + DMSO $d_6$) : δ = 3,7 (2H, s) ; 4,55 (2H, s) ; 7,2 (4H, s) ; 10,75 (1H, s large, échangeable avec $CF_3COOD$).

b) 2-(Bromométhyl)benzèneéthanol

Obtenu en opérant comme dans l'exemple 25 a, à partir de 1,8 g (24 mmoles) du complexe hydrure de bore-diméthylsulfure et de 5 g (21,8 mmoles) du composé préparé dans l'exemple 92a, dans 50 ml de tétrahydrofurane. On obtient 3,5 g (Rdt = 74,6 %) d'huile jaune pâle utilisée sans autre purification.
I.R. (film) : ν (OH) = 3350 $cm^{-1}$.
R.M.N.($CDCl_3$) : δ = 1,2 (2H, t, J = 6,75 Hz) ; 3,4 (1H, s, échangeable avec $D_2O$) ; 3,85 (2H, t, J = 6,75 Hz) ; 4,5 (2H, s) ; 7,2 (4H, s).

c) 1-(Bromométhyl)-2-[2-(triméthylsilyloxy)éthyl]benzène

Obtenu en opérant comme dans l'exemple 26 a, à partir de 47,3 g (220 mmoles) du composé préparé dans l'exemple 92 b, de 35,5 g (220 mmoles) de 1,1,1,3,3,3-hexaméthyldisilazane, de 22,25 g (220 mmoles) de triéthylamine et de 23,9 g (220 mmoles) de chlorure de triméthylsilyle dans 475 ml de tétrahydrofurane. Après distillation, on obtient 42,5 g (Rdt = 67,3 %) d'un liquide incolore. $Eb_{0,7}$ = 98 - 102 °C.
R.M.N.($CDCl_3$) : δ = 0,0 (9H, s) ; 2,9 (2H, t, J = 6,75 Hz) ; 3,8 (2H, t, J = 6,75 Hz) ; 4,5 (2H, s) ; 6,9 - 7,6 (4H, m).

d) 1-[[2-(2-Hydroxyéthyl)phényl]méthyl]cyclopentanecarbonitrile

Obtenu en opérant comme dans l'exemple 87 a, à partir de 7,8 g (77 mmoles) de diisopropylamine, de 48,2 ml de n-butyllithium en solution 1,6 M dans l'hexane, de 6,7 g (70 mmoles) de cyclopentanecarbonitrile commercial, de 18 ml de 1,3-diméthylimidazolidin-2-one et de 20,5 g (71,4 mmoles) du composé préparé dans l'exemple 92c dans 120 ml de tétrahydrofurane. Après distillation, on obtient 8,8 g (Rdt = 55,0 %) d'un liquide visqueux beige. $Eb_{0,4}$ = 165 °C.
I.R. (film) : ν (OH) = 3360 $cm^{-1}$ ; (C≡N) = 2215 $cm^{-1}$.
R.M.N.($CDCl_3$) : δ = 1,4 - 2,25 (8H, m) ; 2,8 (1H, s, échangeable avec $D_2O$) ; 3,0 (4H, m) ; 3,8 (2H, t, J = 6,75 Hz) ; 7,15 (4H, m).

e) 2-[[1-(Aminométhyl)cyclopentyl]méthyl]benzèneéthanol

Obtenu en opérant comme dans l'exemple 15 a, à partir de 1,8 g (45,6 mmoles) de $LiAlH_4$, de 8,8 g (38 mmoles) du composé préparé dans l'exemple 92 d dans 50 ml d'éther. On obtient 3,3 g (Rdt = 37,2 %) d'une huile épaisse jaune pâle utilisée sans autre purification.
I.R. (film) : ν ($NH_2$) = 3360 et 3290 $cm^{-1}$.
R.M.N.($CDCl_3$) : δ = 1,25 - 1,75 (8H, m) ; 2,05 (3H, s, échangeables avec $D_2O$) ; 2,5 (2H, s) ; 2,75 (2H,s) ; 2,9 (2H, t, J = 6,75 Hz) ; 3,8 (2H, t, J = 6,75 Hz) ; 7,2 (4H, s).

f) 4-Chloro-N-[[1-[[2-(2-hydroxyéthyl)phényl]méthyl]cyclopentyl]méthyl]benzènesulfonamide

Obtenu en opérant comme dans l'exemple 32 d, à partir de 3 g (12,8 mmoles) du composé préparé dans l'exemple 92 c, de 1,6 g (16,2 mmoles) de triéthylamine et de 2,65 g (12,5 mmoles) de chlorure de 4-chlorobenzènesulfonyle dans 80 ml de dichlorométhane sec. Après purification par chromatographie sur colonne de silice dans un mélange dichlorométhane-méthanol (98:2), on obtient 2,3 g (Rdt = 45,1 %) d'un solide pâteux.
I.R. (film) : ν (NH) = 3420 $cm^{-1}$ ; (OH) = 3120 $cm^{-1}$ ; ($SO_2$) = 1290 $cm^{-1}$ ; ($SO_2$) = 1125 $cm^{-1}$.
R.M.N.($CDCl_3$) : δ = 1,25 - 1,75 (8H, m) ; 2,5 - 3,1 (7H, m, dont 1H échangeable avec $CF_3COOD$) ; 3,8 (2H, t, J = 6,75 Hz) ; 5,7 (1H, t, J = 6,75 Hz, échangeable avec $CF_3COOD$) ; 7,0 - 7,3 (4H, m) ; 7,3 - 8,0 (4H, m).

g) Acide 2-[[1-[[[4(chlorophényl)sulfonyl]amino]méthyl]cyclopentyl]méthyl]benzèneacétique

Obtenu en opérant comme dans l'exemple 25 g, à partir de 2,3 g (5,6 mmoles) du composé préparé dans l'exemple 92 f, de 5,3 ml (11,2 mmoles) de réactif de Jones dans 70 ml d'acétone. Après chromatographie sur colonne de silice dans un mélange dichlorométhane-méthanol (98:2) et recristallisation dans l'éther isopropylique, on obtient 0.2 g (Rdt = 8,5 %) d'un solide blanc. F = 124 - 6 °C.

| Analyse centésimale : $C_{21}H_{24}ClNO_4S$ (M = 421,95) | | | | | |
|---|---|---|---|---|---|
| | C % | H % | Cl % | N % | S % |
| Calculé | 59,78 | 5,73 | 8,40 | 3,32 | 7,60 |
| Trouvé | 60,09 | 5,80 | 8,45 | 3,38 | 7,30 |

I.R. (KBr) : ν (NH) = 3250 $cm^{-1}$ ; (C=0) = 1715 $cm^{-1}$ ; ($SO_2$) = 1290 $cm^{-1}$ ; ($SO_2$) = 1145 $cm^{-1}$.
R.M.N.($CDCl_3$) : δ = 1,3 - 1,75 (8H, m) ; 2,7 (2H, s) ; 2,8 (2H, d, J = 6,75 Hz, se transforme en singulet avec $CF_3COOD$) ; 3,7 (2H, s) ; 5,3 (1H, t, J = 6,75 Hz, échangeable avec $CF_3COOD$) ; 7,0 - 7,3 (4H, m) ;

7,3 - 7,8 (4H, m) ; 9,6 (1H, s large, échangeable avec $CF_3COOD$).

**Exemple 93**

**Acide 3-[[1-[[[(4-chlorophényl)sulfonyl]amino]méthyl]cyclopentyl]méthyl]benzèneacétique**

a) Acide 3-(bromométhyl)benzèneacétique

Obtenu en opérant comme dans l'exemple 92 a, à partir de 89 g (590 mmoles) d'acide 3-méthylbenzèneacétique commercial, de 123 g (767 mmoles) de brome dans 1200 ml de tétrachlorure de carbone. Après refroidissement, on obtient un précipité blanc que l'on essore. Un second jet est obtenu en ajoutant 1 l d'hexane au filtrat. Les deux précipités rassemblés représentent 40,5 g (Rdt = 30,0 %) d'un produit utilisé sans autre purification. F = 92 - 4 ° C. (Litt. : F = 117 - 20 ° C, selon Snim S.C. et coll., Bull. Korean Chem. Soc. (1988) 9, 185-7 ; CA 110, 74 976 j).
I.R. (KBr) : $\nu$ (C = O) = 1680 $cm^{-1}$.
R.M.N.($CDCl_3$) : $\delta$ = 3,65 (2H, s) ; 4,5 (2H, s) ; 7,25 (4H, s) ; 10,4 (1H, s, large, échangeable avec $CF_3COOD$).

b) 3-(Bromométhyl)benzèneéthanol

Obtenu en opérant comme dans l'exemple 25 a, à partir de 46,7 g (204 mmoles) du composé préparé dans l'exemple 93 a, de 17 g (224,4 mmoles) de complexe d'hydrure de bore-diméthylsulfure en solution 2M dans le toluène, dans 500 ml de tétrahydrofurane sec. On obtient 40 g (Rdt = 93,0 %) d'un solide jaune pâle, utilisé sans autre purification.
I.R. (KBr) : $\nu$ (OH) = 3350 $cm^{-1}$.
R.M.N.($CDCl_3$) : $\delta$ = 1,7 (1H, s, échangeable avec $D_2O$) ; 2,6 (2H, t, J = 6,75 Hz) ; 3,85 (2H, t, J = 6,75 Hz) ; 4,45 (2H, s) ; 7,2 (4H, s).

c) 1-Bromométhyl-3-[2-triméthylsilyloxy)éthyl]benzène

Obtenu en opérant comme dans l'exemple 26 a, à partir de 40 g (186 mmoles) du composé préparé dans l'exemple 93 b, de 30 g (186 mmoles) de 1,1,1,3,3,3-hexaméthyldisilazane, de 18,8 g (186 mmoles) de triéthylamine et de 20,2 g (186 mmoles) de chlorure de triméthylsilyle dans 400 ml de tétrahydrofurane sec. Après distillation, on obtient 45,5 g (Rdt = 85,2 %) d'un liquide incolore. $Eb_8$ = 98 - 104 ° C.
R.M.N.($CDCl_3$) : $\delta$ = 0,0 (9H, s) ; 2,75 (2H, t, J = 6,75 Hz) ; 3,7 (2H, t, J = 6,75 Hz) ; 4,4 (2H, s) ; 6,9 - 7,45 (4H, m).

d) 1-[[3-(2-Hydroxyéthyl)phényl]méthylcyclopentanecarbonitrile

Obtenu en opérant comme dans l'exemple 87 a, à partir de 7,8 g (77 mmoles) de diisopropylamine, de 48,2 ml de n-butyllithium en solution 1,6 M dans l'hexane, de 6,7 g (70 mmoles) de cyclopentanecarbonitrile commercial, de 18 ml de 1,3-diméthylimidazolidin-2-one et de 20,5 g (71,4 mmoles) du composé préparé dans l'exemple 93 c, dans 110 ml de tétrahydrofurane sec. Après distillation, on obtient 9,6 g (Rdt = 60,0 %) d'un liquide visqueux. $Eb_{0,4}$ = 160 ° C.
I.R. (film) : $\nu$ (OH) = 3420 $cm^{-1}$ ; (C≡N) = 2215 $cm^{-1}$.
R.M.N.($CDCl_3$) : $\delta$ = 1,5 - 2,15 (8H, m) ; 2,4 (1H, s, échangeable avec $D_2O$) ; 2,85 (2H, s); 2,85 (2H, t, J = 6,75 Hz) ; 3,8 (2H, t, J = 6,75 Hz) ; 7,0 - 7,3 (4H, m).

e) 3-[[1-(Aminométhyl)cyclopentyl]méthyl]benzèneéthanol

Obtenu en opérant comme dans l'exemple 15 a, à partir de 1,9 g (50,4 mmoles) de $LiAlH_4$, de 9,6 g (42 mmoles) du composé préparé dans l'exemple 93 d dans 55 ml d'éther. On obtient 5,0 g (Rdt = 51,0 %) d'une pâte fluide incolore, utilisée sans autre purification.
I.R. (film) : $\nu$ ($NH_2$) = 3350 et 3300 $cm^{-1}$.
R.M.N.($CDCl_3$) : $\delta$ = 1,25 - 1,75 (8H, m) ; 1,85 (3H, s, échangeables avec $D_2O$) ; 2,45 (2H, s) ; 2,6 (2H, s) ; 3,1 (2H, t, J = 6,75 Hz) ; 3,8 (2H, t, J = 6,75 Hz) ; 6,8 - 7,4 (4H, m).

f) 4-Chloro-N-[[1-[[3-(2-hydroxyéthyl)phényl]méthyl]cyclopentyl]méthyl]benzènesulfonamide

Obtenu en opérant comme dans l'exemple 32 d, à partir de 5 g (21,4 mmoles) du composé préparé dans l'exemple 93 e, de 2,7 g (26,7 mmoles) de triéthylamine et de 4,4 g (20,8 mmoles) de chlorure de 4-chlorobenzènesulfonyle dans 110 ml de dichlorométhane sec. Après purification par chromatographie sur colonne de silice dans un mélange dichlorométhane-méthanol (98:2), on obtient 3,5 g (Rdt = 42,5 %) d'un solide pâteux blanc.

I.R. (film) : $\nu$ (OH) = 3450 $cm^{-1}$ ; (NH) = 3290 $cm^{-1}$ ; ($SO_2$) = 1320 $cm^{-1}$ ; ($SO_2$) = 1150 $cm^{-1}$.

R.M.N.($CDCl_3$) : $\delta$ = 1,1 - 1,9 (9H, m, dont 1H échangeable avec $D_2O$) ; 2,6 (2H, s) ; 2,5 - 3,0 (4H, m) ; 3,8 (2H, t, J = 6,75 Hz) 5,1 (1H, t, J = 6,75 Hz, échangeable avec $D_2O$) ; 6,75 - 7,2 (4H, m) ; 7,3 - 7,5 (2H, m) ; 7,6 - 7,9 (2H, m).

g) Acide 3-[[1-[[[(4-chlorophényl)sulfonyl]amino]méthyl]cyclopentyl]méthyl]benzèneacétique

Obtenu en opérant comme dans l'exemple 25 g, à partir de 3,5 g (8,6 mmoles) du composé préparé dans l'exemple 93 f, de 8,6 ml (17,2 mmoles) de réactif de Jones dans 110 ml d'acétone. Après purification puis recristallisations dans un mélange éther isopropylique-éthanol, on obtient 1,4 g (Rdt = 52,8 %) d'un solide blanc.

F = 139,5 - 40 °C.

| Analyse centésimale : $C_{21}H_{24}ClNO_4S$ (M = 421,939) | | | | | |
|---|---|---|---|---|---|
| | C % | H % | Cl % | N % | S % |
| Calculé | 59,78 | 5,73 | 8,40 | 3,32 | 7,60 |
| Trouvé | 59,63 | 5,87 | 8,38 | 3,31 | 7,76 |

I.R. (KBr) : $\nu$ (NH) = 3270 $cm^{-1}$ ; (C=0) = 1680 $cm^{-1}$ ; ($SO_2$) = 1315 $cm^{-1}$ ; ($SO_2$) = 1140 $cm^{-1}$.

R.M.N.($CDCl_3$ + DMSO $d_6$) : $\delta$ = 1,1 - 1,7 (8H, m) ; 2,4 - 2,75 (4H, m) ; 3,5 (2H, s) ; 6,9 - 7,2 (4H, m) ; 7,2 - 7,6 (3H, m, dont 1H échangeable aveu $CF_3COOD$) ; 7,7 - 8,0 (2H, m) ; 9,6 (1H, s large, échangeable avec $CF_3COOD$).

**Exemples 94 à 102**

Les composés des exemples 94 à 102 :
Acide 4-[[1-[[[(4-hexylphényl)sulfonyl]amino]méthyl]cyclopentyl]méthyl]benzèneacétique
Acide 4-[[1-[[[(4-nitrophényl)sulfonyl]amino]méthyl]cyclopentyl]méthyl]benzèneacétique
Acide 4-[[1-[[[(naphtalen-1-yl)sulfonyl]amino]méthyl]cyclopentyl]méthyl]benzèneacétique
Acide 4-[[1-[[[(4-hexyloxyphényl)sulfonyl]amino]méthyl]cyclopentyl]méthyl]benzèneacétique
Acide 4-[[1-[[[(2-fluorophényl)sulfonyl]amino]méthyl]cyclopentyl]méthyl]benzèneacétique
Acide 4-[[1-[[[(4-cyclohexylphényl)sulfonyl]amino]méthyl]cyclopentyl]méthyl]benzèneacétique
Acide 4-[[1-[[[(pentafluorophényl)sulfonyl]amino]méthyl]cyclopentyl]méthyl]benzèneacétique
Acide 4-[[1-[[([1,1'-biphenyl-4-yl]sulfonyl)amino]méthyl]cyclopentyl]méthyl]benzèneacétique
Acide 4-[[1-[[[(naphtalen-2-yl)sulfonyl]amino]méthyl]cyclopentyl]méthyl]benzèneacétique
ont été préparés en 2 stades à partir de la 1-[[4-[2-[(3,4,5,6-tétrahydro-2H-pyran-2-yl)oxy]éthyl]phényl]-méthyl]cyclopentaneméthanamine préparée dans l'exemple 85 b :

a) Sulfonation en opérant comme dans l'exemple 1 c, à l'aide du sulfochlorure correspondant ;
b) Oxydation de Jones en opérant comme dans l'exemple 25 g.

Les caractéristiques des composés obtenus (A = COOH) et de leurs intermédiaires (A = $CH_2O$-THP)-*sont données dans les tableaux IIIa à IIIe :

* THP = 3,4,5,6-tétrahydro-2H-pyran-2-yl-

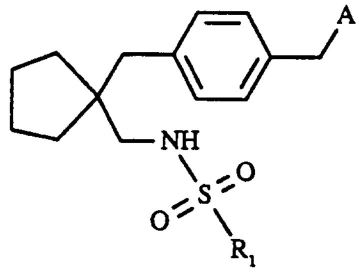

Tableau IIIa

| Ex. N° | $R_1$ | A | Formule brute[a] | F (°C) | RMN ($\delta$,$CDCl_3$) |
|---|---|---|---|---|---|
| 94a | 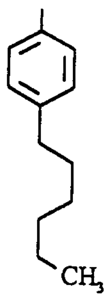 | $CH_2OTHP$ | | solide pâteux | 0,65 - 2,0 (25H, m) ; 2,6 (2H, s) ; 2,7 (2H, d, J = 6,75 Hz, se transforme en s avec $CF_3COOD$); 2,85 (4H, t, J = 6,75 Hz) ; 3,25 - 4,2 (4H, m) ; 4,35 - 4,7 (2H, m, dont 1H échangeable avec $CF_3COOD$) ; 6,8 - 7,1 (4H, m) ; 7,2 - 7,45 (2H, m) ; 7,65 - 7,9 (2H, m) |
| 94b | | COOH | $C_{27}H_{37}NO_4S$ (471,656) | 135-137 (acétate d'éthyle -hexane) | (DMSO $d_6$) : 0,65 - 2,15 (19H, m) ; 2,4 - 3,0 (6H, m) ; 3,5 (2H, s) ; 7,1 (4H, s) ; 7,2 - 7,9 (5H, m, dont 1H échangeable avec $CF_3COOD$) ; 12,25 (1H, s large, échangeable avec $CF_3COOD$) |
| | | | | | |
| 95a | 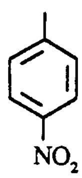 | $CH_2OTHP$ | $C_{26}H_{34}N_2O_6S$ (502,626) | 108-111 (acétate d'éthyle -hexane) | 1,25 - 2,0 (14H, m) ; 2,6 (2H, s) ; 2,7 - 3,0 (4H, m) ; 3,35 - 4,1 (4H, m) ; 4,6 (1H, m) ; 4,9 (1H, t, J = 6,75 Hz, échangeable avec $CF_3COOD$) ; 6,75 - 7,25 (4H, m) ; 7,85 - 8,1 (2H, m) ; 8,2 - 8,45 (2H, m) |
| 95b | | COOH | $C_{21}H_{24}N_2O_6S$ (432,491) | 131-134 (acétate d'éthyle -hexane) | 1,3 - 1,75 (8H, m) ; 2,55 (2H, s) ; 2,7 (2H, d, J = 6 Hz, se transforme en s avec $CF_3COOD$) ; 3,6 (2H, s) ; 5,0 (1H, t, J = 6,0 Hz, échangeable avec $CF_3COOD$) ; 6,9 - 7,2 (4H, m) ; 7,8 - 8,1 (2H, m) ; 8,15 - 8,4 (2H, m) ; 8,9 (1H, s large, échangeable avec $CF_3COOD$) |

[a] Analyse centésimale : C, H, N, S ± 0,19

Tableau IIIb

| Ex. N° | $R_1$ | A | Formule brute[a] | F (°C) | RMN ($\delta$, $CDCl_3$) |
|---|---|---|---|---|---|
| 96a | 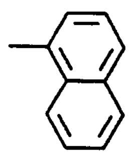 | $CH_2OTHP$ | $C_{30}H_{37}NO_4S$ (507,689) | 104-106 (acétate d'éthyle -hexane) | 1,25 - 1,75 (14H, m) ; 2,45 (2H, s) ; 2,6 (2H, d, J = 6,75 Hz, se transforme en s avec $CF_3COOD$); 2,75 (2H, t, J = 6,75 Hz) ; 3,3 - 4,2 (4H, m) ; 4,45 - 4,9 (2H, m, dont 1H échangeable avec $CF_3COOD$) ; 6,65 - 7,0 (4H, m) ; 7,2 - 8,7 (7H, m) |
| 96b | | COOH | $C_{25}H_{27}NO_4S$ (437,554) | 151-154 (acétate d'éthyle -hexane) | 1,25 - 1,6 (8H, m) ; 2,5 (2H, s) ; 2,6 (2H, d, J = 6,75 Hz, se transforme en s avec $CF_3COOD$) ; 3,55 (2H, s) ; 4,9 (1H, t, J = 6,75 Hz, échangeable avec $CF_3COOD$) ; 6,7 - 7,1 (4H, m) ; 7,3 - 8,8 (7H, m) ; 9,3 (1H, s large, échangeable avec $CF_3COOD$) |
| | | | | | |
| 97a | 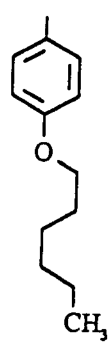 | $CH_2OTHP$ | $C_{32}H_{47}NO_5S$ (557,79) | 83-85 (acétate d'éthyle -hexane) | 0,75 - 2,1 (25H, m) ; 2,5 - 3,1 (6H, m) ; 3,3 - 4,25 (6H, m) ; 4,35 - 4,75 (2H, m, dont 1H échangeable avec $CF_3COOD$) ; 6,8 - 7,3 (6H, m) ; 7,65 - 7,9 (2H, m) |
| 97b | | COOH | $C_{27}H_{37}NO_5S$ (487 655) | 137,5-138,5 (acétate d'éthyle -hexane) | (acétone $d_6$) : 0,75 - 1,9 (19H, m) ; 2,65 (2H, s) ; 2,7 (2H, d, J = 6,75 Hz, se transforme en s avec $CF_3COOD$) ; 3,6 (2H, s) ; 4,1 (2H, t, J = 6,75 Hz) ; 6,1 (1H, t, J = 6,75 Hz, échangeable avec $CF_3COOD$) ; 6,9 - 7,2 (2H, m) ; 7,15 (4H, s) ; 7,6 - 7,9 (2H, m) ; 10,3 (1H, s large, échangeable avec $CF_3COOD$) |

[a] Analyse centésimale : C, H, N, S ± 0,29

Tableau IIIc

| Ex. N° | $R_1$ | A | Formule brute[a] | F (°C) | RMN ($\delta$,$CDCl_3$) |
|---|---|---|---|---|---|
| 98a | 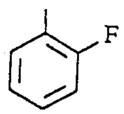 | $CH_2OTHP$ | $C_{26}H_{34}FNO_4S$ (475,619) | 98-100 (acétate d'éthyle -hexane) | 1,3 - 1,8 (14H, m) ; 2,6 (2H, s) ; 2,75 (2H, d, J = 6,75 Hz, se transforme en s avec $CF_3COOD$) ; 2,9 (2H, t, J = 6,75 Hz), 3,35 - 4,2 (4H, m) ; 4,5 - 4,8 (2H, m, dont 1H échangeable avec $CF_3COOD$) ; 7,1 (4H, s) ; 7,15 - 8,1 (4H, m) |
| 98b | | COOH | $C_{21}H_{24}FNO_4S$ (405,484) | 154-155 (acétate d'éthyle -hexane) | (acétone $d_6$) : 1,3 - 1,8 (8H, m) ; 2,7 (2H, s) ; 2,8 (2H, d, J = 6,75 Hz, se transforme en s avec $CF_3COOD$); 3,55 (2H, s) ; 6,5 (1H, t, J = 6,75 Hz, échangeable avec $CF_3COOD$) ; 7,2 (4H, s) ; 7,25 - 8,1 (4H, m) ; 10,5 (1H, s large, échangeable avec $CF_3COOD$) |
| | | | | | |
| 99a | 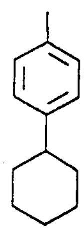 | $CH_2OTHP$ | $C_{32}H_{45}NO_4S$ (539,775) | 108-109 (acétate d'éthyle -hexane) | (acétone $d_6$) ; 1,1 - 2,0 (24H, m) ; 2,6 (2H, s) ; 2,6 - 2,9 (5H, m) ; 3,15 - 4,0 (4H, m) ; 4,55 (1H, m) ; 6,15 (1H, t, J = 6,75 Hz, échangeable avec $CF_3COOD$) ; 7,0 (4H, s) ; 7,25 - 7,5 (2H, m) ; 7,65 - 7,9 (2H, m) |
| 99b | | COOH | $C_{27}H_{35}NO_4S$ (469,64) | 145 - 147 (acétate d'éthyle -hexane) | (acétone $d_6$) : 1,15 - 2,0 (18H, m) ; 2,7 (2H, s) ; 2,6- 2,9 (3H, m) ; 3,55 (2H, s) ; 6,25 (1H, t, J = 6 Hz, échangeable avec $CF_3COOD$) ; 7,15 (4H, s) ; 7,3 - 7,55 (2H, m) ; 7,65 - 7,9 (2H, m) ; 10,65 (1H, s large, échangeable avec $CF_3COOD$) |

[a] Analyse centésimale : C, H, F, N, S ± 0,34 sauf exemple 98 a, S = + 0,56

Tableau IIId

| Ex. N° | $R_1$ | A | Formule brute[a] | F (°C) | RMN ($\delta$, $CDCl_3$) |
|---|---|---|---|---|---|
| 100 a | 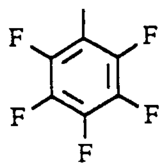 | $CH_2OTHP$ | $C_{26}H_{30}F_5NO_4S$ (547,579) | 95-96 (hexane) | 1,3 - 1,9 (14H, m) ; 2,6 (2H, s) ; 2,7 - 3,1 (4H, m) ; 3,3 - 4,0 (4H, m) ; 4,6 (1H, m) ; 4,9 (1H, t, J = 6,75 Hz, échangeable avec $D_2O$) ; 7,1 (4H, s) |
| 100 b | | COOH | $C_{21}H_{20}F_5NO_4S$ (477,444) | 129-130 (acétate d'éthyle -hexane) | (acétone $d_6$) : 1,4 - 1,8 (8H, m) ; 2,75 (2H, s) ; 3,0 (2H, d, J = 6,75 Hz, se transforme en s avec $CF_3COOD$); 3,6 (2H, s) ; 6,6 (1H, t, J = 6,75 Hz, échangeable avec $CF_3COOD$) ; 7,2 (4H, s) ; 10,6 (1H, s large, échangeable avec $CF_3COOD$) |
| | | | | | |
| 101 a | 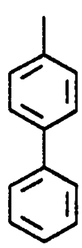 | $CH_2OTHP$ | $C_{32}H_{39}NO_4S$ (533,727) | 118-120 (acétate d'éthyle -hexane) | 1,0 - 2,0 (14H, m) ; 2,55 (2H, s) ; 2,7 (2H, d, J = 6,75 Hz, se transforme en s avec $CF_3COOD$) ; 2,8 (2H, t, J = 6,75 Hz) ; 3,3 - 4,0 (4H, m) ; 4,4 - 4,9 (2H, m, dont 1H échangeable avec $CF_3COOD$) ; 6,75 - 7,2 (4H, m) ; 7,25 - 8,0 (9H, m) |
| 101 b | | COOH | $C_{27}H_{29}NO_4S$ + 1/4 $H_2O$ (472,095) | 170 - 172 (acétate d'éthyle) | ($CDCl_3$ + DMSO $d_6$) : 1,15 - 1,7 (8H, m) ; 2,45 - 2,75 (4H, m) ; 3,45 (2H, s) ; 6,75 - 7,1 (5H, m, dont 1H échangeable avec $CF_3COOD$) ; 7,25 - 8,1 (9H, m) ; 9,4 (1H, s large, échangeable avec $CF_3COOD$) |

[a] Analyse centésimale : C, H, F, N, S ± 0,30 sauf exemple 101b, S = - 0,39

Tableau IIIe

| Ex. N° | $R_1$ | A | Formule brute[a] | F (°C) | RMN (δ, $CDCl_3$) |
|---|---|---|---|---|---|
| 102 a | 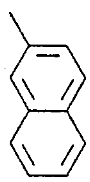 | $CH_2OTHP$ | $C_{30}H_{37}NO_4S$ (507,689) | 132,5 - 133 (acétate d'éthyle) | 1,25 - 2,0 (14H, m) ; 2,55 (2H, s) ; 2,75 (2H, d, J = 6,75 Hz, se transforme en s avec $CF_3COOD$) ; 2,8 (2H, t, J = 6,75 Hz) ; 3,25 - 4,1 ( 4H, m) ; 4,55 (1H, m) ; 4,85 (1H, t, J = 6,75 Hz, échangeable avec $CF_3COOD$) ; 6,9 (4H, s) ; 7,4 - 8,1 (6H, m) ; 8,4 (1H, m) |
| 102 b | | COOH | $C_{25}H_{27}NO_4S$ (437,554) | 131,5 -132,5 (acétate d'éthyle) | 1,2 - 1,8 (8H, m) ; 2,6 (2H, s) ; 2,7 (2H, d, J = 6,75 Hz, se transforme en s avec $CF_3COOD$) ; 3,55 (2H, s) ; 5,2 (1H, t, J= 6,75 Hz, échangeable avec $CF_3COOD$) ; 7,0 (4H, s) ; 7,5 - 8,1 (6H, m) ; 8,35 (1H, m) ; 10,0 (1H, s large, échangeable avec $CF_3COOD$) |

[a] Analyse centésimale : C, H, N, S ± 0,29

## Exemple 103

**Acide 4-[[1-[[[(4-méthylthiophényl)sulfonyl]amino]méthyl]cyclopentyl]méthyl]benzèneacétique**

a) N-[[1-(Phénylméthyl)cyclopentyl]méthyl]-acétamide

A un mélange de 22,7 g (120 mmoles) de l'amine préparée dans l'exemple 15a et de 82 ml de pyridine, on ajoute goutte à goutte, 9,85 g (126 mmoles) de chlorure d'acétyle, en maintenant la température à 30 °C. Puis on porte à reflux 3/4 h. Après refroidissement, on jette sur un mélange glace - HCl concentré, avant d'extraire par le dichlorométhane. La phase organique lavée par l'eau, séchée par $Na_2SO_4$ puis concentrée, est purifiée par distillation, pour donner 23,8 g (Rdt = 85,6 %) d'une huile jaune épaisse, qui cristallise. $Eb_1$ = 200 - 10° C. F = 70 - 2 °C (acétate d'éthyle - pentane).
I.R. (film) : ν (NH) = 3300 $cm^{-1}$ ; (C=O) = 1630 $cm^{-1}$.
R.M.N.($CDCl_3$) : δ = 1,25 - 1,75 (8H, m) ; 1,8 (3H, s) ; 2,6 (2H,s) ; 3,1 (2H, d, J = 6 Hz, se transforme en singulet avec $CF_3COOD$) ; 5,5 (1H, m, échangeable avec $CF_3COOD$) 7,15 (5H, s).

b) N-[[-1-[(4-Acétylphényl)méthyl]cyclopentyl]méthyl]-acétamide

A un mélange, sous azote et maintenu à - 10 °C, de 183,2 g (0,792 mole) du composé préparé dans l'exemple 103 a, de 80,5 g (1,03 mole) de chlorure d'acétyle et de 3 l de dichlorométhane, on ajoute par portions 316,8 g (2,376 moles) de chlorure d'aluminum. On laisse remonter à température ambiante, et on agite 20 h, avant de verser sur un mélange glace - HCl concentré. On extrait par le dichlorométhane. La phase organique lavée par une solution saturée de $NaHCO_3$, puis à l'eau, séchée sur $Na_2SO_4$ et concentrée, est purifiée par recristallisation dans l'acétate d'éthyle, pour donner 177,9 g (Rdt = 82,2 % )

d'un solide jaune. F = 136,7 - 8,7 °C.
I.R. (KBr) : ν (NH) = 3270 $cm^{-1}$ ; (C=O) = 1655 $cm^{-1}$ ; (C=O) = 1615 $cm^{-1}$.
R.M.N.($CDCl_3$) : δ = 1,25 - 1,75 (8H, m) ; 1,9 (3H, s) ; 2,5 (3H, s) ; 2,65 (2H, s) ; 3,1 (2H, d, J = 6 Hz, se transforme en singulet avec $CF_3COOD$) ; 5,6 (1H, m, échangeable avec $CF_3COOD$) ; 7,1 - 7,35 (2H, m) ; 7,7 - 8,0 (2H, m).

c) 4-[[1-[(N-Acétylamino)méthyl]cyclopentyl]méthyl]benzèneacétate de méthyle

Obtenu en opérant comme dans l'exemple 5 e, à partir de 177,9 g (0,651 mole) du composé préparé dans l'exemple 103 b, de 290 ml de méthanol, de 409 g (2,88 moles) d'éthérate de trifluorure de bore, de 433 g (0,976 mole) de tétraacétate de plomb dans 2,8 l de dichlorométhane. On obtient 195,6 g (Rdt = 99,0 %) d'un solide beige, utilisé sans autre purification. F = 99 °C.
I.R. (KBr) : ν (NH) = 3280 $cm^{-1}$ ; (C=O) = 1700 $cm^{1}$ ; (C=O) = 1610 $cm^{-1}$.
R.M.N.($CDCl_3$) : δ = 1,25 - 1,75 (8H, m) 1,9 (3H, s) ; 2,6 (2H, s) ; 3,1(2H, d, J = 6 Hz, se transforme en singulet avec $CF_3COOD$) 3,6 (2H, s) ; 3,7 (3H, s) ; 5,35 (1H, m, échangeable avec $CF_3COOD$) ; 7,15 (4H, s).

d) Acide 4-[[1-(aminométhyl)cyclopentyl]méthyl]benzèneacétique

Un melange de 2,5 g (8,2 mmoles) du composé préparé dans l'exemple 103 c et de 60 ml (110 mmoles) de NaOH 1,83 N, est porté à reflux 7 heures. Après refroidissement, le milieu réactionnel est lavé à l'éther, avant d'être acidifié à pH 6 par HCl 10N. Le précipité formé est filtré et lavé à l'eau froide. Le filtrat concentré jusqu'à un volume de 30 ml est abandonné une nuit à 4 °C, pour donner un nouveau précipité qui est filtré et lavé à l'eau. Les précipités rassemblés sont séchés sous vide à 110 °C et donnent 1,7 g (Rdt = 85,0 %) d'une poudre blanche utilisée sans autre purification. F = 218 °C (dec.).
R.M.N.($CF_3COOD$) : δ = 1,25 - 2,1 (8H, m) ; 2,6 (2H, s) ; 3,2 (2H, m) ; 3,8 (2H, s); 7,25 (4H, s).

e) 4-[[1-(Aminométhyl)cyclopentyl]méthyl]benzèneacétate de méthyle

Obtenu en opérant comme dans l'exemple 1 a, à partir de 9,9 g (46 mmoles) du composé préparé dans l'exemple 103 d dans 60 ml de méthanol. On obtient 8,7 g (Rdt = 82,8 %) d'un liquide épais brun. $Eb_{0,5}$ = 150 - 200 °C.
I.R. (film) : ν ($NH_2$) = 3375 $cm^{-1}$ ; (C=O) = 1720 $cm^{-1}$.
R.M.N.($CDCl_3$) : δ = 1,25 (2H, s, échangeables avec $CF_3COOD$) ; 1,25 - 1,75 (8H, m) ; 2,45 (2H, s) ; 2,6 (2H, s) ; 3,6 (2H, s) ; 3,65 (3H, s) ; 7,1 (4H, s).

f) 4-[[1-[[[(4-Méthylthiophényl)sulfonyl]amino]méthyl]cyclopentyl]méthyl]benzèneacétate de méthyle

Obtenu en opérant comme dans l'exemple 32 d, à partir de 4 g (16 mmoles) du composé préparé dans l'exemple 103 e, de 1,9 g (18,8 mmoles) de triéthylamine, de 3,55 g (15,9 mmoles) de chlorure de 4-(méthylthio)benzènesulfonyle (préparé selon Burton H. et Hu P.F., J. Chem. Soc. (1948), 604-5) dans 70 ml de dichlorométhane sec. Après recristallisation dans un mélange acétate d'éthyle-hexane, on obtient 4,1 g (Rdt = 56,9 %) d'un solide jaune pâle. F = 109 - 14 °C.
I.R. (KBr) : ν (NH) = 3280 $cm^{-1}$ ; (C=0) = 1705 $cm^{-1}$ ; ($SO_2$) = 1320 $cm^{-1}$ ; ($SO_2$) = 1155 $cm^{-1}$.
R.M.N.($CDCl_3$) : δ = 1,25 - 1,75 (8H, m) ; 2,5 (3H, s) ; 2,6 (2H, s) ; 2,7 (2H, d, J = 6,75 Hz, se transforme en singulet avec $D_2O$) ; 3,5 (2H, s) ; 3,7 (3H, s) ; 4,6 (1H, t, J = 6,75 Hz, echangeable avec $D_2O$) ; 6,9 - 7,4 (6H, m) ; 7,55 - 7,8 (2H, m).

g) Acide 4-[[1-[[[(4-méthylthiophényl)sulfonyl]amino]méthyl]cyclopentyl]méthyl]benzèneacétique

Obtenu en opérant comme dans l'exemple 1 d, à partir de 4,1 g (9,2 mmoles) du composé préparé dans l'exemple 103 f, de 0,8 g de KOH en pastilles, de 60 ml d'éthanol et de 60 ml d'eau. Après recristallisation dans le toluène puis dans un mélange acétate d'éthyle-hexane, on obtient 0,7 g (Rdt = 17,6 %) d'un solide blanc cassé. F = 138 - 9 °C.

| Analyse centésimale : $C_{22}H_{27}NO_4S_2$ (M = 433,59) | | | | |
|---|---|---|---|---|
| | C % | H % | N % | S % |
| Calculé | 60,94 | 6,28 | 3,23 | 14,79 |
| Trouvé | 60,58 | 6,35 | 3,18 | 15,05 |

I.R. (KBr) : ν (NH) = 3270 $cm^{-1}$ ; (C=0) = 1715 $cm^{-1}$ ; ($SO_2$) = 1305 $cm^{-1}$ ; ($SO_2$) = 1150 $cm^{-1}$.
R.M.N.($CDCl)_3$ : δ = 1,25 - 1,75 (8H, m) ; 2,5 (3H, s) ; 2,5 - 2,8 (4H, m) ; 3,6 (2H, s) ; 5,0 (1H, t, J = 6,0 Hz, échangeable avec $D_2O$) ; 6,85 - 7,4 (6H, m) ; 7,6 - 7,9 (2H, m) ; 9,5 (1H, s large, échangeable avec $D_2O$).

**Exemple 104**

**Acide 4-[[1-[[[[5-(diméthylamino)naphtalen-1-yl]sulfonyl]amino]méthyl]cyclopentyl]méthyl]-benzèneacétique**

a) 4-[[1-[[[[5-(Diméthylamino)naphtalen-1-yl]sulfonyl]amino]méthyl]cyclopentyl]méthyl]benzèneacétate de méthyle

Obtenu en opérant comme dans l'exemple 32 d, à partir de 2,8 g (10,7 mmoles) du composé préparé dans l'exemple 103 e, de 1,3 g (12,8 mmoles) de triéthylamine, de 2,8 g (10,5 mmoles) de chlorure de 5-(diméthylamino)naphtalènesulfonyle commercial, dans 56 ml de dichlorométhane sec. Après purification par chromatographie sur colonne de silice dans un mélange hexane-acétate d'éthyle (1:1), puis recristallisation dans un mélange hexane-acétate d'éthyle, on obtient 0,9 g (Rdt = 19,5 %) d'un solide jaune. F = 124 - 6°C.
I.R. (KBr) : ν (NH) = 3300 $cm^{-1}$ ; (C=O) = 1705 $cm^{-1}$ ($SO_2$) = 1315 $cm^{-1}$ ; ($SO_2$) = 1115 $cm^{-1}$.
R.M.N.($CDCl_3$) : δ = 1,05 - 1,6 (8H, m) ; 2,5 (2H, s) ; 2,6 (2H, d, J = 6,75 Hz, se transforme en singulet avec $CF_3COOD$) ; 2,9 (6H, s) ; 3,5 (2H, s) ; 3,7 (3H, s) ; 4,65 (1H, t, J = 6,75 Hz, échangeable avec $CF_3COOD$) ; 6,75 - 7,75 (7H, m) ; 8,1 - 8,7 (3H, m).

b) Acide 4-[[1-[[[[5-(diméthylamino)naphtalen-1-yl]sulfonyl]amino]méthyl]cyclopentyl]méthyl]-benzèneacétique

Obtenu en opérant comme dans l'exemple 1d, à partir de 0,9 g (2,1 mmoles) du composé préparé dans l'exemple 104 a, de 0,18 g (3,1 mmoles) de KOH en pastilles, de 90 ml d'eau et 90 ml d'éthanol. Après recristallisations dans l'acétone, on obtient 0,2 g (Rdt = 20,0 %) d'un solide jaune. F = 178 - 81°C.

| Analyse centésimale : $C_{27}H_{32}N_20_4S$ (M = 480,621) | | | | |
|---|---|---|---|---|
| | C % | H % | N % | S % |
| Calculé | 67,47 | 6,71 | 5,83 | 6,67 |
| Trouvé | 67,40 | 6,93 | 5,90 | 6,56 |

I.R. (KBr) : ν (NH) = 3300 $cm^{-1}$ ; (C=O) = 1680 cm-1 ; ($SO_2$) = 1310 $cm^{-1}$ ; ($SO_2$) = 1150 $cm^{-1}$.
R.M.N.(DMSO $d_6$) : δ = 1,1 - 1,55 (8H, m) ; 2,3 - 2,7 (4H, m) ; 2,8 (6H, s) ; 3,4 (2H, s) ; 6,9 (4H, s) ; 7,15 - 8,6 (7H, m, dont 1H échangeable avec $CF_3COOD$) ; 12,1 (1H, s large, échangeable avec $CF_3COOD$).

**Exemple 105**

**Acide 4-[[1-[[[(4-chloro-2-fluorophényl)sulfonyl]amino]méthyl]cyclopentyl]méthyl]benzèneacétique**

a) Chlorure de 4-chloro-2-fluorobenzènesulfonyle

Dans un mélange, maintenu à - 10°C, de 75 ml d'HCl 10 N et de 22 ml d'acide acétique, on ajoute 32,5 g (223 mmoles) de 4-chloro-2-fluoroaniline commerciale, puis 16,7 g (241 mmoles) de nitrite de sodium en solution dans 26 ml d'eau. Ce milieu réactionnel est ajouté par fractions à une solution maintenue à 10°C de 220 ml d'acide acétique saturée en $SO_2$ et contenant 5,5 g (56,3 mmoles) de

chlorure cuivreux (I). On laisse sous agitation 1/2 heure à température ambiante, avant de verser sur un mélange glace-eau et d'extraire par l'éther. La phase organique est lavée par une solution saturée par $NaHCO_3$, séchée sur $Na_2SO_4$ et concentrée. Après distillation, on obtient 44,5 g (Rdt = 87,1 %) d'un liquide jaune pâle qui cristallise. $Eb_{0,1}$ = 81 °C. F = 36 - 8 °C.

| Analyse centésimale : $C_6H_3Cl_2FO_2S$ (M = 229,052) | | | | | |
|---|---|---|---|---|---|
| | C % | H % | Cl % | F % | S % |
| Calculé | 31,46 | 1,32 | 30,96 | 8,29 | 14,00 |
| Trouvé | 31,51 | 1,09 | 31,12 | 8,29 | 13,88 |

I.R. (film) : ν ($SO_2$) = 1375 $cm^{-1}$ ; ($SO_2$) = 1175 $cm^{-1}$.
R.M.N.($CDCl_3$) : δ = 7,1 - 7,5 (2H, m) ; 7,7 - 8,1 (1H, m).

b) Acide 4-[[1-[[[(4-chloro-2-fluorophényl)sulfonyl]amino]méthyl]cyclopentyl]méthyl]benzèneacétique

Un mélange de 3 g (10 mmoles) du composé préparé dans l'exemple 103 c, de 5,6 g (140 mmoles) de NaOH en pastilles et de 72 ml d'eau, est porté à reflux durant 4 heures. Après refroidissement à 40 °C, on ajoute 2,3 g (10 mmoles) du composé préparé dans l'exemple 105 a, puis on laisse sous agitation 16 heures, à température ambiante. On rajoute 0,46 g (2 mmoles) du composé préparé dans l'exemple 105 a, et on agite encore 6 heures à température ambiante. Le milieu réactionnel est alors lavé à l'éther, et acidifié par l'acide chlorhydrique. Le précipité obtenu est essoré, lavé à l'eau et séché, avant d'être purifié par chromatographie sur colonne de silice dans l'acétate d'éthyle puis par recristallisations dans un mélange acétate d'éthyle-hexane, pour donner 0,8 g (Rdt = 18,2 %) d'un solide blanc. F = 151 - 3 °C,

| Analyse centésimale : $C_{21}H_{23}ClFNO_4S$ (M = 439,929) | | | | | | |
|---|---|---|---|---|---|---|
| | C % | H % | Cl % | F % | N % | S % |
| Calculé | 57,33 | 5,27 | 8,06 | 4,32 | 3,18 | 7,29 |
| Trouvé | 57,50 | 5,16 | 8,13 | 4,40 | 3,21 | 7,26 |

I.R. (KBr) : ν (NH) = 3310 $cm^{-1}$ ; (C=O) = 1690 $cm^{-1}$ = 1335 $cm^{-1}$ ; ($SO_2$) = 1155 $cm^{-1}$.
R.M.N.(acétone $d_6$) : δ = 1,3 - 1,75 (8H, m) ; 2,7 (2H, s) ; 2,8 (2H, d, J = 6 Hz, se transforme en singulet avec $CF_3COOD$) ; 3,6 (2H, s) ; 6,6 (1H, t, J = 6 Hz, échangeable avec $CF_3COOD$) ; 7,2 (4H, s) ; 7,3 - 8,0 (3H, m) ; 10,5 (1H, s large, échangeable avec $CF_3COOD$).

## Exemple 106

**Acide 4-[[1-[[[(quinol-8-yl)sulfonyl]amino]méthyl]cyclopentyl]méthyl]benzèneacétique**

Obtenu en opérant comme dans l'exemple 105 b, à partir de 3,0 g (10 mmoles) du composé préparé dans l'exemple 103 c, de 4,8 g (120 mmoles) de NaOH, de 2,3 g (10 mmoles) de chlorure de (quinol-8-yl)-sulfonyle dans 21 ml d'eau. Après 3 recristallisations dans un mélange éthanol-eau, on obtient 1,1 g (Rdt = 25,0 %) de fines aiguilles blanc cassé. F > 320 °C (dec. à partir de 200 °C).

| Analyse centésimale : $C_{24}H_{26}N_2O_4S$ (M = 438,542) | | | | |
|---|---|---|---|---|
| | C % | H % | N % | S % |
| Calculé | 65,73 | 5,98 | 6,39 | 7,31 |
| Trouvé | 65,77 | 6,02 | 6,23 | 7,39 |

I.R. (KBr) : ν (NH) = 3250 $cm^{-1}$ ; (C=O) = 1705 $cm^{-1}$ ; ($SO_2$) = 1300 $cm^{-1}$ ; ($SO_2$) = 1130 $cm^{-1}$.
R.M.N.(DMSO $d_6$) : δ = 1,1 - 1,6 (8H, m) ; 2,4 - 2,7 (4H, m) ; 3,45 (2H, s)) ; 7,0 (4H, s) ; 7,0 (1H, m, échangeable avec $CF_3COOD$) ; 7,5 - 7,9 (2H, m) ; 8,2 - 8,6 (3H, m) ; 9,05 (1H, m) ; 11,8 (1H, s large, échangeable avec $CF_3COOD$).

**Exemple 107**

**Acide 4-[[1-[[[(4-bromophényl)sulfonyl]amino]méthyl]cyclobutyl]méthyl]benzèneacétique**

a) N-[[1-(Phénylméthyl)cyclobutyl]méthyl]-acétamide

Obtenu en opérant comme dans l'exemple 103a, à partir de 55,8 g (318 mmoles) de l'amine préparée dans l'exemple 27c, de 26,2 g (333,8 mmoles) de chlorure d'acétyle dans 200 ml de pyridine. Après trituration dans l'hexane, on obtient 59,4 g (Rdt = 86,0 %) d'un solide crème. F = 74 - 6 °C.
I.R. (KBr) : $\nu$ (NH) = 3310 $cm^{-1}$ ; (C=0) = 1620 $cm^{-1}$.
R.M.N.($CDCl_3$) : $\delta$ = 1,5 - 2,0 (9H, m) ; 2,7 (2H, s) ; 3,25 (2H, d, J = 6 Hz, se transforme en singulet avec $CF_3COOD$) ; 5,4 (1H, m, échangeable avec $CF_3COOD$) ; 7,2 (5H, s).

b) N-[[1-[(4-Acétylphényl)méthyl]cyclobutyl]méthyl]-acétamide

Obtenu en opérant comme dans l'exemple 103b, à partir de 59 g (271 mmoles) du composé préparé dans l'exemple 107a, de 27,5 g (350 mmoles) de chlorure d'acétyle et de 108 g (813 mmoles) de chlorure d'aluminium dans 1000 ml de dichlorométhane. Après recristallisation dans le toluène, on obtient 55,6 g (Rdt = 79,4 %) d'un solide jaune.
F = 106 - 110 °C.
I.R. (KBr) : $\nu$ (NH) = 3310 $cm^{-1}$ ; (C=O) = 1660 $cm^{-1}$ ; (C=0) = 1625 $cm^{-1}$.
R.M.N.($CDCl_3$) : $\delta$ = 1,6 - 2,1 (6H, m) ; 1,95 (3H, s) ; 2,55 (3H, s) ; 2,75 (2H, s) ; 3,25 (2H, d, J = 6 Hz, se transforme en singulet avec $CF_3COOD$) ; 5,7 (1H, m, échangeable avec $CF_3COOD$) ; 7,05 - 7,4 (2H, m) ; 7,7 - 8,0 (2H, m).

c) 4-[[1-[(N-Acétyl-amino)méthyl]cyclobutyl]méthyl]benzèneacétate de méthyle

Obtenu en opérant comme dans l'exemple 5e, à partir de 55,6 g (214 mmoles) du composé préparé dans l'exemple 107b, de 101 ml de méthanol, de 91,1 g (643 mmoles) d'éthérate de trifluorure de bore et de 113,8 g (257 mmoles) de tétraacétate de plomb dans 1090 ml de dichlorométhane. Après distillations, on obtient 18,4 g (Rdt = 29,7 %) d'un liquide épais jaune qui cristallise. $Eb_{0,4}$ = 205 °C. F = 62 - 5 °C.
I.R. (KBr) : $\nu$ (NH) = 3290 $cm^{-1}$ ; (C=O) = 1720 $cm^{-1}$ ; (C=0) = 1620 $cm^{-1}$.
R.M.N.($CDCl_3$) : $\delta$ = 1,6 - 2,1 (9H, m) ; 2,7 (2H, s) ; 3,25 (2H, d, J = 6 Hz, se transforme en singulet avec $CF_3COOD$) ; 3,6 (2H, s) ; 3,7 (3H, s) ; 5,45 (1H, m, échangeable avec $CF_3COOD$) ; 7,1 (4H, s).

d) Acide 4-[[1-(aminométhyl)cyclobutyl]méthyl]benzèneacétique

Obtenu en opérant comme dans l'exemple 103d, à partir de 4,9 g (16,9 mmoles) du composé préparé dans l'exemple 107c et de 4 g (100 mmoles) de NaOH dans 35,5 ml d'eau. On obtient 3,3 g (Rdt = 84,6 %) d'un solide blanc, utilisé sans autre purification. F > 255 °C.
R.M.N.(DMSO $d_6$ + $CF_3COOD$) : $\delta$ = 1,7 - 2,1 (6H, m) ; 2,7 - 2,95 (4H, ; 3,5 (2H, s) ; 7,2 (4H, s).

e) Acide 4-[[1-[[[(4-bromophényl)sulfonyl]amino]méthyl]cyclobutyl]méthyl]benzèneacétique

A un mélange de 3,3 g (14 mmoles) du composé préparé dans l'exemple 107d et de 3,9 g (28 mmoles) de carbonate de potassium dans 400 ml d'eau, on ajoute 3,8 g (14,9 mmoles) de chlorure de 4-bromobenzènesulfonyle. Le milieu réactionnel est ensuite porté à reflux 1 heure. Après refroidissement, lavage à l'éther, puis acidification à pH 1 par HCl dilué, on obtient un précipité qui est purifié par recristallisation dans l'acétate d'éthyle, pour donner 3,3 g (Rdt = 52,4 %) d'un solide blanc. F = 180 - 2 °C.

| Analyse centésimale : $C_{20}H_{22}BrNO_4S$ (M = 452,363) | | | | | |
|---|---|---|---|---|---|
| | C % | H % | Br % | N % | S % |
| Calculé | 53,10 | 4,90 | 17,66 | 3,10 | 7,09 |
| Trouvé | 53,09 | 5,01 | 17,61 | 3,11 | 7,19 |

I.R. (KBr) : ν (NH) = 3240 $cm^{-1}$ ; (C=O) = 1710 $cm^{-1}$ ; ($SO_2$) = 1315 $cm^{-1}$ ; ($SO_2$) = 1155 $cm^{-1}$.
R.M.N.(acétone $d_6$) : δ = 1,55 - 2,15 (6H, m) ; 2,8 (2H, s) ; 2,9 (2H, d, J = 6 Hz, se transforme en singulet avec $CF_3COOD$) ; 3,6 (2H, s) ; 6,45 (1H, t, J = 6 Hz, échangeable avec $CF_3COOD$) ; 7,2 (4H, s) ; 7,8 (4H, s) ; 10,9 (1H, s large, échangeable avec $CF_3COOD$).

## Exemple 108

## Activité biologique

Les produits des exemples 1 à 107 ont été testés sur l'agrégation plaquettaire de cobaye et sur la contraction d'aorte de rat comme décrit précédemment. Les résultats sont donnés dans les tableaux IV a à IV c.

Tableau IV a

| EXEMPLE N° | AGREGATION PLAQUETTAIRE COBAYE CI50 (μM) | VASOCONSTRICTION AORTE RAT CI50 (μM) |
|---|---|---|
| 1 | 0,69 | - |
| 2 | 0,72 | 0,07 |
| 3 | 0,60 | 0,06 |
| 4 | 23,0 | 0,20 |
| 6 | 6,3 | 0,56 |
| 7 | 3,3 | 0,30 |
| 9 | > 20,0 | 1,0 |
| 10 | 1,3 | 5,0 |
| 11 | 0,39 | 0,05 |
| 12 | 1,8 | 0,07 |
| 13 | 0,68 | 0,06 |
| 14 | 5,5 | 0,07 |
| 15 | 1,1 | 0,30 |
| 16 | 2,4 | 0,20 |
| 17 | > 20,0 | > 1,0 |
| 18 | 0,66 | > 1,0 |
| 19 | 0,26 | 0,10 |
| 20 | 0,28 | 0,07 |
| 22 | 0,71 | 0,15 |
| 23 | 0,48 | 0,04 |
| 24 | 0,57 | 0,30 |
| 25 | 1,5 | 0,06 |
| 26 | 3,1 | 0,46 |
| 27 | 0,29 | 0,02 |
| 28 | 2,6 | > 1,0 |
| 29 | 5,3 | 1,0 |
| 30 | 0,92 | 1,0 |
| 31 | 2,5 | ≧ 1,0 |
| 32 | 1,8 | 0,40 |
| 33 | > 5,0 | > 1,0 |
| 43 | > 5,0 | > 1,0 |

Tableau IV b

| EXEMPLE N° | AGREGATION PLAQUETTAIRE COBAYE CI50 (µM) | VASOCONSTRICTION AORTE RAT CI50 (µM) |
|---|---|---|
| 44 | > 5,0 | - |
| 45 | > 5,0 | - |
| 52 | > 5,0 | > 1,0 |
| 53 | 0,16 | 0,01 |
| 54 | 1,1 | 0,04 |
| 55 | 2,9 | 0,1 |
| 56 | 5,0 | 1,0 |
| 57 | 0,69 | 0,10 |
| 58 | 6,3 | 1,0 |
| 59 | 1,4 | 0,08 |
| 60 | > 5,0 | > 1,0 |
| 61 | > 5,0 | > 1,0 |
| 62 | 3,0 | 0,75 |
| 63 | > 5,0 | > 1,0 |
| 64 | 2,6 | 0,13 |
| 65 | 0,35 | 0,27 |
| 66 | > 5,0 | > 1,0 |
| 67 | 1,5 | 0,28 |
| 68 | 4,0 | 0,40 |
| 69 | > 5,0 | > 1,0 |
| 70 | > 5,0 | 1,0 |
| 71 | 3,0 | 0,04 |
| 72 | 0,64 | 0,04 |
| 73 | 0,70 | 0,20 |
| 74 | > 5,0 | > 1,0 |
| 75 | > 5,0 | > 1,0 |
| 76 | 2.9 | > 1,0 |
| 77 | 2,4 | > 1,0 |
| 79 | 0,10 | 0,17 |

Tableau IV c

| EXEMPLE N° | AGREGATION PLAQUETTAIRE COBAYE CI50 (μM) | VASOCONSTRICTION AORTE RAT CI50 (μM) |
|---|---|---|
| 80 | 2,9 | 0,19 |
| 81 | 1,6 | 0,06 |
| 82 | 2,0 | 1,0 |
| 83 | 0,94 | 1,0 |
| 84 | > 5,0 | 1,0 |
| 85 | 1,3 | > 1,0 |
| 86 | 0,51 | 1,0 |
| 87 | 4,0 | 0,60 |
| 88 | 0,31 | 1,0 |
| 89 | 0,26 | 0,03 |
| 90 | > 5,0 | > 1,0 |
| 91 | 3,1 | 1,0 |
| 92 | 0,37 | 1,0 |
| 93 | 1,6 | 0,05 |
| 94 | 1,8 | > 1,0 |
| 95 | 0,57 | 0,28 |
| 96 | 0,75 | > 1,0 |
| 97 | 5,9 | > 1,0 |
| 98 | 0,25 | 0,32 |
| 99 | > 5,0 | > 1,0 |
| 100 | > 5,0 | > 1,0 |
| 101 | > 5,0 | > 1,0 |
| 102 | 1,0 | - |
| 103 | 1,0 | 0,18 |
| 104 | > 5,0 | > 1,0 |
| 105 | 0,57 | 0,11 |
| 106 | > 5,0 | - |

## Revendications

1. Nouveaux sulfonamides substitués, leurs sels, complexes, esters et amides physiologiquement acceptables de formule

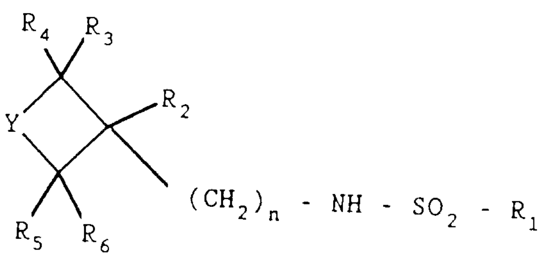

dans laquelle :
$R_1$ est un radical aryle ou un hétérocycle, éventuellement substitué par un ou plusieurs des groupes suivants :
halogène, trifluorométhyle, trifluorométhoxy, alkyle inférieur éventuellement substitué par un groupe hydroxyle, cycloalkyle, alkoxy inférieur, alkoxycarbonyle, carboxy, alkylthio inférieur, alkylsulfinyle inférieur, alkylsulfonyle inférieur, acyle inférieur, thioacyle inférieur, amine éventuellement substituée par un ou plusieurs groupes choisis parmi alkyle inférieur, acétamide, nitro, nitrile, azide et aryle ;
$R_2$ et $R_3$ sont différents ; l'un des deux représente W ; lorsque $R_2$ est W, $R_3$ représente l'hydrogène ou un alkyle inférieur et lorsque $R_3$ est W, $R_2$ représente l'hydrogène ; W est un groupe $-Z-Ar-(CH_2)_q-A$,

dans lequel A est $CO_2H$ ou un groupement hydrolysable en $CO_2H$, $SO_3H$, $PO_3H_2$, un hétérocycle, un radical acyle inférieur, un radical oxoalkylcarboxylique ou bien le groupe hydroxyéthyle ;
q est 0, 1, 2, 3 ou 4 ; Ar est un radical aryle ; Z est oxygène, $CH_2$, ou une liaison ;
$R_4$, $R_5$ et $R_6$ représentent indépendamment l'un de l'autre un hydrogène ou un alkyle inférieur ;
Y est un groupe $-(CH_2)_s-B-(CH_2)_t$ ; s et t sont 0, 1 ou 2 ;
B est un atome d'oxygène, de soufre oxydé ou non, d'azote éventuellement substitué par un radical alkyle inférieur, un radical acyle inférieur ou un radical aryle, une fonction carbonyle ou thiocarbonyle, un hétérocycle ou une liaison ;
n est 0 ou 1, étant entendu que :

. le terme "aryle" désigne un groupement mono- ou bicyclique aromatique de 6 à 10 atomes de carbone,
. le terme "hétérocycle" désigne un cycle aromatique ou non comprenant de 3 à 10 atomes dont 1 à 4 hétéroatomes choisis parmi O, S et N,
. le terme "alkyle inférieur" désigne un radical hydrocarboné linéaire ou ramifié de 1 à 7 atomes de carbone,
. les termes "acyle inférieur" et "thioacyle inférieur" désignent un groupe alkyle inférieur lié respectivement à une fonction carbonyle ou thiocarbonyle,
. le terme "oxoalkylcarboxylique" désigne un groupe acyle inférieur porteur d'une fonction acide carboxylique,
. le terme "groupement hydrolysable" désigne un groupe acide carboxylique sous la forme de sel métallique, d'un ester d'alkyle inférieur, d'un ester d'alkylaminoalkyle, d'un ester de dialkylaminoalkyle, ou bien d'un amide,
. le terme "alkoxy inférieur" désigne un groupe alkyle inférieur lié à un atome d'oxygène,
. les termes "alkylthio inférieur", "alkylsulfinyle inférieur" et "alkylesulfonyle inférieur" désignent un groupe alkyle inférieur lié à un atome de soufre respectivement non oxydé, mono-oxydé ou di-oxydé,
. le terme "cycloalkyle" désigne un cycle hydrocarboné saturé de 3 à 12 atomes de carbone,
. le terme "halogène" désigne un atome de fluor, de chlore, de brome ou d'iode.

**2.** Les produits selon la revendication 1, caractérisés en ce que $R_3$ est un groupement W et que $R_2$ est différent de W.

**3.** Les produits selon la revendication 1, caractérisés en ce que $R_2$ est W, et que $R_3$ est différent de W.

**4.** Les produits selon la revendication 1, caractérisés en ce que $R_3$ est un groupe W, et Z contenu dans W est un atome d'oxygène.

**5.** Les produits selon la revendication 1, caractérisés en ce que $R_2$ est W, et Z contenu dans W est une liaison.

**6.** Un produit selon les revendications 1 et 2, choisi parmi le groupe comprenant l'acide *trans*-4-[[2-[[(4-chlorophényl)sulfonyl]amino]cyclopentyl]méthyl]benzèneacétique, l'acide *trans*-4-[[2-[[(4-chlorophényl)-sulfonyl]amino]cyclohexyl]méthyl]benzèneacétique, l'acide *cis*-4-[[2-[[(4-chlorophényl)sulfonyl]amino]-cyclopentyl]méthyl]benzèneacétique.

**7.** Le produit selon les revendications 1, 2 et 4, l'acide 4-[2-[[(4-chlorophényl)sulfonyl]amino]-cyclopentyloxy]benzèneacétique

**8.** Un produit selon les revendications 1 et 3, choisi parmi le groupe comprenant l'acide 4-[[1-[[[(4-chlorophényl)sulfonyl]amino]méthyl]cyclopentyl]méthyl]benzèneacétique, l'acide 4-[[1-[[[(4-méthylphényl)sulfonyl]amino]méthyl]cyclopentyl]méthyl]benzèneacétique, l'acide 4-[[1-[[[(4-bromophényl)-sulfonyl]amino]méthyl]cyclopentyl]méthyl]benzèneacétique, l'acide 4-[[1-[[[(4-chloro-2-fluorophényl)-sulfonyl]amino)méthyl]cyclopentyl]méthyl]benzèneacétique, l'acide 4-[[1-[[[(4-chlorophényl)sulfonyl]-amino]méthyl]cyclopropyl]méthyl]benzèneacétique, l'acide 4-[[1-[[[(4-chlorophényl)sulfonyl]amino]-méthyl]cyclobutyl]méthyl]benzèneacétique, l'acide 4-[[1-[[[(4-bromophényl)sulfonyl]amino]méthyl]-cyclobutyl]méthyl]benzèneacétique, l'acide 4-[[1-[[[(4-chlorophényl)sulfonyl]amino]méthyl]-3,3-diméthyl-cyclobutyl]méthyl]benzèneacétique.

**9.** Un produit selon les revendications 1, 3 et 5, choisi parmi le groupe comprenant l'acide 4-[1-[[[(4-chlorophényl)sulfonyl]amino]méthyl]cyclopentyl]benzèneacétique, l'acide 4-[1-[[[(4-chlorophényl)-sulfonyl]amino]méthyl]cyclobutyl]benzèneacétique, l'acide 4-[1-[[[(4-chlorophényl)sulfonyl]amino]-méthyl]cyclopropyl]benzèneacétique.

**10.** Un médicament renfermant comme principe actif une quantité efficace d'au moins un composé décrit dans la revendication 1, en association avec un véhicule pharmaceutiquement acceptable, sous forme de comprimés, comprimés pelliculés, gélules, solutés injectables, solutions, suppositoires, crèmes ou aérosols.

**11.** Utilisation d'un produit selon l'une des revendications 1 à 9 pour l'obtention d'un médicament destiné à traiter les troubles de l'appareil circulatoire ou de l'appareil respiratoire.

**12.** Un procédé pour la préparation d'un produit défini dans une des revendications 1 à 9, caractérisé en ce qu'on traite une amine de formule

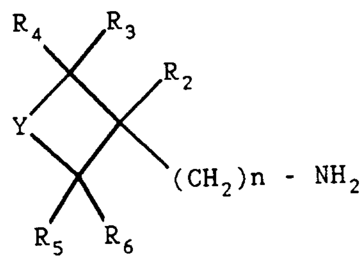

par un chlorure d'arylsulfonyle ou d'hétéroarylsulfonyle $R_1$-$SO_2Cl$. W représenté par $R_2$ ou $R_3$ possède les significations énoncées dans 14 revendication 1 et peut prendre aussi la valeur -Z-Ar; les substituants $R_{1-6}$ et les paramètres Y, Z, Ar et n possèdent les significations énoncées dans la revendication 1.

**13.** Un procédé pour la préparation d'un produit défini dans une des revendications 1, 2 et 6, caractérisé en ce qu'on traite une aziridine de formule

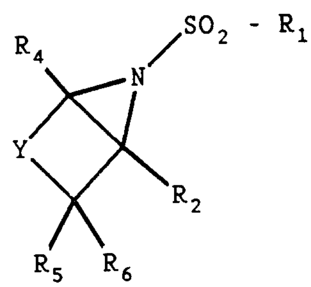

par un dérivé organométallique d'un halogénure $R_3$-X, X est un halogène, $R_3$ représente W qui possède les significations énoncées dans la revendication 1 et qui peut prendre aussi la valeur -Z-Ar, Z étant uniquement $CH_2$ ou liaison. Les substituants $R_2$, $R_{4-6}$ et les paramètres Y et Ar possèdent les significations énoncees dans la revendication 1, n étant égal à 0.

## Claims

**1.** New substituted sulphonamides and their physiologically acceptable salts, complexes, esters and amides of the formula

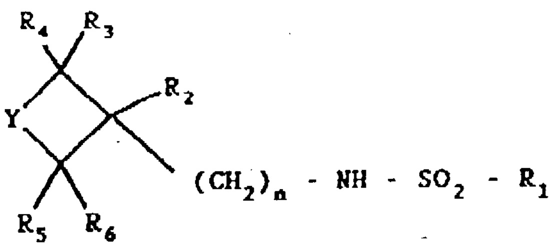

in which:

$R_1$ is an aryl radical or a heterocyclic group, possibly substituted by one or more of the following groups:

halogen, trifluoromethyl, trifluoromethoxy, lower alkyl possibly substituted by a hydroxyl group, cycloalkyl, lower alkoxy, alkoxycarbonyl, carboxy, lower alkylthio, lower alkylsulphinyl, lower alkylsulphonyl, lower acyl, lower thioacyl, amine possibly substituted by one or more groups selected from lower alkyl, acetamide, nitro, nitrile, azide and aryl;

$R_2$ and $R_3$ are different; one of them represents W; when $R_2$ is W, $R_3$ represents hydrogen or a lower alkyl and when $R_3$ is W, $R_2$ represents hydrogen; W is a group $-Z-Ar-(CH_2)_q-A$, in which A is $CO_2H$ or a group which is hydrolysable to $CO_2H$, $SO_3H$ or $PO_3H_2$, a heterocyclic group, a lower acyl radical, an oxoalkylcarboxylic radical or the hydroxyethyl group, q is 0, 1, 2, 3 or 4; Ar is an aryl radical; and Z is oxygen, $CH_2$ or a bond;

$R_4$, $R_5$ and $R_6$ independently of each other represent hydrogen or a lower alkyl;

Y is a group $-(CH_2)_s-B-(CH_2)_t$; s and t are 0, 1 or 2; B is an oxygen atom, an oxidised or non-oxidised sulphur atom, a nitrogen atom possibly substituted by a lower alkyl radical, a lower acyl radical or an aryl radical, a carbonyl or thiocarbonyl function, a heterocyclic group or a bond;

n is 0 or 1, it being understood that:

- the term "aryl" designates a mono- or bicyclic aromatic group of 6 to 10 carbon atoms,
- the term "heterocyclic group" designates an aromatic or non-aromatic cyclic group comprising from 3 to 10 atoms of which 1 to 4 are heteroatoms selected from O, S and N,
- the term "lower alkyl" designates a linear or branched hydrocarbon radical with 1 to 7 carbon atoms,
- the terms "lower acyl" and "lower thioacyl" designate a lower alkyl group linked to a carbonyl or thiocarbonyl function respectively,
- the term "oxoalkylcarboxylic" designates a lower acyl group bearing a carboxylic acid function,
- the term "hydrolysable group" designates a carboxylic acid group in the form of a metal salt, a lower alkyl ester, an alkylaminoalkyl ester, a dialkylaminoalkyl ester or an amide,
- the term "lower alkoxy" designates a lower alkyl group linked to an oxygen atom,
- the terms "lower alkylthio", "lower alkylsulphinyl" and "lower alkylsulphonyl" designate a lower alkyl group linked to a sulphur atom that is non-oxidised, mono-oxidised or dioxidised respectively
- the term "cycloalkyl" designates a saturated hydrocarbon ring with 3 to 12 carbon atoms,
- the term "halogen" designates an atom of fluorine, chlorine, bromine or iodine.

**2.** The products according to claim 1, characterised in that $R_3$ is a group W and that $R_2$ is different from W.

**3.** The products according to claim 1, characterised in that $R_2$ is W and that $R_3$ is different from W.

**4.** The products according to claim 1, characterised in that $R_3$ is a group W and that Z contained in W is an oxygen atom.

**5.** The products according to claim 1, characterised in that $R_2$ is W and that Z contained in W is a bond.

**6.** A product according to claims 1 and 2, selected from the group comprising
*trans*-4-[[2-[[(4-chlorophenyl)sulphonyl]amino]cyclopentyl]methyl]benzeneacetic acid,
*trans*-4-[[2-[[(4-chlorophenyl)sulphonyl]amino]cyclohexyl]methyl)benzeneacetlc acid and
*cis*-4-[[2-[[(4-chlorophenyl)sulphonyl]amino]cyclopentyl]methyl]benzeneacetic acid.

**7.** The product according to claims 1, 2 and 4, 4-[2-[[(4-chlorophenyl)sulphonyl]amino]cyclopentyloxy]-benzeneaceticacid.

**8.** A product according to claims 1 and 3, selected from the group comprising 4-[[1-[[[(4-chlorophenyl)-sulphonyl]amino]methyl]cyclopentyl]methyl]benzeneacetic acid,
4-[[1-[[[(4-methylphenyl)sulphonyl]amino]methyl]cyclopentyl]methyl]benzeneacetic acid,
4-[[1-[[[(4-bromophenyl)sulphonyl]amino]methyl]cyclopentyl]methyl]benzeneacetic acid,
4-[[1-[[[(4-chloro-2-fluorophenyl)sulphonyl]amino]methyl]cyclopentyl]methyl]benzeneacetic acid,
4-[[1-[[[(4-chlorophenyl)sulphonyl]amino]methyl]cyclopropyl]methyl]benzeneacetic acid,
4-[[1-[[[(4-chlorophenyl)sulphonyl]amino]methyl]cyclobutyl]methyl]benzeneacetic acid,
4-[[1-[[[(4-bromophenyl)sulphonyl]amino]methyl]cyclobutyl]methyl]benzeneacetic acid,
and 4-[[1-[[[(4-chlorophenyl)sulphonyl]amino]methyl]-3,3-dimethylcyclobutyl]methyl]benzeneacetic acid.

**9.** A product according to claims 1, 3 and 5, selected from the group comprising 4-[1-[[[(4-chlorophenyl)-sulphonyl]amino]methyl]cyclopentyl]benzeneacetic acid, 4-[1-[[[(4-chlorophenyl)sulphonyl]amino]-methyl]cyclobutyl]benzeneacetic acid and 4-[1-[[[(4-chlorophenyl)sulphonyl]amino]methyl]cyclopropyl]-benzeneacetic acid.

**10.** A medicament containing as active principle an effective quantity of at least one compound described in claim 1, in association with a pharmaceutically acceptable excipient, in the form of tablets, coated tablets, capsules, injectable solutes, solutions, suppositories, creams or aerosols.

**11.** Use of a product according to one of claims 1 to 9 for obtaining a medicament intended to treat the disorders of the circulatory system or of the respiratory system

**12.** A process for the preparation of a product defined in one of claims 1 to 9, characterised in that an amine of formula

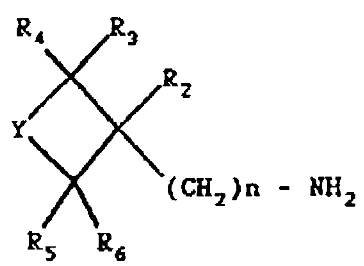

is treated with an arylsulphonyl or heteroarylsulphonyl chloride $R_1$-$SO_2Cl$. W, represented by $R_2$ or $R_3$, has the meanings set out in claim 1 and can also have the value -Z-Ar; the substituents $R_{1-6}$ and the parameters Y, Z, Ar and n have the meanings set out in claim 1.

**13.** A process for the preparation of a product defined in one of claims 1, 2 and 6, characterised in that an aziridine of formula

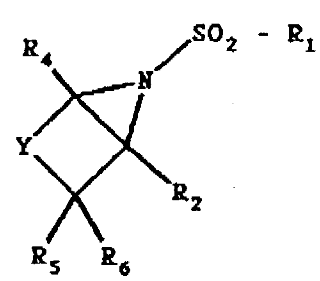

is treated with an organometallic derivative of a halide $R_3$-X, X is a halogen, $R_3$ represents W, which

has the meanings set out in claim 1 and can also take the value -Z-Ar, Z being solely $CH_2$ or a bond The substituents $R_2$, $R_{4-6}$ and the parameters Y and Ar have the meanings set out in claim 1, n being equal to 0.

## Patentansprüche

1. Neue substituierte Sulfonamide und deren physiologisch annehmbare Salze, Komplexe, Ether und Amide der Formel

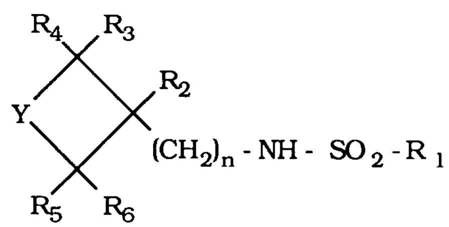

in der:
$R_1$ eine Arylgruppe oder eine heterocyclische Gruppe, die gegebenenfalls durch eine oder mehrere der folgenden Gruppen substituiert ist: Halogenatome, Trifluormethylgruppen, Trifluormethoxygruppen, niedrigmolekulare Alkylgruppen, die gegebenenfalls durch eine Hydroxylgruppe substituiert sind, Cycloalkylgruppen, niedrigmolekulare Alkoxygruppen, Alkoxycarbonylgruppen, Carboxygruppen, niedrigmolekulare Alkylthiogruppen, niedrigmolekulare Alkylsulfinylgruppen, niedrigmolekulare Alkylsulfonylgruppen, niedrigmolekulare Acylgruppen, niedrigmolekulare Thioacylgruppen, Amingruppen, die gegebenenfalls durch eine oder mehrere Gruppen ausgewählt aus niedrigmolekularen Alkylgruppen, Acetamidgruppen, Nitrogruppen, Nitrilgruppen, Azidgruppen und Arylgruppen substituiert sind;
$R_2$ und $R_3$ voneinander verschieden sind und eine der beiden Gruppen W darstellt; wobei dann, wenn $R_2$ W bedeutet, $R_3$ ein Wasserstoffatom oder eine niedrigmolekulare Alkylgruppe darstellt und wenn $R_3$ W bedeutet, $R_2$ ein Wasserstoffatom darstellt; W eine Gruppe $-Z-Ar-(CH_2)_q-A$, worin A $CO_2H$ oder eine zu einer $CO_2H$-Gruppe hydrolysierbare Gruppe, $SO_3H$, $PO_3H_2$, eine heterocyclisehe Gruppe, eine niedrigmolekulare Acylgruppe, eine Oxoalkylcarboxylgruppe oder eine Hydroxyethylgruppe darstellt; q 0, 1, 2, 3 oder 4; Ar eine Arylgruppe; Z ein Sauerstoffatom, $CH_2$ oder eine Bindung darstellen;
$R_4$, $R_5$ und $R_6$ unabhängig voneinander Wasserstoffatome oder niedrigmolekulare Alkylgruppen;
Y eine Gruppe $-(CH_2)_s-B-(CH_2)_t$; worin s und t 0, 1 oder 2 und B ein Sauerstoffatom, ein gegebenenfalls oxidiertes Schwefelatom, ein gegebenenfalls durch eine niedrigmolekulare Alkylgruppe, eine niedrigmolekulare Acylgruppe oder eine Arylgruppe substituiertes Stickstoffatom, eine Carbonylgruppe oder eine Thiocarbonylgruppe, eine heterocyclische Gruppe oder eine Bindung darstellen;
n 0 oder 1, mit der Maßgabe, daß:

- der Begriff "Arylgruppe" für eine monocyclische oder bicyclische aromatische Gruppe mit 6 bis 10 Kohlenstoffatomen steht,
- der Begriff "heterocyclische Gruppe" für einen gegebenenfalls aromatischen Ring mit 3 bis 10 Atomen, darunter 1 bis 4 Heteroatomen ausgewählt aus O, S und N steht,
- der Begriff"niedrigmolekulare Alkylgruppe" für eine geradkettige oder verzweigte Kohlenwasserstoffgruppe mit 1 bis 7 Kohlenstoffatomen steht,
- die Begriffe "niedrigmolekulare Acylgruppe" und "niedrigmolekulare Thioacylgruppe" für eine über eine Carbonylgruppe oder eine Thiocarbonylgruppe gebundene niedrigmolekulare Alkylgruppe stehen,
- der Begriff "Oxoalkylcarboxylgruppe" für eine niedrigmolekulare Acylgruppe steht, die eine Carbonsäuregruppe trägt,
- der Begriff "hydrolysierbare Gruppe" für eine Carbonsäuregruppe in Form des Metallsalzes, eine niedrigmolekulare Alkylestergruppe, eine Alkylaminoalkylestergruppe, eine Dialkylaminoalkylestergruppe oder eine Amidgruppe steht,
- der Begriff"niedrigmolekulare Alkoxygruppe" für eine an ein Sauerstoffatom gebundene niedrigmolekulare Alkylgruppe steht,
- die Begriffe "niedrigmolekulare Alkylthiogruppe", "niedrigmolekulare Alkylsulfinylgruppe" und "niedrigmolekulare Alkylsulfonylgruppe" für eine niedrigmolekulare Alkylgruppe steht, die an ein

nicht-oxidiertes, mono-oxidiertes bzw. dioxidiertes Schwefelatom gebunden ist,

- der Begriff "Cycloalkylgruppe" für einen gesättigten Kohlenwasserstoffring mit 3 bis 12 Kohlenstoffatomen steht, und
- der Begriff"Halogenatom" für ein Fluoratom, ein Chloratom, ein Bromatom oder ein Iodatom steht.

**2.** Produkte nach Anspruch 1, **dadurch gekennzeichnet,** daß $R_3$ eine Gruppe W bedeutet und $R_2$ von W verschieden ist.

**3.** Produkte nach Anspruch 1, **dadurch gekennzeichnet,** daß $R_2$ W bedeutet und $R_3$ von W verschieden ist.

**4.** Produkte nach Anspruch 1, **dadurch gekennzeichnet,** daß $R_3$ eine Gruppe W bedeutet und der in W enthaltene Rest Z ein Sauerstoffatom darstellt.

**5.** Produkte nach Anspruch 1, **dadurch gekennzeichnet,** daß $R_2$ W bedeutet und der in W enthaltene Rest Z eine Bindung darstellt.

**6.** Produkt nach den Ansprüchen 1 und 2, ausgewählt aus der Gruppe, die trans-4-[[2-[[(4-Chlorphenyl)-sulfonyl]-amino]-cyclopentyl]-methyl]-benzolessigsäure, trans-4-[[2-[[[4-Chlorphenyl)-sulfonyl]-amino]-cyclohexyl]-methyl]-benzolessigsäure, cis-4-[[2-[[(4-Chlorphenyl)-sulfonyl]-amino]-cyclopentyl]-methyl]-benzolessigsäure umfaßt.

**7.** Produkt der Ansprüche 1, 2 und 4, nämlich 4-[2-[[(4-Chlorphenyl)-sulfonyl]-amino]-cyclpentyloxy]-benzolessigsäure.

**8.** Produkt nach den Ansprüchen 1 und 3, ausgewählt aus der Gruppe, die 4-[[1-[[[(4-Chlorphenyl)-sulfonyl]-amino]-methyl]-cyclopentyl]-methyl]-benzolessigsäure, 4-[[1-[[[(4-Methylphenyl)-sulfonyl]-amino]-methyl]-cyclopentyl]-methyl]-benzolessigsäure, 4-[[1-[[[(4-Bromphenyl)-sulfonyl]-amino]-methyl]-cyclopentyl]-methyl]-benzolessigsäure, 4-[[1-[[[(4-Chlor-2-fluorphenyl)-sulfonyl]-amino]-methyl]-cyclopentyl]-methyl]-benzolessigsäure, 4-[[1-[[[(4-Chlorphenyl)-sulfonyl]-amino]-methyl]-cyclopropyl]-methyl]-benzolessigsäure, 4-[[1-[[[(4-Chlorphenyl)-sulfonyl]-amino]-methyl]-cyclobutyl]-methyl]-benzolessigsäure, 4-[[1-[[[(4-Bromphenyl)-sulfonyl]-amino]-methyl]-cyclobutyl]-methyl]-benzolessigsäure und 4-[[1-[[[-(4-Chlorphenyl)-sulfonyl]-amino]-methyl]-3,3-dimethylcyclobutyl]-methyl]-benzolessigsäure umfaßt.

**9.** Produkt nach den Ansprüchen 1, 3 und 5, ausgewählt aus der Gruppe, die 4-[1-[[[(4-Chlorphenyl)-sulfonyl]-amino]-methyl]-cyclopentyl]-benzolessigsäure, 4-[1-[[[(4-Chlorphenyl)-sulfonyl]-amino]-methyl]-cyclobutyl]-benzolessigsäure und 4-[1-[[[(4-Chlorphenyl)-sulfonyl]-amino]-methyl]-cyclopropyl]-benzolessigsäure umfaßt.

**10.** Arzneimittel enthaltend als Wirkstoff eine wirksame Menge mindestens einer Verbindung, die in Anspruch 1 beschrieben ist, in Kombination mit einem pharmazeutisch annehmbaren Trägermaterial in Form von Tabletten, umhüllten Tabletten, Gelkapseln, injizierbaren Lösungen, Lösungen, Suppositorien, Cremes oder Aerosolen.

**11.** Verwendung eines Produkts nach einem der Ansprüche 1 bis 9 zur Herstellung eines Arzneimittels zur Behandlung von Störungen des Kreislaufsystems oder des Atmungssystems.

**12.** Verfahren zur Herstellung eines Produkts, wie es in einem der Ansprüche 1 bis 9 definiert ist, **dadurch gekennzeichnet,** daß man ein Amin der Formel

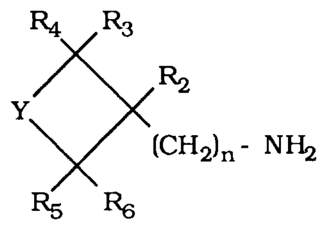

mit einem Arylsulfonylchlorid oder einem Heteroarylsulfonylchlorid $R_1$-$SO_2Cl$, worin die durch $R_2$ oder $R_3$ dargestellte Gruppe W die in Anspruch 1 angegebenen Bedeutungen besitzt und darüber hinaus die Bedeutung -Z-Ar aufweisen kann; und die Substituenten $R_{1-6}$ und die Symbole Y, Z, Ar und n die in Anspruch 1 angegebenen Bedeutungen besitzen, behandelt.

**13.** Verfahren zur Herstellung eines Produkts, wie es in einem der Ansprüche 1, 2 und 6 definiert ist, **dadurch gekennzeichnet,** daß man ein Aziridin der Formel

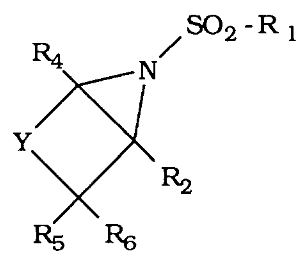

mit einem metallorganischen Halogenderivat $R_3$-X, worin X ein Halogenatom darstellt, $R_3$ W darstellt, welches die in Anspruch 1 angegebenen Bedeutungen besitzt und auch die Bedeutung -Z-Ar haben kann, worin Z lediglich $CH_2$ oder eine Bindung darstellt, und die Substituenten $R_2$, $R_{4-6}$ und die Symbole Y und Ar die in Anspruch 1 angegebenen Bedeutungen besitzen und n 0 ist, behandelt.